(19) Europäisches Patentamt
European Patent Office
Office européen des brevets

(11) EP 4 467 538 A1

(12) **EUROPEAN PATENT APPLICATION**
published in accordance with Art. 153(4) EPC

(43) Date of publication:
**27.11.2024 Bulletin 2024/48**

(21) Application number: **23807034.6**

(22) Date of filing: **18.05.2023**

(51) International Patent Classification (IPC):
***C07D 405/14*** (2006.01) ***C07D 413/14*** (2006.01)
***C07D 417/14*** (2006.01) ***C07D 491/048*** (2006.01)
***C07D 471/04*** (2006.01) ***A61K 31/4545*** (2006.01)
***A61K 31/444*** (2006.01) ***A61K 31/4439*** (2006.01)
***A61K 31/497*** (2006.01) ***A61K 31/496*** (2006.01)
***A61P 3/10*** (2006.01)

(52) Cooperative Patent Classification (CPC):
Y02P 20/55

(86) International application number:
**PCT/CN2023/095059**

(87) International publication number:
**WO 2023/222084 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.05.2022 CN 202210548598**
**05.08.2022 CN 202210936272**

(71) Applicant: **Chengdu Di'Ao Jiuhong Pharmaceutical Factory**
**Chengdu, Sichuan 610041 (CN)**

(72) Inventors:
- **HE, Peng**
  **Chengdu, Sichuan 610041 (CN)**
- **YU, Zhou**
  **Chengdu, Sichuan 610041 (CN)**
- **DONG, Guangxin**
  **Chengdu, Sichuan 610041 (CN)**
- **DENG, Ta**
  **Chengdu, Sichuan 610041 (CN)**
- **ZHANG, Rui**
  **Chengdu, Sichuan 610041 (CN)**
- **YE, Qijun**
  **Chengdu, Sichuan 610041 (CN)**
- **ZHANG, Shaofeng**
  **Chengdu, Sichuan 610041 (CN)**
- **ZHANG, Yong**
  **Chengdu, Sichuan 610041 (CN)**
- **ZHANG, Juan**
  **Chengdu, Sichuan 610041 (CN)**
- **HUANG, Pei**
  **Chengdu, Sichuan 610041 (CN)**
- **LI, Shan**
  **Chengdu, Sichuan 610041 (CN)**
- **LI, Bogang**
  **Chengdu, Sichuan 610041 (CN)**

(74) Representative: **Dehns**
**10 Old Bailey**
**London EC4M 7NG (GB)**

(54) **BENZIMIDAZOLE OR AZABENZIMIDAZOLE COMPOUND, PREPARATION METHOD THEREFOR AND USE THEREOF**

(57) The invention discloses a benzimidazole or azabenzimidazole compound, a preparation method therefor and use thereof. The benzimidazole or azabenzimidazole compound has a structure shown in formula (I). Use of the compound and a pharmaceutically acceptable salt, a stereoisomer, a solvate or a hydrate thereof in preparing a GLP-1 receptor agonist and use thereof in preparation of a medicament for treating and/or preventing metabolic diseases.

EP 4 467 538 A1

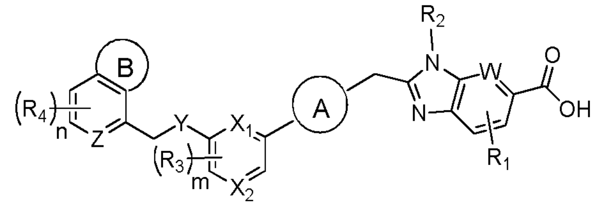

formula (I).

## Description

TECHNICAL FIELD

The present invention belongs to the technical field of chemical medicine, in particular to a benzimidazole or azabenzimidazole compound, a preparation method therefor and use thereof.

BACKGROUND

Glucagon-like peptide-1 (GLP-1) is a peptide hormone secreted by intestinal epithelial L cells, which is widely distributed in the mucosa of pancreas, stomach and small intestine, as well as in the heart, lung and central nervous system. GLP-1 specifically binds to a GLP-1 receptor (GLP-1R) in vivo, which activates cyclic adenosine monophosphate (cAMP) and mitogen-activated protein kinase (MAPK) pathways in cell membrane and then plays a variety of roles, such as promoting glucose-dependent insulin secretion, inhibiting glucagon secretion, inhibiting islet β cell apoptosis, delaying gastric emptying, inhibiting food intake and the like. At present, GLP-1 receptor agonist (GLP-1RA) has been developed as a treatment for type II diabetes. More and more clinical experiments have also confirmed that GLP-1RA not only plays a dominant role in the treatment of diabetes, but also plays different roles in obesity, nonalcoholic steatohepatitis (NASH), cardiovascular diseases and nervous system diseases. In addition, GLP-1 can bind with related receptors such as kidney and skin to affect tissue metabolism (Sichuan Medical Journal, 2020, 41(10):1089-1093.).

At present, GLP-1 receptor agonists on the market are polypeptide medicaments, such as Semaglutide, Liraglutide, Exenatide, Dulaglutide, etc. However, the peptide GLP-1 has poor oral bioavailability and is inconvenient to take, so small-molecular GLP-1 receptor agonists with good oral bioavailability are highly expected. At present, small-molecule GLP-1 receptor agonists in the clinical development stage include TTP-273 from vTv Company and PF-06882961 from Pfizer, so it is urgent to develop more GLP-1 receptor agonists.

SUMMARY

One object of the present invention is to provide a benzimidazole or azabenzimidazole compound, such as the benzimidazole or azabenzimidazole compound shown in general formula (I), general formula (II), general formula (II'), general formula (III), general formula (IV), general formula (V), general formula (VI), general formula (VII), general formula (VIII), general formula (IX), general formula (II-3), general formula (V-3) , etc., and a pharmaceutically acceptable salt, a stereoisomer, a solvate or a hydrate thereof.

Another object of the present invention is to provide a pharmaceutical composition formed by any of the above compounds or the pharmaceutically acceptable salts, the stereoisomers, the solvates or the hydrates thereof, as an active ingredient, with a pharmaceutically acceptable carrier.

Another object of the present invention is to provide use of the compounds of the above general formula or the pharmaceutically acceptable salts, the stereoisomers, the solvates or the hydrates thereof as a GLP-1R agonist.

Another object of the present invention is to provide use of the compounds of the above general formula or the pharmaceutically acceptable salts, the stereoisomers, the solvates or the hydrates thereof in preparing a medicament for treating and/or preventing type I diabetes, type II diabetes, malnutrition-related diabetes, diabetic complications, obesity, metabolic syndrome, hyperglycemia, glucose intolerance, nonalcoholic fatty liver disease (NAFLD), nonalcoholic steatohepatitis (NASH), cardiovascular disease, dyslipidemia, cerebral infarction, stroke, Parkinson's disease, dementia, insulin resistance and hepatic insulin resistance; and preferably the use in preparing the medicament for treating and/or preventing type I diabetes, type II diabetes, malnutrition-related diabetes mellitus, diabetic complications, obesity, metabolic syndrome, hyperglycemia, glucose intolerance, nonalcoholic fatty liver disease (NAFLD), nonalcoholic steatohepatitis (NASH), cardiovascular disease and dyslipidemia.

Another object of the present invention is to provide a preparation method for the compound of the general formula above.

In order to achieve the above objects, the present invention employs the following technical solutions.

In some embodiments of the present invention, the present invention provides a compound shown in general formula (I) or a pharmaceutically acceptable salt, a stereoisomer, a solvate or a hydrate thereof:

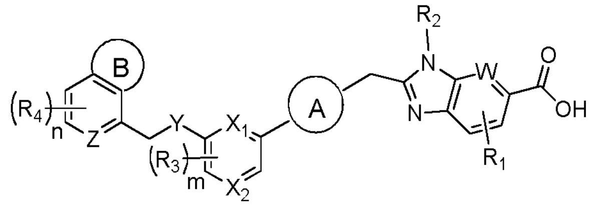

formula (I)

wherein:

$R_1$ is selected from : hydrogen atom, halogen, cyano, C1-C6 alkyl, alkenyl, alkynyl, C1-C6 alkoxy, amino, hydroxyl, cycloalkyl, heterocyclyl, aryl and heteroaryl, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl;

$R_2$ is selected from:

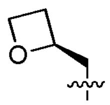 or 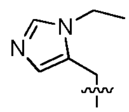 ;

$R_3$ are the same or different and are independently selected from: hydrogen atom, halogen, cyano, C1-C6 alkyl, alkenyl, alkynyl, C1-C6 alkoxy, amino, hydroxyl, cycloalkyl, heterocyclyl, aryl and heteroaryl, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl;

$R_4$ are the same or different and are independently selected from hydrogen atom, halogen, cyano, C1-C6 alkyl, alkenyl, alkynyl, C1-C6 alkoxy, amino, hydroxyl, cycloalkyl, heterocyclyl, aryl and heteroaryl, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl;

ring A is selected from

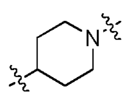 , 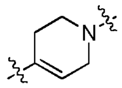 , 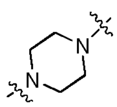 , 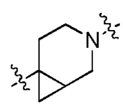 , 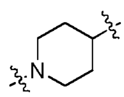 , 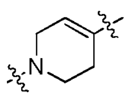 ,

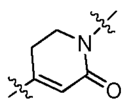 , 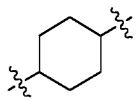 , 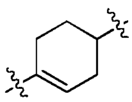 , 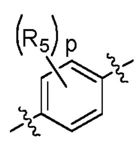 or 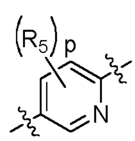 ;

ring B is selected from

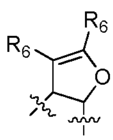 , 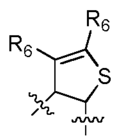 , 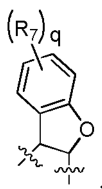 , 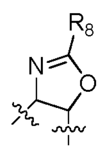 , 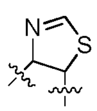 , 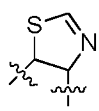 ,

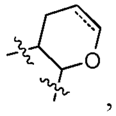 , 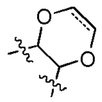 or 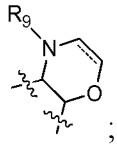 ;

$R_5$ are the same or different and are independently selected from hydrogen atom, halogen, cyano, C1-C6 alkyl, alkenyl, alkynyl, C1-C6 alkoxy, amino, hydroxyl, cycloalkyl, heterocyclyl, aryl and heteroaryl, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl;

$R_6$ are the same or different and are independently selected from hydrogen atom, halogen, cyano, C1-C6 alkyl, alkenyl, alkynyl, C1-C6 alkoxy, amino, hydroxyl, cycloalkyl, heterocyclyl, aryl and heteroaryl, or two adjacent $R_6$ are connected to form a 3-6 membered carbocyclic ring or heterocyclic ring and benzene ring, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl;

$R_7$ are the same or different and are independently selected from hydrogen atom, halogen, cyano, C1-C6 alkyl, alkenyl, alkynyl, C1-C6 alkoxy, amino, hydroxyl, cycloalkyl, heterocyclyl, aryl and heteroaryl, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl;

a restrictive condition thereof is that when ring A is:

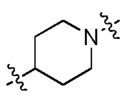 , 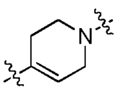 and 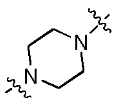

and when Z is selected from $CR_{10}$, ring B is not selected from

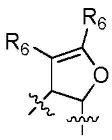 and 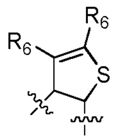 ;

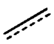

is a single bond or a double bond;

W is selected from N and $CR_{10}$;

$X_1$ is selected from N and $CR_{10}$;

$X_2$ is selected from N and $CR_{10}$;

Y is selected from O and NH;

Z is selected from N and $CR_{10}$;

m is selected from 0, 1 or 2;

n is selected from 0, 1 or 2;

p is selected from 0, 1, 2 or 3;

q is selected from 0, 1, 2, 3 or 4;

$R_8$ is selected from hydrogen atom, halogen, C1-C6 alkyl, C1-C6 cycloalkyl, heterocyclyl and aryl;

$R_9$ is selected from hydrogen atom, C1-C6 alkyl and alkenyl; and

$R_{10}$ is selected from hydrogen atom, halogen, cyano, C1-C6 alkyl, alkenyl, alkynyl, C1-C6 alkoxy, amino, hydroxyl, cycloalkyl, heterocyclyl, aryl and heteroaryl, wherein the C1-C6 alkyl, the alkenyl, the alkynyl, the C1-C6 alkoxy, the amino, the cycloalkyl, the heterocyclyl, the aryl and the heteroaryl are optionally substituted by one or more substituents selected from halogen, cyano, C1-C6 alkyl, alkenyl, alkynyl, C1-C6 alkoxy, amino, hydroxyl, cycloalkyl, heterocyclyl, aryl and heteroaryl.

In some embodiments of the present invention, in the compound shown in general formula (I) above or the pharmaceutically acceptable salt, the stereoisomer, the solvate or the hydrate thereof, preferably:

$R_1$ is selected from hydrogen atom, halogen, cyano and C1-C6 alkyl, wherein the C1-C6 alkyl is optionally substituted by one or more halogens;
$R_3$ are the same or different and are independently selected from hydrogen atom, halogen, cyano and C1-C6 alkyl, wherein the C1-C6 alkyl is optionally substituted by one or more halogens; and
$R_4$ are the same or different and are independently selected from hydrogen atom, halogen, cyano and C1-C6 alkyl, wherein the C1-C6 alkyl is optionally substituted by one or more halogens.

In some embodiments of the present invention, in the compound shown in general formula (I) above or the pharmaceutically acceptable salt, the stereoisomer, the solvate or the hydrate thereof, preferably:

$R_5$ are the same or different and are independently selected from hydrogen atom, halogen, cyano and C1-C6 alkyl, wherein the C1-C6 alkyl is optionally substituted by one or more halogens;
$R_6$ are the same or different and are independently selected from hydrogen atom, halogen, cyano and C1-C6 alkyl, or two adjacent $R_6$ are connected to form a 3-6 membered carboatomic ring or heterocyclic ring and benzene ring, wherein the C1-C6 alkyl is optionally substituted by one or more halogens;
$R_7$ are the same or different and are independently selected from hydrogen atom, halogen, cyano and C1-C6 alkyl, wherein the C1-C6 alkyl is optionally substituted by one or more helogens; and
$R_{10}$ is selected from hydrogen atom, halogen, cyano and C1-C6 alkyl, wherein C1-C6 alkyl is optionally substituted by one or more halogens.

In some embodiments of the present invention, in the compound shown in general formula (I) above or the pharmaceutically acceptable salt, the stereoisomer, the solvate or the hydrate thereof, preferably:

W is selected from N and $CR_{10}$;
$X_1$ is selected from N and $CR_{10}$;
$X_2$ is selected from N and $CR_{10}$;
Y is selected from O;
Z is selected from N and $CR_{10}$; and
$R_{10}$ is selected from hydrogen atom, halogen, cyano and C1-C6 alkyl, wherein the C1-C6 alkyl is optionally substituted by one or more halogens.

In some embodiments of the present invention, in the compound shown in general formula (I) above or the pharmaceutically acceptable salt, the stereoisomer, the solvate or the hydrate thereof, preferably:

ring A is selected from

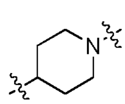, 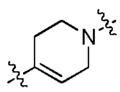, 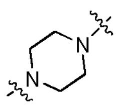, 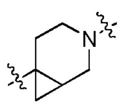, 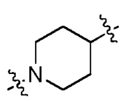, 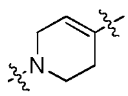,

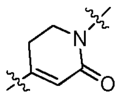, 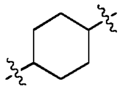, 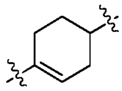, 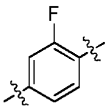, 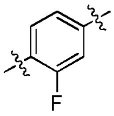, 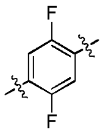, 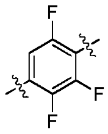, 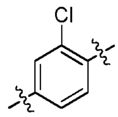, 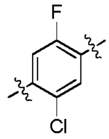,

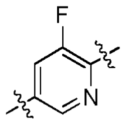 or 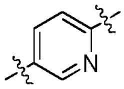;

ring B is selected from

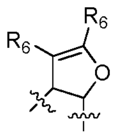, 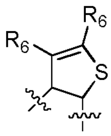, 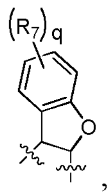, 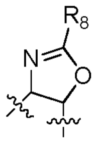, 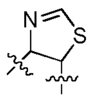, 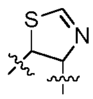, 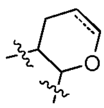,

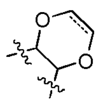 or 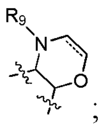;

a restrictive condition thereof is that when ring A is:

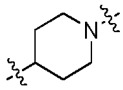, 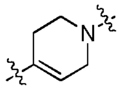 and 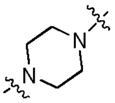

and when Z is selected from $CR_{10}$, ring B is not selected from

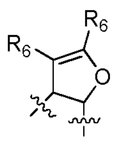 and 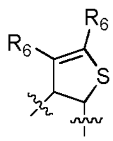

In some embodiments of the present invention, in the compound shown in general formula (I) above or the pharmaceutically acceptable salt, the stereoisomer, the solvate or the hydrate thereof, preferably:

ring B is selected from

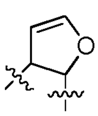, 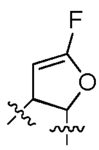, 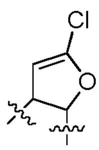, 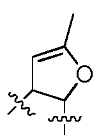, 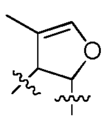, 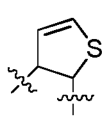, 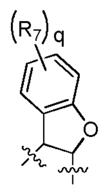,

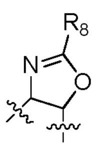, 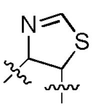, 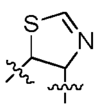, 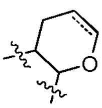, 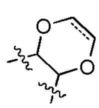, 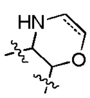 or 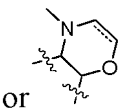;

$R_7$ are the same or different and are independently selected from hydrogen atom and halogen; and
$R_8$ is selected from hydrogen atom, F, Cl, methyl, ethyl, cyclopropyl, phenyl and furyl.

In some embodiments of the present invention, the present invention provides a compound shown in general formula (II) or a pharmaceutically acceptable salt, a stereoisomer, a solvate or a hydrate thereof:

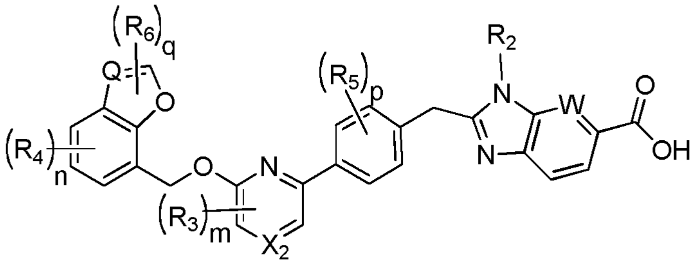

formula (II)

wherein:

$R_2$ is selected from

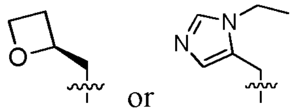

;

$R_3$ is independently selected from hydrogen atom and halogen;
$R_4$ is independently selected from hydrogen atom, halogen, cyano and C1-C6 alkyl, wherein C1-C6 alkyl can further be substituted by one or more halogens;
$R_5$ is independently selected from halogen;
$R_6$ is independently selected from hydrogen atom, halogen, C1-C6 alkyl, C3-C6 cycloalkyl, phenyl, furan and C2-C4 alkenyl; or, two adjacent $R_6$ are connected to form a ring;
W is selected from N and $CR_{10}$;
$X_2$ is selected from N and $CR_{10}$;
Q is selected from N and CH;
p is selected from 0, 1, 2 or 3;
m is selected from 0, 1 or 2;
n is selected from 0, 1, 2 or 3;
q is selected from 0, 1 or 2; and
$R_{10}$ is selected from hydrogen atom and halogen.

In some embodiments of the present invention, the compound shown in general formula (II) above or the pharmaceutically acceptable salt, the stereoisomer, the solvate or the hydrate thereof has a structure shown in general formula (II-1):

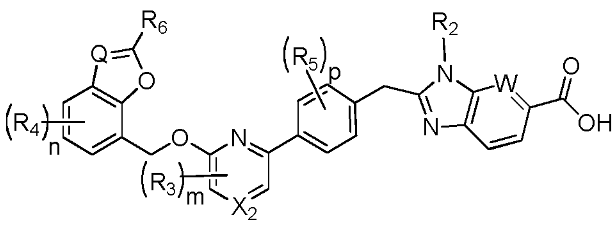

formula (II-1)

wherein:

$R_2$ is selected from

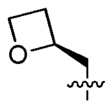 or 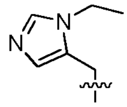 ;

$R_3$ is independently selected from hydrogen atom and halogen;
$R_4$ is independently selected from hydrogen atom, halogen, cyano and C1-C6 alkyl, wherein the C1-C6 alkyl can further be substituted by one or more halogens;
$R_5$ is independently selected from halogen;
$R_6$ is independently selected from hydrogen atom, halogen, C1-C6 alkyl, C3-C6 cycloalkyl, phenyl, furan and C2-C4 alkenyl; or, two adjacent $R_6$ are connected to form a ring;
W is selected from N and $CR_{10}$;
$X_2$ is selected from N and $CR_{10}$;
Q is selected from N and CH;
p is selected from 0, 1, 2 or 3;
m is selected from 0, 1 or 2;
n is selected from 0, 1, 2 or 3; and
$R_{10}$ is selected from hydrogen atom and halogen.

In some embodiments of the present invention, in the compound shown in general formula (II-1) above or the pharmaceutically acceptable salt, the stereoisomer, the solvate or the hydrate thereof, preferably: $R_3$ is selected from hydrogen atom and F; $R_4$ is selected from hydrogen atom, F, Cl, cyano, $CF_3$ and $CHF_2$; $X_2$ and Q are selected from CH; and $R_{10}$ is selected from hydrogen atom and F.

In some embodiments of the present invention, the compound shown in general formula (II-1) above or the pharmaceutically acceptable salt, the stereoisomer, the solvate or the hydrate thereof has a structure shown in general formula (II-1-1):

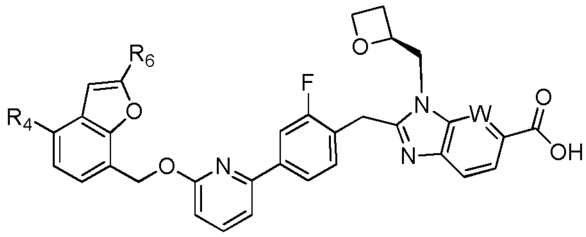

formula (II-1-1),

wherein preferably: $R_4$ is selected from Cl and cyano; $R_6$ is selected from hydrogen atom and F; and $R_{10}$ is selected from hydrogen atom.

In some embodiments of the present invention, the compound shown in general formula (II) above or the pharmaceutically acceptable salt, the stereoisomer, the solvate or the hydrate thereof has a structure shown in general formula (II-2):

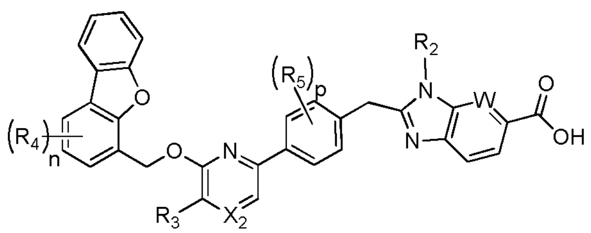

formula (II-2),

wherein:

$R_2$ is selected from

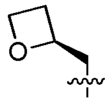 or 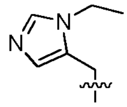 ;

$R_3$ is independently selected from hydrogen atom and halogen;
$R_4$ is independently selected from hydrogen atom, halogen, cyano and C1-C6 alkyl, wherein the C1-C6 alkyl may further be substituted by one or more halogens;
$R_5$ is independently selected from halogen;
W is selected from N and $CR_{10}$;
$X_2$ is selected from N and $CR_{10}$;
p is selected from 0, 1, 2 or 3;
n is selected from 0, 1, 2 or 3; and
$R_{10}$ is selected from hydrogen atom and halogen.

In some embodiments of the present invention, in the compound shown in general formula (II-2) above or the pharmaceutically acceptable salt, the stereoisomer, the solvate or the hydrate thereof, preferably: $R_5$ is selected from F; $R_3$ is selected from hydrogen atom and F; $X_2$ is selected from CH; and $R_{10}$ is selected from hydrogen atom and F.

In some embodiments of the present invention, the compound shown in general formula (II-2) above or the pharmaceutically acceptable salt, the stereoisomer, the solvate or the hydrate thereof has a structure shown in general formula (II-2-1):

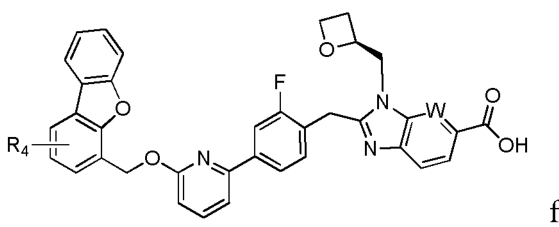

formula (II-2-1),

wherein preferably: $R_4$ is selected from hydrogen atom, Cl and cyano; and $R_{10}$ is selected from hydrogen atom.

In some embodiments of the present invention, the present invention provides a compound of general formula (III) or a pharmaceutically acceptable salt, a stereoisomer, a solvate or a hydrate thereof:

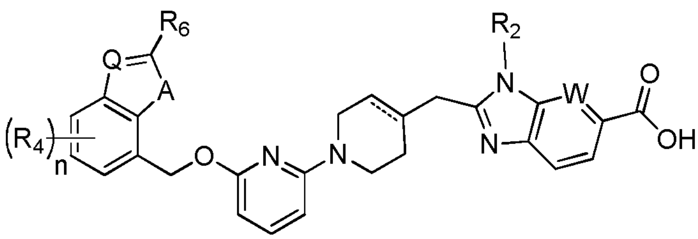

formula (III)

wherein:

$R_2$ is selected from

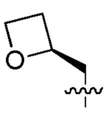 or 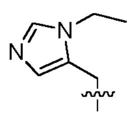 ;

$R_4$ is independently selected from hydrogen atom, halogen and cyano;
$R_6$ is independently selected from hydrogen atom, halogen, C1-C6 alkyl, C3-C6 cycloalkyl, phenyl and furan;
W is selected from N and $CR_{10}$;
Q is selected from N and CH;
A is selected from O and S;

n is selected from 0, 1, 2 or 3;
$R_{10}$ is selected from hydrogen atom and halogen;
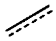 is a single bond or a double bond; and
preferably, $R_6$ is independently selected from hydrogen atom and halogen; and $R_{10}$ is selected from hydrogen atom.

In some embodiments of the present invention, the present invention provides a compound of general formula (IV) or a pharmaceutically acceptable salt, a stereoisomer, a solvate or a hydrate thereof:

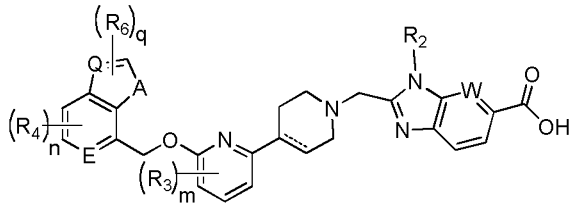
formula (IV)

wherein:

$R_2$ is selected from

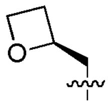 or 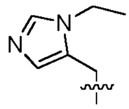 ;

$R_3$ is independently selected from hydrogen atom and halogen;
$R_4$ is independently selected from hydrogen atom, halogen and cyano;
$R_6$ is independently selected from hydrogen atom, halogen, C1-C6 alkyl, C3-C6 cycloalkyl, phenyl, furan and C2-C4 alkenyl; or, two adjacent $R_6$ are connected to form a ring.
W is selected from N and $CR_{10}$;
Q is selected from N and CH;
A is selected from O and S;
E is selected from N and CH;
m is selected from 0, 1 or 2;
n is selected from 0, 1 or 2;
q is selected from 0, 1 or 2;
$R_{10}$ is selected from hydrogen atom and halogen;
when E is CH and Q is CH, two adjacent R6 are connected to form a ring;
preferably, the compound or the pharmaceutically acceptable salt, the stereoisomer, the solvate or the hydrate thereof has a structure shown in general formula (IV-1):

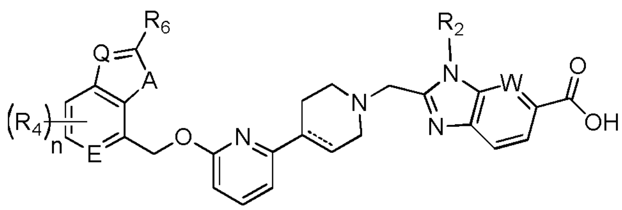
formula (IV-1),

wherein $R_{10}$ is selected from hydrogen atom and F; at least one of Q and E is N; A is selected from O; $R_6$ is selected from hydrogen atom and F; and n is 1;
or, preferably, the compound or the pharmaceutically acceptable salt, the stereoisomer, the solvate or the hydrate thereof has a structure shown in general formula (IV-2):

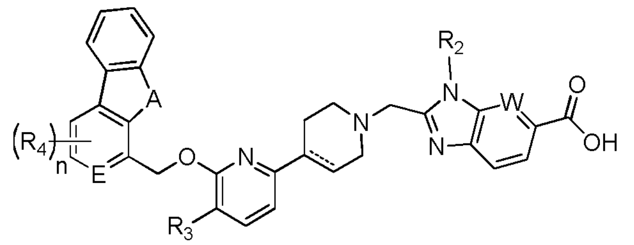

formula (IV-2);

further preferably, $R_3$ is selected from hydrogen atom and F; $R_{10}$ is selected from hydrogen atom and F; A is selected from O; E is selected from CH; and n is 1; further preferably, $R_2$ is selected from

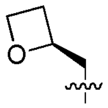

;

and $R_{10}$ is selected from hydrogen atom.

In some embodiments of the present invention, the present invention provides a compound of general formula (V) or a pharmaceutically acceptable salt, a stereoisomer, a solvate or a hydrate thereof:

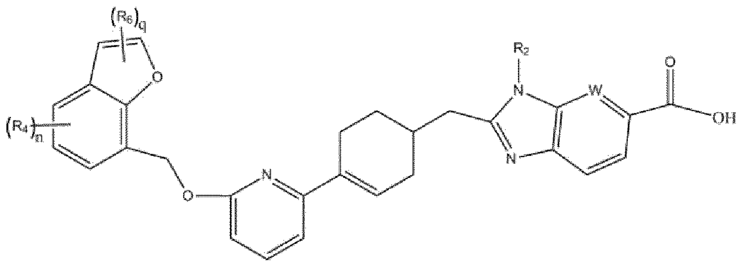

formula (V),

wherein: $R_2$ is selected from

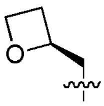

or

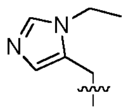

;

$R_4$ is independently selected from hydrogen atom, halogen, cyano and C1-C6 alkyl, wherein the C1-C6 alkyl can be further substituted by one or more halogens; $R_6$ is independently selected from hydrogen atom, halogen, C1-C6 alkyl, phenyl, furan and C2-C4 alkenyl; or, two adjacent $R_6$ are connected to form a ring; W is selected from N and $CR_{10}$; n is selected from 0, 1, 2 or 3; q is selected from 0, 1 or 2; and $R_{10}$ is selected from hydrogen atom and halogen;

preferably, the compound or the pharmaceutically acceptable salt, the stereoisomer, the solvate or the hydrate thereof has a structure shown in general formula (V-1):

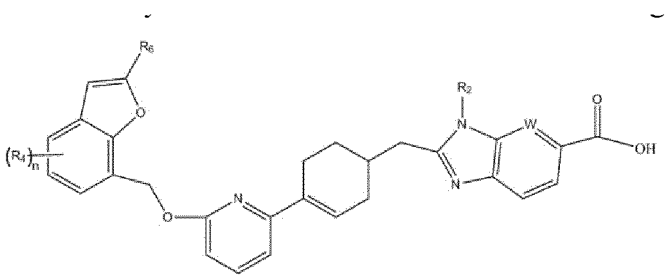

formula (V-1);

further preferably, $R_4$ is selected from hydrogen atom, F, Cl, cyano, $CF_3$ and $CHF_2$; $R_6$ is selected from hydrogen atom and F; $R_{10}$ is selected from hydrogen atom; and n is 1; further preferably, $R_4$ is selected from hydrogen atom, Cl and

cyano;

or preferably, the compound or the pharmaceutically acceptable salt, the stereoisomer, the solvate or the hydrate thereof has a structure shown in general formula (V-2):

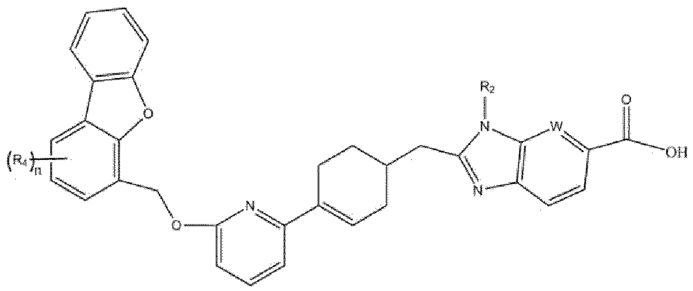

formula (V-2);

further preferably, $R_4$ is selected from hydrogen atom and Cl; $R_{10}$ is selected from hydrogen atom; and n is 1.

In some embodiments of the present invention, the present invention provides a compound of general formula (VI) or a pharmaceutically acceptable salt, a stereoisomer, a solvate or a hydrate thereof:

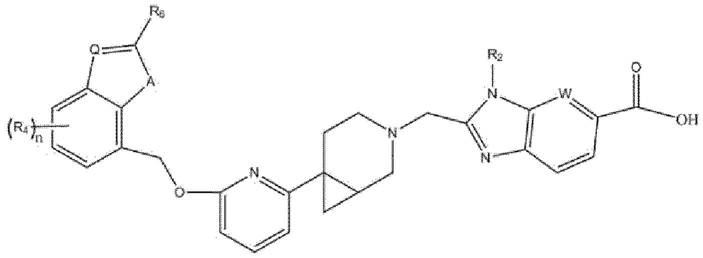

formula (VI),

wherein: $R_2$ is selected from

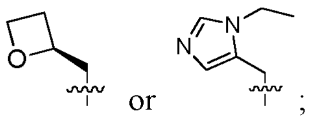

$R_4$ is independently selected from hydrogen atom, halogen, and cyano; $R_6$ is independently selected from hydrogen atom, halogen and C1-C6 alkyl; W is selected from N and $CR_{10}$; Q is selected from N and CH; A is selected from O and S; n is selected from 0, 1 or 2; and $R_{10}$ is selected from hydrogen atom and halogen.

In some embodiments of the present invention, the present invention provides a compound of general formula (VII) or a pharmaceutically acceptable salt, a stereoisomer, a solvate or a hydrate thereof:

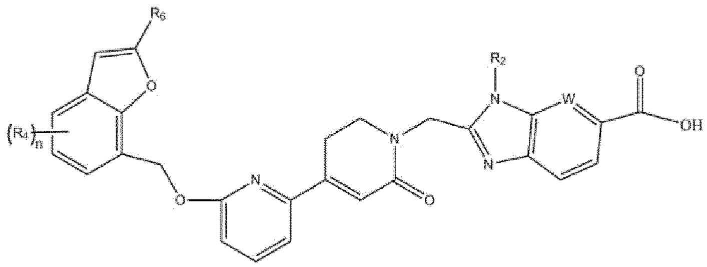

formula (VII),

wherein: $R_2$ is selected from

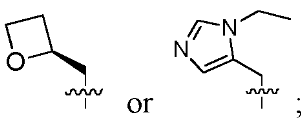

$R_4$ is independently selected from hydrogen atom, halogen, and cyano; $R_6$ is independently selected from hydrogen atom and halogen; W is selected from N and $CR_{10}$; n is selected from 0, 1 or 2; and $R_{10}$ is selected from hydrogen atom and halogen.

In some embodiments of the present invention, the present invention provides a compound of general formula (VIII) or a pharmaceutically acceptable salt, a stereoisomer, a solvate or a hydrate thereof:

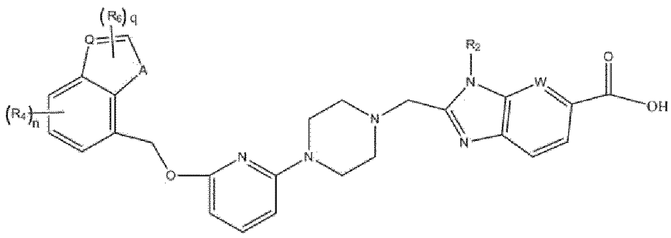

formula (VIII),

wherein: $R_2$ is selected from

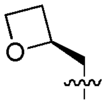 or 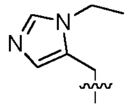 ;

$R_4$ is independently selected from hydrogen atom, halogen, and cyano; $R_6$ is independently selected from hydrogen atom and C2-C4 alkenyl; or, two $R_6$ are connected to form a ring; W is selected from N and $CR_{10}$; Q is selected from N and CH; A is selected from O and S; n is selected from 0, 1 or 2; q is selected from 0 or 1; and $R_{10}$ is selected from hydrogen atom and halogen.

In some embodiments of the present invention, the present invention provides a compound of general formula (IX) or a pharmaceutically acceptable salt, a stereoisomer, a solvate or a hydrate thereof:

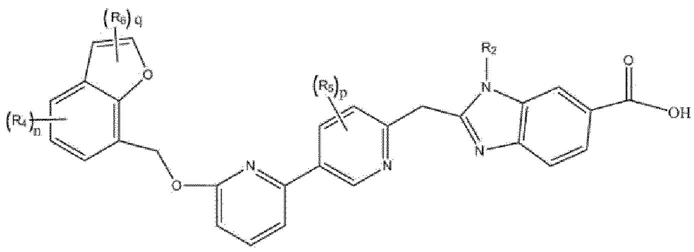

formula (IX),

wherein: $R_2$ is selected from

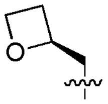 or 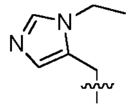 ;

$R_4$ is independently selected from hydrogen atom, halogen, and cyano; $R_5$ is selected from hydrogen atom and F; $R_6$ is independently selected from hydrogen atom and halogen; n is selected from 0, 1 or 2; and q is selected from 0 or 1.

In some embodiments of the present invention, the present invention provides a compound of general formula (II-3) or a pharmaceutically acceptable salt, a stereoisomer, a solvate or a hydrate thereof:

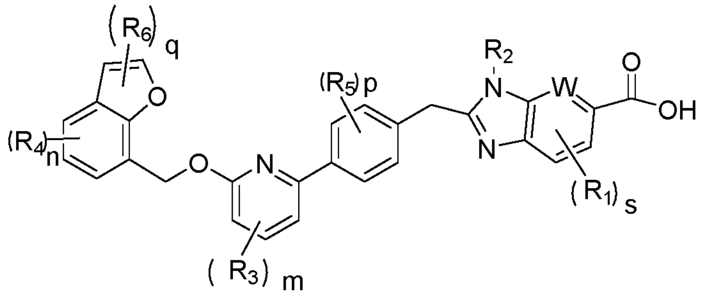

formula (II-3)

wherein:

$R_1$ is selected from hydrogen atom and halogen;
$R_2$ is independently selected from

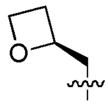 or 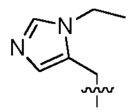 ;

$R_3$ is independently selected from hydrogen atom and halogen;
$R_4$ is independently selected from hydrogen atom, halogen, and cyano
$R_5$ is independently selected from halogen;
$R_6$ is independently selected from hydrogen atom, halogen and C1-C6 alkyl;
W is selected from N and CH;
p is selected from 0, 1, 2 or 3;
m is selected from 0, 1 or 2, 3;
n is selected from 0, 1, 2 or 3;
q is selected from 0, 1 or 2;
s is selected from 0, 1 or 2;
preferably, the compound or the pharmaceutically acceptable salt, the stereoisomer, the solvate or the hydrate thereof has a structure shown in general formula (II-3-1):

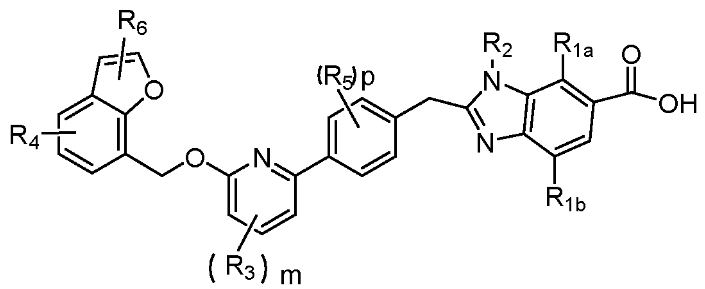

formula (II-3-1)

Wherein $R_{1a}$ and $R_{1b}$ are independently selected from hydrogen atom and F;

$R_2$ is

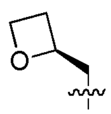 ;

$R_6$ is independently selected from hydrogen atom and halogen;
m is selected from 1 or 2;
p is selected from 1, 2 or 3;
further preferably, the compound or the pharmaceutically acceptable salt, the stereoisomer, the solvate or the hydrate

thereof has a structure shown in general formula (II-3-2):

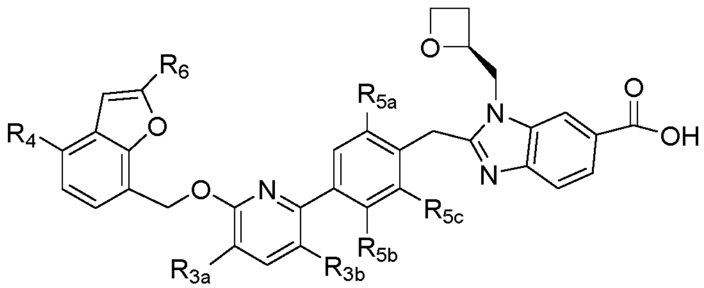

formula (II-3-2)

wherein, $R_{3a}$ and $R_{3b}$ are independently selected from F and hydrogen atom;
$R_4$ is independently selected from F, Cl and cyano;
$R_{5a}$, $R_{5b}$ and $R_{5c}$ are independently selected from hydrogen atom and F;
$R_6$ is independently selected from hydrogen atom, F, and Cl; and
further preferably, $R_{3a}$ and $R_{3b}$ are not F at the same time, $R_{5a}$ and $R_{5c}$ are independently selected from hydrogen atom and F, and $R_{5b}$ is hydrogen atom.

In some embodiments of the present invention, the present invention provides a compound of general formula (V-3) or a pharmaceutically acceptable salt, a stereoisomer, a solvate or a hydrate thereof:

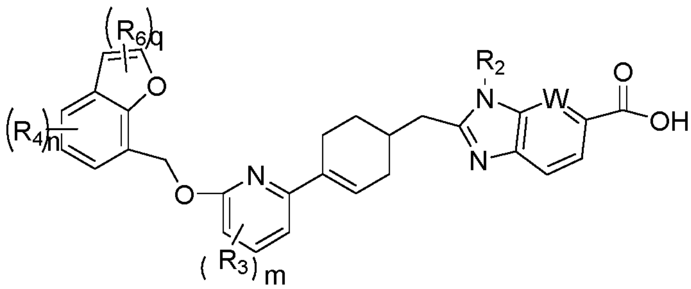

formula (V-3);

wherein:

$R_2$ is selected from

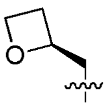

or

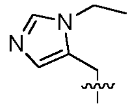

;

$R_3$ is selected from hydrogen atom and halogen;
$R_4$ is selected from hydrogen atom, halogen and cyano;
$R_6$ is selected from hydrogen atom, halogen and C1-C6 alkyl;
W is selected from N and CH;
m is selected from 0, 1 or 2;
n is selected from 0, 1, 2 or 3;
q is selected from 0, 1 or 2;
preferably, the compound or the pharmaceutically acceptable salt, the stereoisomer, the solvate or the hydrate thereof has a structure shown in general formula (V-3-1):

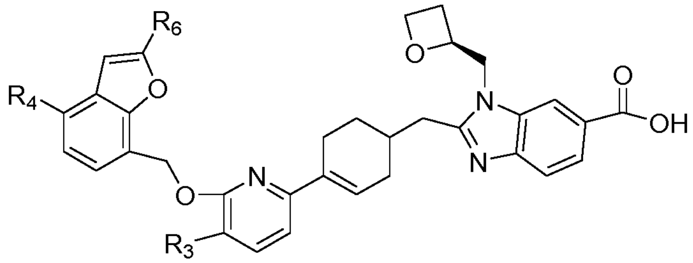

formula (V-3-1)

wherein $R_3$ is selected from hydrogen atom and halogen; $R_4$ is selected from hydrogen atom, halogen, and cyano; and $R_6$ is selected from hydrogen atom and halogen;

further preferably, $R_3$ is selected from hydrogen atom and F; $R_4$ is selected from hydrogen atom, Cl, and cyano; and $R_6$ is selected from hydrogen atom and Cl; and

further preferably, $R_4$ is Cl.

In some embodiments of the present invention, the present invention provides a compound of general formula (II') or a pharmaceutically acceptable salt, a stereoisomer, a solvate or a hydrate thereof

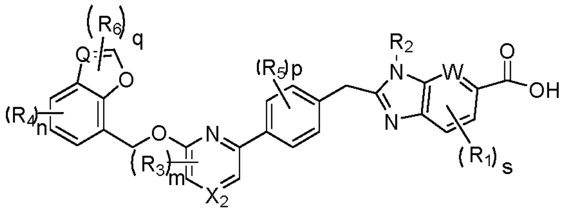

formula (II')

wherein:

$R_1$ is selected from hydrogen atom and halogen;

$R_2$ is selected from

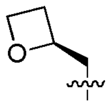 or 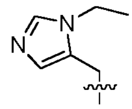 ;

$R_3$ is independently selected from hydrogen atom and halogen;

$R_4$ is independently selected from hydrogen atom, halogen, cyano and C1-C6 alkyl, wherein C1-C6 alkyl can further substituted by one or more halogens;

$R_5$ is independently selected from hydrogen atom and halogen;

$R_6$ is independently selected from hydrogen atom, halogen, C1-C6 alkyl, C3-C6 cycloalkyl, phenyl, furan and C2-C4 alkenyl;

W is selected from N and $CR_{10}$;

$X_2$ is selected from N and $CR_{10}$;

Q is selected from N and CH;

p is selected from 0, 1, 2 or 3;

m is selected from 0, 1, 2 or 3;

n is selected from 0, 1, 2 or 3;

q is selected from 0, 1 or 2;

s is selected from 0, 1 or 2;

$R_{10}$ is selected from hydrogen atom and halogen;

preferably, the compound or the pharmaceutically acceptable salt, the stereoisomer, the solvate or the hydrate thereof has a structure shown in general formula (II'-1):

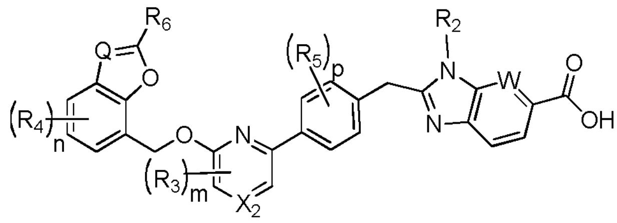

formula (II'-1)

wherein:

- $R_3$ is selected from hydrogen atom and F;
- $R_4$ is selected from hydrogen atom, F, Cl, cyano, $CF_3$ and $CHF_2$;
- $X_2$ and Q are selected from CH; and $R_{10}$ is selected from hydrogen atom and F;
- or,
- preferably, the compound or the pharmaceutically acceptable salt, the stereoisomer, the solvate or the hydrate thereof has a structure shown in general formula (II' -3):

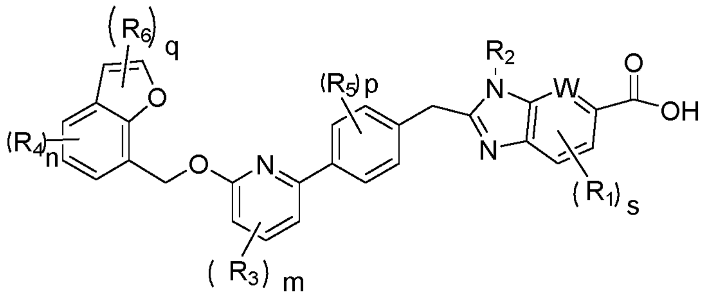

formula (II'-3)

wherein:

- $R_4$ is independently selected from hydrogen atom, halogen and cyano
- $R_6$ is independently selected from hydrogen atom, halogen and C1-C6 alkyl; and
- W is selected from N and CH.

In some embodiments of the present invention, the structure shown in general formula (II'-1) is further the structure shown in general formula (II'-1-1):

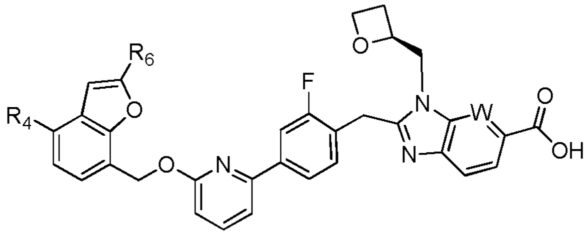

formula (II'-1-1)

wherein:

- $R_4$ is selected from Cl and cyano;
- $R_6$ is selected from hydrogen atom and F; and
- $R_{10}$ is selected from hydrogen atom.

In some embodiments of the present invention, the structure shown in general formula (II'-3) is further the structure shown in general formula (II'-3-1):

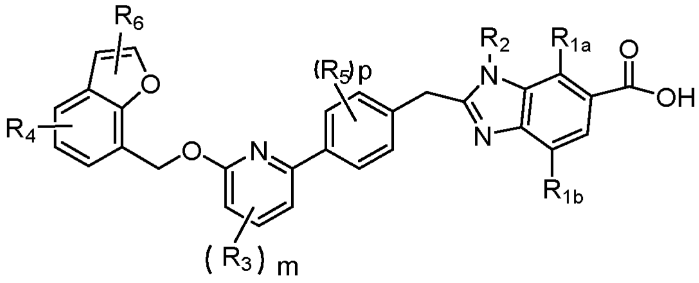

formula (II'-3-1)

wherein:

$R_{1a}$ and $R_{1b}$ are independently selected from hydrogen atom and F;
$R_2$ is

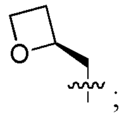
;

$R_6$ is independently selected from hydrogen atom and halogen;
m is selected from 1 or 2;
p is selected from 1, 2 or 3;
preferably, the structure shown in general formula (II'-3) is further the structure shown in general formula (II'-3-2):

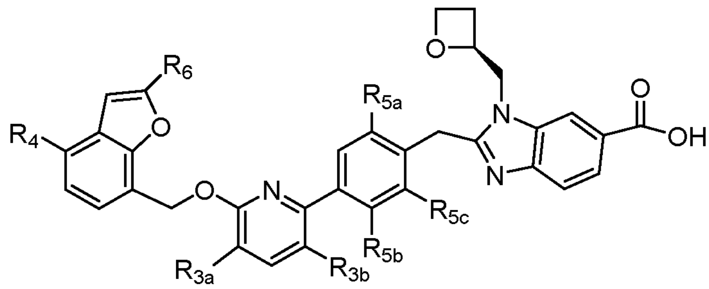

formula (II'-3-2)

wherein:

$R_{3a}$ and $R_{3b}$ are independently selected from F and hydrogen atom;
$R_4$ is independently selected from F, Cl and cyano;
$R_{5a}$, $R_{5b}$ and $R_{5c}$ are independently selected from hydrogen atom and F;
$R_6$ is independently selected from hydrogen atom, F and Cl; and
further preferably, $R_{3a}$ and $R_{3b}$ are not F at the same time, $R_{5a}$ and $R_{5c}$ are independently selected from hydrogen atom and F, and $R_{5b}$ is hydrogen atom.

In some embodiments of the present invention, the following compounds are provided, but the present invention are not limited to the following specific compounds:

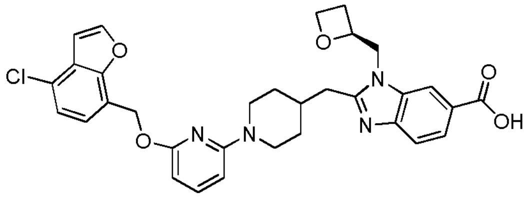
,

compound 1

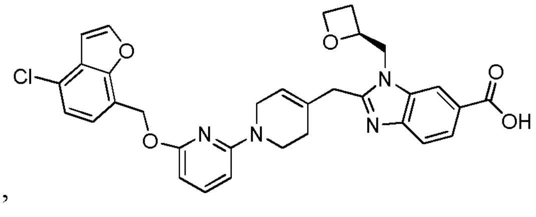
,

compound 2

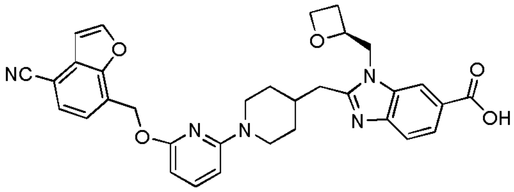

,

compound 3

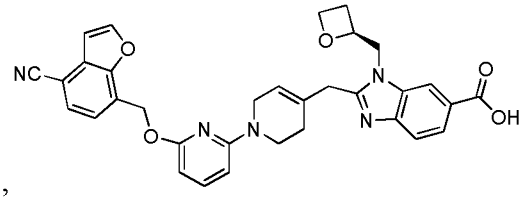

,

compound 4

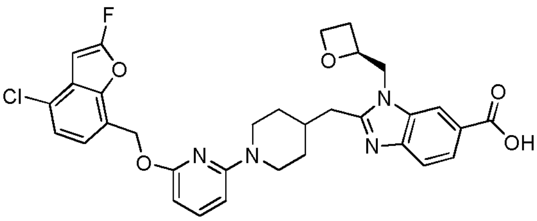

,

compound 5

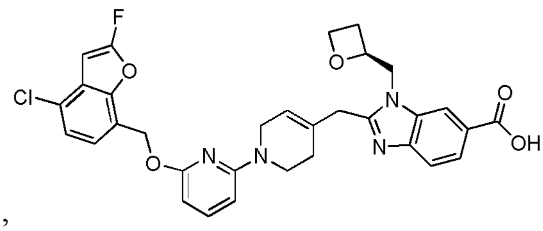

,

compound 6

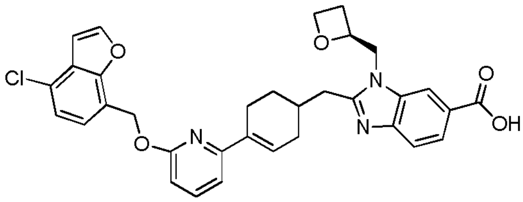

,

compound 7

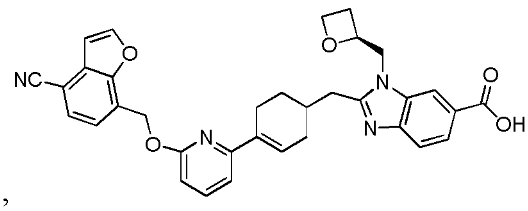

,

compound 8

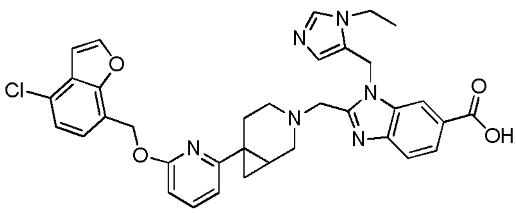

,

compound 9

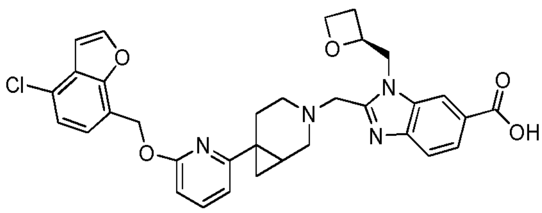

,

compound 10

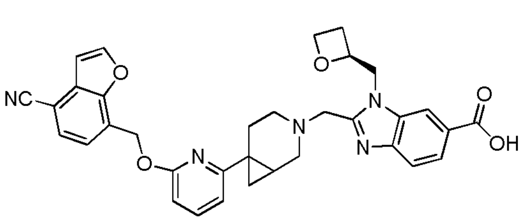

,

compound 11

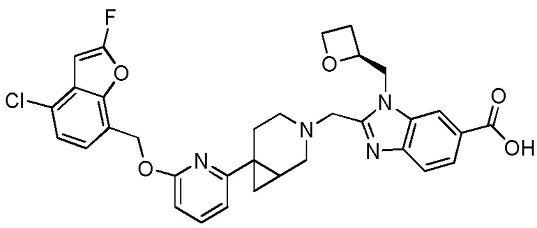

,

compound 12

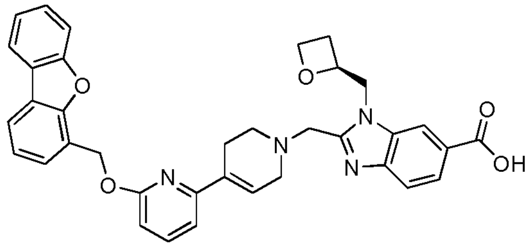
,

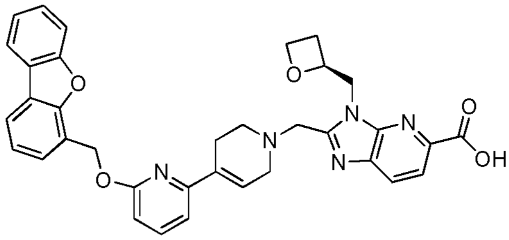
,

compound 13

compound 14

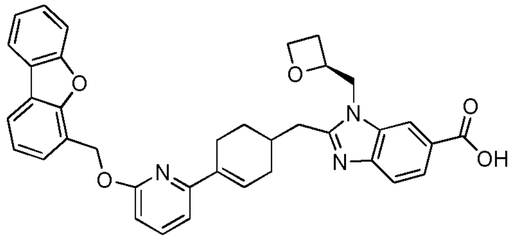
,

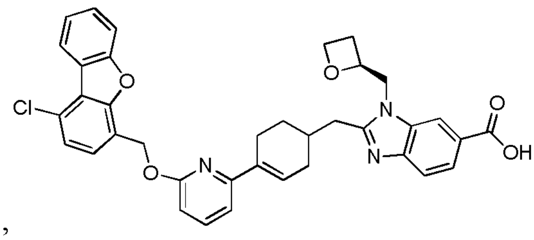
,

compound 15

compound 16

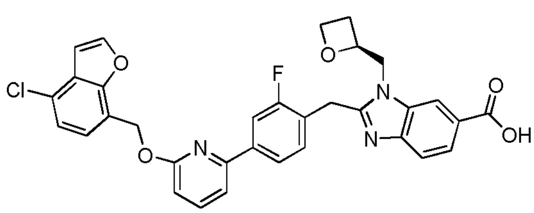
,

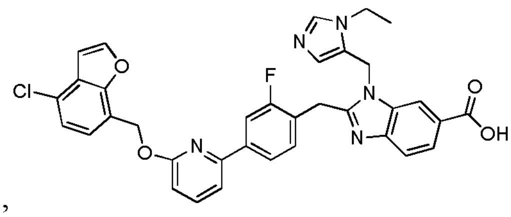
,

compound 17

compound 18

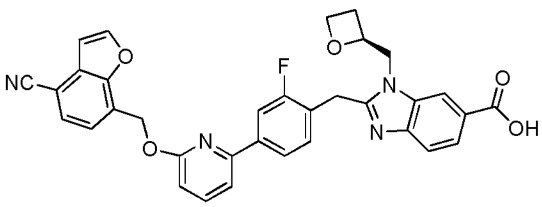
,

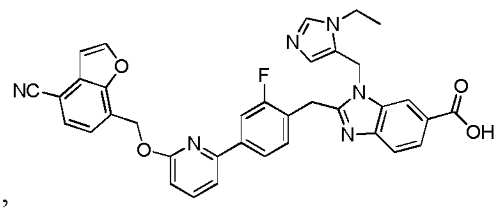
,

compound 19

compound 20

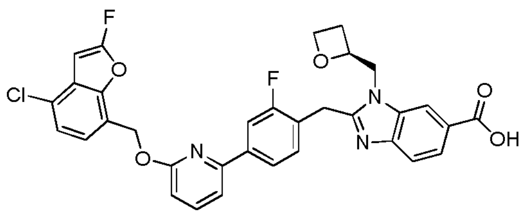
,

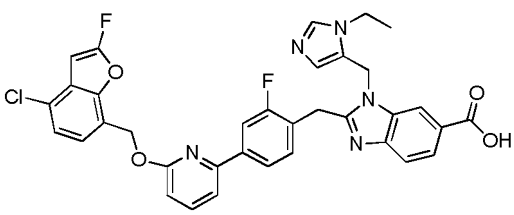
,

compound 21

compound 22

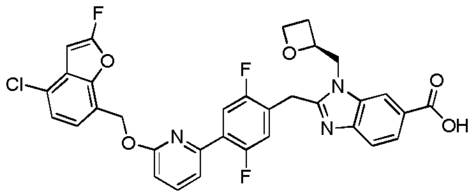
,

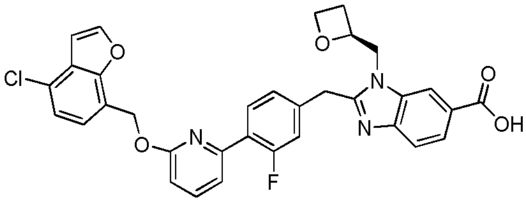
,

compound 23

compound 24

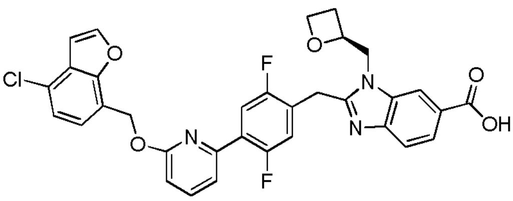
,

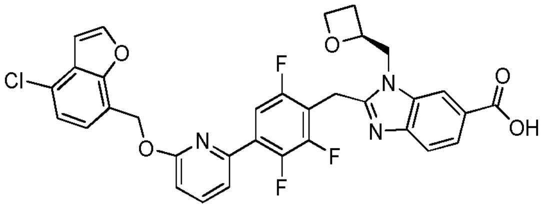
,

compound 25

compound 26

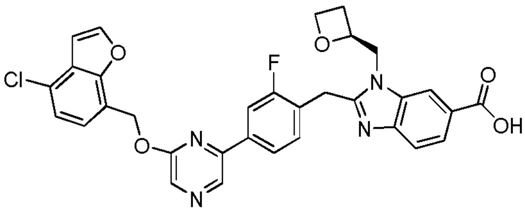
,

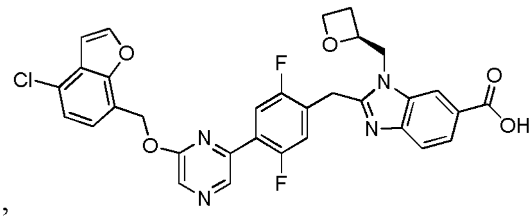
,

compound 27

compound 28

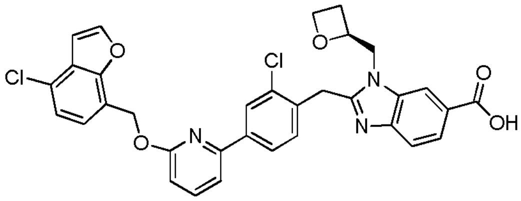
,

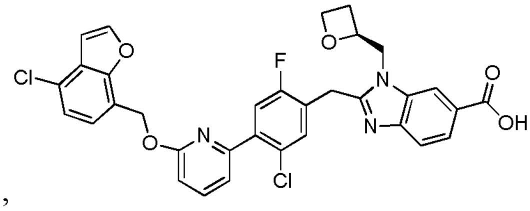
,

compound 29

compound 30

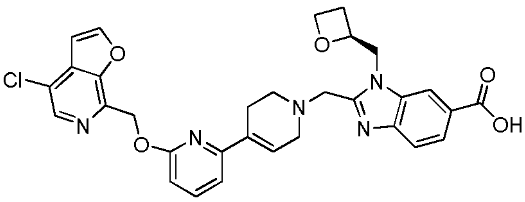
,

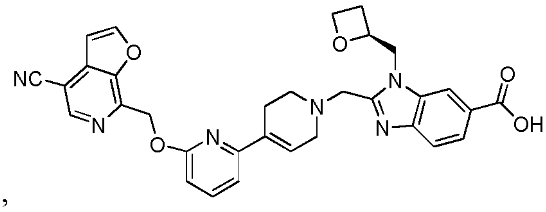
,

compound 31

compound 32

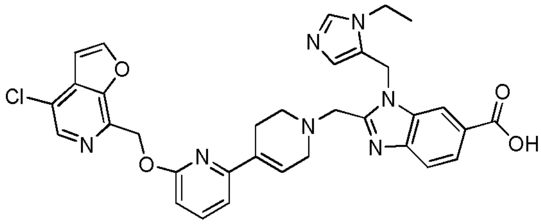

,

compound 33

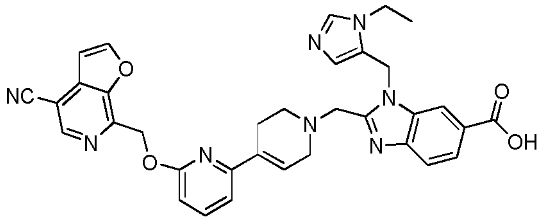

,

compound 34

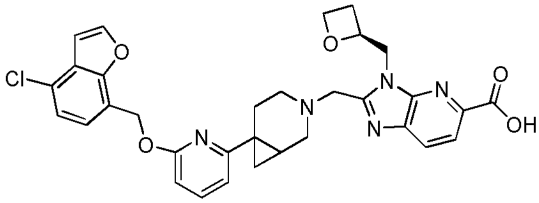

,

compound 35

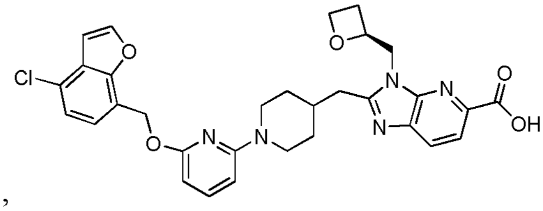

,

compound 36

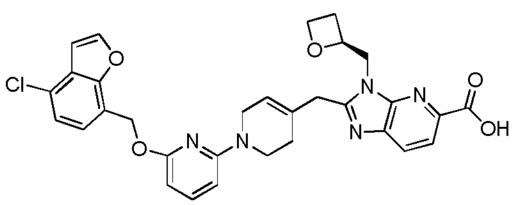

,

compound 37

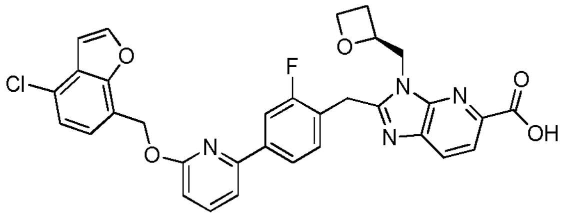

,

compound 38

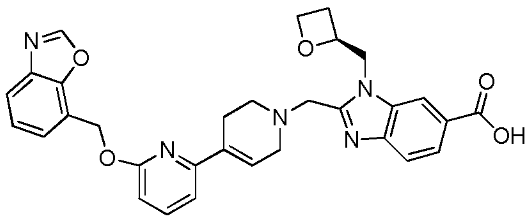

,

compound 39

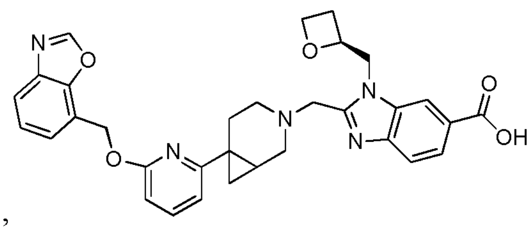

,

compound 40

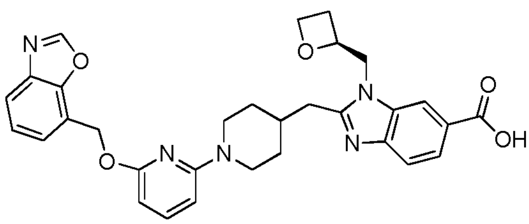

,

compound 41

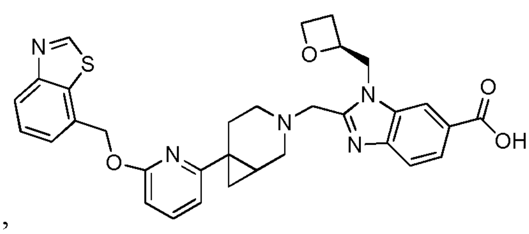

,

compound 42

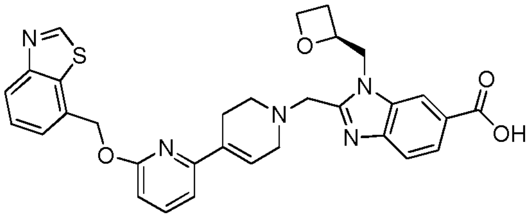
,
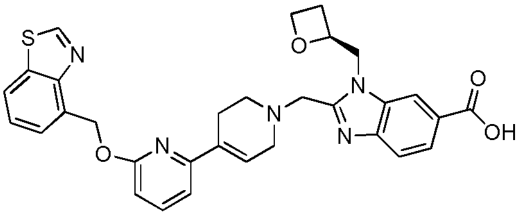
,

compound 43

compound 44

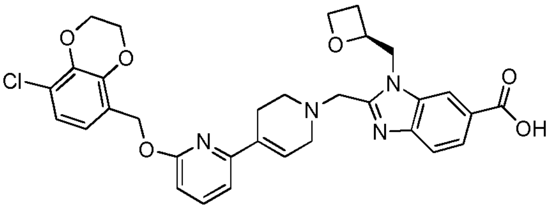
,
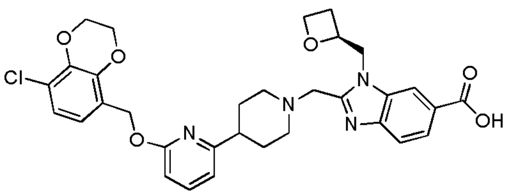
,

compound 45

compound 46

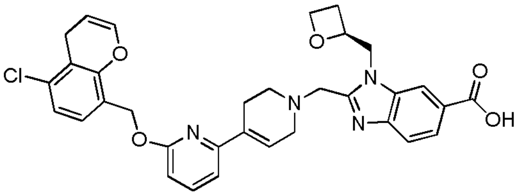
,
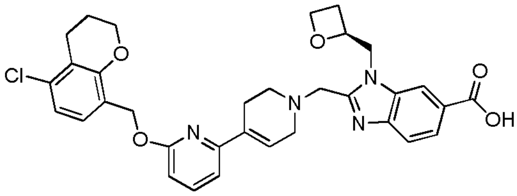
,

compound 47

compound 48

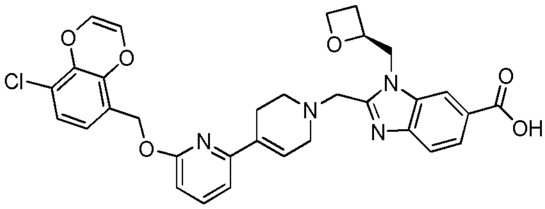
,
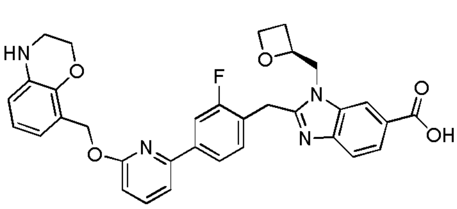
,

compound 49

compound 50

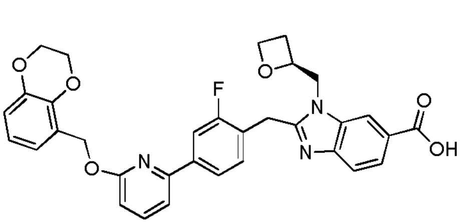
,
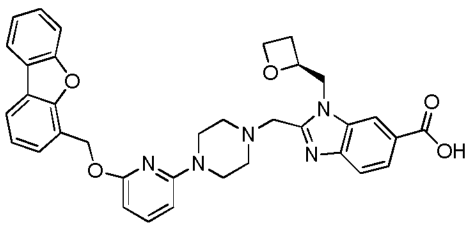
,

compound 51

compound 52

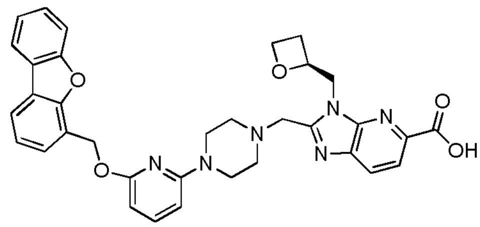
,

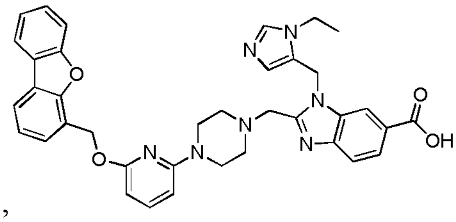
,

compound 53

compound 54

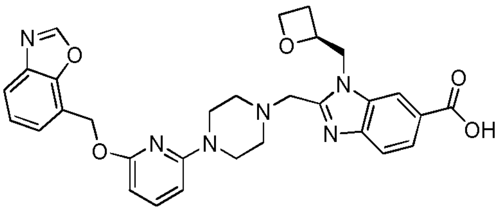
,

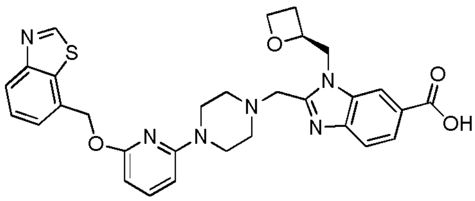
,

compound 55

compound 56

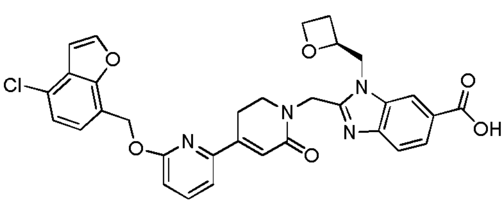
,

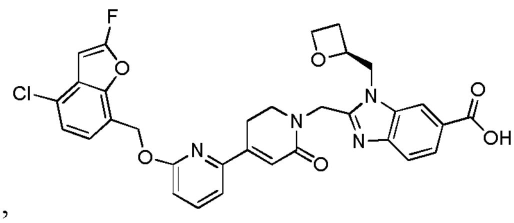
,

compound 57

compound 58

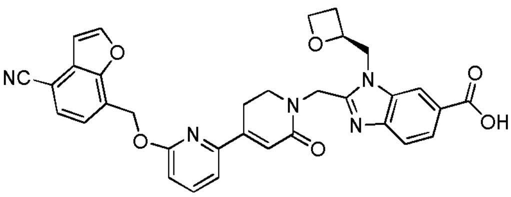
,

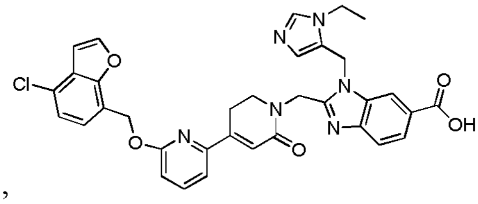
,

compound 59

compound 60

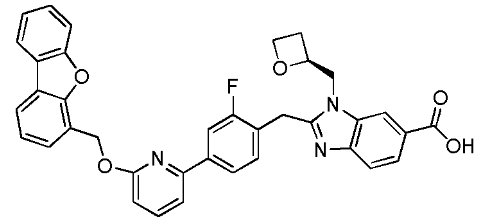
,

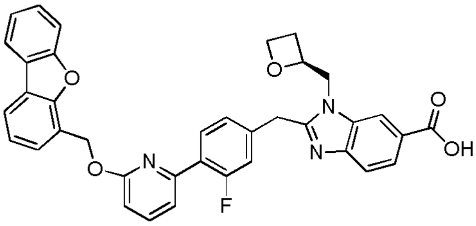
,

compound 61

compound 62

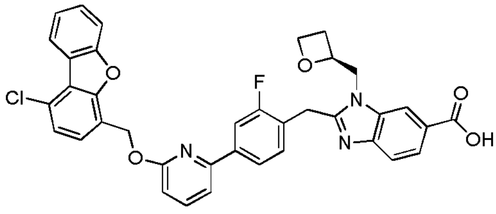

,

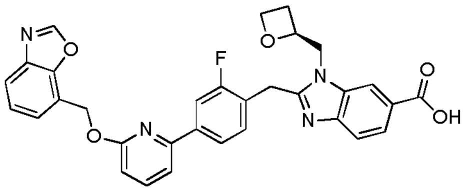

,

compound 63

compound 64

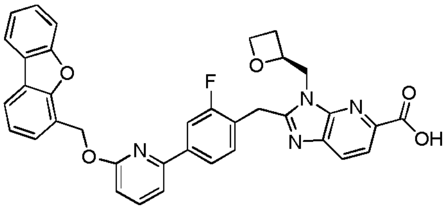

,

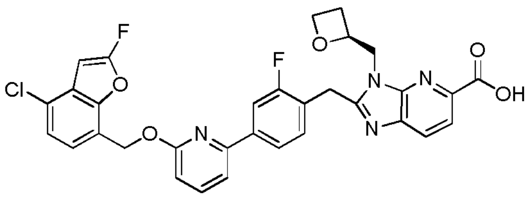

,

compound 65

compound 66

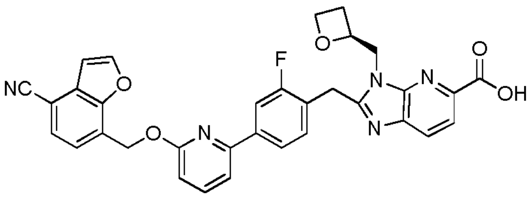

,

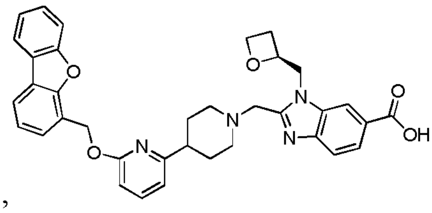

,

compound 67

compound 68

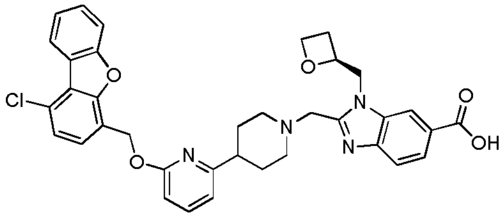

,

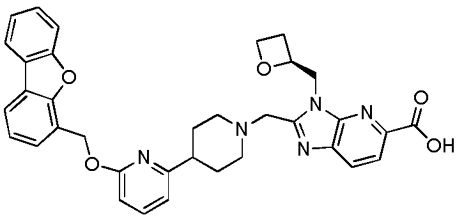

,

compound 69

compound 70

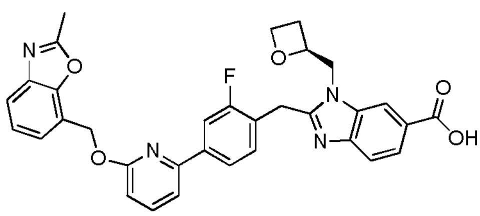

,

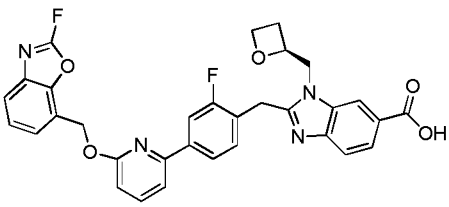

,

compound 71

compound 72

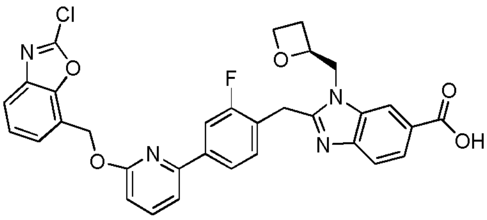
,

compound 73

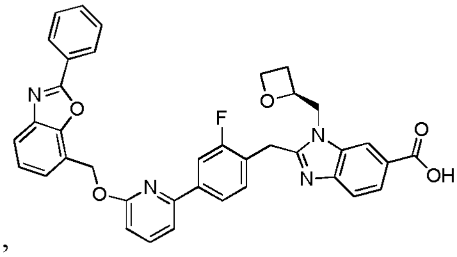
,

compound 74

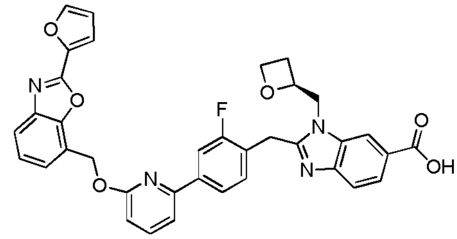
,

compound 75

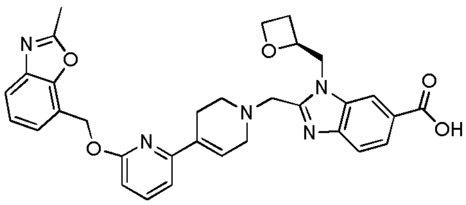
,

compound 76

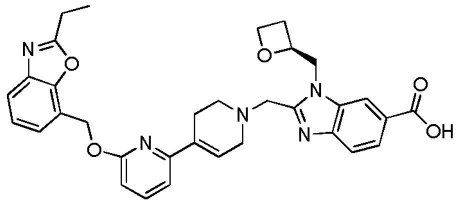
,

compound 77

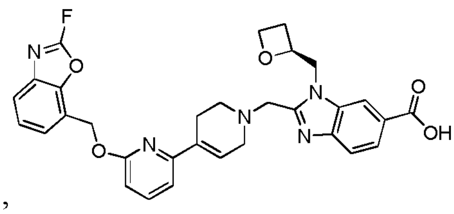
,

compound 78

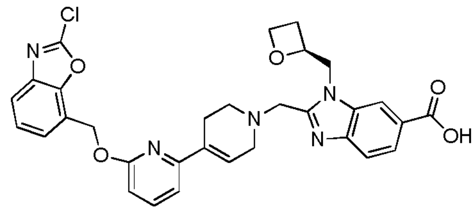
,

compound 79

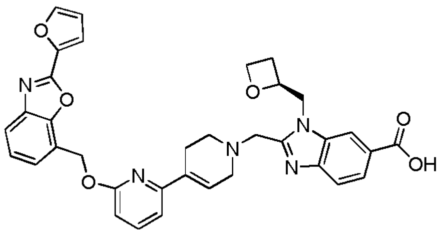
,

compound 80

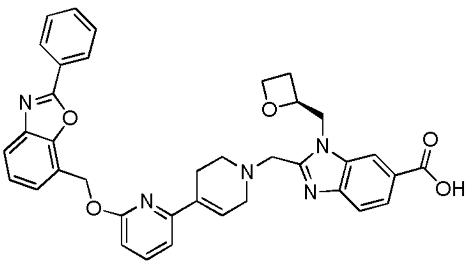
,

compound 81

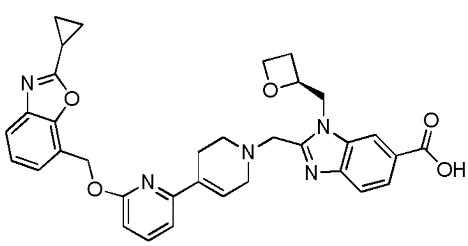
,

compound 82

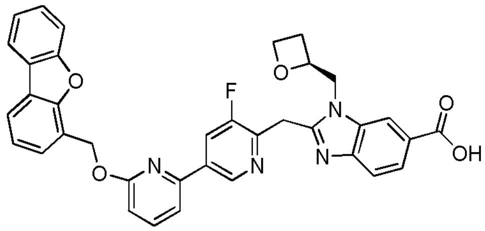

,

compound 83

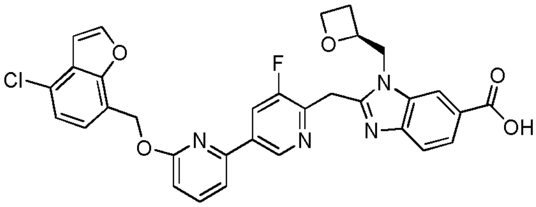

,

compound 84

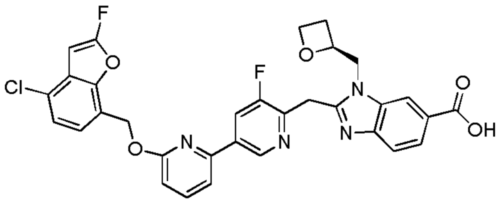

,

compound 85

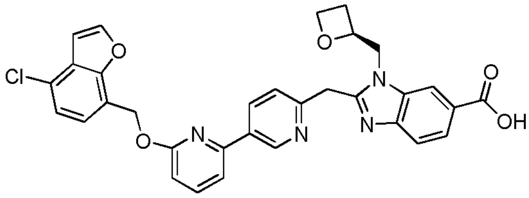

,

compound 86

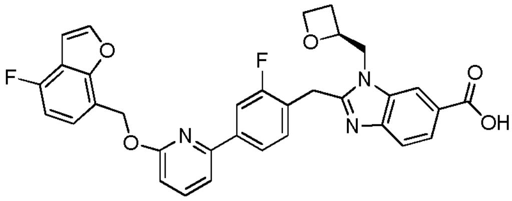

,

compound 87

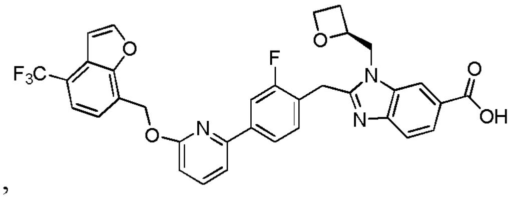

,

compound 88

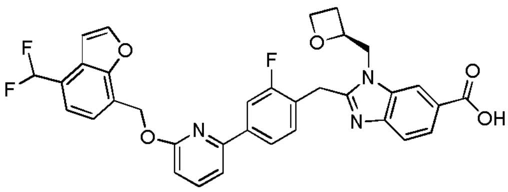

,

compound 89

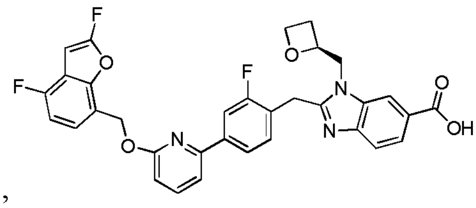

,

compound 90

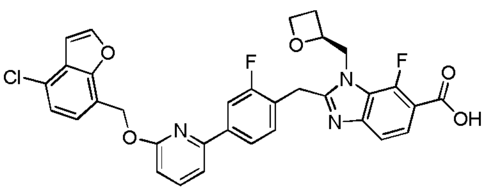

,

compound 91

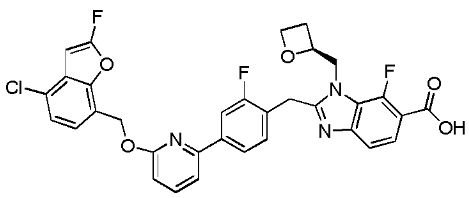

,

compound 92

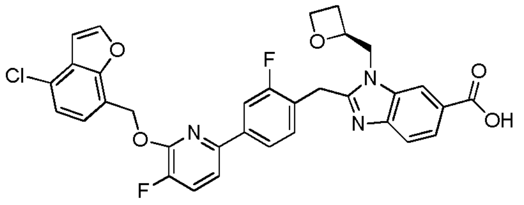,

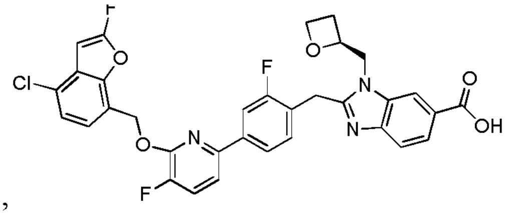,

compound 93

compound 94

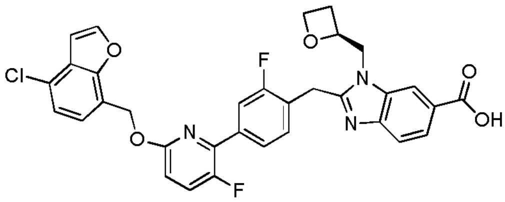,

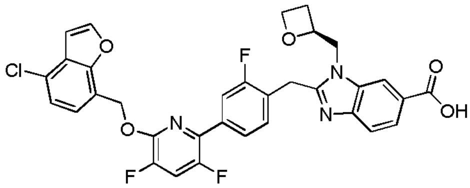,

compound 95

compound 96

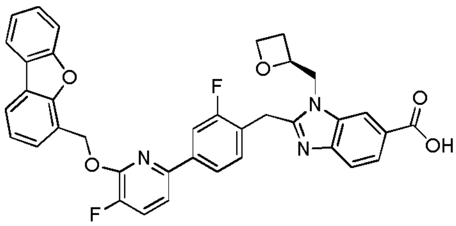,

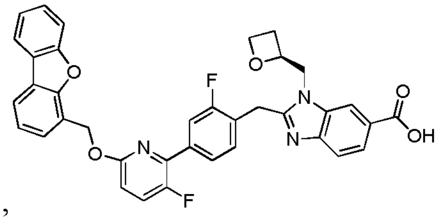,

compound 97

compound 98

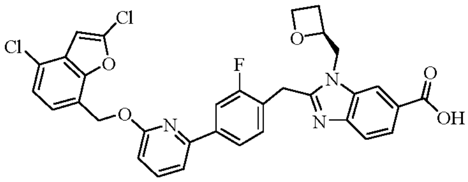,

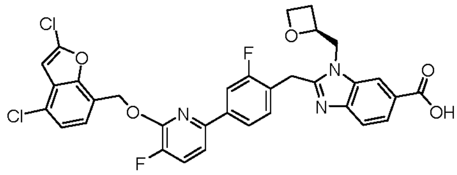,

compound 99

compound 100

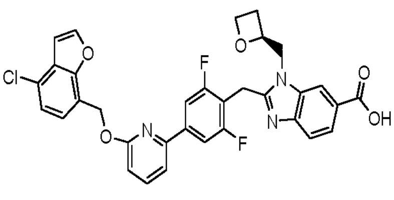,

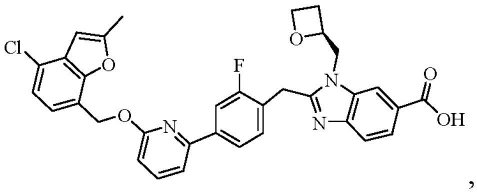,

compound 101

compound 102

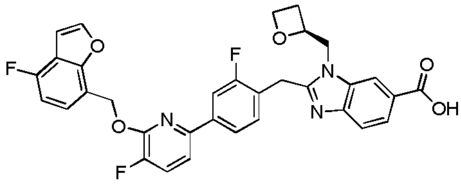,

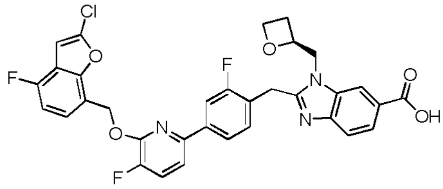,

compound 103

compound 104

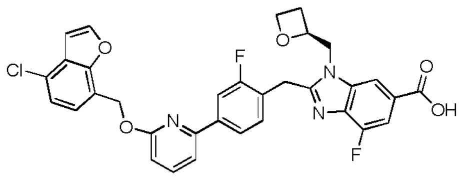,

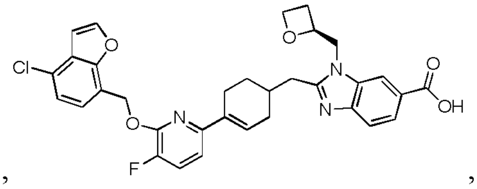,

compound 105 compound 106

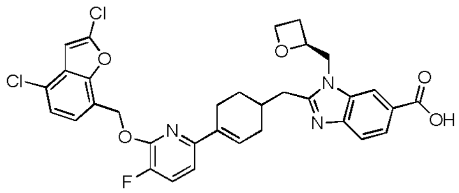,

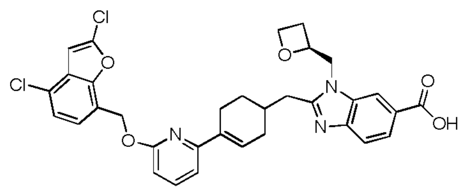

compound 107 compound 108

In some embodiments of the present invention, a pharmaceutical composition is provided, the composition comprises at least one compound of the present invention described herein or a pharmaceutically acceptable salt, a stereoisomer, a solvate or a hydrate thereof, and a pharmaceutically acceptable carrier, an additive or an excipient.

In some embodiments of the present invention, use of the compound of the present invention described herein or the pharmaceutically acceptable salt, the stereoisomer, the solvate or the hydrate thereof as well as the aforementioned pharmaceutical composition in preparing a medicament for a GLP-1 receptor agonist is provided.

In some embodiments of the present invention, use of the compound of the present invention described herein or the pharmaceutically acceptable salt, the stereoisomer, the solvate or the hydrate thereof as well as the aforementioned pharmaceutical composition in preparing a medicament for treating and/or preventing type I diabetes, type II diabetes, malnutrition-related diabetes, diabetic complications, obesity, metabolic syndrome, hyperglycemia, glucose intolerance, nonalcoholic fatty liver disease (NAFLD), nonalcoholic steatohepatitis (NASH), cardiovascular disease, dyslipidemia, cerebral infarction, stroke, Parkinson's disease, dementia, insulin resistance and hepatic insulin resistance is provided; and preferably the use in preparing the medicament for treating and/or preventing type I diabetes, type II diabetes, malnutrition-related diabetes, diabetic complications, obesity, metabolic syndrome, hyperglycemia, glucose intolerance, nonalcoholic fatty liver disease (NAFLD), nonalcoholic steatohepatitis (NASH), cardiovascular disease and dyslipidemia.

Definitions and general terms:

The following terms and the like are used to describe the present invention. It should be understood that, as understood by those skilled in the art, terms without specific definitions are given meanings consistent with those of terms used in the context of the present invention.

The term "alkyl" used in the present invention refers to a saturated aliphatic hydrocarbyl group, and the term comprises straight-chain and branched-chain hydrocarbyl, such as $C_1$-$C_6$ alkyl. "$C_1$-$C_6$ alkyl" refers to alkyl with 1 to 6 carbon atoms, such as alkyl with 1 carbon atom, 2 carbon atoms, 3 carbon atoms, 4 carbon atoms, 5 carbon atoms and 6 carbon atoms, comprising but being not limited to methyl, ethyl, propyl (such as n-propyl and isopropyl), butyl (such as n-butyl, isobutyl and tert-butyl), pentyl (such as n-pentyl, isopentyl and neopentyl), hexyl (such as n-hexyl), and the like.

The term "halogen" used in the present invention refers to fluorine, chlorine, bromine or iodine, preferably fluorine and chlorine.

The term "alkenyl" used in the present invention refers to an aliphatic hydrocarbon group containing a carbon-carbon double bond, preferably a straight-chain or a branched-chain with 2 to 10 carbon atoms, such as vinyl, 1-propenyl, 2-propenyl (allyl), isopropenyl, 2-methyl-1-propenyl, 1-butenyl and 2-butenyl.

The term "alkynyl" used in the present invention refers to straight-chain or branched-chain hydrocarbyl with at least one carbon-carbon triple bond, preferably straight-chain or branched-chain hydrocarbyl with 2 to 10 carbon atoms, such as ethynyl, propynyl and butynyl.

The term "cycloalkyl" used in the present invention refers to a non-aromatic monocyclic or polycyclic system, such as cyclopropyl, cyclobutyl, cyclopentyl and cyclohexyl, preferably cyclopropyl.

The term "heterocyclyl" used in the present invention refers to "heterocyclyl" or "heterocycle", which is a saturated or unsaturated monocyclic or polycyclic system containing one or more heteroatoms selected from O, N or S, preferably a 3-membered to 10-membered monocycle or polycycle.

The term "aryl" used in the present invention refers to an aromatic group, such as phenyl, naphthyl, tetrahydronaphthyl, indanyl and biphenyl, preferably phenyl.

The term "heteroaryl" used in the present invention refers to an aromatic ring containing one or more heteroatoms

selected from O, N or S, the heteroaryl may be a monocyclic, bicyclic or tricyclic ring system, such as quinolyl, pyrazolyl, pyrrolyl, thienyl, furyl, pyridyl, pyrimidinyl, pyrazinyl, triazolyl, imidazolyl, oxazolyl, isoxazolyl, pyridazinyl, benzofuryl, benzothiophenyl, benzoxazolyl, indolyl, and the like.

The term "pharmaceutically acceptable salt" used in the present invention refers to the salt of the compound of the present invention, which is prepared from the compound with specific substituent found in the present invention and a pharmaceutically acceptable acid or base.

The term "stereoisomer" used in the present invention refers to a compound with the same chemical composition but different spatial arrangement of atoms and groups, comprising an enantiomer, a diastereomer, a geometric isomer, a trans-hindered isomer or a conformational isomer.

The term "racemate" used in the present invention refers to a mixture of two equimolar enantiomers, which lacks optical activity, and a mixture thereof, such as a racemic mixture.

The term "solvate" used in the present invention is used to describe a molecular complex comprising a compound of any chemical formula above or a pharmaceutically acceptable salt thereof and one or more pharmaceutically acceptable solvent molecules (such as ethanol). When the solvent is water, the term "hydrate" is used.

The term "pharmaceutically acceptable carrier" used in the present invention refers to any preparation carrier or medium which can deliver an effective amount of active substance of the present invention, does not interfere with biological activity of an active substance, and has no toxic or side effects on a host or patient, and representative carriers comprise water, oil, vegetables and minerals, cream base, lotion base, ointment base, and the like. These bases comprise a suspending agent, a tackifier, a transdermal enhancer, and the like.

The compound of the present invention may also contain atomic isotopes in unnatural proportions at one or more atoms constituting such compound. For example, the compound may be radiolabeled with a radioactive isotope, such as deuterium (D), tritium ($^3H$), iodine -125 ($^{125}I$) or carbon-14 ($^{14}C$). All isotopic variations of the compound of the present invention, whether irradiated or not, are included in the scope of the present invention.

In addition, a prodrug and ester of the compound of any formula above are also included in the scope of the present invention.

The beneficial technical effects obtained are as follows:

1) The compounds of the present invention have excellent biological activities, for example, the cell activities in vitro of the 25 compounds such as the compound 14, the compound 17, the compound 18, the compound 19, the compound 21, the compound 38, the compound 67, the compound 87, the compound 91, the compound 92, the compound 93, the compound 94, the compound 95, the compound 96, the compound 97, the compound 99, the compound 100, the compound 101, the compound 102, the compound 103, the compound 104, the compound 105, the compound 106, the compound 107 and the compound 108 are significantly better than those of the compounds in the prior art. For example, the hypoglycemic effects in vivo of the compound 17, the compound 19, the compound 21, the compound 38, the compound 61, the compound 93, the compound 94, the compound 99, the compound 100, the compound 106 and the compound 107 are significantly better than those of the compounds in the prior art.
2) The compounds of the present invention have good pharmacokinetic properties, such as effectively prolonging the half-life and increasing the plasma exposure, and the in vivo half-lives of the compound 21, the compound 93, the compound 94, the compound 99, the compound 100, and the like, are significantly better than those of the compounds in the prior art.
3) The compounds of the present invention also have safe hERG cardiotoxicity.

## BRIEF DESCRIPTION OF THE DRAWINGS

FIG. 1 shows blood glucose concentration curves of compounds 17, 19, 38 and 61 of the present invention and comparative examples.
FIG. 2 shows blood glucose AUC (0 to 120 minutes) of compounds 17, 19, 38 and 61 of the present invention and comparative examples.
FIG. 3 shows blood glucose concentration curves of compounds 17, 93 and 100 of the present invention and comparative examples.
FIG. 4 shows blood glucose AUC (0 to 120 minutes) of compounds 17, 93 and 100 of the present invention and comparative examples.
FIG. 5 shows blood glucose concentration curves of compounds 21, 94, 99, 106 and 107 of the present invention and comparative examples.
FIG. 6 shows blood glucose AUC (0 to 120 minutes) of compounds 21, 94, 99, 106 and 107 of the present invention and comparative examples.

DETAILED DESCRIPTION

The present application is further described below in conjunction with the embodiments below, which are set forth to provide further illustration of the practice of the present application, but it should be noted that the embodiments described below are exemplarily, which are only used to illustrate the present application, but are not to be construed as limiting the present application. Modifications and substitutions thereof by those skilled in the art are also within the scope of the present application as determined by the appended claims. The reagents used in the embodiments of the present application are all commercially available.

Example 1:

Synthesis of Compound 1

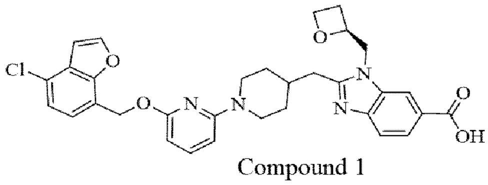

Compound 1

Synthesis route:

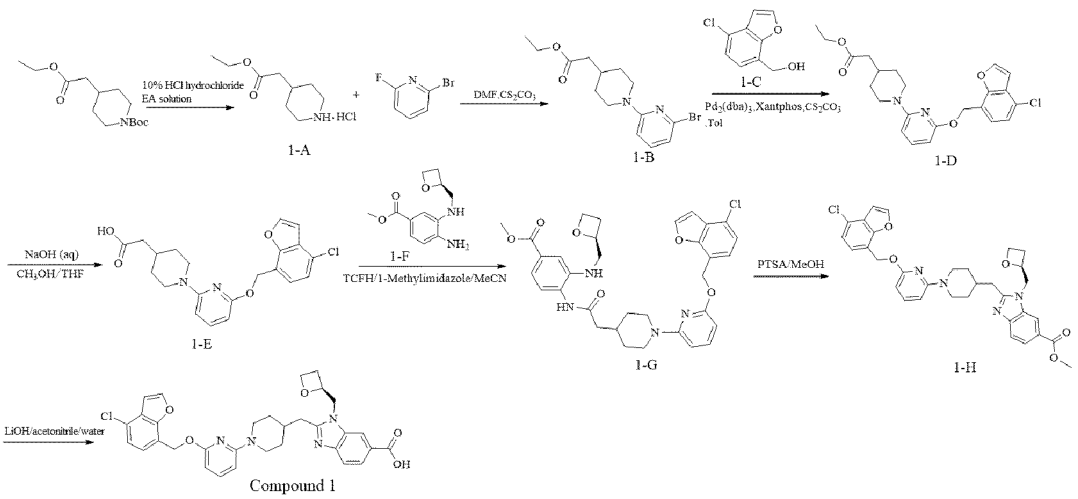


Compound 1-A:

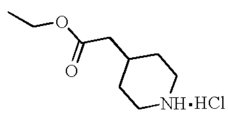

1-A

2.0 g of compound 1-Boc-4-piperidine acetic acid ethyl ester (1.0 eq.) was weighed and added to a 50mL round-bottom flask, 5 mL of ethyl acetate was added, followed by 20 mL of 10% ethyl acetate hydrochloride solution, and the reaction mixture was stirred at room temperature for 3-4 hours. TLC showed that the raw material 1-Boc-4-piperidine acetic acid ethyl ester was completely reacted, and then the reaction solution was concentrated under reduced pressure to obtain compound 1-A (ethyl 4-piperidineacetate hydrochloride). The product could be directly used in next reaction without purification.

Compound 1-B:

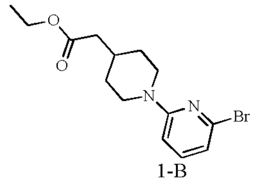

1-B

589.9 mg of compound 1-A (1.0 eq.) was weighed and added to a 50mL round-bottom flask, 10 ml of DMF was added, and followed by 1.85 g of $Cs_2CO_3$ (2.0 eq.). After the reaction solution was stirred at room temperature for 30 minutes, 500 mg of 2-bromo-6-fluoropyridine (1.0 eq.) was added, and the reaction solution was stirred evenly, placed at 70°C for reaction under heating for 1-2 hours. TLC showed that the raw material 2-bromo-6-fluoropyridine was completely reacted. Then, the heating was stopped and the reaction solution was cooled to room temperature, 16 mL of water was added, and then the system was extracted with 10 mL×2 of ethyl acetate twice. The organic phases were combined and washed with 10 mL×2 of water twice and then washed with saturated salt solution. The organic phases were dried with anhydrous sodium sulfate, and then filtered. The filtrate was concentrated under reduced pressure to obtain brownish red oily compound 1-B (850 mg, yield 91.50%). The product was directly used in the next step without purification.

Compound 1-C:

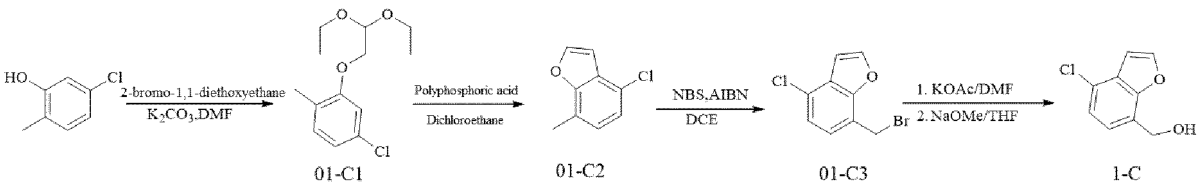


01-C1 01-C2 01-C3 1-C

Compound 01-C1:

10.0 g of 5-chloro-2-methylphenol (1.0 eq.), 20.74 g of 2-bromo-1,1-diethoxyethane (1.5 eq.) and 14.54 g of $K_2CO_3$ (1.5 eq.) were weighed and added to a 500mL round-bottom flask and 200 mL of DMF was added. Then, under the protection of nitrogen, the reaction solution was stirred evenly and heated at 120°C for 16 hours. TLC showed that the raw material 5-chloro-2-methylphenol was completely reacted, the heating was stopped and the reaction solution was cooled to room temperature. Then, 400 mL of water was added to the reaction solution and extracted with 150 mL×3 of ethyl acetate for thrice. The organic phases were combined, washed with 200 mL×2 of water twice and washed with 200 mL×2 of saturated salt solution twice. Then the organic phases were dried with anhydrous sodium sulfate and filtered. The filtrate was concentrated under reduced pressure to obtain an oily crude product, which was purified by column chromatography (PE as eluent) to obtain compound 01-C1 (12.39 g, yield 68.41%).

Compound 01-C2:

12.39 g of compound 01-C1 (1.0 eq.) and 24.33 g of polyphosphoric acid (1.5 eq.) were weighed and added to a 500mL round-bottom flask. 250 ml of toluene was added. Then, under the protection of nitrogen, the reaction solution was stirred evenly and heated at 90°C for 15 hours. TLC showed that the compound 01-C1 was completely reacted. After the heating was stopped and the reaction solution was cooled to room temperature, the reaction solution was quenched with 250 mL of water and left to stand for stratification. The organic phases were washed with 150 mL of water once, washed with 150 mL×2 of saturated $NaHCO_3$ solution twice, and then washed with 150 mL of saturated salt solution once. The organic phases were collected and dried with anhydrous sodium sulfate and filtered. The filtrate was concentrated under reduced pressure to obtain oily compound 01-C2, which was directly used in the next step without purification.

Compound 01-C3:

9.25 g of crude compound 01-C2 obtained above, 11.85 g of NBS (1.2 eq.) and 1.82 g of 2-2-azodiisobutyronitrile (0.2 eq.) were added to a 250mL round-bottom flask. 100 ml of DCE (1,2-dichloroethane) was added. The reaction mixture was stirred evenly and then heated at 72°C for 12 hours. TLC showed that the raw materials were basically reacted completely and the heating was stopped. After cooling slightly, the solvent was removed under reduced pressure. The residue was purified by silica gel column chromatography (PE as eluent) to obtain oily compound 01-C3 (9.0 g, yield

66.04%).

Compound 1-C:

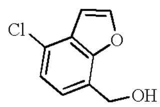

1-C

9.0 g of compound 01-C3 (1.0 eq.) was weighed and added to a 250mL round-bottom flask. 90 mL of DMF was added and stirred to dissolve, then 36.0 g of potassium acetate (10.0 eq.) was added, and the mixture was stirred at room temperature for 2.5 hours. TLC showed the raw materials were completely reacted. 270 mL of water and 100 mL of ethyl acetate were added to the system, and the system was left to stand for liquid separation. The aqueous phase was extracted with 100 mL×2 of ethyl acetate twice. The ethyl acetate organic phases were combined and washed with 100 mL×2 of water twice and then washed with 100 mL×2 of saturated salt solution twice, dried with anhydrous sodium sulfate and filtered. The filtrate was concentrated under reduced pressure to obtain a yellow oily substance. Subsequently, 40 mL of THF was added to the oily substance, and stirred evenly, then 14.68 mL of 5 mol/L sodium methoxide solution was added, and the mixture was stirred at room temperature for 1.5 hours. TLC showed that the raw materials disappeared. The reaction was stopped, 120 mL of water and 100 mL of ethyl acetate were added to the reaction solution, and the reaction solution was left to stand for liquid separation. The aqueous phase was extracted with 100 mL×2 of ethyl acetate twice. The organic phases were combined and washed with 100 mL×2 of saturated salt solution twice. The organic phases were dried with anhydrous sodium sulfate and filtered. The filtrate was concentrated under reduced pressure to obtain a crude yellow oily substance, which was purified by column chromatography to obtain off-white solid compound 1-C (4.5 g, yield 67.5%). $^1$H NMR (400 MHz, DMSO-$d_6$) δ = 8.12 (s, 1H), 7.34-7.32 (m, 2H), 7.01 (s, 1H), 5.39 (t, *J* = 5.6Hz, 1H), 4.79 (d, *J* = 5.6 Hz, 2H).

Compound 1-D:

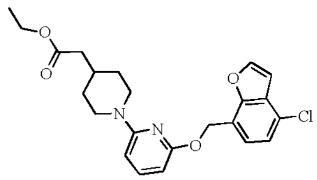

1-D

850 mg of compound 1-B (1.0 eq.) was weighed and added to a 100mL round-bottom flask. 473.63 mg of compound 1-C (1.0 eq.), 1.69 g of $Cs_2CO_3$ (2.0 eq.), 300.42 mg of Xantphos (0.2 eq.), and 237.54 mg of $Pd_2(dba)_3$ (0.1 eq.), then 32 mL of toluene were added. The reaction mixture was stirred evenly, and replaced with nitrogen thrice, heated at 110°C under the protection of nitrogen for 4-5 hours. TLC showed that two raw materials were completely reacted. The heating was stopped, and the reaction solution was cooled to room temperature, and filtered with diatomite. Then, 50 mL of water was added to the filtrate and the system was left to stand for liquid separation. The toluene phase was washed with 30 mL×2 of saturated salt solution twice, dried with anhydrous sodium sulfate and filtered. The filtrate was concentrated to obtain a yellow oily substance, which was purified by column chromatography (petroleum ether/ethyl acetate = 95/5 as eluent) to obtain oily compound 1-D (640 mg, yield 57.45%).

Compound 1-E:

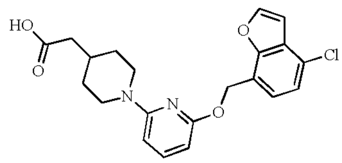

1-E

640 mg of compound 1-D (1.0 eq.) was weighed and added to a 100mL round-bottom flask. 30 mL of mixed solution of MeOH/THF = 1/1 (volume ratio)was added, and 4.5 mL of 2 mol/L NaOH solution was added. The reaction mixture was heated at 40°C for 2 hours. TLC showed that the raw materials were completely reacted. The heating was

stopped, and the reaction solution was cooled to room temperature, concentrated under reduced pressure, and then added with 15 mL of water. The pH of the reaction solution was adjusted to be about 4 with 2 mol/L hydrochloric acid solution. Then, the reaction solution was extracted with 15 mL×2 of ethyl acetate twice, and the organic phases were combined and washed with 30 mL×2 of saturated salt solution twice, dried with anhydrous sodium sulfate, and filtered. The filtrate was concentrated under reduced pressure to obtain yellow oily compound 1-E (560 mg, yield 93.76%).

Compound 1-F:

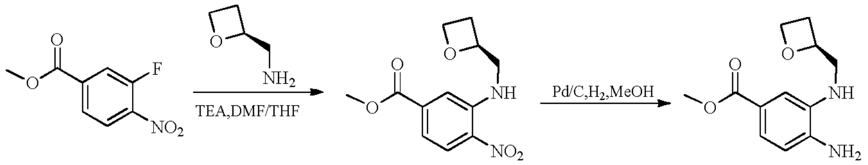


01-F1 1-F

Compound 01-F1:

9.0 g of methyl 3-fluoro-4-nitrobenzoate (1.0 eq.) was weighed and added to a 500mL round-bottom flask. 180 mL of DMF and 180 mL of THF, then 13.00 mL of TEA (2.0 eq.) were added. The reaction mixture was stirred evenly, and 4.8 g of (*S*)-2-(aminomethyl)oxetane (1.2 eq.) was added. The reaction solution was stirred at room temperature to react for 16 hours. TLC showed that the raw materials were basically reacted completely. Subsequently, the mixture was concentrated under reduced pressure to remove THF, then 540 mL of water was added to the remaining DMF solution, and the mixture was stirred and crystallized for 3-4 hours. The mixture was filtered to obtain yellow solid compound 01-F1 (9.88 g, yield 82.47%).

Compound 1-F:

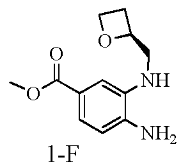

1-F

6.0 g of compound 01-F1 (1.0 eq.) was weighed and added to a 250mL round-bottom flask. 90 mL of methanol was added and stirred to form a suspension and then 600 mg of Pd/C (10%) was added, the system was replaced with hydrogen thrice. The reaction solution was reacted at room temperature in hydrogen atmosphere for 3 hours. TLC showed that the raw materials were completely reacted. Subsequently, the mixed reaction solution was filtered with diatomite, and the filtrate was concentrated under reduced pressure to obtain brown oily compound 1-F (5.3 g, yield 100%). $^1$HNMR (400 MHz, $CDCl_3$) δ=7.41 (dd, J = 8.1, 1.5 Hz, 1H), 7.30 (s, 1H), 6.62 (d, J = 8.1 Hz, 1H), 5.05-5.02 (m, 1H), 4.67 (dd, J = 14.1, 7.7 Hz, 1H), 4.57-4.52 (m, 1H), 4.16(brs, 3H), 3.81 (s, 3H), 3.38 (dd, J = 12.9, 6.9 Hz, 1H), 3.28 (dd, J = 12.9, 3.5 Hz, 1H), 2.72 - 2.61 (m, 1H), 2.55 - 2.42 (m, 1H).

Compound 1-G:

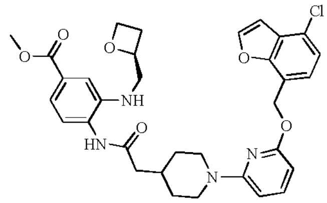

1-G

560 mg of compound 1-E (1.0 eq.) was weighed and added to a 50mL round-bottom flask. 10 mL of acetonitrile was added, and then 314 mg of compound 1-F (0.95 eq.) was added. The reaction solution was stirred at 0°C for 10 minutes. Then, 460 mg of N-methylimidazole (4.0 eq.) and 550 mg TCFH (N,N,N',N'-tetramethylchloroformamidinium hexafluorophosphate, 1.4 eq.) were added. After addition, the reaction solution was kept at 0°C to react for 1 hour. Then, the ice bath was removed and the reaction was returned to room temperature and reacted for 1-2 hours. TLC showed that

two raw materials were reacted completely. A large amount of solids were precipitated and filtered, and the filter cake was washed with 1 mL of acetonitrile to obtain off-white solid compound 1-G (310 mg, yield 35.75%).

Compound 1-H:

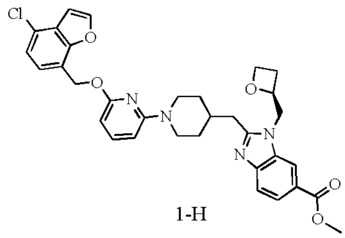

1-H

310 mg of compound 1-G (1.0 eq.) was weighed and added to a 50mL round-bottom flask. 9 mL of methanol, and then 94.71 mg pTSA (p-toluenesulfonic acid, 1.1 eq.) were added. The reaction solution was placed at 85°C for reaction under heating for 1.5 hours. TLC showed that the raw materials were reacted completely. The reaction solution was then cooled to room temperature, concentrated under reduced pressure, and then 10 mL of water and 15 mL of ethyl acetate were added and the thus obtained system was left to stand for liquid separation. The aqueous phase was extracted with 10 mL of ethyl acetate, and the organic phases were combined and washed with 30 mL×2 saturated salt solution twice, then dried with anhydrous sodium sulfate, and filtered. The filtrate was concentrated under reduced pressure to obtain a crude product, which was purified by column chromatography (dichloromethane/methanol = 98/2 as eluent) to obtain white foamy compound 1-H (240 mg, yield 79.85%). MS m/z (ESI):601.2213 $(M+H)^+$, $^1$HNMR (400 MHz, $CDCl_3$) δ =8.12 (s, 1H), 8.02 (d, J = 8.5 Hz, 1H), 7.80 (d, J = 8.5 Hz, 1H), 7.68 (d, J = 2.1 Hz, 1H), 7.41 (t, J = 7.9 Hz, 1H), 7.35 (d, J = 8.0 Hz, 1H), 7.22 (d, J = 8.0 Hz, 1H), 6.88 (d, J = 2.1 Hz, 1H), 6.20 (d, J = 8.0 Hz, 1H), 6.13 (d, J = 7.8 Hz, 1H), 5.62 (s, 2H), 5.24 - 5.15 (m, 1H), 4.63 (dd, J = 13.9, 7.8 Hz, 1H), 4.47 - 4.34 (m, 3H), 4.27 (d, J = 12.9 Hz, 2H), 3.97 (s, 3H), 2.97 (d, J = 7.1 Hz, 2H), 2.91 - 2.81 (m, 3H), 2.80 - 2.71 (m, 1H), 2.47 - 2.41 (m, 1H), 1.85 (d, J = 12.8 Hz, 2H), 1.40 - 1.34 (m, 2H).

Compound 1:

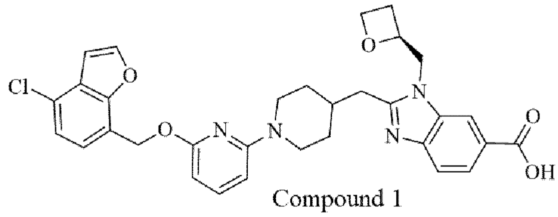

Compound 1

240 mg of compound 1-H (1.0 eq.) was weighed and added to a 50mL round-bottom flask. 5 mL of acetonitrile, 2.5 mL of methanol and 2.5 mL of water were added, and then 25.18 mg of $LiOH.H_2O$ (1.5 eq.) was added. The reaction mixture was stirred evenly and heated at 45°C for reaction for 4-5 hours. TLC showed that the raw materials were completely reacted. Subsequently, the reaction solution was cooled to room temperature, concentrated under reduced pressure, and then 5 mL of water was added. The pH of the reaction solution was adjusted to be 4-5 with citric acid. Then, 10 mL of ethyl acetate was added and the thus obtained system was left to stand for liquid separation The aqueous phase was extracted with 10 mL×2 of ethyl acetate twice, and the organic phases were combined and washed with saturated salt solution, dried with anhydrous sodium sulfate, and filtered. The filtrate was concentrated under reduced pressure and purified by column chromatography to obtain compound 1 (150 mg, yield 63.87%). MS m/z (ESI): 587.2058$(M+H)^+$, $^1$HNMR (400 MHz, MeOD) δ =8.29 (s, 1H), 7.99 (d, J = 8.5 Hz, 1H), 7.84 (d, J = 2.1 Hz, 1H), 7.68 (d, J = 8.5 Hz, 1H), 7.40 (t, J = 7.9 Hz, 1H), 7.32 (d, J = 8.0 Hz, 1H), 7.21 (d, J = 8.0 Hz, 1H), 6.89 (d, J = 2.1 Hz, 1H), 6.25 (d, J = 8.1 Hz, 1H), 6.07 (d, J = 7.8 Hz, 1H), 5.58 (s, 2H), 5.17 (m, 1H), 4.69-4.56 (m, 2H), 4.50 (dd, J = 15.6, 2.3 Hz, 1H), 4.37-4.32 (m, 1H), 4.23 (d, J = 13.0 Hz, 2H), 2.99-2.92 (m, 2H), 2.81-2.70 (m, 3H), 2.52-2.40 (m, 1H), 2.33-2.18 (m, 1H), 1.71 (d, J = 11.5 Hz, 2H), 1.31-1.25 (m, 2H).

Example 2:

Synthesis of compound 2:

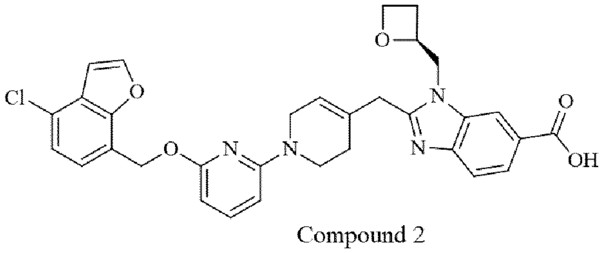

Compound 2

Synthesis route:

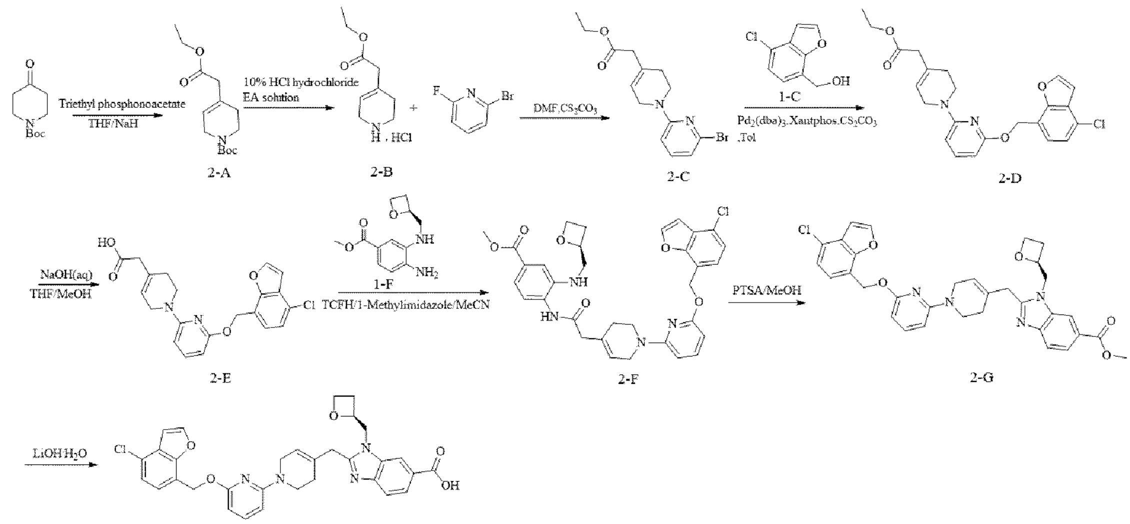


Compound 2

Compound 2-A:

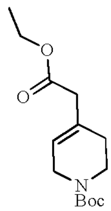

2-A

75 mL of anhydrous THF was added to a 250mL round-bottom flask, and 2.9 g of NaH (60%, 2.3 eq.) was added in batches at room temperature. The reaction mixture was then stirred for 30 minutes, and 9.13 mL of triethyl phosphonoacetate (1.4 eq.) was dropwise added. After the dropwise addition, the reaction solution was stirred for 1 hour. Subsequently, 6.5 g of 1-Boc-4-piperidone (1.0 eq.) was added in batches. Subsequently, the reaction solution was stirred at room temperature for 2-3 hours, and TLC showed that the raw materials were completely reacted. 150 mL of water was slowly dropwise added into the reaction solution for quenching, and the solution was extracted with 100 mL×3 of ethyl acetate thrice. The organic phases were combined and washed with 100 mL×2 of saturated salt solution twice. The organic phases were dried with anhydrous sodium sulfate and filtered. The filtrate was concentrated under reduced pressure to obtain an oily substance, which was purified by column chromatography (PE/EA = 95/5) to obtain compound 2-A (1.67 g, yield 18.9%). $^1$HNMR(400MHz, $CDCl_3$)δ =5.54 (tt, J = 3.2, 1.5 Hz, 1H), 4.16 (q, J = 7.1 Hz, 2H), 3.91 (tq, J = 2.8, 1.4 Hz, 2H), 3.52 (t, J = 5.7 Hz, 2H), 3.03 (d, J = 1.7 Hz, 2H), 2.16 (tt, J = 5.7, 2.4 Hz, 2H), 1.48 (s, 9H), 1.28 (t, J = 7.2 Hz, 3H).

Compound 2-B:

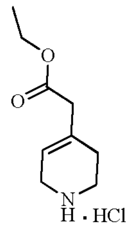

2-B

1.67 g of compound 2-A (1.0 eq.) was weighed and added to a 50mL round-bottom flask. 4 mL of ethyl acetate was added and the thus obtained system was stirred to dissolve. Then, 20 mL of 10% hydrogen chloride ethyl acetate solution was added, and the reaction solution was stirred at room temperature for 4 hours. TLC showed that the raw materials were completely reacted. The reaction solution was concentrated under reduced pressure to obtain compound 2-B hydrochloride. The compound 2-B hydrochloride was directly used in the next step without purification.

Compound 2-C:

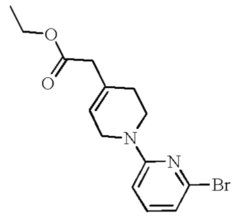

2-C

The compound 2-B hydrochloride (1.0 eq.) obtained in the previous step was weighed and added to a 250mL round-bottom flask. 25 mL of DMF and 6.17 g of cesium carbonate (3.0 eq.) were added. The reaction solution was stirred at room temperature for 30 minutes, and then 1.11 g of 2-bromo-6-fluoropyridine (1.0 eq.) was added. Subsequently, the reaction solution was heated at 70°C for 1 hour. TLC showed that the raw materials were completely reacted. Subsequently, the reaction solution was cooled to room temperature, 50 mL of water was added, and the thus obtained system was extracted with 20 mL×2 of ethyl acetate twice. The organic phases were combined and washed with 25 mL×2 of saturated sodium chloride solution twice. The organic phases were dried with anhydrous sodium sulfate and filtered. The filtrate was concentrated under reduced pressure to obtain oily compound 2-C (1.57 g, yield 77.72%). The compound 2-C was directly used to for the next reaction without purification.

Compound 2-D:

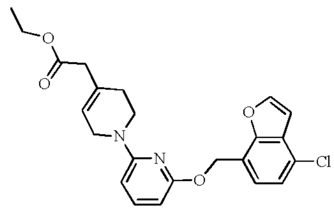

2-D

960.0 mg of compound 2-C (1.0 eq.) was weighed and added to a 100mL round-bottom flask. 512.0 mg of compound 1-C (0.95 eq.), 1.92 g of $Cs_2CO_3$ (2.0 eq.), 340.8 mg of Xantphos (0.2 eq.), 269 mg of $Pd_2(dba)_3$ (0.1 eq.) and 32 mL of toluene were added. The reaction mixture was then stirred evenly, replaced with nitrogen thrice, and subsequently heated at 110°C for 16 hours under the protection of nitrogen. TLC showed that two raw materials were completely reacted. The heating was stopped and the reaction solution was cooled to room temperature, filtered with diatomite, and the filtrate was concentrated under reduced pressure to obtain a yellow oily substance, which was purified by column chromatography (petroleum ether/ethyl acetate = 95/5 as eluent) to obtain oily compound 2-D (402 mg, yield 33.5%).

Compound 2-E:

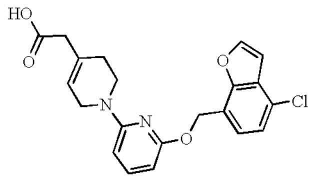

2-E

402 mg of compound 2-D (1.0 eq.) was weighed and added to a 100mL round-bottom flask. 10 mL of mixed solution of MeOH/THF = 1/1 was added. 1.6 mL of 2 mol/L NaOH solution was then added. Subsequently, the reaction solution was heated at 40°C for 2-3 hours. TLC showed that the raw materials were completely reacted. Subsequently, the reaction solution was cooled to room temperature and concentrated under reduced pressure. 15 mL of water was added to the concentrated solution, and the pH of the reaction solution was adjusted to be about 4 with 2 mol/L hydrochloric acid solution. The aqueous phase was extracted with 15 mL×2 of ethyl acetate twice, and the organic phases were combined and washed with 15 mL×2 of saturated salt solution twice, dried with anhydrous sodium sulfate and filtered. The filtrate was concentrated under reduced pressure to obtain light green oily compound 2-E crude product with a total of 402 mg, which was directly used in the next reaction without purification.

Compound 2-F:

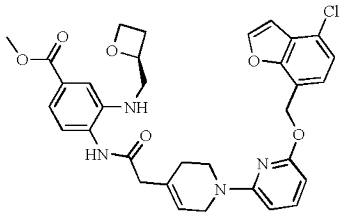

2-F

402 mg of compound 2-E (1.0 eq.) was weighed and added to a 50mL round-bottom flask. 8 mL of acetonitrile and 226.23 mg of compound 1-F (0.95 eq.) were added. Subsequently, the reaction solution was stirred at 0°C for 10 minutes. 336.0 mg of N-methylimidazole (4.0 eq.) and 396.0 mg of TCFH (1.4 eq.) were added. After the addition, the reaction solution was kept at 0°C for reaction for 3-4 hours. TLC showed that two raw materials were completely reacted. 10 mL of water was added to the reaction solution and the thus obtained system was extracted with 15 mL of ethyl acetate. The organic phases were washed with 15 mL×2 of saturated salt solution twice, dried with anhydrous sodium sulfate and filtered. The filtrate was concentrated under reduced pressure to obtain light green oily compound, which was purified by silica gel column chromatography (DCM: MeOH = 98: 2) to obtain compound 2-F (365 mg, yield 61.66%).

Compound 2-G:

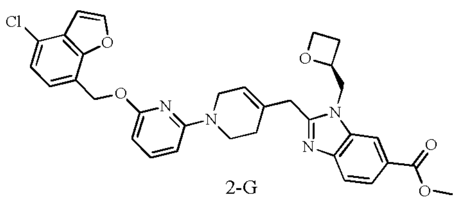

2-G

365 mg of compound 2-F (1.0 eq.) was weighed and added to a 50mL round-bottom flask. 11 mL of methanol and 112.10 mg of pTSA (1.1 eq.) were added. The reaction solution was heated at 80°C for 0.5 hours. TLC showed that the raw materials were completely reacted. Subsequently, the reaction solution was cooled to room temperature. 5 mL of water and saturated $NaHCO_3$ solution were added to the reaction solution to adjust the pH of the reaction solution to be about 8. Then, 15 mL of ethyl acetate was added and the thus obtained system was left to stand for liquid separation. The aqueous phase was extracted with 15mL of ethyl acetate, and the organic phases were combined and washed with 10 mL×2 of saturated salt solution twice, dried with anhydrous sodium sulfate and filtered. The filtrate was concentrated under reduced pressure and purified by column chromatography (DCM: MeOH = 98: 2 as eluent) to obtain yellow foamy compound 2-G (270 mg, yield 76.3%).

Compound 2:

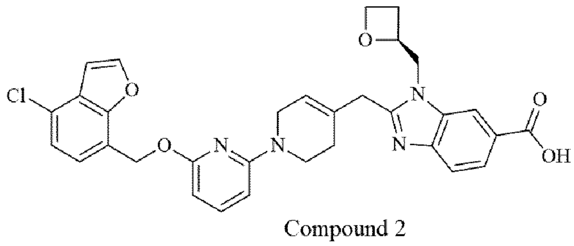

Compound 2

270 mg of compound 2-G (1.0 eq.) was weighed and added to a 50mL round-bottom flask. A mixed solution of THF/MeOH/ water = 6 mL/6 mL/3 mL, and 28.37 mg of LiOH·$H_2O$ (1.5 eq.) were added. The reaction solution was heated at room temperature overnight. TLC showed that the raw materials were completely reacted. Subsequently, the reaction solution was cooled to room temperature and concentrated under reduced pressure. 5 mL of water was added to the concentrated solution, and the pH of the reaction solution was adjusted to be 4-5 with citric acid. The aqueous phase was extracted with 10 mL×2 of ethyl acetate twice, and the organic phases were combined and washed with 10 mL×2 of saturated salt solution twice, dried with anhydrous sodium sulfate and filtered. The filtrate was concentrated under reduced pressure to obtain an oily substance. 1 mL of methanol was added to the oily substance for pulping, and then filtered to obtain compound 2 (180 mg, yield 68.2%). MS m/z (ESI): 585.1902$(M+H)^+$, $^1$HNMR (400 MHz, $CDCl_3$) δ= 8.22 (d, J = 1.5 Hz, 1H), 8.11 (dd, J = 8.5, 1.5 Hz, 1H), 7.88 (d, J = 8.4 Hz, 1H), 7.67 (d, J = 2.2 Hz, 1H), 7.40 (t, J = 7.9 Hz, 1H), 7.34 (d, J = 8.0 Hz, 1H), 7.21 (d, J = 8.0 Hz, 1H), 6.86 (d, J = 2.2 Hz, 1H), 6.19-6.08 (m, 2H), 5.66-5.59 (m, 2H), 5.56 (s, 1H), 5.18 (qd, J = 7.0, 3.0 Hz, 1H), 4.63 (td, J = 8.0, 6.0 Hz, 1H), 4.52-4.33 (m, 3H), 4.03-3.83 (m, 4H), 3.71 (m, 2H), 2.78-2.67 (m, 1H), 2.42 (m, 1H), 2.24 (s, 2H).

Example 3:

Synthesis of Compound 3:

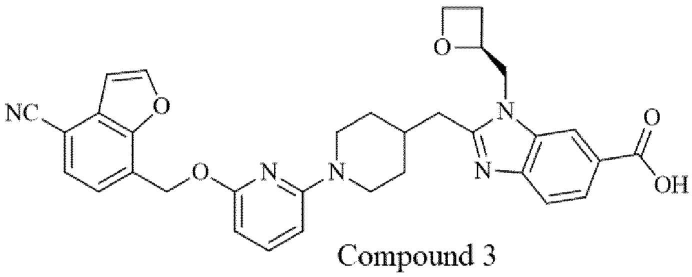

Compound 3

Synthesis route:

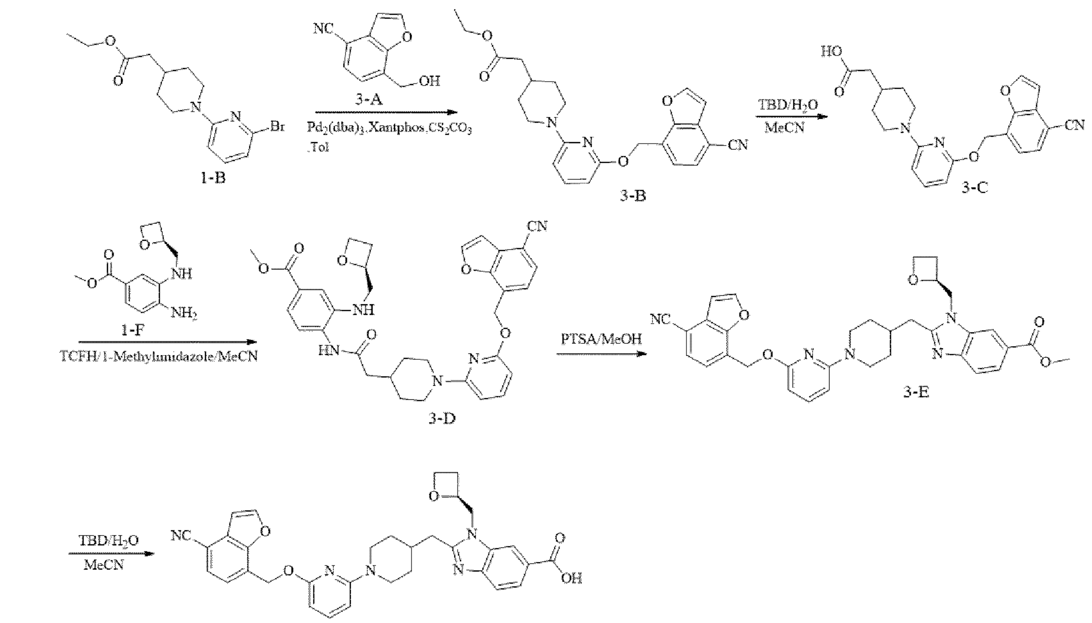


Compound 3

## Compound 3-A:

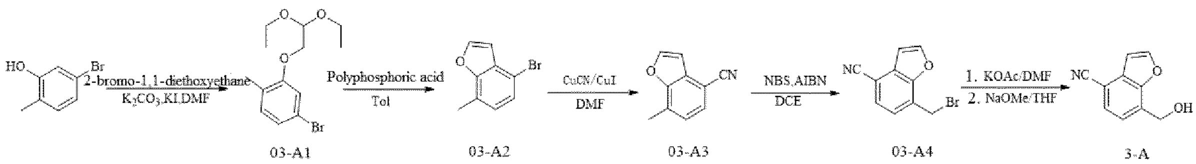


Compound 03-A1:

10.0 g of 5-bromo-2-methylphenol (1.0 eq.) was weighed and added to a 500mL round-bottom flask. 200 mL of DMF, 12.5 g of 2-bromo-1,1-diethoxyethane (1.2 eq.), 15.0 g of potassium carbonate (2.0 eq.) and 1.0 g of KI (0.11 eq.) were added. The reaction solution was stirred evenly and then was placed at 120°C for a reaction under heating and stirring for 16 hours. TLC showed that the raw materials were basically reacted completely. Subsequently, the reaction solution was cooled to room temperature and 200 mL of water was added for quenching, and then the thus obtained system was extracted with 200 mL×2 of ethyl acetate twice. The ethyl acetate organic phases were combined and washed with 200 mL×2 of water twice, then washed with 200 mL of saturated salt solution, dried with anhydrous sodium sulfate and filtered. The filtrate was concentrated under reduced pressure to obtain a crude product, which was purified by column chromatography (PE as eluent) to obtain oily compound 03-A1 (7.6 g, yield 46.9%).

Compound 03-A2:

7.6 g of compound 03-A1 (1.0 eq.) was weighed and added to a 500mL round-bottom flask. 150 mL of toluene and 20.96 g of polyphosphoric acid (2.5 eq.) were added. Subsequently, the reaction solution was placed at 90°C for reaction under heating and stirring for 10 hours. TLC showed that the raw materials were basically reacted completely. Subsequently, after cooled to room temperature, 200 mL of water was added to the reaction solution, the thus obtained system was stirred, and left to stand for stratification. The toluene phases were washed with 150 mL of saturated $NaHCO_3$, and washed with 150 mL of saturated salt solution, then dried with anhydrous sodium sulfate and filtered. The filtrate was concentrated under reduced pressure to obtain oily compound 03-A2 (5.4 g), which was directly used in the next step without purification.

Compound 03-A3:

3.76 g of compound 03-A2 (1.0 eq.), 4.79 g of CuCN (3.0 eq.) and 6.79 g of CuI (2.0 eq.) were weighed and added to a 150mL round-bottom flask. 56.4 mL of DMF was added. The reaction solution was stirred evenly and placed at 150°C

for reaction under heating and stirring for 9 hours. TLC showed that the raw materials were basically reacted completely. Subsequently, after cooled to room temperature, 150 mL of water and 150 mL of ethyl acetate were added, and a large amount of solids were generated in the reaction system. Subsequently, the reaction solution was filtered with diatomite. The filtrate was left to stand for stratification to collect the ethyl acetate phases. The organic phases were washed with 30 mL×2 of 25% ammonia water solution twice, then washed with 50 mL×2 of water twice, and washed with 50 mL×2 of saturated salt solution twice, dried with anhydrous sodium sulfate and filtered. The filtrate was concentrated under reduced pressure to obtain yellow solid crude product, which was purified by column chromatography (PE as eluent) to obtain white solid compound 03-A3 (2.1 g, yield 75%). $^1$HNMR (400 MHz, $CDCl_3$) δ =7.80 (d, J = 2.3 Hz, 1H), 7.51 (d, J = 7.7 Hz, 1H), 7.18 (d, J = 7.7 Hz, 1H), 7.00 (d, J = 2.2 Hz, 1H), 2.61 (s, 3H).

Compound 03-A4:

2.1 g of compound 03-A3 (1.0 eq.), 2.85 g of NBS (1.2 eq.) and 438.7 mg of 2,2-azobisisobutyronitrile (0.2 eq.) were weighed and added to a 50mL round-bottom flask. 25 mL of dichloroethane was added. The reaction solution was stirred evenly and placed at 72°C for reaction under heating and stirring for 12 hours. TLC showed that the raw materials were basically reacted completely. Subsequently, the reaction solution was cooled to room temperature, and the solvent was removed under reduced pressure. The residue was purified by silica gel column chromatography (PE as eluent) to obtain off-white solid compound 03-A4 (2.4 g, yield 76.20%).

Compound 3-A:

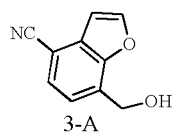

3-A

2.4 g of compound 03-A4 (1.0 eq.) was weighed and added to a 150mL round-bottom flask. 24 mL of DMF, then 9.84 g of potassium acetate (10.0 eq.) were added. The reaction solution was stirred at room temperature for 3.0 hours. TLC showed that the raw materials were completely reacted. Subsequently, 72 mL of water was added for quenching, and the thus obtained system was extracted with 50 mL×2 of ethyl acetate twice. The ethyl acetate organic phases were combined and washed with 50 mL×2 of water twice, and then washed with 50 mL×2 of saturated salt solution. The organic phases were dried with anhydrous sodium sulfate and filtered. The filtrate was concentrated under reduced pressure to obtain an oily substance. 12 mL of THF was added to the oily substance, the thus obtained system was stirred evenly, and then 4.1 mL of 5mol/L sodium methoxide solution was added, and the reaction solution was stirred at room temperature for reaction for 1 hour. TLC showed that the raw materials disappeared. The reaction was stopped. 36 mL of water and 50 mL of ethyl acetate were added to the reaction solution, and the thus obtained system was left to stand for liquid separation. The aqueous phase was extracted with 50 mL×2 of ethyl acetate twice, and the organic phases were combined and washed with 75 mL×2 of saturated salt solution twice, dried with anhydrous sodium sulfate and filtered. The filtrate was concentrated under reduced pressure to obtain a crude yellow oily substance, which was purified by column chromatography (petroleum ether/ ethyl acetate = 85/15 as eluent) to obtain light yellow solid compound 3-A (1.2 g, 68.18%). $^1$HNMR (400 MHz, $CDCl_3$) δ 7.81 (d, J = 2.2 Hz, 1H), 7.62 (d, J = 7.7 Hz, 1H), 7.47 (d, J = 7.7 Hz, 1H), 7.03 (d, J = 2.2 Hz, 1H), 5.10 (s, 2H).

Compound 3-B:

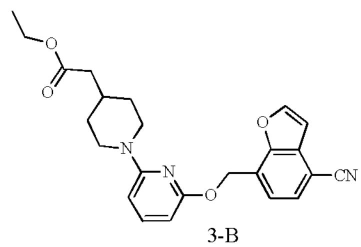

3-B

850 mg of compound 1-B (1.0 eq.) was weighed and added to a 100mL round-bottom flask. 450 mg of compound 3-A (1.0 eq.), 1.69 g of $Cs_2CO_3$ (2.0 eq.), 300.0 mg of Xantphos (0.2 eq.), 237.7 mg of $Pd_2(dba)_3$ (0.1 eq.) and 32 mL of toluene were added. The reaction mixture was then stirred evenly, replaced with nitrogen thrice, heated at 110°C for reaction for 16 hours under the protection of nitrogen. TLC showed that two raw materials were completely reacted.

Subsequently, the heating was stopped and the reaction solution was cooled to room temperature, and then filtered with diatomite. The filtrate was concentrated under reduced pressure to obtain a crude product, which was purified by column chromatography (petroleum ether/ethyl acetate = 95/5 as eluent) to obtain compound 3-B (720 mg, yield 66.06%).

Compound 3-C:

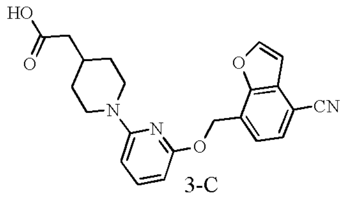

3-C

720 mg of compound 3-B (1.0 eq.) was weighed and added to a 50mL round-bottom flask. 15 mL of acetonitrile, and then TBD solution (1,5,7-triazabicyclo[4.4.0]dec-5-ene, 475.9 mg of TBD (2.0 eq.) was dissolved in 3.0 mL of water) were added. The reaction solution was stirred at room temperature for 24 hours. TLC showed that the raw materials were basically reacted completely. The reaction was stopped, and the pH of the reaction solution was adjusted to be about 5.0 with citric acid. The reaction solution was extracted with 15 mL×2 of ethyl acetate twice. The organic phases were combined and washed with 30 mL×2 of saturated salt solution twice, dried with anhydrous sodium sulfate and filtered. The filtrate was concentrated under reduced pressure to obtain compound 3-C (640 mg, yield 95.06%).

Compound 3-D:

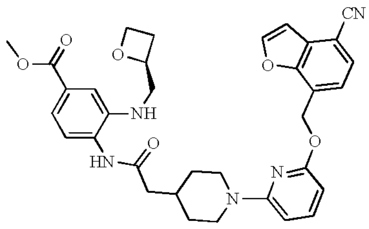

3-D

400 mg of compound 3-C (1.0 eq.) was weighed and added to a 50mL round-bottom flask. 8 mL of acetonitrile and 241.50 mg of compound 1-F (1.0 eq.) were added. The reaction solution was placed at 0°C for reaction under stirring. 335.6 mg of N-methylimidazole (4.0 eq.) and 401.5 mg of TCFH (1.4 eq.) were then added. Subsequently, the reaction solution was kept at 0°C for 1 hour. The ice bath was removed and the reaction was returned to room temperature and continuously reacted for 2 hours. TLC showed that the raw material compound 1-F was completely reacted. The reaction solution was concentrated under reduced pressure and evaporated to dryness, 4 mL of acetonitrile was added for pulping, and then the thus obtained system was filtered. The filter cake was washed with 0.5 mL of acetonitrile to obtain solid compound 3-D (340 mg, yield 54.6%).

Compound 3-E:

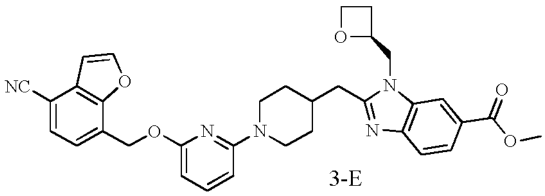

3-E

310 mg of compound 3-D (1.0 eq.) was weighed and added to a 50mL round-bottom flask. 9 mL of methanol and 96.23 mg of pTSA (1.1 eq.) were added. The reaction solution was stirred evenly and placed at 80°C for reaction under heating for 1 hour. TLC showed that the raw materials were completely reacted. Subsequently, the reaction solution was cooled to room temperature and concentrated under reduced pressure to evaporate methanol. 10 mL of water and 15 mL of ethyl acetate were added to the concentrated solution, and the thus obtained system was left to stand for liquid separation. The aqueous phase was then extracted with 10 mL of ethyl acetate. The organic phases were combined and washed with 15 mL×2 of saturated salt solution twice, dried with anhydrous sodium sulfate and filtered. The filtrate was

concentrated under reduced pressure to obtain a crude product, which was purified by column chromatography (DCM: MeOH = 98: 2 as eluent) to obtain white foamy compound 3-E (220 mg, yield 73.2%).

Compound 3:

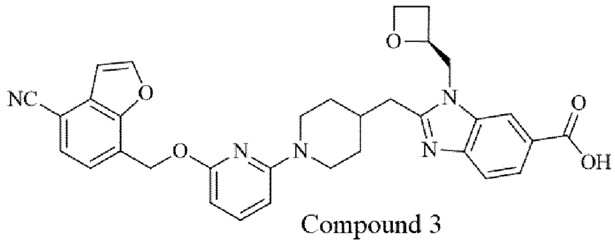

Compound 3

220 mg of compound 3-E (1.0 eq.) was weighed and added to a 50mL round-bottom flask. 5 mL of acetonitrile, and then TBD solution (103.6 mg (2.0 eq.) dissolved in 1.0 mL of water) were added. The reaction solution was stirred at room temperature for 24 hours. TLC showed that the raw materials were basically reacted completely. The reaction was stopped, and the pH of the reaction solution was adjusted to be about 5.0 with citric acid. The reaction solution was extracted with 10 mL×2 of ethyl acetate twice. The organic phases were combined and washed with 20 mL×2 of saturated salt solution twice, dried with anhydrous sodium sulfate and filtered. The filtrate was concentrated under reduced pressure and purified by column chromatography to obtain compound 3 (110 mg, yield 51.14%). MS m/z (ESI): 578.2408$(M+H)^+$, $^1$HNMR (400 MHz, $CDCl_3$) $\delta$= 8.22 (s, 1H), 8.10 (dd, J = 8.5, 1.2 Hz, 1H), 7.88 (d, J = 8.5 Hz, 1H), 7.81 (d, J = 2.2 Hz, 1H), 7.57 (d, J = 7.8 Hz, 1H), 7.48 (d, J = 7.8 Hz, 1H), 7.42 (t, J = 8.0 Hz, 1H), 7.00 (d, J = 2.2 Hz, 1H), 6.19 (d, J = 8.1 Hz, 1H), 6.15 (d, J = 7.8 Hz, 1H), 5.70 (s, 2H), 5.26 - 5.17 (m, 1H), 4.64 (d, J = 6.0 Hz, 1H), 4.55 - 4.33 (m, 3H), 4.25 - 4.17 (m, 2H), 3.00 (d, J = 7.1 Hz, 2H), 2.86 - 2.71 (m, 3H), 2.51 - 2.31 (m, 2H), 1.83 (d, J = 12.8 Hz, 2H), 1.37 - 1.30 (m, 2H).

Example 4:

Synthesis of Compound 4:

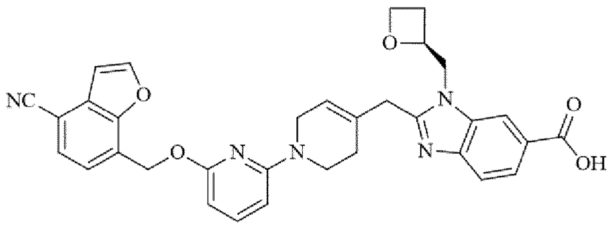

Compound 4

Synthesis route:

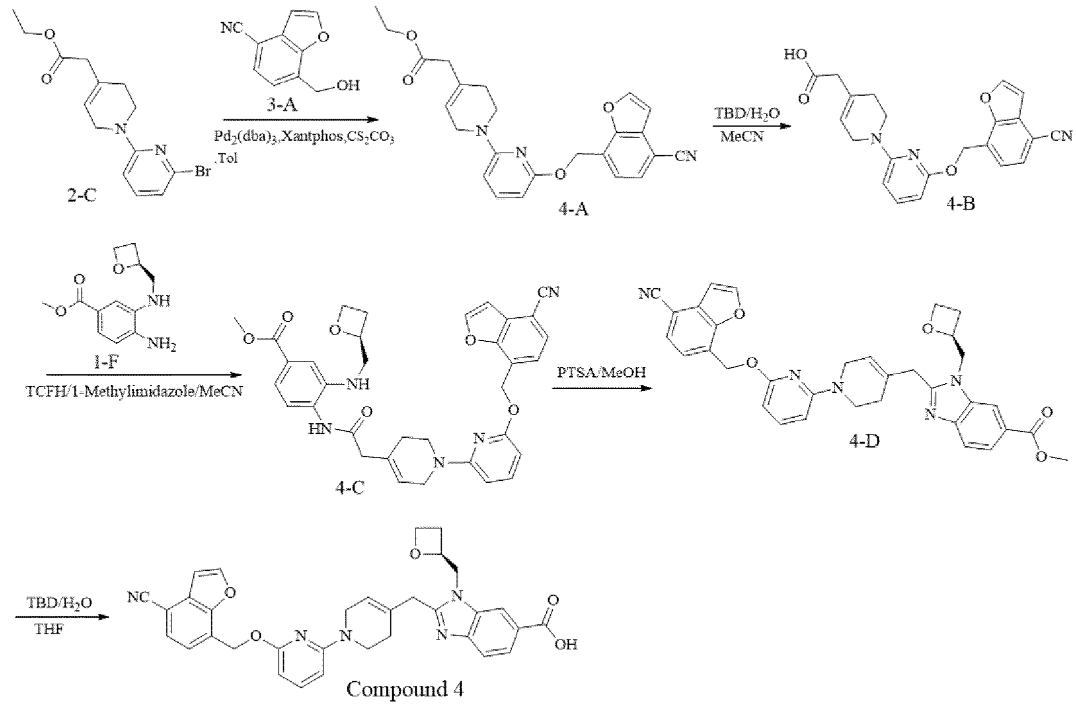

Compound 4

Referring to the preparation method of compound 2 in Example 2, compound 1-C therein was replaced with compound 3-A to obtain compound 4. MS m/z (ESI):576.2243 (M+H)$^+$.

Example 5:

Synthesis of Compound 7:

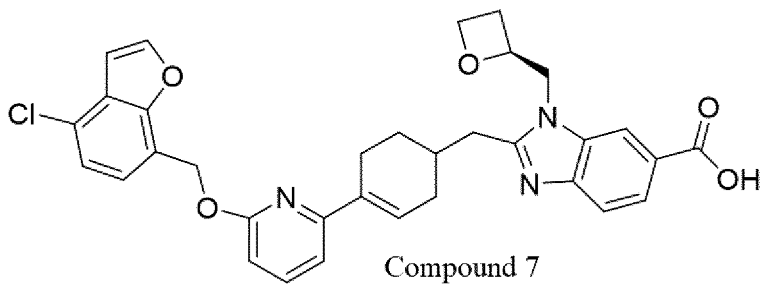
Compound 7

Synthesis route:

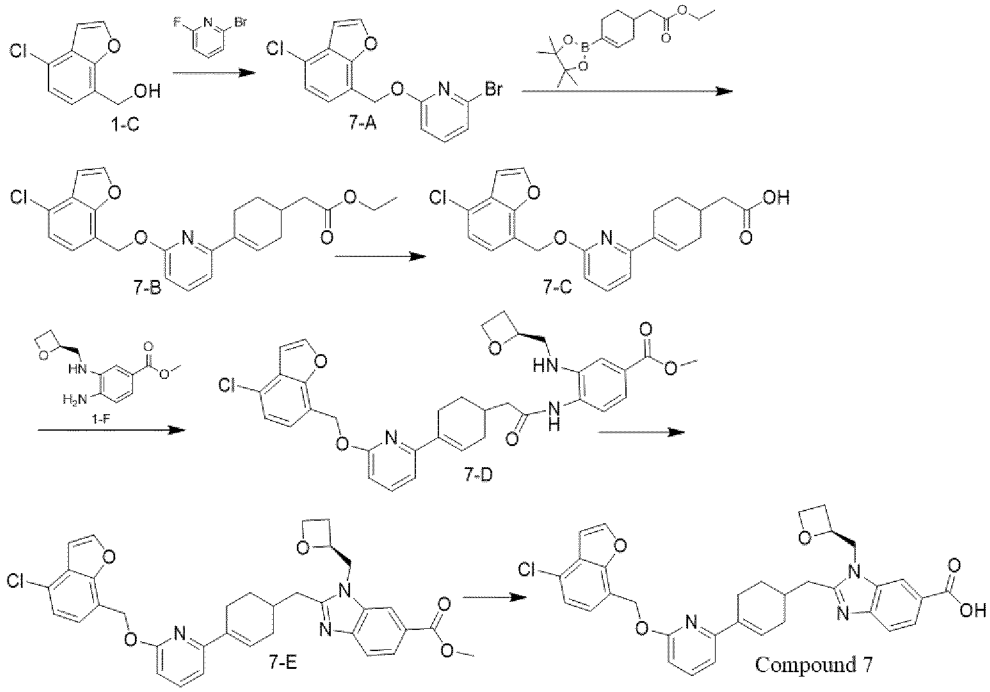
1-C 7-A 7-B 7-C 1-F 7-D 7-E Compound 7

Compound 7-A:

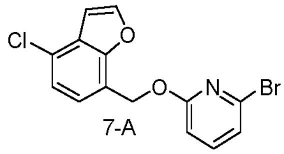
7-A

1 g of compound 1-C (1.0 eq.), 1.45 g of 2-bromo-6-fluoropyridine (1.5 eq.) and 3.75 g of cesium carbonate (2.0 eq.) were weighed and added to a 100mL round-bottom flask. 30 mL of DMF was then added. Subsequently, the reaction solution was heated to 90°C and reacted for 4 hours. Then, the heating was stopped and the reaction solution was cooled to room temperature. 120 mL of water was added, and the thus obtained system was stirred for 0.5 hours at room temperature, and then filtered. The filter cake was dried at 50°C for 12 hours to obtain 1.8 g of compound 7-A with a yield of 97%.

Compound 7-B:

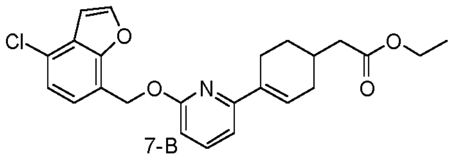
7-B

0.5 g of compound 7-A (1.0 eq.), 0.478 g of compound 1-pinacol borate-4-ethyl acetate-1-cyclohexene (1.1 eq.), 0.108 g of Pd(dppf)$Cl_2$ (0.1 eq.), and 0.249 g of sodium hydrogen carbonate (2 eq.) were weighed and added to a 50mL round-bottom flask. 7.5 mL of toluene, 2.5 mL of ethanol and 2.5 mL of water were then added. Subsequently, the reaction solution was degassed with nitrogen twice, and heated to 110°C for reaction under nitrogen atmosphere. After 2 hours of reaction, the heating was stopped, and then the reaction solution was cooled to room temperature, filtered by a funnel with diatomite, and the filter cake was washed with 15 mL×2 of ethyl acetate. Subsequently, the filtrate was concentrated to dryness and purified by column chromatography to obtain 0.6 g of compound 7-B with a yield of 95.4%.

Compound 7-C:

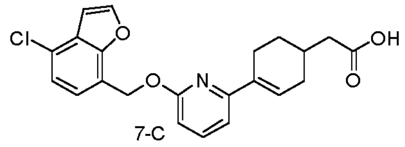

7-C

0.6 g of compound 7-B (1.0 eq.) was weighed and added to a 50mL round-bottom flask. 6 mL of methanol and 9 mL of tetrahydrofuran were added. Subsequently, 1.4 mL of 2 mol/L NaOH solution (2.0 eq.) was dropwise added to the reaction solution at room temperature. After the dropwise addition, the reaction solution was continuously reacted at room temperature for 1.5 hours. After the reaction, the reaction solution was concentrated. 15 mL of water and 30 mL of ethyl acetate were added after the concentration, and the pH of the system was adjusted to be 4-5 with 1 mol/L HCl solution, then the organic phases were separated, and the aqueous phase was continuously washed with 35 mL×2 of ethyl acetate twice. The organic phases were combined and washed with saturated salt solution, dried with anhydrous $Na_2SO_4$ for 5 minutes and then filtered. The filtrate was concentrated to dryness to obtain 0.468 g of compound 7-C with a yield of 83.5%.

Compound 7-D:

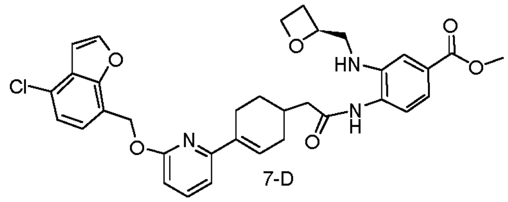

7-D

0.465 g of compound 7-C (1.0 eq.) and 0.304 g of compound 1-F (1.1 eq.) were weighed and added to a 50mL round-bottom flask. 10.0 mL of acetonitrile was added. After the reaction solution was stirred for 15 minutes in an ice bath, 0.304 g of N, N, N', N'-tetramethylchloroformamidinium hexafluorophosphate (1.1 eq.) and 0.202 g of N-methylimidazole (2.1 eq.) were weighed and added. The reaction solution was continuously stirred and reacted for 0.5 hours under the ice bath. Subsequently, the reaction solution was transferred to room temperature and continuously reacted for 3 hours. After the reaction, the reaction system was added with 30 mL of $H_2O$, stirred for 0.5 hours, and then filtered. The filter cake was washed with 20 mL×3 of $H_2O$ and dried at 50°C to obtain 0.607 g of compound 7-D with a yield of 84%.

Compound 7-E:

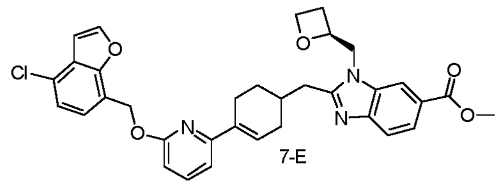

7-E

0.605 g of compound 7-D (1.0 eq.) was weighed and added to a 50mL round-bottom flask. 10.0 mL of 1,4-dioxane was added. 2.95 g of acetic acid (50.0 eq.) was then added dropwise. After the dropwise addition, the reaction solution was heated at 80°C for 15 hours. The reaction solution was cooled to room temperature. 10 mL of water and 25 mL of ethyl acetate were added, and the pH of the system was adjusted to be 7-8 with 5% $NaHCO_3$ solution. Subsequently, the reaction solution was left to stand. The organic phases were separated, and the aqueous phase was continuously washed with 25 mL×2 of ethyl acetate twice. The organic phases were combined and washed with saturated salt solution, dried with anhydrous $Na_2SO_4$ for 5 minutes, filtered, concentrated to dryness, and purified by column chromatography to obtain 0.345 g of compound 7-E with a yield of 58.7%. MS m/z (ESI): 598.2109 $(M+H)^+$.

Compound 7:

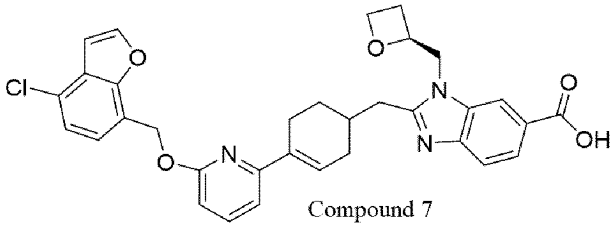

Compound 7

0.34 g of compound 7-E (1.0 eq.) was weighed and added to a 25mL round-bottom flask. 1.7 mL of methanol and 5.0 mL of tetrahydrofuran were added. 0.56 mL of 2N sodium hydroxide solution was then added. Subsequently, the reaction solution was reacted at room temperature. After 20 hours, the reaction solution was concentrated. After the concentration, 6 mL of water was added, and the pH of the reaction solution was adjusted to be 4-5 with 1 mol/L HCl solution. Subsequently, the reaction solution was filtered. The filter cake was pulped with 5 mL of ethyl acetate and 1 mL of methanol, and then filtered. The filter cake was washed with 3 mL×3 of ethyl acetate, and then dried to obtain 0.177 g of compound 7 with a yield of 53.3%. MS m/z (ESI): 584.1953 $(M+H)^+$, $^1H$ NMR (400 MHz, DMSO-$d_6$) δ= 8.21 (s, 1H), 8.17 (d, *J* = 2.0Hz, 1H), 7.81 (d, *J* = 8.4 Hz, 1H), 7.68-7.60 (m, 2H), 7.42 (d, *J* = 8.4 Hz, 1H), 7.34 (d, *J* = 8.0 Hz, 1H), 7.07-7.04 (m, 2H), 6.74-6.70 (m, 2H), 5.65 (s, 2H), 5.03 (d, *J* = 6.4 Hz, 1H), 4.63 (dd, *J* = 15.2, 6.8 Hz, 1H), 4.52-4.43 (m, 2H), 4.32-4.28 (m, 1H), 3.04-2.92 (m, 2H), 2.72-2.64 (m, 2H), 2.41-2.33 (m, 4H), 2.07-1.96 (m, 2H), 1.50-1.47 (m, 1H).

Example 6:

Synthesis of Compound 8:

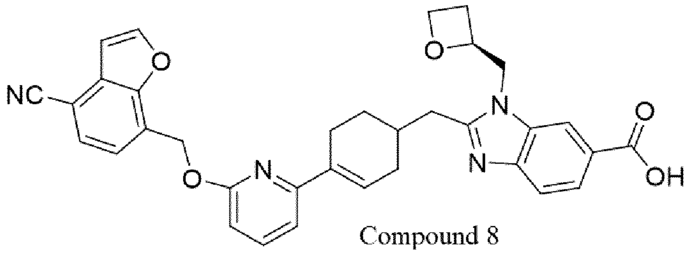

Compound 8

Synthesis route:

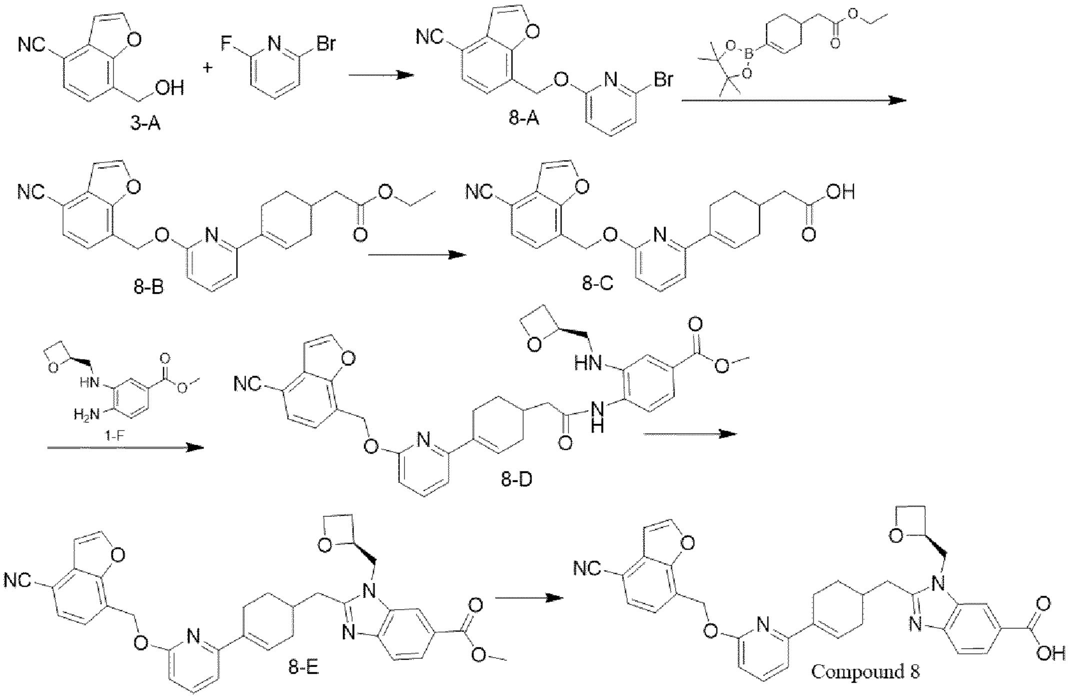


Compound 8-A:

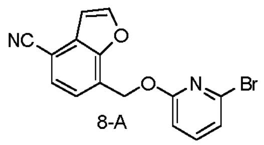

8-A

1 g of compound 3-A (1.0 eq.), 1.53 g of 2-bromo-6-fluoropyridine (1.5 eq.) and 3.76 g of cesium carbonate (2.0 eq.) were weighed and added to a 100mL round-bottom flask. 30 mL of DMF was added. Subsequently, the reaction solution was heated to 90°C for reaction, and the heating was stopped after 4 hours of reaction. The reaction solution was cooled to room temperature, and 120 mL of water was added, stirred at room temperature for 0.5 hours and filtered. The filter cake was washed with water and dried at 50°C for 12 hours to obtain 1.7g of compound 8-A with a yield of 89.5%.

Compound 8-B:

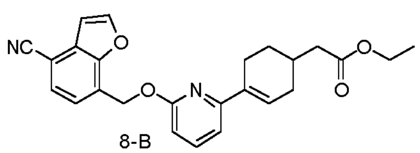

8-B

0.5 g of compound 8-A (1.0 eq.), 0.492 g of 1-pinacol borate-4-ethyl acetate-1-cyclohexene (1.1 eq.), 0.111 g of Pd(dppf)$Cl_2$ (0.1 eq.) and 0.255 g of sodium hydrogen carbonate (2 eq.) were weighed and added to a 50mL round-bottom flask. 7.5 mL of toluene, 2.5 mL of ethanol and 2.5 mL of water were added. Subsequently, the reaction solution was degassed with nitrogen twice, and heated to 110°C for reaction under nitrogen atmosphere. After reaction for 2 hours, the heating was stopped, and the reaction solution was cooled to room temperature, filtered by a funnel with diatomite, and the filter cake was washed with 15 mL×2 of ethyl acetate. Subsequently, the filtrate was concentrated to dryness and purified by column chromatography to obtain 0.506 g of compound 8-B with a yield of 80%.

Compound 8-C:

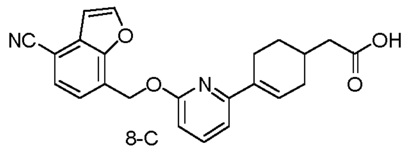

8-C

0.5 g of compound 8-B (1.0 eq.) was weighed and added to a 50mL round-bottom flask. 15 mL of acetonitrile, then TBD solution (1,5,7-triazabicyclo[4.4.0]dec-5-ene, TBD (2.0 eq.) was dissolved in 4.0 mL of water) were added. The reaction solution was stirred at room temperature for 24 hours. After the reaction was completed, the reaction solution was concentrated , 15 mL of water and 35 mL of ethyl acetate were added, and the pH of the reaction system was adjusted to be 4-5 with citric acid. Subsequently, the reaction solution was left to stand, the organic phases were separated, and the aqueous phase was continuously washed with 35 mL×2 of ethyl acetate twice. The organic phases were combined and washed with saturated salt solution, dried with anhydrous $Na_2SO_4$ for 5 minutes, and then filtered. The filtrate was concentrated to dryness to obtain 0.364 g of compound 8-C with a yield of 78%.

Compound 8-D:

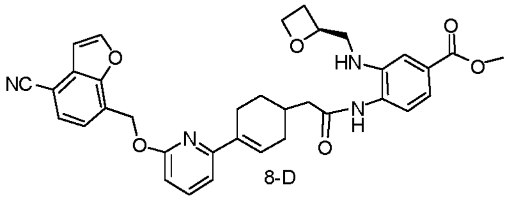

8-D

0.3 g of compound 8-C (1.0 eq.), 0.201 g of compound 1-F (1.1 eq.) and 0.298 g of TBTU (1.2 eq.) were weighed and added to a 50mL round-bottom flask. 15.0 mL of DMF was added. The reaction solution was stirred for 10 minutes in an ice bath, and then 0.199 g of N,N-diisopropylethylamine (2.0 eq.) was added dropwise. After the dropwise addition, the reaction solution was continuously stirred for 3 hours in the ice bath. After the reaction, 30 mL of $H_2O$ and 30 mL of ethyl acetate were added to the reaction system, the thus obtained system was stirred for 5 minutes, and left to stand. The organic phases were separated, and the aqueous phase was continuously washed with 30 mL×2 of ethyl acetate twice. The organic phases were combined and washed with saturated salt solution, dried with anhydrous $Na_2SO_4$ for 5 minutes and filtered. The filtrate was concentrated to dryness and purified by column chromatography to obtain 0.265 g of compound 8-D with a yield of 56%. MS m/z (ESI):607.2553 $(M+H)^+$.

Compound 8-E:

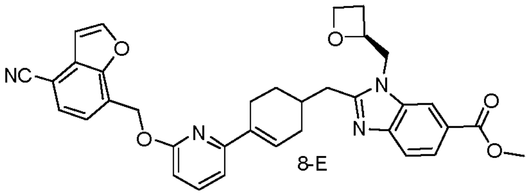

8-E

0.25 g of compound 8-D was weighed and added to a 50mL round-bottom flask. 10 mL of methanol and 78.0 mg of pTSA (1.1 eq.) were added. The reaction solution was stirred evenly and was placed at 80°C for reaction under heating for 2 hours. Subsequently, the reaction solution was cooled to room temperature, concentrated and purified by column chromatography to obtain 0.143 g of compound 8-E with a yield of 59%.

Compound 8:

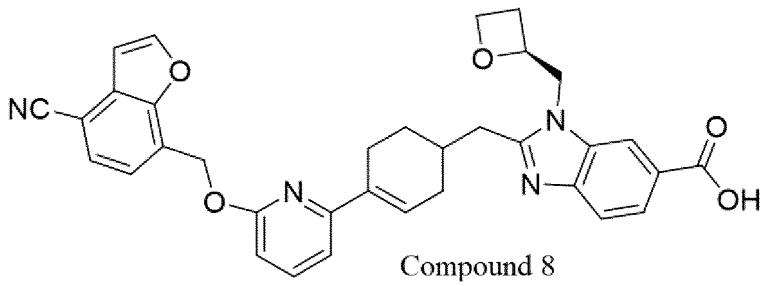

Compound 8

0.1 g of compound 8-E (1.0 eq.) was weighed and added to a 25mL round-bottom flask. 5 mL of acetonitrile, and then TBD solution (TBD (2.0 eq.) dissolved in 1.0 mL of water) were added. The reaction solution was stirred at room temperature for 24 hours. Subsequently, the reaction solution was concentrated and 30 mL of ethyl acetate was added. The pH of the reaction system was then adjusted to be 4-5 with citric acid. Subsequently, the reaction solution was left to stand, the organic phases were separated, and the aqueous phase was continuously washed with 20 mL×2 of ethyl acetate twice. The organic phases were combined and washed with saturated salt solution, dried with anhydrous $Na_2SO_4$ for 5 minutes, and then filtered. The filtrate was concentrated to dryness and purified by column chromatography to obtain 19.5 mg of compound 8 with a yield of 20%. MS m/z (ESI):575.2291 $(M+H)^+$.

Example 7:

Synthesis of Compound 10:

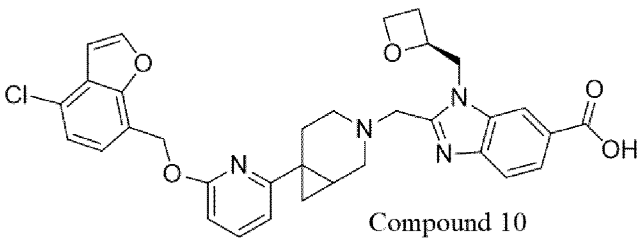

Compound 10

Synthesis route:

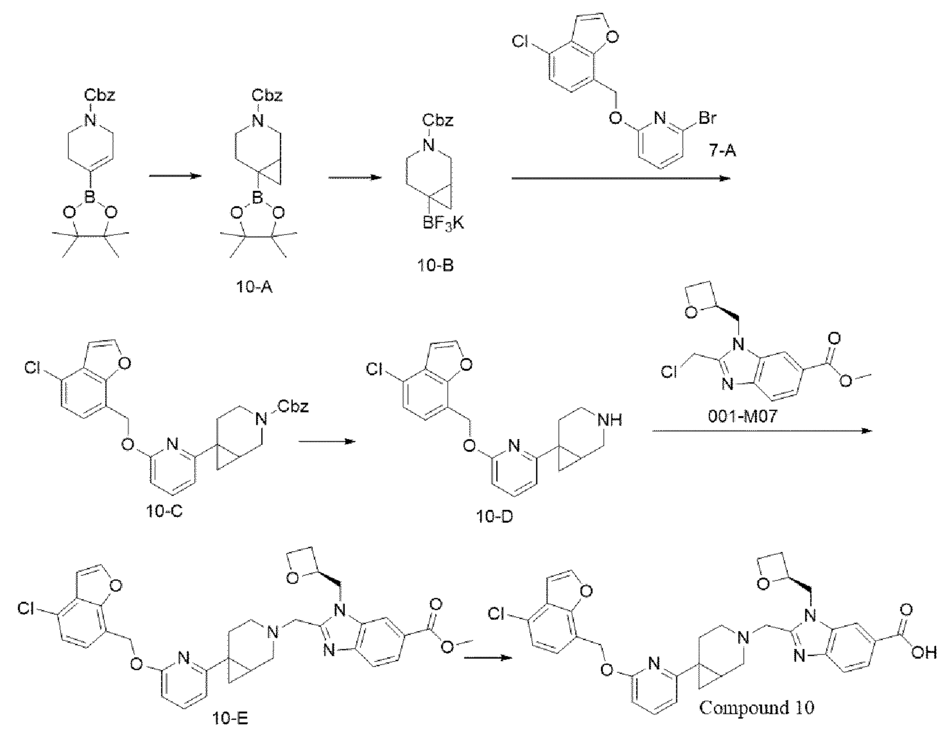


Compound 001-M7:

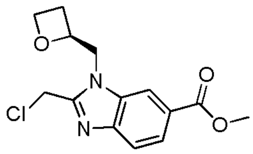

001-M07

5.30 g of compound 1-F (1.0 eq.) was weighed and added to a 250mL round-bottom flask. 64 mL of acetonitrile, 3.81 g of 2-chloro-1,1,1-trimethoxyethane (1.1 eq.) and 213.0 mg of pTSA·$H_2O$ (0.05 eq.) were then added. Subsequently, the reaction solution was heated at 50°C for 1.5 hours. TLC showed that the raw materials were completely reacted. The reaction solution was slightly cooled and then concentrated under reduced pressure. The concentrated solution was added with 50 mL of water and 35 mL of ethyl acetate, and left to stand for liquid separation. The aqueous phase was washed with 20 mL×2 of ethyl acetate twice, and the organic phases were combined and then washed with saturated salt solution, dried with anhydrous sodium sulfate and filtered. The filtrate was concentrated under reduced pressure to obtain an oily substance. Subsequently, 8 mL of ethyl acetate and 40 mL of petroleum ether were added to the oily substance for pulping, and then the thus obtained system was filtered to obtain solid compound 001-M07 (5.2 g, 78.67%). $^1$HNMR (400 MHz, $CDCl_3$) δ =8.16 (s, 1H), 8.04 (d, J = 8.4 Hz, 1H), 7.83 (d, J = 8.5 Hz, 1H), 5.24 (d, J = 6.4 Hz, 1H), 5.08 (s, 2H), 4.65 (dd, J = 14.8, 6.9 Hz, 2H), 4.56 (d, J = 13.9 Hz, 1H), 4.36 (dd, J = 5.9, 3.2 Hz, 1H), 3.98 (s, 3H), 2.79 (dt, J = 14.0, 7.8 Hz, 1H), 2.51-2.38 (m, 1H).

Compound 10-A:

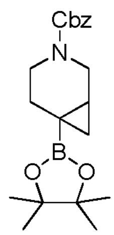

10-A

Under the protection of nitrogen, 45 mL of dichloromethane and 70 mL of 1 M $ZnEt_2$ in Toluene (8.0 eq.) were

added to a 250mL three-neck flask. The thus obtained system was cooled to -40°C and stirred. 10 mL of dichloromethane diluent containing 37.46 g of diiodomethane (16.0 eq.) was slowly added dropwise. After the dropwise addition, the reaction solution was kept at -40°C and stirred for about 1 hour, and then 7.97 g of trifluoroacetic acid (8.0 eq.) was added dropwise, the thus obtained system was heated to -15°C, and continuously stirred for 1 hour, and then 10 mL of dichloromethane diluent containing 3 g of N-Cbz-3,6-dihydro-2H-pyridine-4-boronic acid pinacol ester (1.0 eq.) was continuously added dropwise. After the dropwise addition, the reaction solution was stirred at room temperature for about 15 hours, and then filtered with diatomite. The filtrate was added with 100 mL of dichloromethane and 50 mL of saturated sodium bicarbonate solution, and then stirred for liquid separation. The organic phases were washed with 50 mL of water and 50 mL of saturated sodium chloride solution in turn, dried with anhydrous sodium sulfate and filtered. The filtrate was concentrated. After the concentration, 60 mL of tetrahydrofuran and 30 mL of water were added to the residue, and the thus obtained system was stirred to dissolve. 3.11 g of N-bromosuccinimide (2 eq.) was added. The thus obtained system was continuously stirred for about 2 hours, then 100 mL of ethyl acetate and 50 mL of saturated sodium bicarbonate solution were added, and the thus obtained system was stirred for liquid separation. The organic phases were washed with 50 mL of water and 50 mL of saturated sodium chloride solution again, dried with anhydrous sodium sulfate and filtered. The filtrate was concentrated and purified by column chromatography separation to obtain 830 mg of compound 10-A with a yield of 26.6%. MS m/z (ESI):358.2192 $(M+H)^+$, $^1H$ NMR (400 MHz, $CDCl_3$) $\delta$ =7.43-7.29 (m, 5H), 5.13 (s, 2H), 3.92 (t, *J* = 11.5 Hz, 1H), 3.57 (dd, *J* = 10.3, 6.6 Hz, 2H), 2.97 (s, 1H), 2.13 (dd, *J*= 9.4, 4.8 Hz, 1H), 1.31-1.12 (m, 14H), 0.96-0.87 (m, 1H), 0.45 (m, 1H).

Compound 10-B:

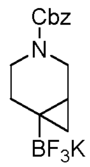

10-B

800 mg of compound 10-A (1.0 eq.) and 1.22 g of $KHF_2$ (7.0 eq.) were weighed and added to a 25mL round-bottom flask. 8 mL of methanol was added. The reaction mixture was reacted to reflux for about 15 hours until TLC showed that the reaction was completed. After cooled to room temperature, the reaction solution was concentrated, and then 5 mL of petroleum ether and 1 mL of methyl tert-butyl ether were added, pulped at room temperature for about 0.5 hours, and then the thus obtained system was filtered. The filter cake was added with 8 mL of acetonitrile and stirred under reflux for about 15 hours, and then filtered. The filtrate was concentrated to obtain 630 mg of compound 10-B with a yield of 83.4%.

Compound 10-C:

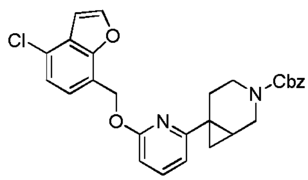

10-C

400 mg of compound 7-A (1.0 eq.), 438 mg of compound 10-B (1.1eq.), 47 mg of tBuXPhos-Pd-G3 (0.05 eq.) and 1.15 g of cesium carbonate (3.0 eq.) were weighed and added to a 25mL round-bottom flask. 5 mL of toluene and 1 mL of water were then added. The reaction solution was stirred evenly and then degassed with nitrogen twice. Subsequently, the reaction solution was heated to 80°C under nitrogen atmosphere and stirred for about 6 hours until TLC showed that the reaction was completed. The reaction solution was cooled to room temperature and filtered with diatomite. The filter cake was washed with 50 mL of ethyl acetate, and the filtrate was collected. The filtrate was respectively washed with 20 mL of water and 20 mL of saturated sodium chloride solution in turn, dried with anhydrous sodium sulfate and filtered. The filtrate was concentrated and purified by column chromatography separation to obtain 350 mg of compound 10-C with a yield of 60.6%.

Compound 10-D:

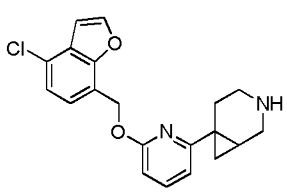

10-D

340 mg of compound 10-C (1.0 eq.), 202 mg of triethylsilane (2.5 eq.), 35 mg of triethylamine (0.5 eq.) and 16 mg of palladium acetate (0.1 eq.) were weighed and added to a 25mL round-bottom flask. 5 mL of ethanol was added. The reaction solution was stirred evenly, and then reacted under nitrogen atmosphere at room temperature for about 16 hours until TLC showed that the reaction was completed. The reaction solution was filtered by a funnel with diatomite. The filter cake was washed with a proper amount of ethanol and the filtrate was collected. Then the filtrate was concentrated and purified by column chromatography separation to obtain 198 mg of compound 10-D with a yield of 80.2%.

Compound 10-E:

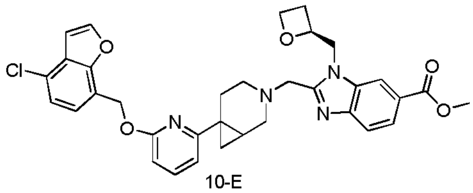

10-E

190 mg of compound 10-D (1.0 eq.), 158 mg of compound 001-M07 (1.0 eq.) and 148 mg of potassium carbonate (2.0 eq.) were weighed and added to a 50mL round-bottom flask. 10 mL of acetonitrile was added. The reaction mixture was heated to 50°C and reacted for about 4 hours until TLC showed that the reaction was completed. Subsequently, the reaction solution was cooled to room temperature, 20 mL of water and 30 mL of ethyl acetate were added, and the thus obtained system was left to stand for liquid separation. The aqueous phase was extracted with 20 mL×3 of ethyl acetate thrice, and the organic phases were combined and respectively washed with 20 mL of water and 20 mL of saturated sodium chloride solution, dried with anhydrous sodium sulfate and filtered. After concentration, the filtrate was purified by column chromatography separation to obtain 220 mg of compound 10-E with a yield of 67.1%. MS m/z (ESI):613.2215 $(M+H)^+$.

Compound 10:

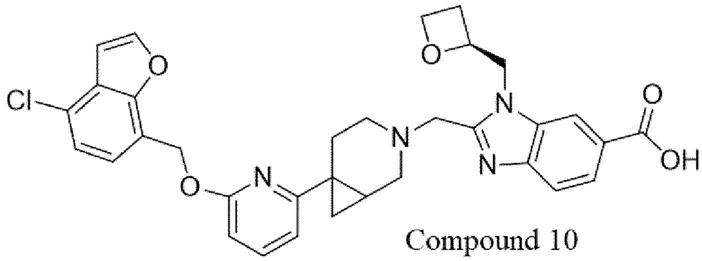

Compound 10

200 mg of compound 10-E (1.0 eq.) and 21 mg of lithium hydroxide monohydrate (1.5 eq.) were weighed and added to a 50mL round-bottom flask. 3 mL of acetonitrile, 1 mL of methanol and 1 mL of water were then added. The reaction solution was heated to 40°C and reacted for about 5.5 hours until TLC showed that the reaction was completed. Subsequently, the reaction solution was cooled to room temperature and concentrated to remove most organic solvent. 15 mL of water was added to the residue for dilution, and the pH of the reaction solution was adjusted to be 4-5 with citric acid. Subsequently, the aqueous phase was extracted with 15 mL×3 of ethyl acetate, and the organic phases were combined and respectively washed with 15 mL of water and 15 mL of saturated sodium chloride solution, dried with anhydrous $Na_2SO_4$ and filtered. After concentration, the filtrate was purified by column chromatography to obtain 124 mg of compound 10 with a yield of 63.6%. MS m/z (ESI):599.2063 $(M+H)^+$.

Example 8:

Synthesis of Compound 11:

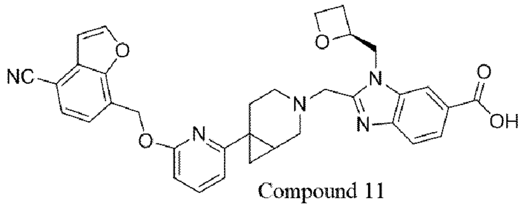

Compound 11

Synthesis route:

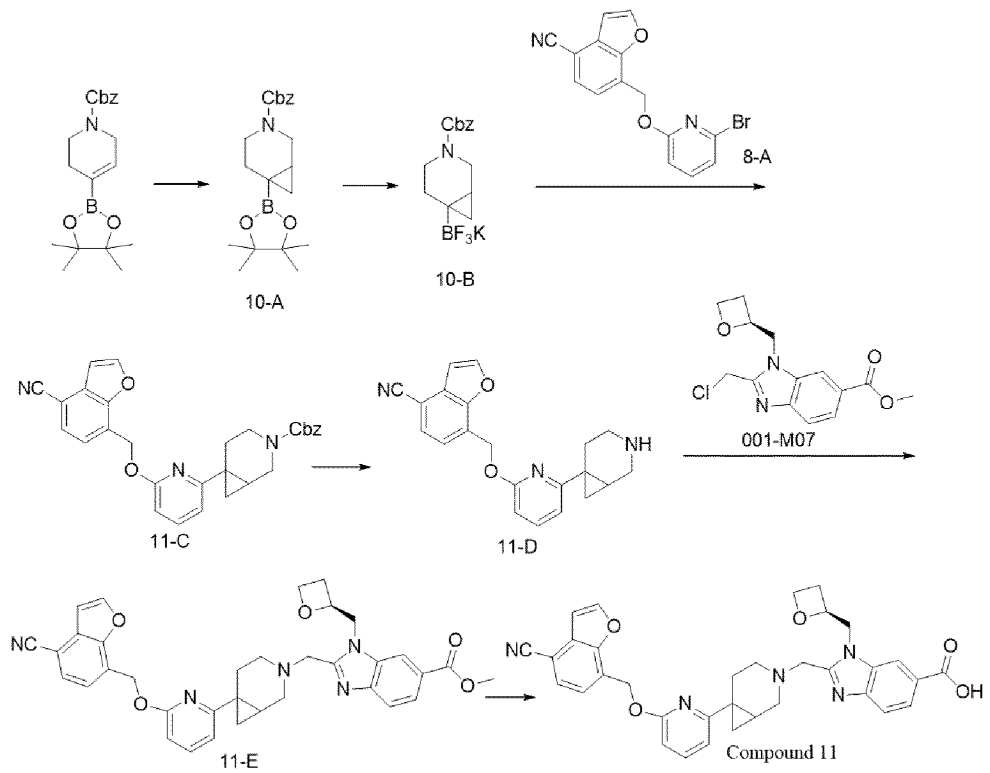

8-A

10-A

10-B

001-M07

11-C

11-D

11-E

Compound 11

Referring to the preparation method of compound 10, compound 7-A therein was replaced with compound 8-A to obtain compound 11. MS m/z (ESI):590.2409 $(M+H)^+$.

Example 9:

Synthesis of Compound 13:

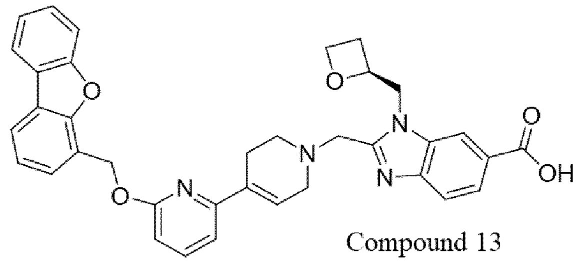

Compound 13

Synthesis route:

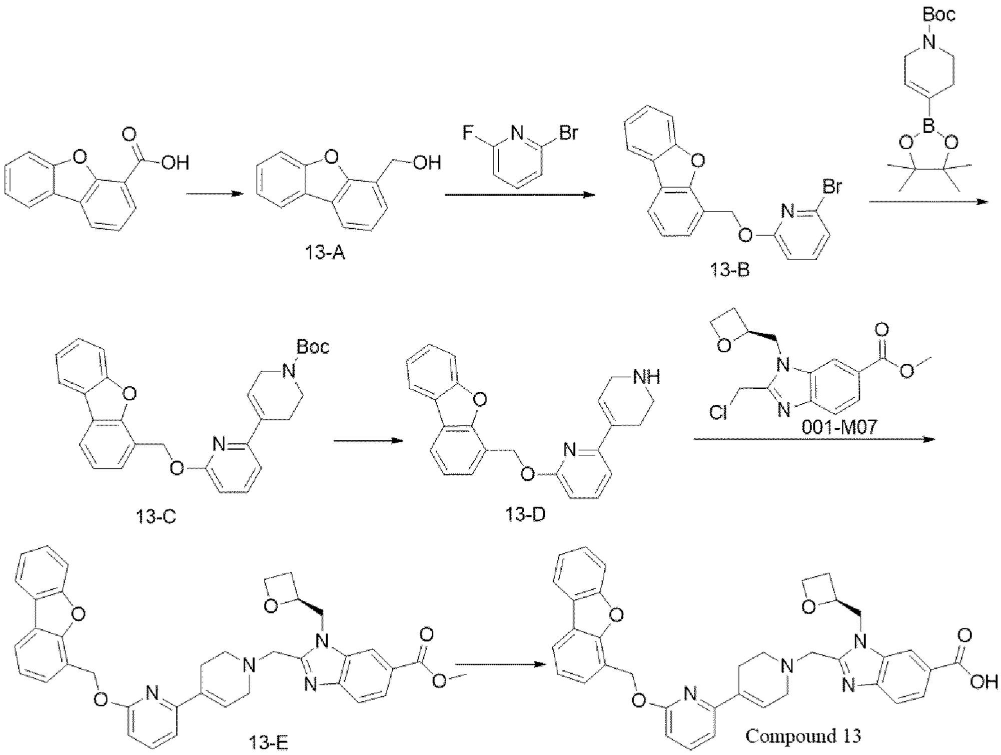

13-A

13-B

001-M07

13-C

13-D

13-E

Compound 13

Compound 13-A:

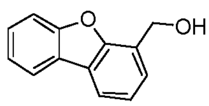

13-A

600 mg of dibenzofuran-4-carboxylic acid (1.0 eq.) was weighed and added to a 50mL round-bottom flask. 10 mL of tetrahydrofuran was added. The thus obtained system was stirred to dissolve, then stirred for 5 minutes in an ice bath, and then 215 mg of lithium aluminum hydride (2.0 eq.) was added. After the addition, the reaction solution was continuously reacted at 0-10°C for about 1 hour. TLC showed that the reaction was completed. Subsequently, 10 mL of saturated aqueous ammonium chloride solution was dropwise added to the reaction solution until no bubbles were generated. Then, the reaction solution was filtered by a funnel with diatomite, and the filter cake was washed with 50 mL of ethyl acetate. After concentration, the filtrate was purified by column chromatography to obtain 447 mg of compound 13-A with a yield of 79.8%.

Compound 13-B:

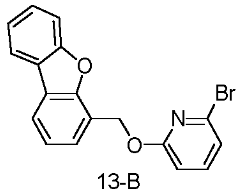

13-B

420 mg of compound 13-A (1.0 eq.), 410 mg of 2-bromo-6-fluoropyridine (1.1 eq.) and 1.38 g of cesium carbonate (2.0 eq.) were weighed and added to a 50mL round-bottom flask. 5 mL of N,N-dimethylformamide was added. The reaction solution was heated to 90°C and reacted for about 2 hours until TLC showed that the reaction was completed. Subsequently, the reaction solution was cooled to room temperature and 15 mL of water was added to precipitate a large amount of solids, and then the thus obtained system was continuously stirred for about 0.5 hours and filtered. The filter cake was washed with a small amount of water and then dried to obtain 600 mg of compound 13-B with a yield of 80.0%.

Compound 13-C:

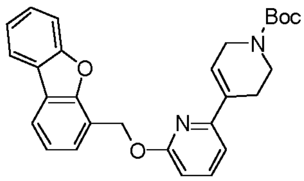

13-C

600 mg of compound 13-B (1.0 eq.), 576 mg of N-Boc-1,2,5,6-tetrahydropyridine-4-boronic acid pinacol ester (1.1 eq.), 124 mg of Pd(dppf)$Cl_2$ (0.1 eq.) and 285 mg of sodium hydrogen carbonate (2.0 eq.) were weighed and added to a 100mL round-bottom flask. 7.5 mL of toluene, 2.5 mL of ethanol and 2.5 mL of water were added. The reaction solution was stirred evenly, degassed with nitrogen twice, and then heated to 110°C and reacted for about 2 hours under nitrogen atmosphere until TLC showed that the reaction was completed. Subsequently, the reaction solution was cooled to room temperature and filtered by a funnel with diatomite and the filter cake was washed with a small amount of dichloromethane. The filtrate was concentrated to dryness, and purified by column chromatography separation to obtain 710 mg of compound 13-C with a yield of 91.8%.

Compound 13-D:

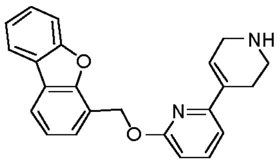

13-D

700 mg of compound 13-C (1.0 eq.), 875 mg of p-toluenesulfonic acid monohydrate (3.0 eq.) were weighed and added to a 25mL round-bottom flask. 15 mL of ethyl acetate was added. The reaction mixture was heated to 60°C and reacted for about 2 hours until TLC showed that the reaction was completed. Subsequently, the reaction solution was cooled to room temperature, and 30 mL of ethyl acetate was added for dilution. The pH of the reaction solution was adjusted to be 8-9 with saturated sodium bicarbonate solution, and then the reaction solution was left to stand for liquid separation. The ethyl acetate phases were dried with anhydrous sodium sulfate and filtered. The filtrate was concentrated to dryness to obtain 510 mg of compound 13-D with a yield of 93.2%.

Compound 13-E:

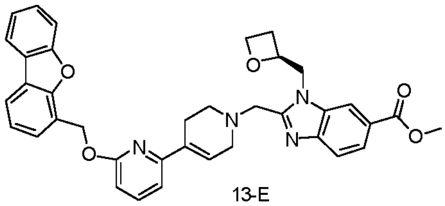

13-E

250 mg of compound 001-M07 (1.0 eq.), 317 mg of compound 13-D (1.05 eq.) and 234 mg of cesium carbonate (2.0 eq.) were weighed and added to a 100mL round-bottom flask. 15 mL of acetonitrile was added. The reaction solution was heated to 50°C for about 3 hours until TLC showed that the reaction was completed. Subsequently, the reaction solution was cooled to room temperature and 30 mL of water was added to precipitate a large amount of solids, and then the thus obtained system was continuously stirred for about 0.5 hours and then subjected to suction filtration. The filter cake was washed with a small amount of water and then dried to obtain 385 mg of compound 13-E with a yield of 73.9%. MS m/z (ESI):615.2605 $(M+H)^+$.

Compound 13:

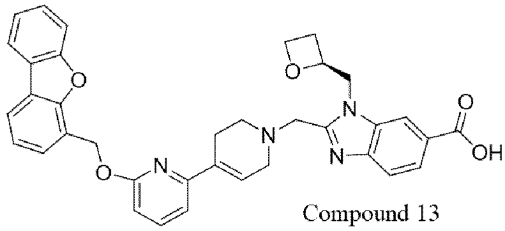

Compound 13

385 mg of compound 13-E (1.0 eq.) and 39 mg of lithium hydroxide monohydrate (1.5 eq.) were weighed and added to a 25mL round-bottom flask. 6 mL of acetonitrile, 2 mL of methanol and 2 mL of water were then added. The reaction mixture was heated to 40°C and reacted for about 6 hours until TLC showed that the reaction was completed. After cooled to room temperature, the reaction solution was concentrated to remove most organic solvents. 20 mL of water was added for dilution, and the pH of the reaction solution was adjusted to be 4-5 with citric acid solution. The aqueous phase was extracted with 20 mL×3 of ethyl acetate. The ethyl acetate phases were combined, and respectively washed with 20 mL of water and 20 mL of saturated sodium chloride solution, dried with anhydrous $Na_2SO_4$ and then filtered. The filtrate was concentrated to dryness and recrystallized with dichloromethane/methanol to obtain 241 mg of compound 13 with a yield of 64.1%. MS m/z (ESI):601.2457 $(M+H)^+$, $^1H$ NMR (400 MHz, DMSO) $\delta$ =12.90 (s, 1H), 8.28 (s, 1H), 8.09 (t, J = 8.1 Hz, 2H), 7.85 (d, *J* = 8.4 Hz, 1H), 7.67 (dd, *J* = 14.6, 8.0 Hz, 3H), 7.59 (d, *J* = 7.3 Hz, 1H), 7.37 (t, *J* = 7.6 Hz, 2H), 7.27 (t, *J* = 7.4 Hz, 1H), 7.05 (d, *J* = 7.4 Hz, 1H), 6.75 (d, *J* = 8.1 Hz, 1H), 6.67 (s, 1H), 5.73 (s, 2H), 5.03 (d, *J* = 5.3 Hz, 1H), 4.76 (dd, *J* = 15.2, 7.1 Hz, 1H), 4.61 (d, *J* = 14.2 Hz, 1H), 4.46 (dd, *J* = 13.6, 7.0 Hz, 1H), 4.34 (dd, *J* = 14.3, 5.9 Hz, 1H), 4.02 (d, *J* = 13.5 Hz, 1H), 3.86 (d, *J* = 13.4 Hz, 1H), 3.10 (q, *J* = 16.9 Hz, 2H), 2.62 (s, 3H), 2.39 (d, *J* = 18.4 Hz, 3H).

Example 10:

Synthesis of Compound 14:

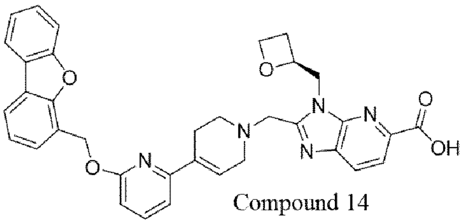

Compound 14

Synthesis route:

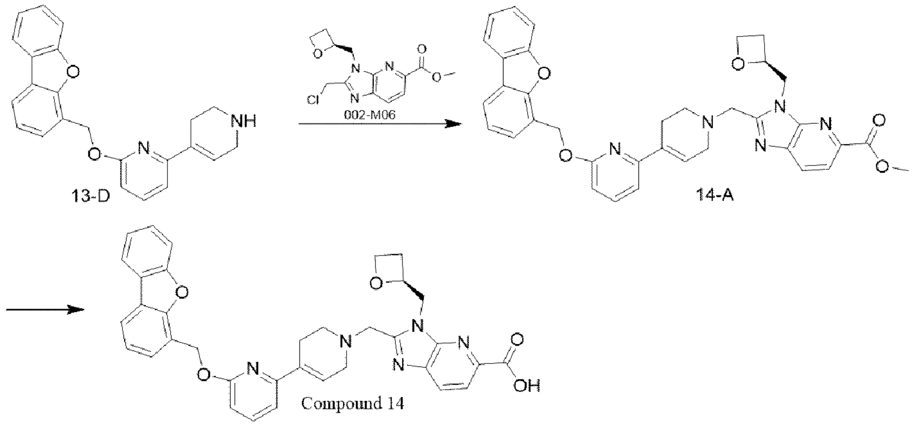


13-D 14-A Compound 14

Synthesis of Compound 002-M06:

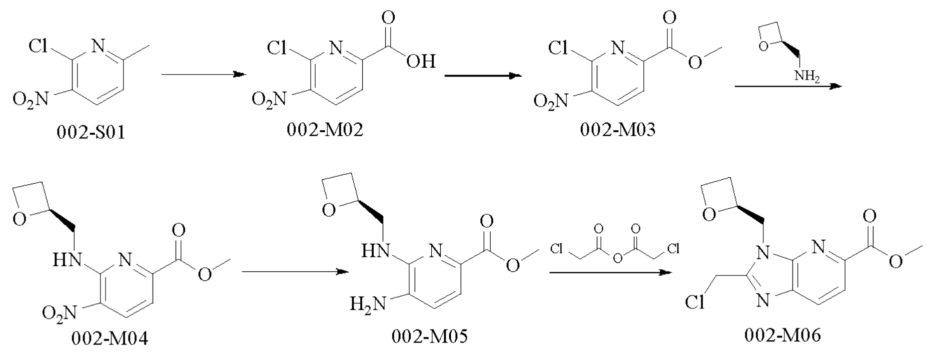

002-S01 002-M02 002-M03 002-M04 002-M05 002-M06

Compound 002-M02:

9.7 g of compound 002-S01 (1.0 eq.) was weighed and added to a 100mL round-bottom flask. 40 mL of concentrated sulfurinc acid was added. 16.9 g of chromium trioxide (3.0 eq.) were weighed and added in batches. The internal temperature of the flask was kept lower than 50°C, and the reaction solution was stirred at room temperature. After reaction for 24 hours, the reaction solution was poured into 200 g of ice, stirred at room temperature for 1 hour, and filtered. The filter cake was washed with water and dried at 50°C for 12 hours to obtain 9.625 g of compound 002-M02 with a yield of 85%.

Compound 002-M03:

8.5 g of compound 002-M02 (1.0 eq.) was weighed and added to a 250mL round-bottom flask. 85 ml of dichloromethane was added. The reaction solution was stirred for 0.5 hours in an ice bath. 10.65 g of oxalyl chloride (2.0 eq.) and 0.52 mL of DMF (0.16 eq.) were weighed and added. The reaction solution was slowly heated to room temperature, and continuously reacted for 1.0 hour. 4.03 g of methanol (3.0 eq.) was weighed and added. The reaction solution was continuously reacted at room temperature for 15 hours. After the reaction, the reaction solution was concentrated, and purified by column chromatography to obtain 8.0 g of compound 002-M03 with a yield of 88%.

Compound 002-M04:

4.0 g of compound 002-M03 (1.0 eq.), 1.93 g of compound (S)-oxetan-2-ylmethanamine, 3.74 g of triethylamine (2.0 eq.), 80 mL of DMF and 80 mL of THF were weighed and added to a 250mL round-bottom flask. Subsequently, the reaction solution was reacted at room temperature for 15 hours. After the reaction, the reaction solution was concentrated

to remove THF, then 160 mL of water was added, the thus obtained system was stirred for 2 hours, and filtered. The filter cake was washed with water, and then dried at 50°C for 12 hours to obtain 3.35 g of compound 002-M04 with a yield of 68%. MS m/z (ESI):268.0925 $(M+H)^+$.

Compound 002-M05:

3.35 g of compound 002-M04 (1.0 eq.), 0.335 g of palladium on carbon (10%) and 50 mL of methanol were weighed and added to a 100mL round-bottom flask. Subsequently, the reaction solution was degassed with nitrogen twice, and degassed with hydrogen twice, and reacted for 15 hours at room temperature under hydrogen atmosphere. After the reaction, the reaction solution was filtered by a funnel with diatomite, and the filter cake was washed with 50 mL×2 of ethyl acetate. Subsequently, the filtrate was concentrated to dryness to obtain 2.97 g of compound 002-M05, which was directly used in the next reaction without purification.

Compound 002-M06:

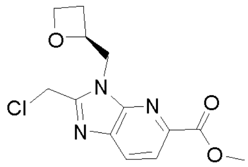

002-M06

1.275 g of compound 002-M05 (1.0 eq.), 0.965 g of chloroacetic anhydride (1.05 eq.) and 25 mL of THF were weighed and added to a 50mL round-bottom flask. The reaction mixture was heated to 60°C for reaction. After reaction for 1.5 hours, the heating was stopped, the reaction solution was cooled to room temperature and was concentrated. After the concentration, 25 mL of saturated sodium bicarbonate solution and 25 mL of ethyl acetate were added to the residue, and then the organic phases were separated. The aqueous phase was continuously washed with 25 mL×2 of ethyl acetate twice, and the organic phases were combined and washed with saturated salt solution, dried with anhydrous $Na_2SO_4$ for 5 minutes, and then filtered. The filtrate was concentrated to dryness and purified by column chromatography to obtain 1.07 g of compound 002-M06 with a yield of 67.3%. MS m/z (ESI):296.0798 $(M+H)^+$, $^1$HNMR(400MHz, $CDCl_3$)δ =8.14-8.10 (m, 2H), 5.23-5.17 (m, 1H), 5.07 (q, *J* = 12.4 Hz, 2H), 4.82-4.70 (m, 2H), 4.58 (q, *J* = 7.6 Hz, 1H), 4.32-4.26 (m, 1H), 3.99 (s, 3H), 2.80-2.71 (m, 1H), 2.46-2.37 (m, 1H).

Compound 14-A:

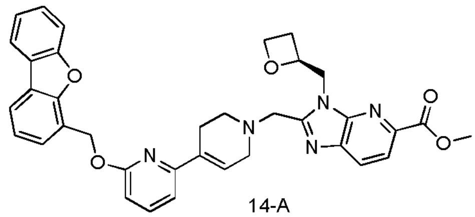

14-A

0.15 g of compound 002-M06 (1.0 eq.), 0.199 g of compound 13-D (1.1 eq.) and 91 mg of potassium carbonate (2.0 eq.) were weighed and added to a 25mL round-bottom flask. 5 mL of acetonitrile was added. The reaction solution was heated to 60°C for reaction. After reaction for 3 hours, the heating was stopped, and the reaction solution was cooled to room temperature and was concentrated, and the residue was added with 10 mL of water after the concentration to precipitate a large amount of solids which were continuously stirred for about 0.5 hours and then subjected to suction filtration. The filter cake was washed with 10 mL×3 of water, dried and then purified by column chromatography to obtain 0.165 g of compound 14-A with a yield of 53%.

Compound 14:

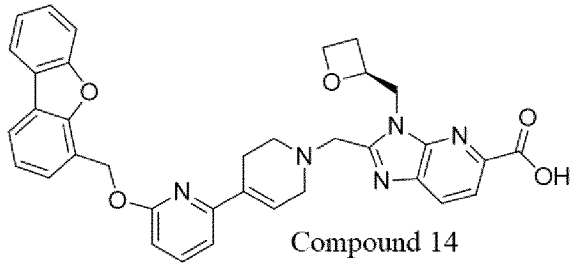

Compound 14

0.164 g of compound 14-A (1.0 eq.) was weighed and added to a 25mL round-bottom flask. 1.0 mL of methanol and 3.0 mL of tetrahydrofuran were added, and then 0.27 mL of 2N sodium hydroxide solution was added. Subsequently, the reaction solution was reacted at room temperature. After 15 hours, the reaction solution was concentrated. After the concentration, 4 mL of water was added to the residue, and the pH of the reaction solution was adjusted to be 4-5 with 1 mol/L HCl. Subsequently, the reaction solution was filtered. The filter cake was pulped with 2 mL of ethyl acetate and 0.5 mL of methanol for pulping, and then filtered. The filter cake was washed with 2 mL×3 of ethyl acetate, and dried to obtain 0.118 g of compound 14 with a yield of 73.6%. MS m/z (ESI):602.2403 $(M+H)^+$, $^1H$ NMR (400 MHz, DMSO-$d_6$) δ =8.17 (d, *J* = 8.2 Hz, 1H), 8.10-8.02 (m, 3H), 7.69-7.64 (m, 2H), 7.58 (d, *J* = 7.2 Hz, 1H), 7.39-7.34 (m, 2H), 7.25 (t, *J* = 7.6 Hz, 1H), 7.04 (d, *J* = 7.2 Hz, 1H), 6.75 (d, *J* = 8.4 Hz, 1H), 6.67 (s, 1H), 5.72 (s, 2H), 5.14-5.08 (m, 1H), 4.82-4.64 (m, 2H), 4.47-4.30 (m, 2H), 4.02 (dd, *J* = 38.4, 13.2 Hz, 2H), 3.19-3.08 (m, 2H), 2.67-2.62 (m, 3H), 2.47-2.42 (m, 3H).

Example 11:

Synthesis of Compound 15:

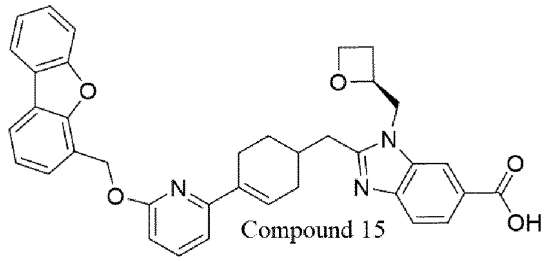

Compound 15

Synthesis route:

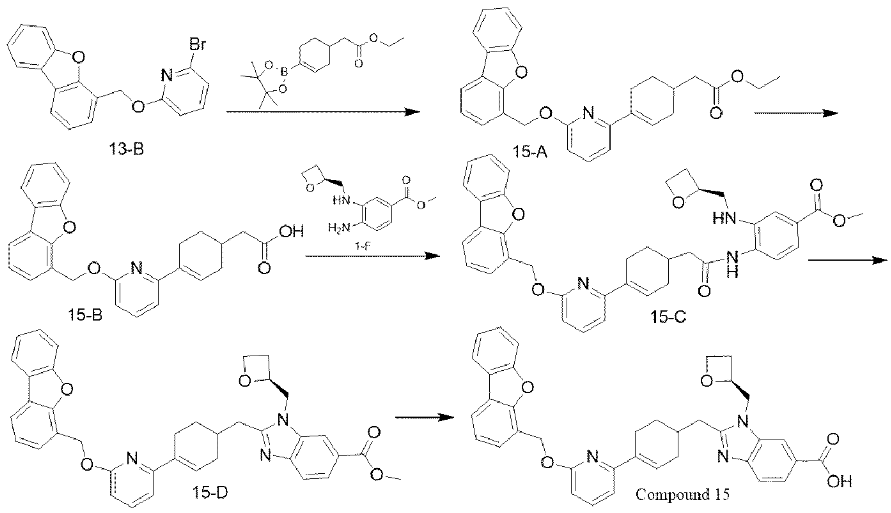


Compound 15-A:

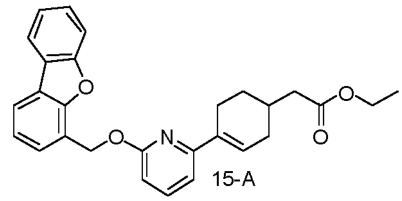
15-A

0.5 g of compound 13-B (1.0 eq.), 0.457 g of 1-pinacol borate-4-ethyl acetate-1-cyclohexene (1.1 eq.), 0.103 g of Pd(dppf)$Cl_2$ (0.1 eq.) and 0.237 g of sodium hydrogen carbonate (2.0 eq.) were weighed and added to a 50mL round-bottom flask. 7.5 mL of toluene, 2.5 mL of ethanol and 2.5 mL of water were added. Subsequently, the reaction solution was degassed with nitrogen twice and heated to 110°C for reaction under nitrogen atmosphere. After reaction for 2-3 hours, the heating was stopped, and the reaction solution was cooled to room temperature and filtered by a funnel with diatomite, and the filter cake was washed with 15 mL×2 of ethyl acetate. The filtrate was collected, and then the filtrate was concentrated to dryness and purified by column chromatography to obtain 0.467 g of compound 15-A with a yield of 75%.

Compound 15-B:

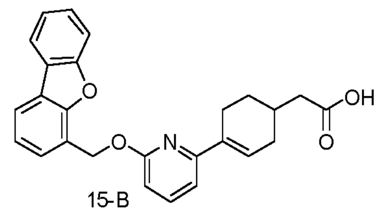
15-B

0.45 g of compound 15-A (1.0 eq.) was weighed and added to a 50mL round-bottom flask. 6.5 mL of methanol and 10 mL of tetrahydrofuran were added. Subsequently, 1.02 mL of 2 mol/L NaOH solution (2.0 eq.) was dropwise added to the reaction solution at room temperature. After the dropwise addition, the reaction solution was continuously reacted for 1.5 hours at room temperature. After the reaction was completed, the reaction solution was concentrated. After the concentration, 15 mL of water and 35 mL of ethyl acetate were added to the residue, and the pH of the reaction system was adjusted to be 4-5 with 1 mol/L HCl solution, and then the thus obtained system was left to stand. The organic phases were separated, the aqueous phase was continuously washed with 35 mL×2 of ethyl acetate twice, and the organic phases were combined, and washed with saturated salt solution, then dried with anhydrous $Na_2SO_4$ for 5 minutes, and then filtered. The filtrate was concentrated to dryness to obtain 0.316 g of compound 15-B with a yield of 75%.

Compound 15-C:

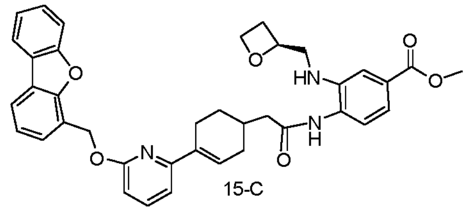

15-C

0.3 g of compound 15-B (1.0 eq.), 0.188 g of compound 1-F (1.1 eq.) and 0.279 g of TBTU (1.2 eq.) were weighed and added to a 50mL round-bottom flask. 15.0 mL of DMF was added. The reaction solution was stirred for 10 minutes in an ice bath, and then dropwise added with 0.188 g of diisopropylethylamine (2.0 eq.). After the dropwise addition, the reaction solution was continuously stirred for 3 hours in the ice bath. After the reaction, 30 mL of $H_2O$ and 30 mL of ethyl acetate were added to the reaction system, the thus obtained system was stirred for 5 minutes, and left to stand. The organic phases were separated, and the aqueous phase was continuously washed with 30 mL×2 of ethyl acetate twice. The organic phases were combined and washed with saturated salt solution, dried with anhydrous $Na_2SO_4$ for 5 minutes and filtered. The filtrate was concentrated to dryness and purified by column chromatography to obtain 0.247 g of compound 15-C with a yield of 53.9%.

Compound 15-D:

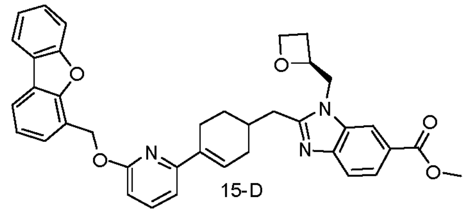

15-D

0.2 g of compound 15-C was weighed and added to a 50mL round-bottom flask. 4.0 mL of 1,4-dioxane was added. 1 mL of acetic acid was then added dropwise. After the dropwise addition, the reaction solution was heated at 80°C for reaction. After 5 hours, the reaction solution was cooled to room temperature. Subsequently, the reaction solution was concentrated and purified by column chromatography to obtain 0.12 g of compound 15-D with a yield of 62%.

Compound 15:

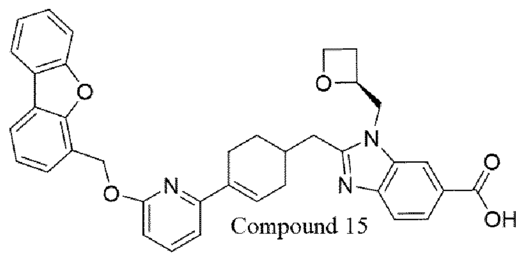

Compound 15

0.1 g of compound 15-D (1.0 eq.) and 20.5 mg of lithium hydroxide monohydrate were weighed and added to a 25mL round-bottom flask. 1.0 mL of methanol, 1.0 mL of tetrahydrofuran and 0.1 mL of $H_2O$ were added. Subsequently, the reaction solution was heated at 40°C for reaction. After 5 hours, the reaction solution was cooled to room temperature, and was concentrated. After the concentration, 30 mL of ethyl acetate was added to the residue, and the pH of the reaction system was adjusted to be 4-5 with 1 mol/L HCl solution, and then the reaction solution was left to stand. The organic phases were separated, the aqueous phase was continuously washed with 20 mL×2 of ethyl acetate twice, and the organic phases were combined and washed with saturated salt solution, dried with anhydrous $Na_2SO_4$ for 5 minutes, and then filtered. The filtrate was concentrated to dryness and purified by column chromatography to obtain 53.7 mg of compound 15 with a yield of 55%. MS m/z (ESI):600.2493 $(M+H)^+$, $^1H$ NMR (400 MHz, DMSO-d6) δ=8.23 (s, 1H), 8.13-8.10 (m, 2H), 7.83-7.59 (m, 5H), 7.44-7.33 (m, 3H), 7.04 (d, J = 7.2 Hz, 1H), 6.73 (d, J = 7.2 Hz, 2H), 5.74 (s, 2H), 5.01 (d, J = 5.2 Hz, 1H), 4.64-4.59 (m, 1H), 4.51-4.44 (m, 2H), 4.29 (d, J = 6.0 Hz, 1H), 4.05-4.01 (m, 1H), 2.94-2.93 (m, 3H), 2.72-2.64 (m, 1H), 2.33-2.29 (m, 4H), 1.99 (s, 2H).

Example 12:

Synthesis of Compound 16:

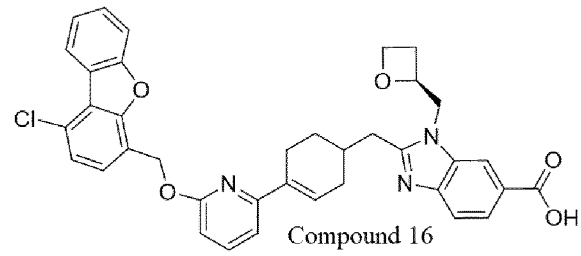

Compound 16

Synthesis route:

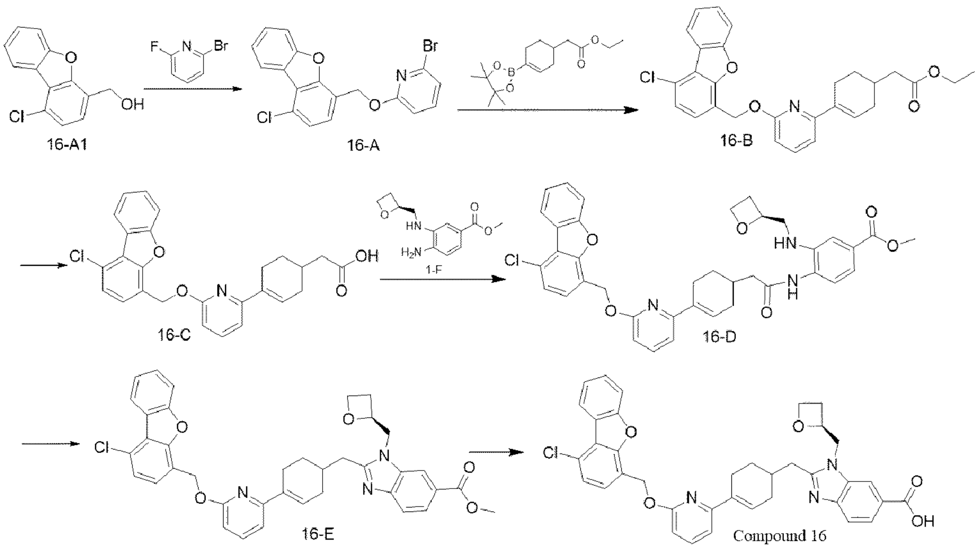


Referring to the synthesis method of compound 15, compound 13-B therein was replaced with compound 16-A to finally obtain compound 16. MS m/z (ESI):634.2103 $(M+H)^+$.

Example 13:

Synthesis of Compound 17:

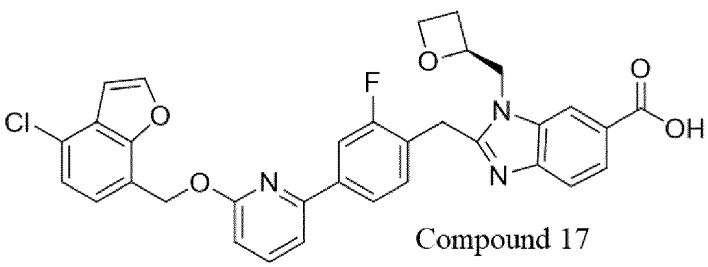

Compound 17

Synthesis route:

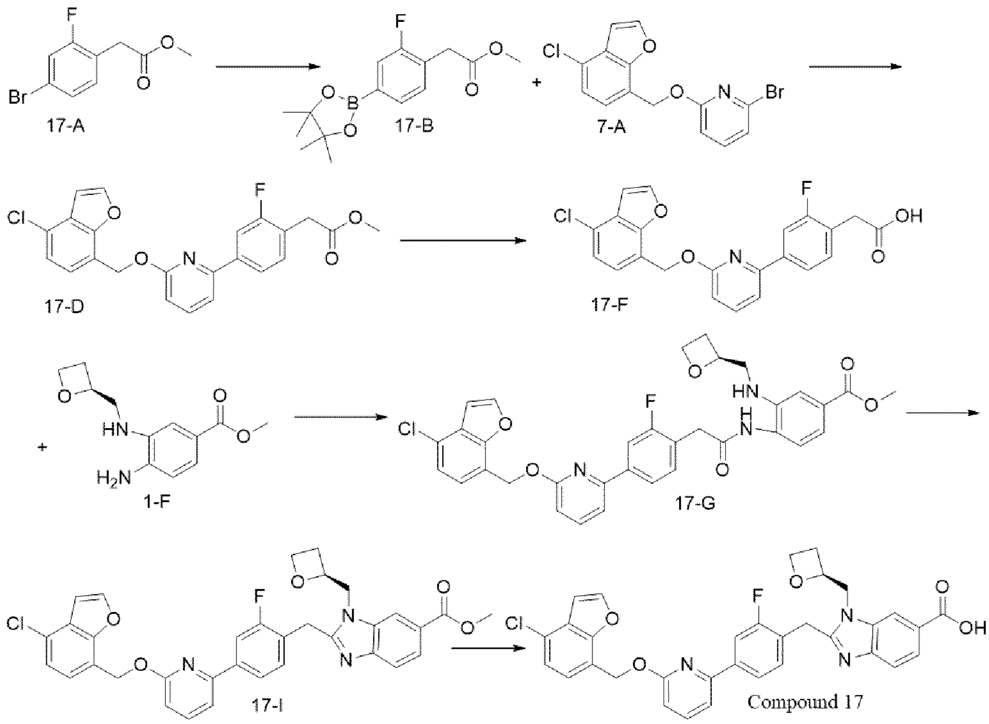

17-A 17-B 7-A

17-D 17-F

1-F 17-G

17-I Compound 17

Compound 17-B:

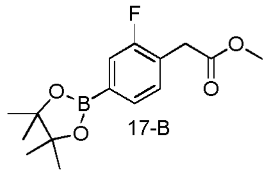

17-B

4.94 g of compound 17-A (1.0 eq.), 6.09 g of bis(pinacolato)diboron (1.2 eq.), 0.44 g of Pd(dppf)$Cl_2$ (0.03 eq.) and 4.91 g of potassium acetate (2.5 eq.) were weighed and added to a round-bottom flask. 30 mL of 1,4-dioxane was added. Subsequently, the reaction solution was degassed with nitrogen twice, and heated to 100°C for reaction under nitrogen atmosphere. After reaction for 2 hours, the heating was stopped, and the reaction solution was cooled to room temperature. Subsequently, the reaction solution was filtered by a funnel with diatomite, and the filter cake was washed with 20 mL×2 of ethyl acetate. Subsequently, the filtrate was concentrated to dryness to obtain 6.87 g of compound 17-B, which was directly used in the next reaction without purification.

Compound 17-D:

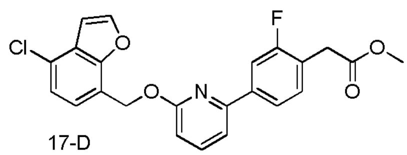

17-D

0.353 g of compound 17-B (1.2 eq.), 0.338 g of compound 7-A (1.0 eq.), 0.022 g of Pd(dppf)$Cl_2$ (0.03 eq.) and 0.815 g of $Cs_2CO_3$ (2.5 eq.) were weighed and added to a 50mL round-bottom flask. 13.0 mL of 1,4-dioxane and 2.0 mL of $H_2O$ were added. Subsequently, the reaction solution was degassed with nitrogen twice, and heated to 90°C for reaction under nitrogen atmosphere. After reaction for 2 hours, the heating was stopped, and the reaction solution was cooled to room temperature. Subsequently, the reaction solution was filtered by a funnel with diatomite, and the filter cake was

washed with 10 mL×2 of ethyl acetate. Subsequently, the filtrate was concentrated to dryness and purified by column chromatography to obtain 0.328 g of compound 17-D with a yield of 77.0%.

Compound 17-F:

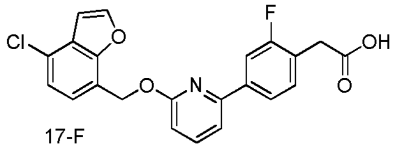

17-F

0.328 g of compound 17-D (1.0 eq.) was weighed and added to a 50mL round-bottom flask. 4.0 mL of methanol and 6.0 mL of tetrahydrofuran were added. Subsequently, 0.77 mL of 2 mol/L NaOH solution (2.0 eq.) was dropwise added to the reaction solution at room temperature. After the dropwise addition, the reaction solution was continuously reacted for 1.5 hours at room temperature. After the reaction was completed, the reaction solution was concentrated. After the concentration, 10 mL of water and 25 mL of ethyl acetate were added to the reaction solution, and the pH of the reaction solution was adjusted to be 4-5 with 1 mol/L HCl solution. Subsequently, the reaction solution was left to stand, the organic phases were separated, the aqueous phase was continuously washed with 25 mL×2 of ethyl acetate twice, and the organic phases were combined and washed with saturated salt solution, dried with anhydrous $Na_2SO_4$ for 5 minutes, and then filtered. The filtrate was concentrated to dryness, after drying, 0.261 g of white solid compound 17-F was obtained with a yield of 82.3%. MS m/z (ESI):412.0751 $(M+H)^+$.

Compound 17-G:

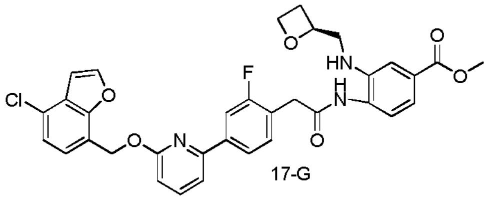

17-G

0.261 g of compound 17-F (1.0 eq.), 0.165 g of compound 1-F (1.1 eq.) and 0.250 g of TBTU (1.2 eq.) were weighed and added to a 50mL round-bottom flask. 10.0 mL of DMF was added. The reaction solution was stirred for 10 minutes in an ice bath, and then 0.22 mL of diisopropylethylamine (2.0 eq.) was added dropwise. After the dropwise addition, the reaction solution was continuously stirred for 3 hours in the ice bath. After the reaction was completed, 30 mL of $H_2O$ and 30 mL of ethyl acetate were added to the reaction system, stirred for 5 minutes, and left to stand. The organic phases were separated, the aqueous phase was continuously washed with 25 mL×2 of ethyl acetate twice, and the organic phases were combined and washed with saturated salt solution, dried with anhydrous $Na_2SO_4$ for 5 minutes, and then filtered. The filtrate was concentrated to dryness and purified by column chromatography to obtain 0.160 g of compound 17-G with a yield of 40.0%. MS m/z (ESI):630.1805 $(M+H)^+$.

Compound 17-I:

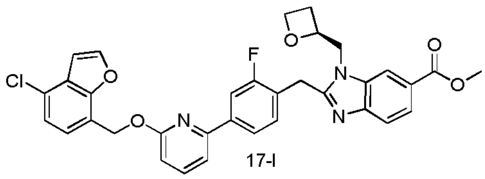

17-I

0.160 g of compound 17-G was weighed and added to a 50mL round-bottom flask. 4.0 mL of 1,4-dioxane was added, and then 0.58 mL of acetic acid was then added dropwise. After the dropwise addition, the reaction solution was heated for reaction at 80°C. After reaction for 5 hours, the reaction solution was cooled to room temperature. Subsequently, the reaction solution was concentrated and purified by column chromatography to obtain 86 mg of compound 17-I with a yield of 55.5%. MS m/z (ESI):612.1707 $(M+H)^+$.

Compound 17:

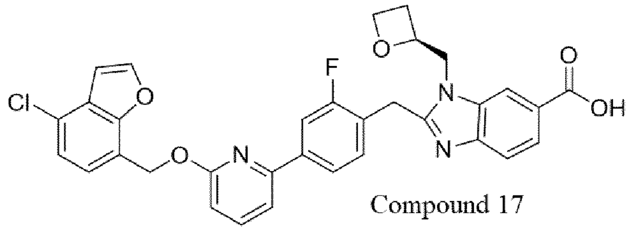

Compound 17

86 mg of compound 17-I and 17.6 mg of lithium hydroxide monohydrate were weighed and added to a 25mL round-bottom flask. 1.0 mL of methanol, 1.0 mL of tetrahydrofuran and 0.1 mL of $H_2O$ were added. Subsequently, the reaction solution was heated for reaction at 40°C. After reaction for 5 hours, the reaction solution was cooled to room temperature. Subsequently, the reaction solution was concentrated. After the concentration, 30 mL of ethyl acetate was added to the residue, and the pH of the reaction solution was adjusted to be 4-5 with 1 mol/L HCl solution. Subsequently, the reaction solution was left to stand, the organic phases were separated, the aqueous phase was continuously washed with 20 mL×2 of ethyl acetate twice, and the organic phases were combined and washed with saturated salt solution, dried with anhydrous $Na_2SO_4$ for 5 minutes, and then filtered. The filtrate was concentrated to dryness and purified by column chromatography to obtain 60 mg of compound 17 with a yield of 71.8%. MS m/z (ESI):598.1536 $(M+H)^+$, $^1H$ NMR (400 MHz, DMSO) δ =12.70 (s, 1H), 8.25 (s, 1H), 8.19 (d, *J* = 2.0 Hz, 1H), 7.93 - 7.77 (m, 4H), 7.61 (dd, *J* = 21.2, 8.0 Hz, 2H), 7.52 - 7.33 (m, 3H), 7.06 (d, *J* = 2.4 Hz, 1H), 6.89 (d, *J* = 8.0 Hz, 1H), 5.77 (s, 2H), 5.05 (d, *J* = 4.8 Hz, 1H), 4.72 (dd, *J* = 15.5, 7.0 Hz, 1H), 4.63 - 4.32 (m, 5H), 2.70 (m, 1H), 2.44 - 2.32 (m, 1H).

Example 14:

Synthesis of Compound 18:

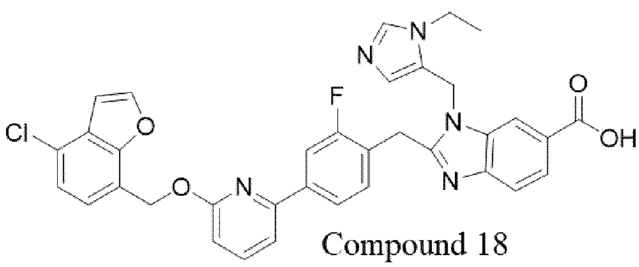

Compound 18

Synthesis route:

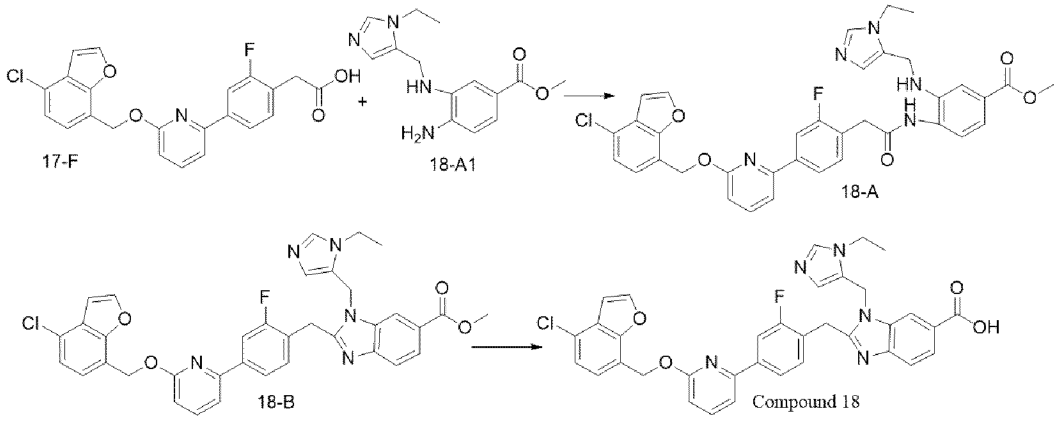

17-F

18-A1

18-A

18-B

Compound 18

Referring to the synthesis of compound 17, wherein compound 1-F was replaced with compound 18-A1 to finally obtain compound 18. MS m/z (ESI):636.1810 $(M+H)^+$, $^1$HNMR (400 MHz, DMSO-d6) $\delta$=12.81 (brs, 1H), 8.18 (dd, J = 7.0, 1.9 Hz, 2H), 7.82 (m, 4H), 7.64 (dd, J = 15.6, 8.9 Hz, 3H), 7.48 (d, J = 8.0 Hz, 1H), 7.39 (dd, J = 15.2,8.0 Hz, 2H), 7.06 (d, J = 2.3 Hz, 1H), 6.89 (d, J = 8.2 Hz, 1H), 6.48 (s, 1H), 5.77 (s, 2H), 5.73 (s, 2H), 4.39 (s, 2H), 3.97 (q, J = 7.2 Hz, 2H), 1.17 (t, J = 7.2 Hz, 3H).

Example 15:

Synthesis of Compound 19:

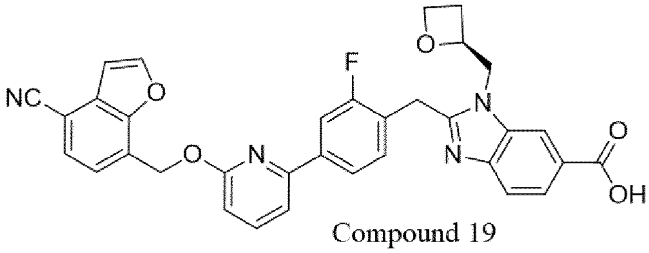

Compound 19

Synthesis route:

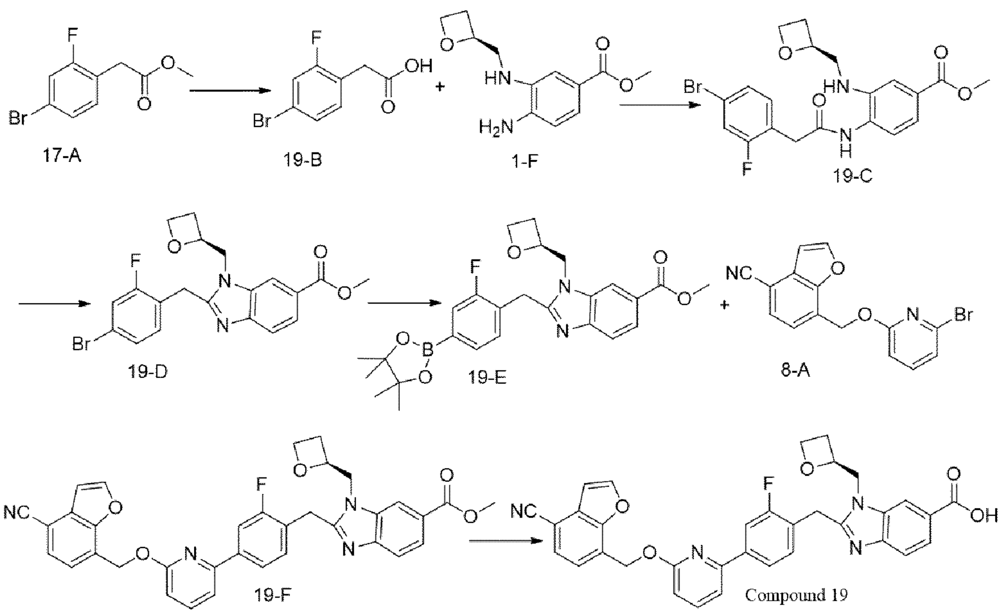

17-A, 19-B, 1-F, 19-C, 19-D, 19-E, 8-A, 19-F, Compound 19

Compound 19-B:

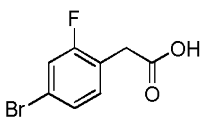

19-B

4.94 g of compound 17-A (1.0 eq.) was weighed and added to a 150 mL round-bottom flask. 20.0 mL of methanol and 30.0 mL of tetrahydrofuran were added. Subsequently, 20.0 mL of 2mol/L NaOH solution (2.0 eq.) was dropwise added to the reaction solution at room temperature. After the dropwise addition, the reaction solution was continuously reacted for 1.5 hours at room temperature. After the reaction, the reaction solution was concentrated. After the

concentration, 20 mL of water was added to the residue, and the pH of the reaction solution was adjusted to be 4-5 with 1 mol/L HCl solution. A large amount of white solids were precipitated from the system, the system was stirred for 5 minutes and then filtered. The filter cake was washed with 20 mL×2 of water twice and collected. The filter cake was dried by forced air drying at 50°C to obtain 4.0 g of white solid compound 19-B with a yield of 85.9%.

Compound 19-C:

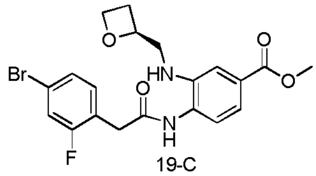

19-C

0.70 g of compound 19-B (1.0 eq.), 0.78 g of compound 1-F (1.1 eq.) and 1.16 g of TBTU (1.2 eq.) were weighed and added to a 100mL round-bottom flask. 15.0 mL of DMF was added. The reaction solution was stirred for 10 minutes in an ice bath, and then 1.0 mL of diisopropylethylamine (2.0 eq.) was added dropwise. After the dropwise addition, the reaction solution was continuously stirred for 3 hours in the ice bath. After the reaction was completed, 30 mL of $H_2O$ and 30 mL of ethyl acetate were added to the reaction system and the thus obtained system was stirred for 5 minutes. The reaction solution was left to stand, the organic phases were separated, the aqueous phase was continuously washed with 25 mL×2 of ethyl acetate twice, and the organic phases were combined and washed with saturated salt solution, dried with anhydrous $Na_2SO_4$ for 5 minutes, and then filtered. The filtrate was concentrated to dryness and purified by column chromatography to obtain 0.70 g of white solid compound 19-C with a yield of 51.9%. MS m/z (ESI): 451.0664 $(M+H)^+$.

Compound 19-D:

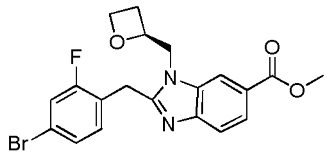

19-D

0.32 g of compound 19-C was weighed and added to a 50mL round-bottom flask. 5.0mL of 1,2-dichloroethane was added, and then 0.81 mL of acetic acid (20.0 eq.) was dropwise added. After the dropwise addition, the reaction solution was heated for reaction at 60°C. After reaction for 5 hours, the reaction solution was cooled to room temperature, and then the reaction solution was concentrated. After the concentration, 25.0 mL of ethyl acetate was added to the residue, then 30.0 mL of 3% $K_2CO_3$ aqueous solution was added under stirring. Subsequently, the reaction solution was left to stand, the organic phases were separated, the aqueous phase was continuously washed with 15 mL×2 of ethyl acetate twice, and the organic phases were combined and washed with saturated salt solution, dried with anhydrous $Na_2SO_4$ for 5 minutes, and then filtered. The filtrate was concentrated to dryness to obtain 0.31 g of compound 19-D with a yield of 100% (crude product), MS m/z (ESI): 433.0565$(M+H)^+$, which was directly used in the next reaction without purification.

Compound 19-E:

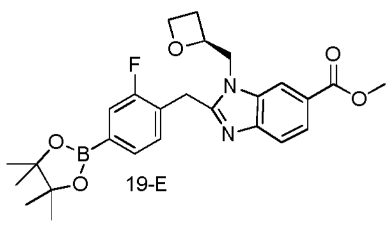

19-E

0.31 g of compound 19-D (1.0 eq.), 0.22 g of bis(pinacolato)diboron (1.2 eq.), 16.0 mg of Pd(dppf)$Cl_2$ (0.03 eq.) and 176 mg of potassium acetate (2.5 eq.) were weighed and added to a 50mL round-bottom flask. 12.0 mL of 1,4-dioxane was added. Subsequently, the reaction solution was degassed with nitrogen twice, and heated to 100°C for reaction under nitrogen atmosphere. After reaction for 2 hours, the heating was stopped, and the reaction solution was cooled to room

temperature and was filtered by a funnel with diatomite, and the filter cake was washed with 20 mL×2 of ethyl acetate. Subsequently, the filtrate was concentrated to dryness and purified by column chromatography to obtain 0.16 g of compound 19-E with a yield of 47.6%.

Compound 19-F:

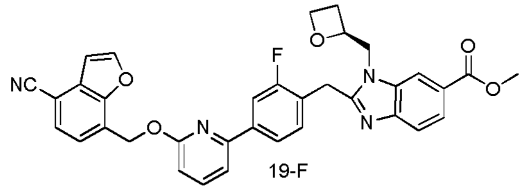

19-F

0.16 g of compound 19-E (1.05 eq.), 0.104 g of compound 8-A (1.0 eq.), 8.0 mg of Pd(dppf)$Cl_2$ (0.03 eq.) and 260 mg of $Cs_2CO_3$ (2.5 eq.) were weighed and added to a 50mL round-bottom flask. 5.0 mL of 1,4-dioxane and 2.0mL of $H_2O$ were added. Subsequently, the reaction solution was degassed with nitrogen twice, and heated to 90°C for reaction under nitrogen atmosphere. After reaction for 2 hours, the heating was stopped, the reaction solution was cooled to room temperature and was filtered by a funnel with diatomite, and the filter cake was washed with 10 mL×2 of ethyl acetate. Subsequently, the filtrate was concentrated to dryness and purified by column chromatography to obtain 0.12 g of compound 19-F with a yield of 62.8%.

Compound 19:

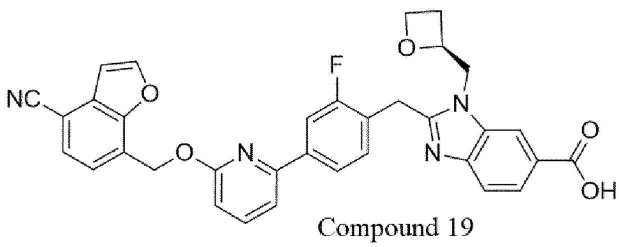

Compound 19

0.12 g of compound 19-F (1.0 eq.) and 55 mg of TBD (2.0 eq.) were weighed and added to a 25mL round-bottom flask. 2.5 mL of acetonitrile and 0.5 mL of water were added. The reaction mixture reacted for about 36 hours at room temperature until TLC showed that the reaction was completed. The reaction solution was concentrated and 5 mL of water was added, and then the pH of the reaction solution was adjusted to be 4-5 with citric acid solution. A large amount of off-white solids were precipitated, then the reaction solution was continuously stirred for about 0.5 hours, filtered and washed. The filter cake was recrystallized with dichloromethane/methanol to obtain 74 mg of compound 19 with a yield of 58.3%. MS m/z (ESI):589.1881 $(M+H)^+$; $^1$H NMR (400 MHz, DMSO) δ =12.85 (s, 1H), 8.36 (d, *J* = 1.9 Hz, 1H), 8.25 (s, 1H), 7.93 - 7.74 (m, 5H), 7.69 - 7.55 (m, 3H), 7.40 (t, *J* = 8.0 Hz, 1H), 7.24 (d, *J* = 2.0 Hz, 1H), 6.94 (d, *J* = 8.2 Hz, 1H), 5.88 (d, *J* = 11.9 Hz, 2H), 5.07 (dd, *J* = 15.9, 9.4 Hz, 1H), 4.80 - 4.26 (m, 6H), 2.69 (dd, *J* = 16.6, 8.7 Hz, 1H), 2.37 (dd, *J* = 18.2, 8.3 Hz, 1H).

Example 16:

Synthesis of Compound 21:

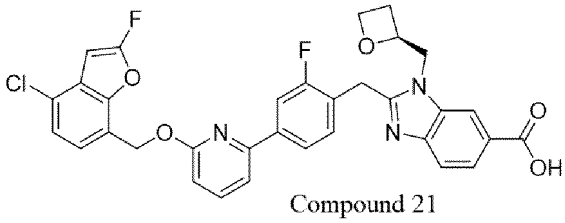

Compound 21

Synthesis route:

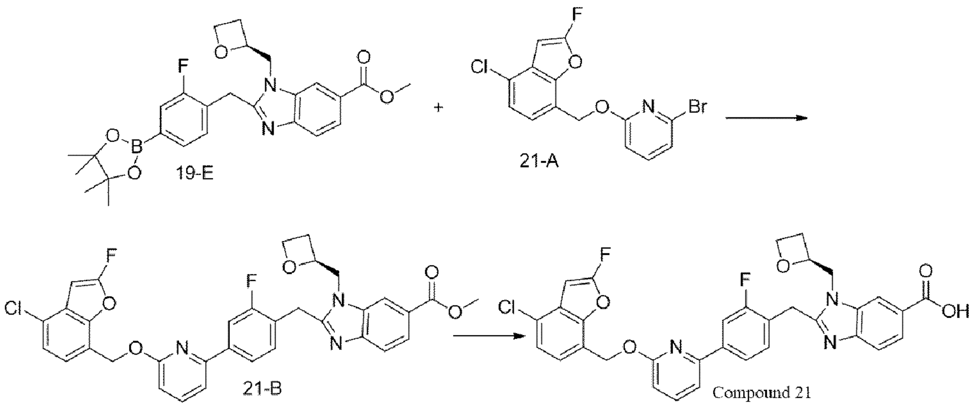

19-E 21-A 21-B Compound 21

Referring to the synthesis method of compound 19, wherein compound 8-A was replaced with compound 21-A to finally obtain compound 21. MS m/z (ESI): 616.1447 $(M+H)^+$, $^1H$ NMR (400 MHz, DMSO-d6) δ=12.74 (brs, 1H), 8.25 (s, 1H), 7.86-7.79 (m, 4H), 7.61 (dd, J = 15.6, 7.2 Hz, 2H), 7.46 (d, J = 8.4 Hz, 1H), 7.41 (t, J = 8.4 Hz, 2H), 6.90 (d, J = 8.4 Hz, 1H), 6.51 (d, J = 6.4 Hz, 1H), 5.71 (s, 2H), 5.07-5.02 (m, 1H), 4.72 (dd, J = 15.6, 7.2 Hz, 1H), 4.61-4.33 (m, 5H), 2.74-2.66 (m, 1H), 2.42-2.38 (m, 1H).

Example 17:

Synthesis of Compound 23:

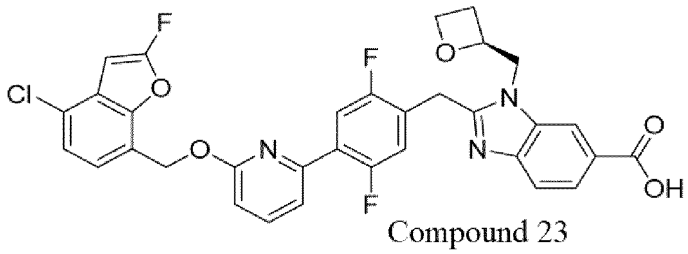

Compound 23

Synthesis route:

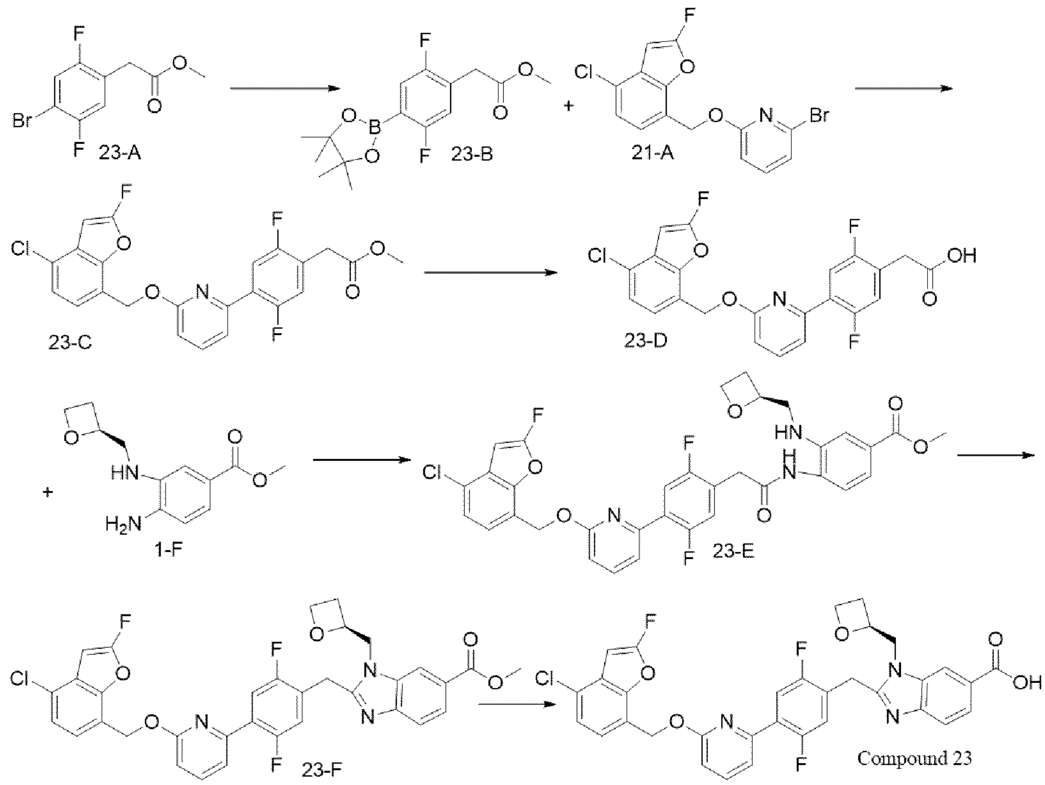
23-A
23-B
21-A
23-C
23-D
1-F
23-E
23-F
Compound 23

Referring to the synthesis method of compound 17, wherein compound 17-B was replaced with compound 23-B and compound 7-A was replaced with compound 21-A to finally obtain compound 23. MS m/z (ESI): 634.1352 $(M+H)^+$.

Example 18:

Synthesis of Compound 24:

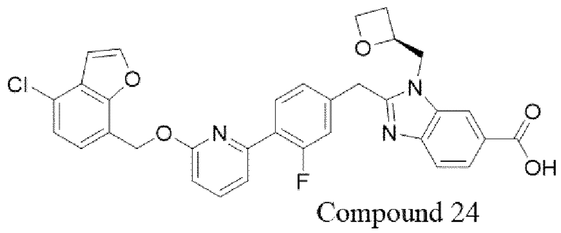

Compound 24

Synthesis route:

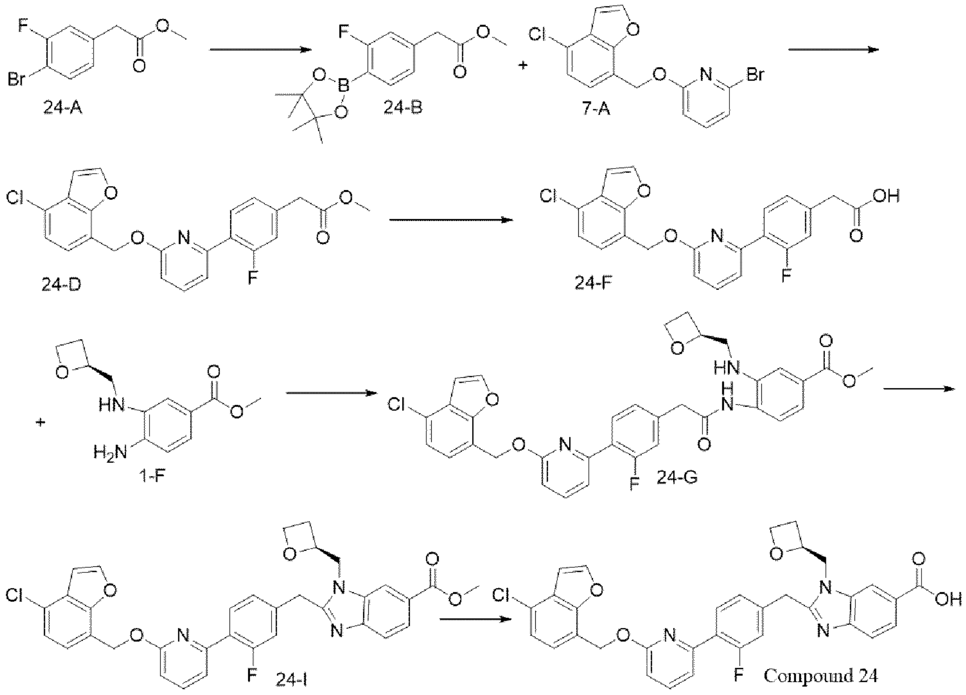

24-A
24-B
7-A
24-D
24-F
1-F
24-G
24-I
Compound 24

Referring to the synthesis method of compound 17, wherein compound 17-A was replaced with compound 24-A to finally obtain compound 24. MS m/z (ESI): 598.1541(M+H)$^+$. $^1$H NMR (400 MHz, DMSO-$d_6$) δ=12.34 (brs, 1H), 8.25 (s, 1H), 8.19 (d, J = 1.8 Hz, 1H), 7.89 - 7.73 (m, 4H), 7.67-7.64 (m, 1H), 7.58 (d, J = 8.4 Hz, 1H), 7.49 (d, J = 8.0 Hz, 1H), 7.45 - 7.32 (m, 3H), 7.06 (d, J = 1.8 Hz, 1H), 5.85 (s, 2H), 5.05 (d, J = 5.3 Hz, 1H), 4.74-4.69 (m, 1H), 4.64 - 4.23 (m, 5H), 2.72-2.65(m, 1H), 2.45 - 2.25 (m, 1H).

Example 19:

Synthesis of Compound 25:

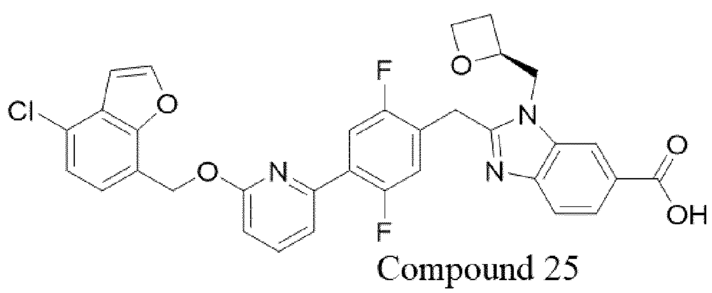

Compound 25

Synthesis route:

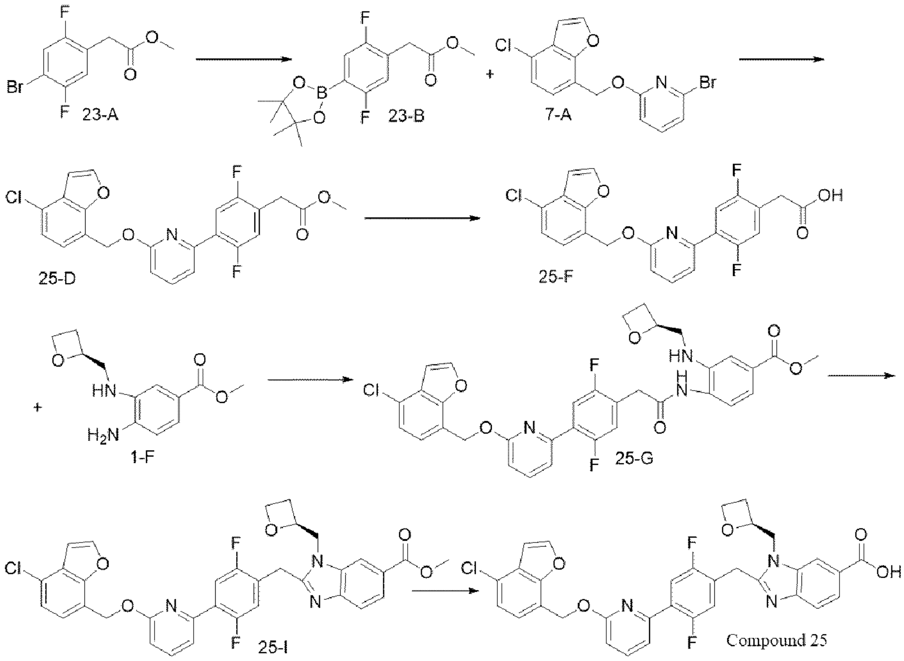
23-A 23-B 7-A 25-D 25-F 1-F 25-G 25-I Compound 25

Referring to the synthesis method of compound 17, wherein compound 17-A was replaced with compound 23-A to finally obtain compound 25. MS m/z (ESI): 616.1447(M+H)$^+$. $^1$H NMR (400 MHz, DMSO-$d_6$) δ =12.65 (s, 1H), 8.21 (s, 1H), 8.20 (d, *J* = 2.0 Hz, 1H), 7.91-7.76 (m, 4H), 7.61 (dd, *J* = 21.2, 8.0 Hz, 2H), 7.52 - 7.35 (m, 2H), 7.06 (d, *J* = 2.4 Hz, 1H), 6.87 (d, *J* = 8.0 Hz, 1H), 5.75 (s, 2H), 5.03 (d, *J* = 4.8 Hz, 1H), 4.71 (dd, *J* = 15.5, 7.0 Hz, 1H), 4.65 - 4.35 (m, 5H), 2.70 (m, 1H), 2.44 - 2.35 (m, 1H).

Example 20:

Synthesis of Compound 26:

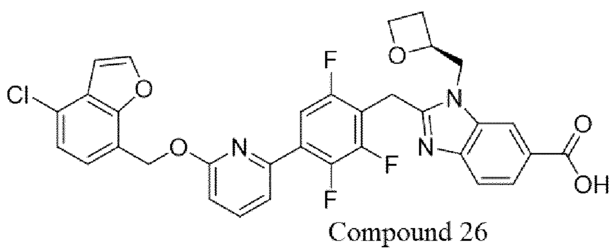

Compound 26

Synthesis route:

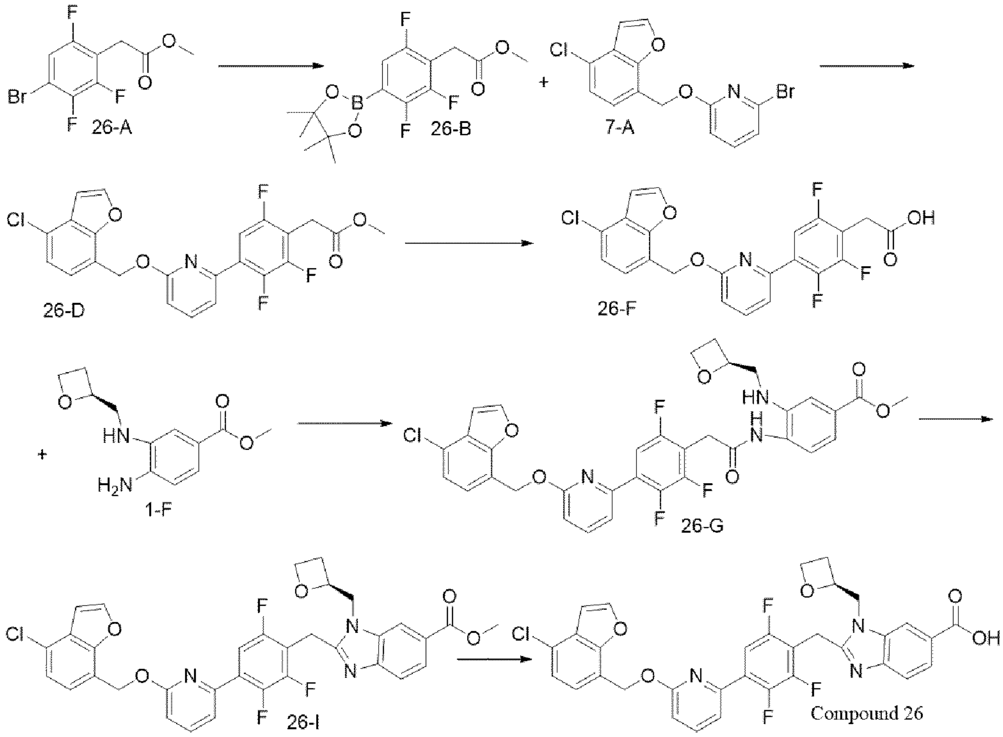

26-A 26-B 7-A 26-D 26-F 1-F 26-G 26-I Compound 26

Referring to the synthesis method of compound 17, wherein compound 17-A was replaced with compound 26-A to finally obtain compound 26. MS m/z (ESI): 634.1353 $(M+H)^+$.

Example 21:

Synthesis of Compound 27:

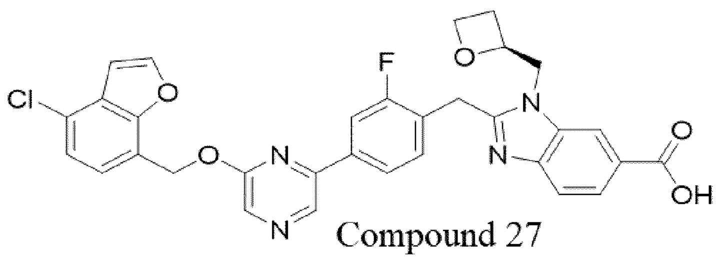

Compound 27

Synthesis route:

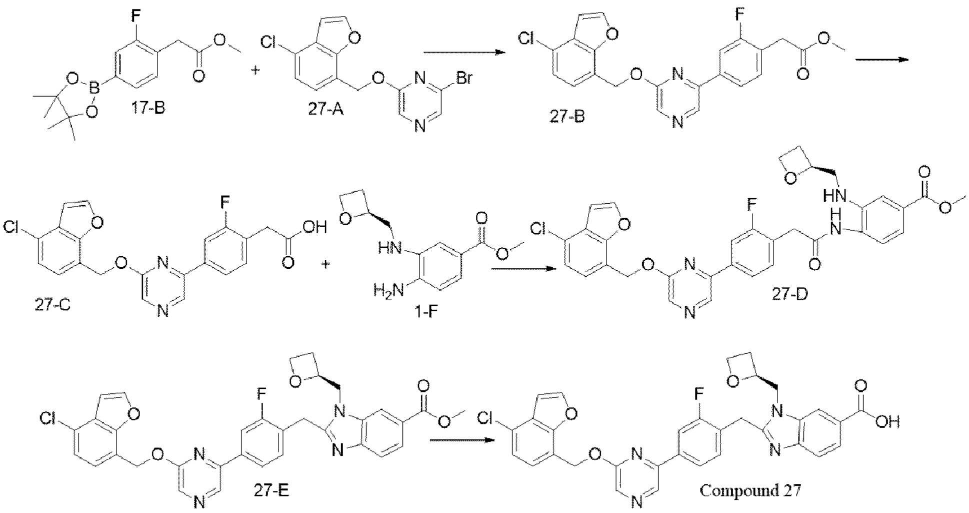
17-B
27-A
27-B
27-C
1-F
27-D
27-E
Compound 27

Referring to the synthesis of compound 17, wherein compound 7-A was replaced with compound 27-A to finally obtain compound 27 (200 mg, yield 66.0%).MS m/z (ESI): 599.1493 $(M+H)^+$, $^1H$ NMR (400 MHz, DMSO) δ=12.71 (s, 1H), 8.91 (s, 1H), 8.37 (s, 1H), 8.26 (s, 1H), 8.20 (s, 1H), 8.02 - 7.89 (m, 2H), 7.79 (d, J = 8.4 Hz, 1H), 7.59 (d, J = 8.4 Hz, 1H), 7.51 (t, J = 7.7 Hz, 2H), 7.38 (dd, J = 8.0, 1.8 Hz, 1H), 7.07 (t, J = 2.1 Hz, 1H), 5.82 (s, 2H), 5.06 (td, J = 9.0, 6.3 Hz, 1H), 4.74 (dd, J = 15.6, 7.0 Hz, 1H), 4.65 - 4.55 (m, 1H), 4.55 - 4.30 (m, 4H), 2.71 (tt, J = 15.1, 7.4 Hz, 1H), 2.39 (m, 1H).

Example 22:

Synthesis of Compound 31:

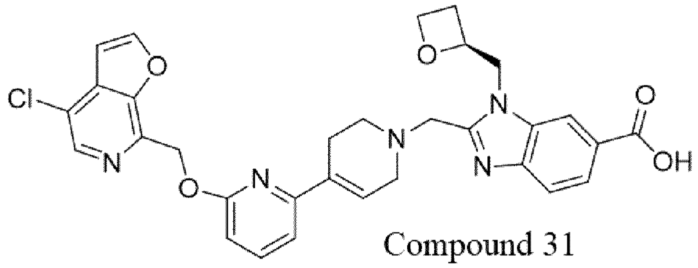
Compound 31

Synthesis route:

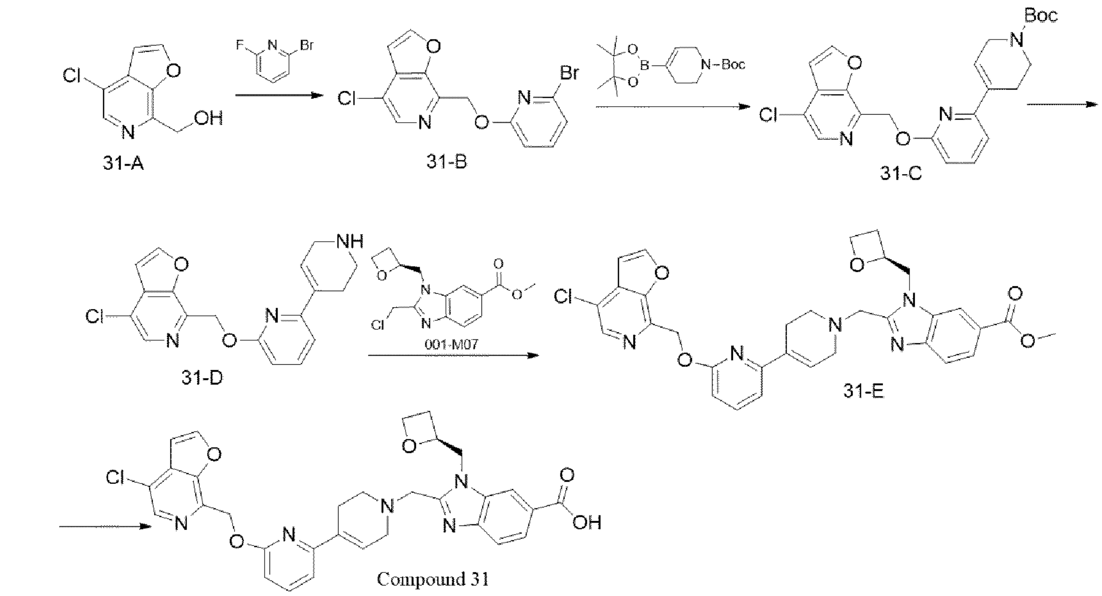

31-A 31-B 31-C 31-D 31-E Compound 31

Referring to the synthesis method of compound 13, wherein compound 13-A was replaced with compound 31-A to finally obtain compound 31. MS m/z (ESI): 586.1853 $(M+H)^+$.

Example 23:

Synthesis of Compound 38:

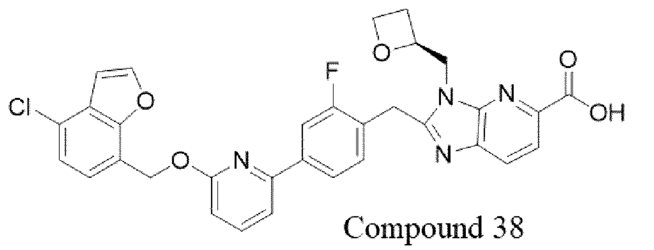

Compound 38

Synthesis route:

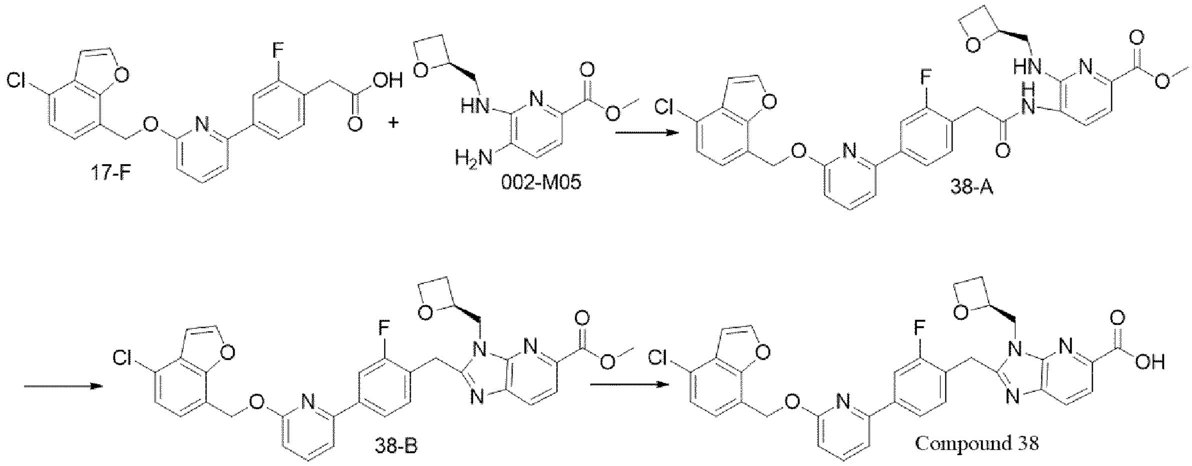
17-F 002-M05 38-A 38-B Compound 38

Compound 38-A:

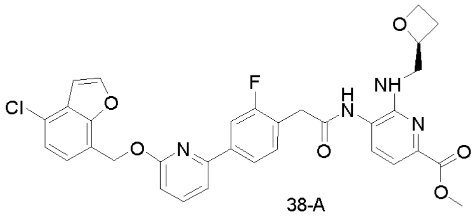

38-A

0.2 g of compound 17-F (1.0 eq.) and 0.127 g of compound 002-M05 (1.1 eq.) were weighed and added to a 25mL round-bottom flask. 4.0 mL of acetonitrile was added. The reaction solution was stirred for 15 minutes in an ice bath, and then 0.15 g of N,N,N',N'-tetramethylchloroformamidinium hexafluorophosphate (1.1 eq.) and 84 mg of N-methylimidazole (2.1 eq.) were weighed and added. The reaction solution was continuously stirred for 0.5 hours in the ice bath. Subsequently, the reaction solution was transferred to room temperature and continuously reacted for 3 hours. After the reaction was completed, the reaction system was added with 12 mL of $H_2O$, stirred for 0.5 hours, and then filtered. The filter cake was washed with 4 mL×3 of water, and purified by column chromatography to obtain 0.122 g of compound 38-A with a yield of 39.8%.

Compound 38-B:

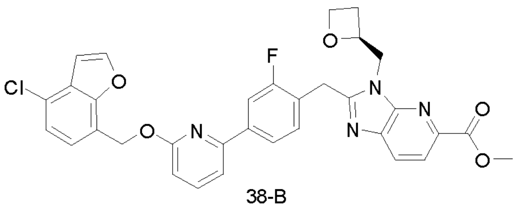

38-B

0.12 g of compound 38-A (1.0 eq.) was weighed and added to a 50mL round-bottom flask. 1.8 mL of 1,4-dioxane was added. 0.47 g of acetic acid (40.0 eq.) was then added dropwisae. After the dropwise addition, the reaction solution was heated for reaction at 80°C. After 3 hours, the reaction solution was cooled to room temperature, added with 5 mL of water and 10 mL of ethyl acetate, and the pH of the reaction solution was adjusted to be 7-8 with 5% $NaHCO_3$ solution. Then, the reaction solution was left to stand, the organic phases were separated, the aqueous phase was continuously washed with 10 mL×2 of ethyl acetate twice, and the organic phases were combined and washed with saturated salt solution, dried with anhydrous $Na_2SO_4$ for 5 minutes, and then filtered. The filtrate was concentrated to dryness and purified by column chromatography to obtain 90 mg of compound 38-B with a yield of 77.2%.

Compound 38:

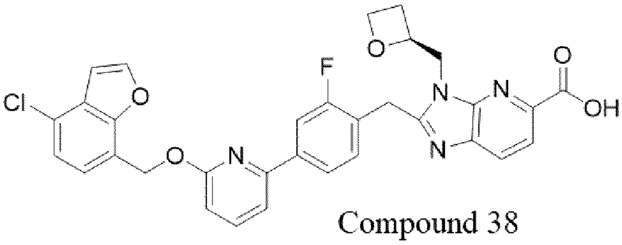

Compound 38

85 mg of compound 38-B (1.0 eq.) was weighed and added to a 25mL round-bottom flask. 0.5 mL of methanol and 1.3 mL of tetrahydrofuran were added. 0.14 mL of 2 mol/L sodium hydroxide solution was then added. Subsequently, the reaction solution was reacted at room temperature. After 15 hours, the reaction solution was concentrated. After the concentration, 2.5 mL of water was added to the residue, and the pH of the reaction system was adjusted to be 4-5 with 1 mol/L HCl solution. Subsequently, the reaction solution was filtered, and the filter cake was purified by column chromatography to obtain 32 mg of compound 38 with a yield of 38.5%. MS m/z (ESI): 599.1494 $(M+H)^+$, $^1H$ NMR (400 MHz, DMSO-$d_6$) δ= 9.08 (s, 1H), 8.17 (s, 1H), 8.05 (d, *J* = 7.6 Hz, 1H), 7.98 (d, *J* = 8.0 Hz, 1H), 7.89-7.82 (m, 2H), 7.49-7.36 (m, 2H), 7.14-7.07 (m, 2H), 6.99 (d, *J* = 7.6 Hz, 1H), 6.89 (d, *J* = 7.6 Hz, 1H), 6.70 (m, 1H), 5.78 (s, 2H), 5.15 (s,

1H), 4.76-4.37 (m, 4H), 3.99-3.96 (m, 1H), 2.76-2.67 (m, 1H), 2.04-1.96 (m, 1H).

Example 24:

Synthesis of Compound 39:

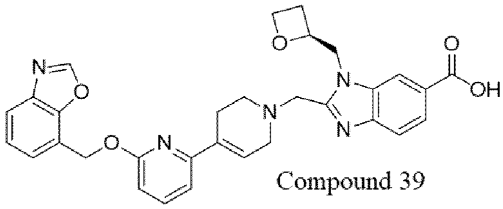

Compound 39

Synthesis route:

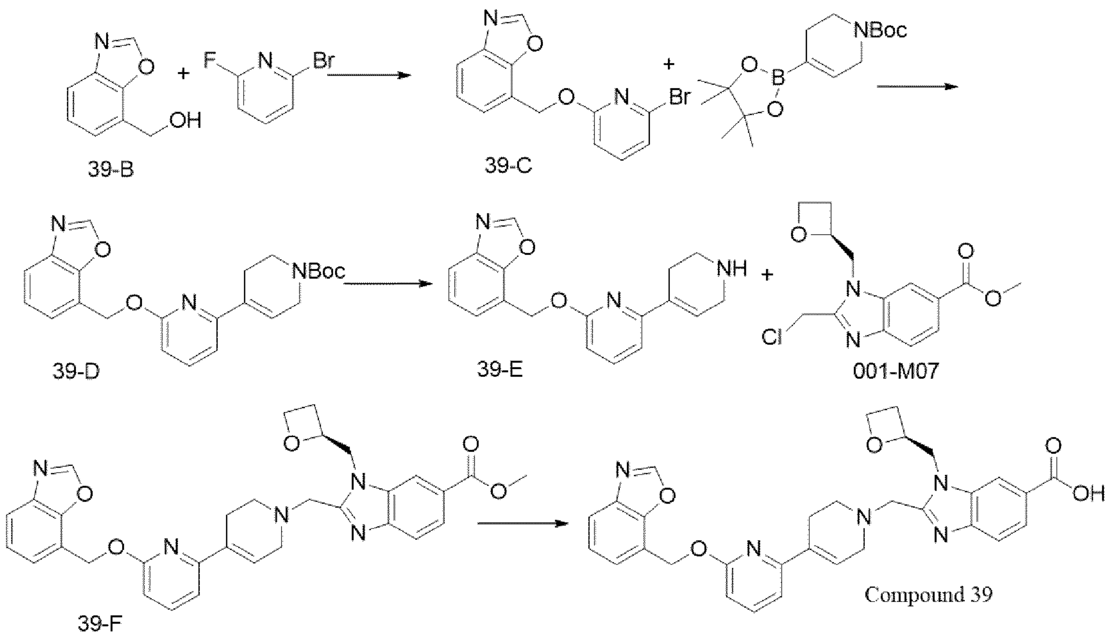

39-B

39-C

39-D

39-E

001-M07

39-F

Compound 39

Referring to the synthesis method of compound 13, wherein compound 13-B was replaced with compound 39-C to finally obtain compound 39.MS m/z (ESI): 552.2243 $(M+H)^+$.

Example 25:

Synthesis of Compound 45:

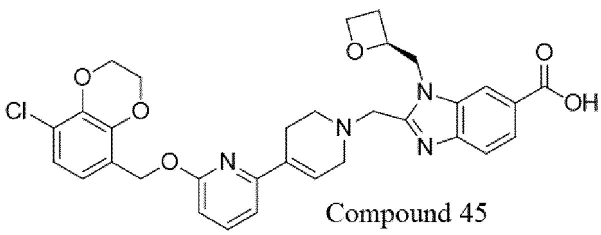

Compound 45

Synthesis route:

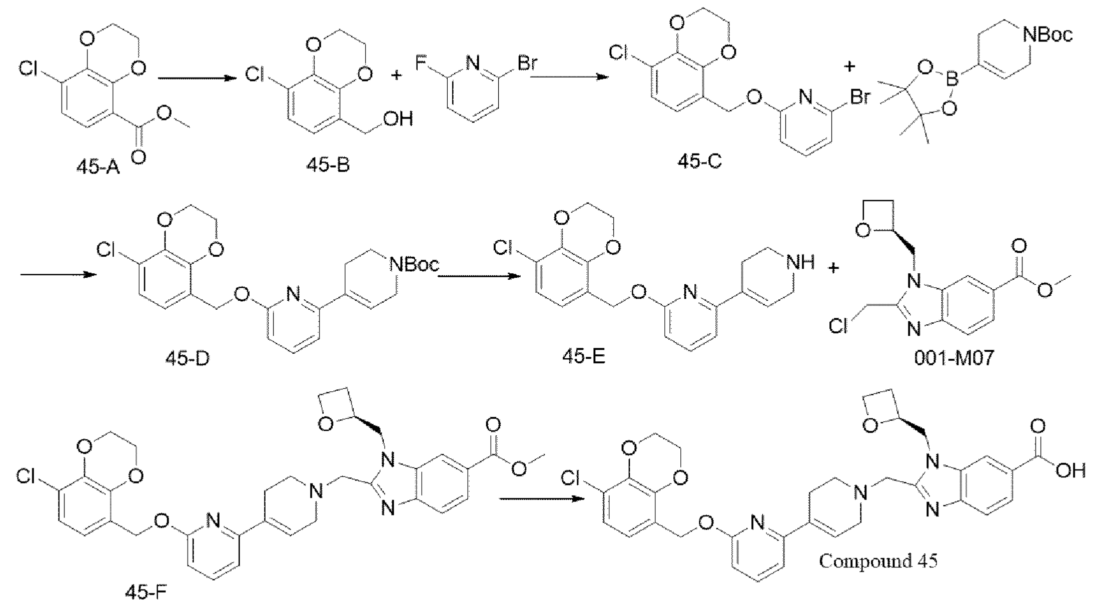

45-A 45-B 45-C

45-D 45-E 001-M07

45-F Compound 45

Referring to the synthesis method of compound 13, wherein compound 13-B was replaced with compound 45-C to finally obtain compound 45. MS m/z (ESI): 603.2007 (M+H)+.

Example 26:

Synthesis of Compound 61:

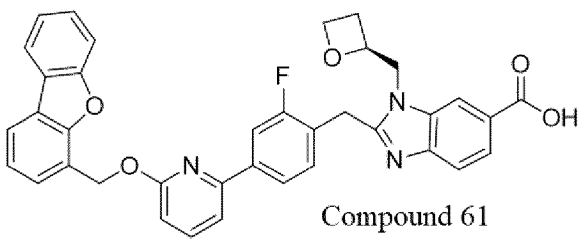

Compound 61

Synthesis route:

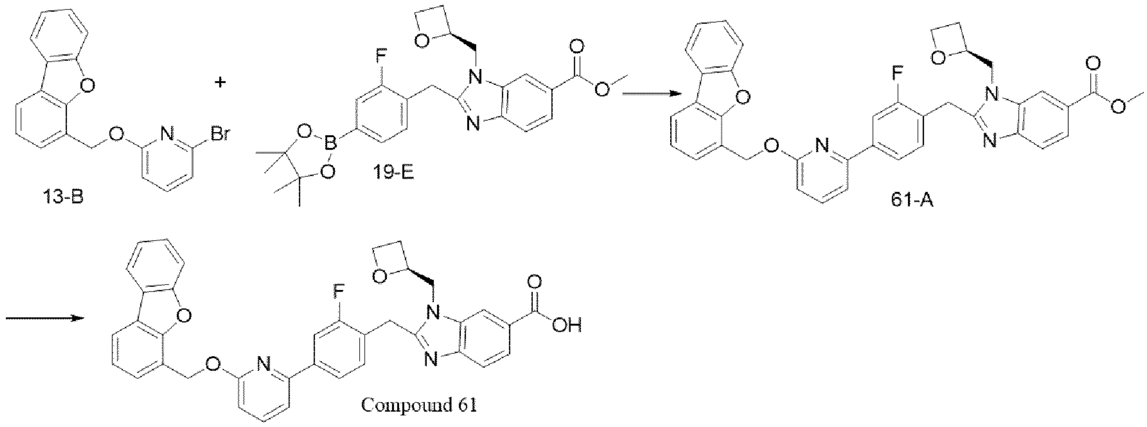

13-B 19-E 61-A

Compound 61

Compound 61-A:

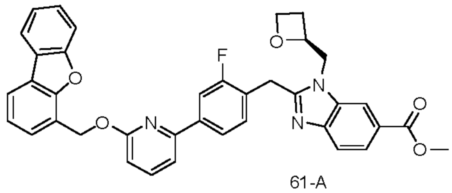

61-A

200 mg of compound 19-E (1.0 eq.), 155 mg of compound 13-B (1.05 eq.), 9 mg of Pd(dppf)$Cl_2$ (0.03 eq.) and 339 mg of cesium carbonate (2.5 eq.) were weighed and added to a 25mL round-bottom flask. 5 mL of 1,4-dioxane and 1 mL of water were added. The reaction solution was stirred evenly and then degassed with nitrogen twice, and heated to 90°C under nitrogen atmosphere for about 2 hours until TLC showed that the reaction was completed. Subsequently, the reaction solution was cooled to room temperature and was filtered by a funnel with diatomite, the filter cake was washed with a small amount of dichloromethane, and the filtrate was concentrated to dryness and separated and purified by column chromatography to obtain 131 mg of compound 61-A with a yield of 50.2%. MS m/z (ESI): 628.2249 $(M+H)^+$.

Compound 61:

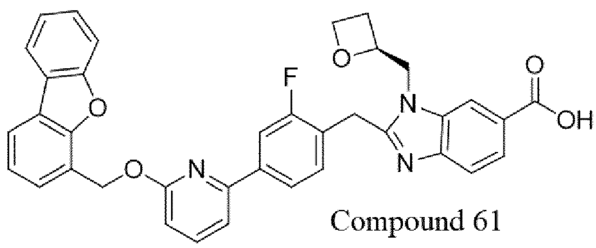

Compound 61

126 mg of compound 61-A (1.0 eq.) was weighed and added to a 25mL round-bottom flask. 3 mL of tetrahydrofuran and 1 mL of methanol were added, the thus obtained system was stirred evenly, and 0.2 mL of 2 mol/L NaOH solution (2.0 eq.) was slowly dropwise added, and then the thus obtained system was continuously reacted overnight at room temperature until TLC showed that the reaction was completed. The reaction solution was concentrated, and about 10 mL of water was added. The pH of the reaction solution was adjusted to be 4-5 with citric acid solution, and a large amount of off-white solids were precipitated. The reaction solution was continuously stirred for about 0.5 hours, filtered, and washed with water. The filter cake was recrystallized with dichloromethane/methanol to obtain 79 mg of compound 61 with a yield of 64.2%. MS m/z (ESI): 614.2094 $(M+H)^+$; $^1$H NMR (400 MHz, DMSO) $\delta$ =12.82 (s, 1H), 8.26 (s, 1H), 8.13 (dd, $J$ = 12.6, 7.6 Hz, 2H), 7.91 (d, $J$ = 11.5 Hz, 1H), 7.82 (dd, $J$ = 13.6, 5.7 Hz, 3H), 7.74 (d, $J$ = 8.2 Hz, 1H), 7.70 - 7.56 (m, 3H), 7.42 (ddd, $J$ = 23.4, 15.3, 7.4 Hz, 3H), 7.29 (t, $J$ = 8.0 Hz, 1H), 6.91 (d, $J$ = 8.2 Hz, 1H), 5.86 (s, 2H), 5.04 (d, $J$ = 5.4 Hz, 1H), 4.70 (dd, $J$ = 15.6, 7.0 Hz, 1H), 4.62 - 4.29 (m, 5H), 2.75 - 2.58 (m, 1H), 2.45 - 2.27 (m, 1H).

Example 27:

Synthesis of Compound 66:

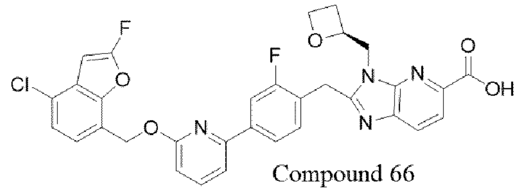

Compound 66

Synthesis route:

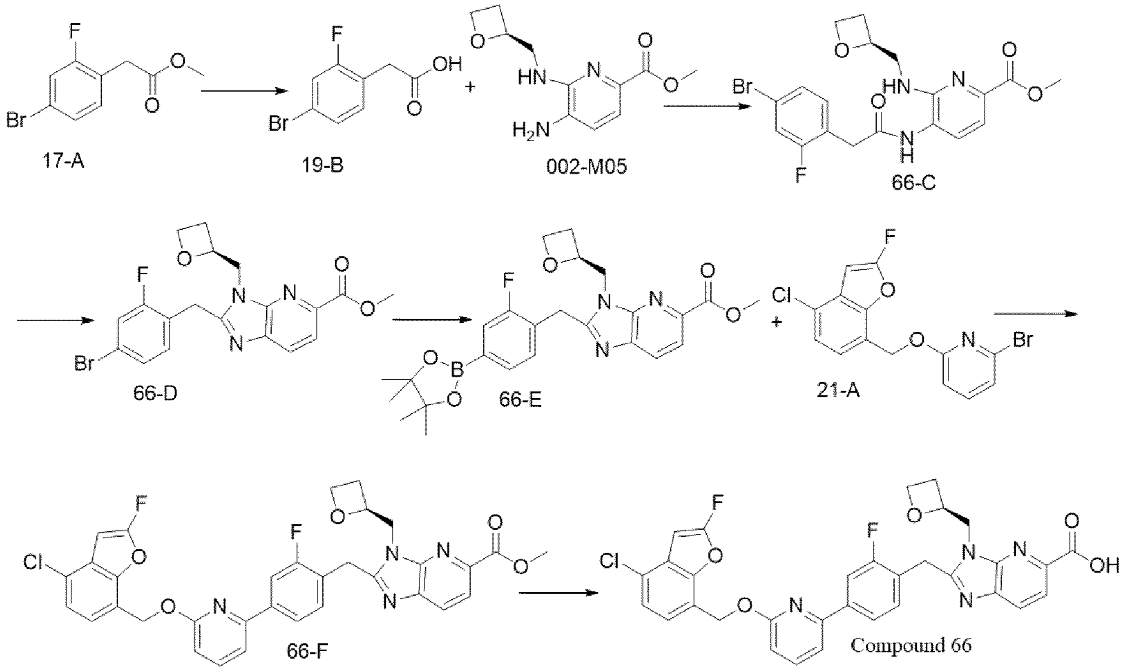

17-A 19-B 002-M05 66-C 66-D 66-E 21-A 66-F Compound 66

Referring to the synthesis method of compound 21, wherein compound 19-E was replaced with compound 66-E to finally obtain compound 66. MS m/z (ESI):617.1401(M+H)+.

Example 28:

Synthesis of Compound 67:

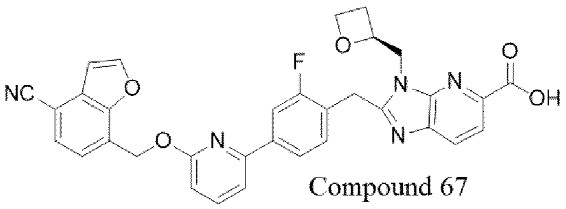

Compound 67

Synthesis route:

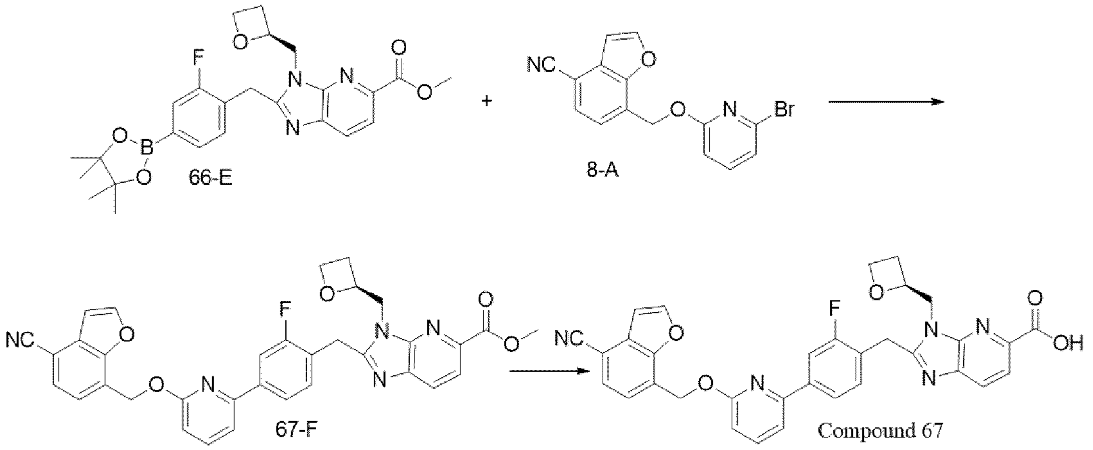

66-E

8-A

67-F

Compound 67

Referring to the synthesis method of compound 19, wherein compound 19-E was replaced with compound 66-E to finally obtain compound 67. MS m/z (ESI): 590.1831(M+H)$^+$. $^1$H NMR (400 MHz, DMSO-$d_6$) δ= 12.64 (brs, 1H), 8.25 (s, 1H), 8.11 (d, J = 7.6 Hz, 1H), 7.89 (d, J = 8.0 Hz, 1H), 7.84-7.81 (m, 2H), 7.49-7.36 (m, 2H), 7.14-7.07 (m, 2H), 6.93 (d, J = 7.6 Hz, 1H), 6.85 (d, J = 7.6 Hz, 1H), 6.70 (m, 1H), 5.78 (s, 2H), 5.15-5.13 (m, 1H), 4.76-4.60 (m, 1H), 4.57-4.33 (m, 5H), 2.76-2.67 (m, 1H), 2.01-1.93 (m, 1H).

Example 29:

Synthesis of Compound 71:

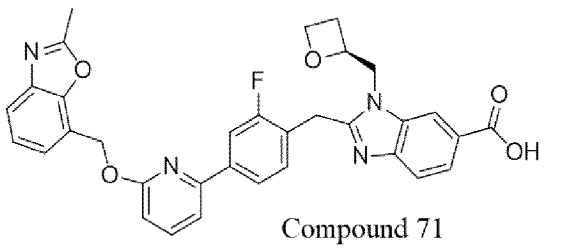

Compound 71

Synthesis route:

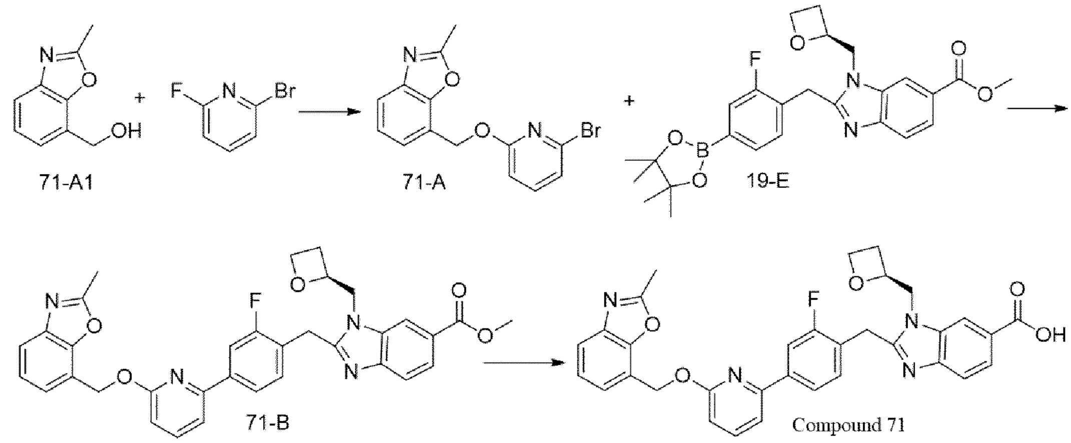

71-A1 71-A 19-E

71-B Compound 71

Referring to the synthesis method of compound 19, wherein compound 8-A was replaced with compound 71-A to finally obtain compound 71 (177 mg, yield 49.03%). MS m/z (ESI): 579.2039(M+H)$^+$, $^1$HNMR (400 MHz, DMSO-d6) δ=12.69 (s, 1H), 8.25 (s, 1H), 7.83 (m, 4H), 7.68-7.56 (m, 3H), 7.51-7.38 (m, 2H), 7.33 (t, J = 7.7 Hz, 1H), 6.90 (d, J = 8.2 Hz, 1H), 5.74 (s, 2H), 5.05 (dd, J = 15.0, 7.6 Hz, 1H), 4.73 (dd, J = 15.6, 7.1 Hz, 1H), 4.60 (dd, J = 15.8, 2.8 Hz, 1H), 4.55-4.40 (m, 3H), 4.36 (dt, J = 9.0, 5.8 Hz, 1H), 2.78-2.65 (m, 1H), 2.61 (s, 3H), 2.39 (m, 1H).

Example 30:

Synthesis of Compound 72:

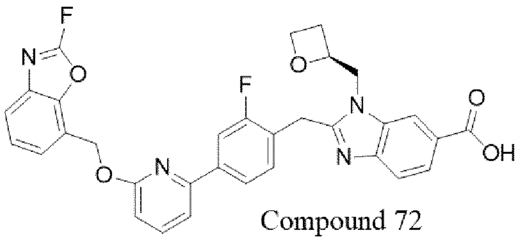

Compound 72

Synthesis route:

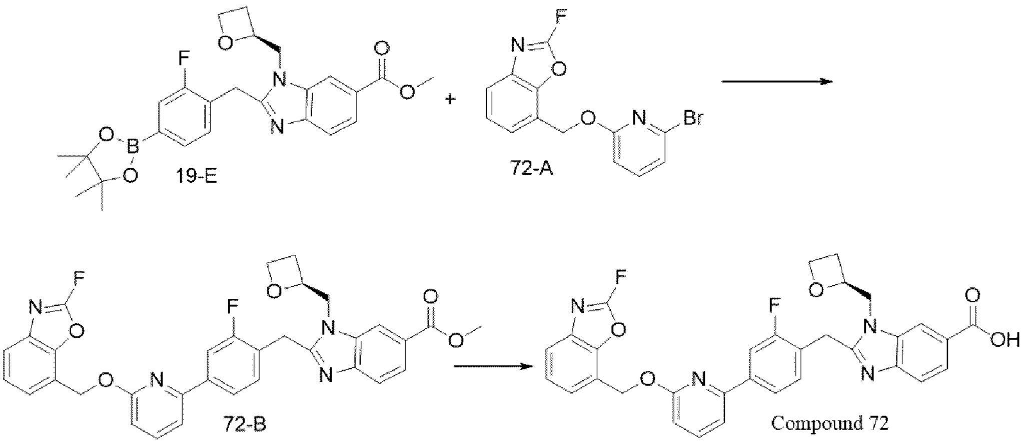

19-E 72-A

72-B Compound 72

Referring to the synthesis method of compound 19, wherein compound 8-A was replaced with compound 72-A to finally obtain compound 72. MS m/z (ESI): 583.1789(M+H)$^+$.

Example 31:

Synthesis of Compound 76:

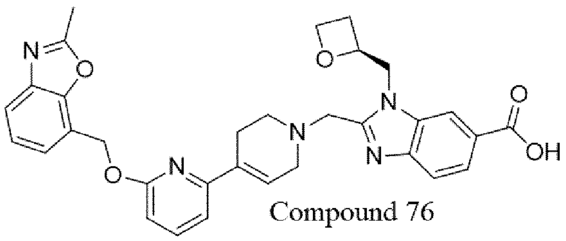

Compound 76

Synthesis route:

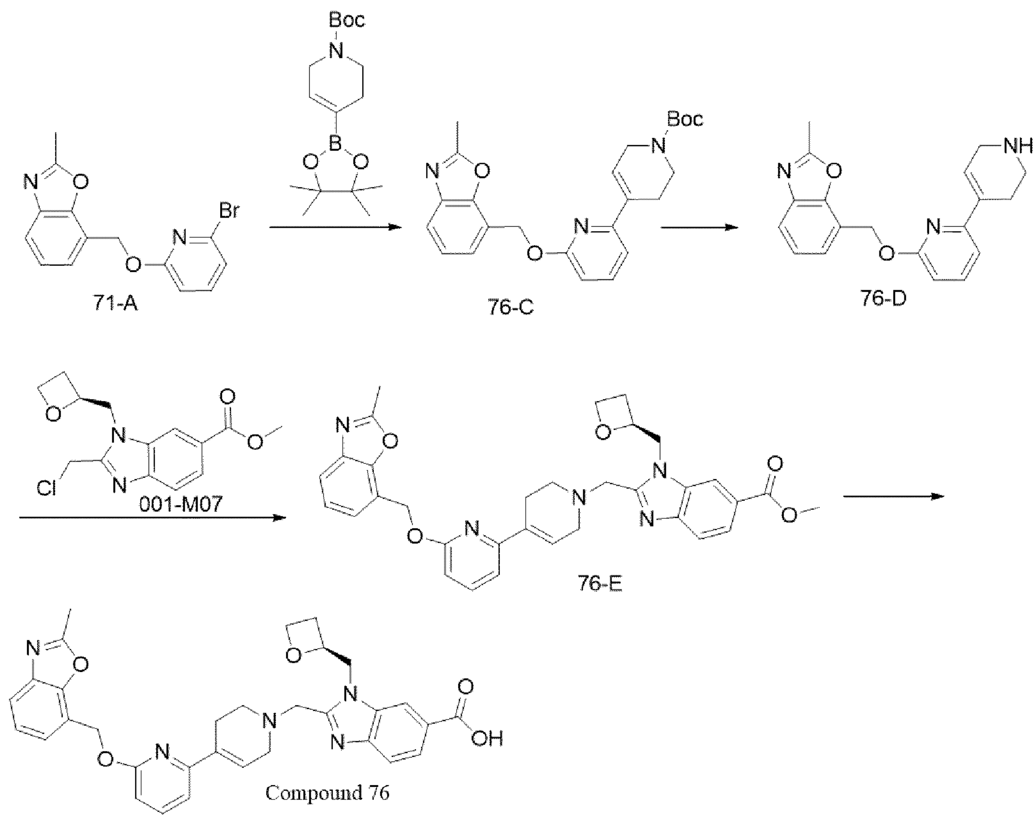

71-A

76-C

76-D

001-M07

76-E

Compound 76

Referring to the synthesis method of compound 13, wherein compound 13-B was replaced with compound 71-A to finally obtain compound 76. MS m/z (ESI): 566.2401(M+H)$^+$.

Example 32:

Synthesis of Compound 82:

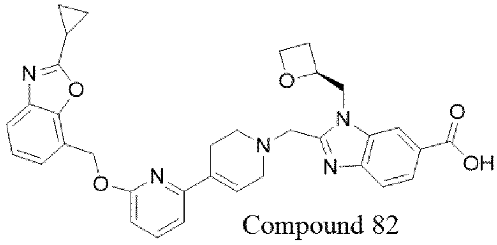

Compound 82

Synthesis route:

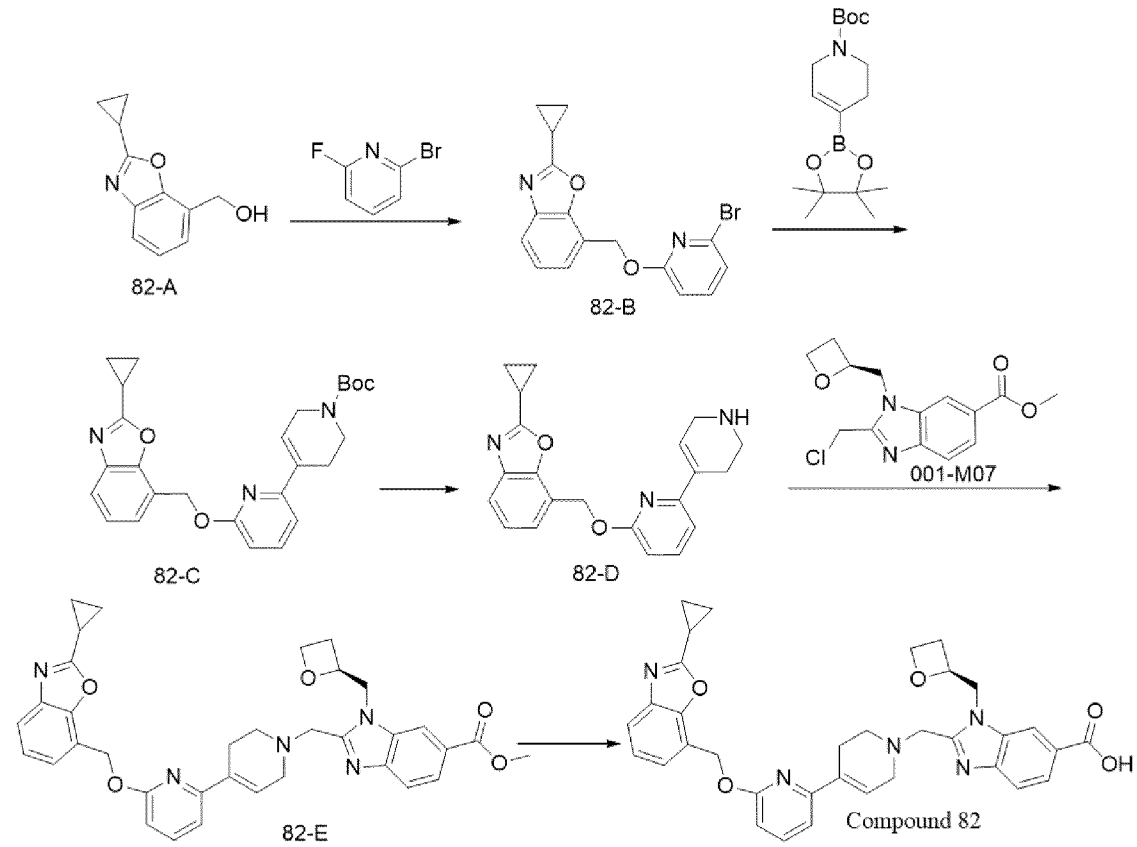


Referring to the synthesis method of compound 13, wherein compound 13-A was replaced with compound 82-A to finally obtain compound 82. MS m/z (ESI): 592.2553(M+H)$^+$.

Example 33:

Synthesis of Compound 83:

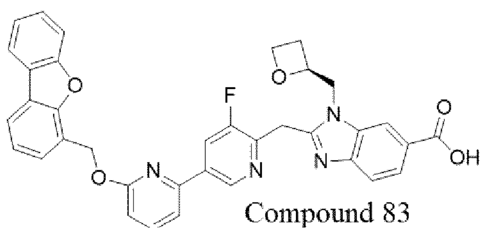

Compound 83

Synthesis route:

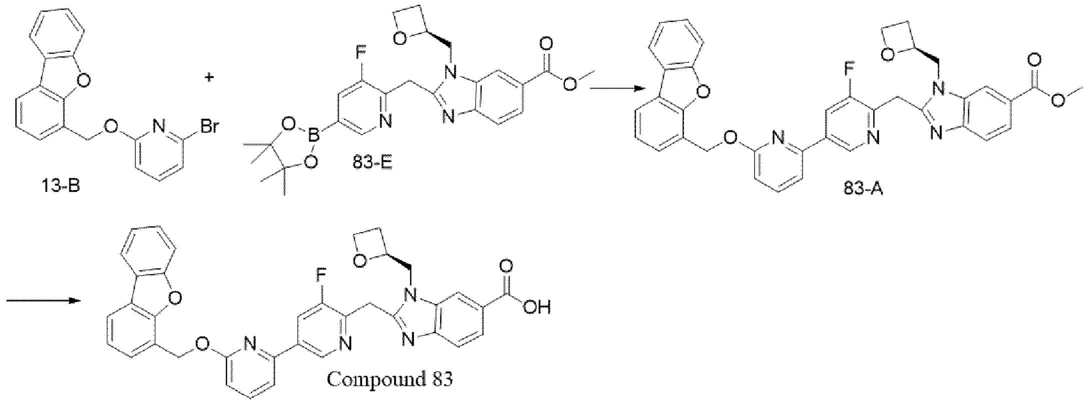


Referring to the synthesis method of compound 61, wherein compound 19-E was replaced with compound 83-E to finally obtain compound 83. MS m/z (ESI): 615.2039$(M+H)^+$.

Example 34:

Synthesis of Compound 84:

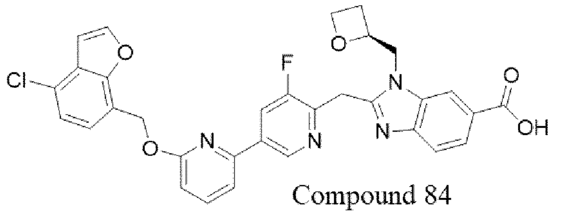

Compound 84

Synthesis route:

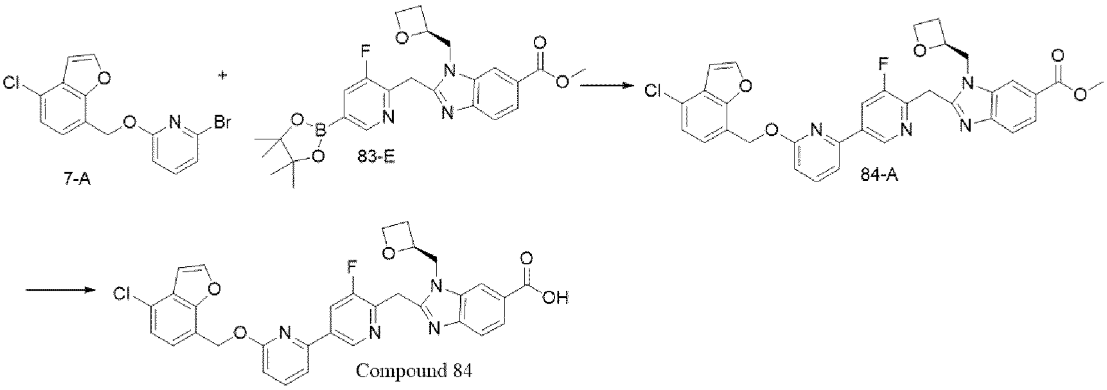


Referring to the synthesis method of compound 61, wherein compound 13-B was replaced with compound 7-A and compound 19-E was replaced with compound 83-E to finally obtain compound 84. MS m/z (ESI): 599.1492$(M+H)^+$.

Example 35:

Synthesis of Compound 87:

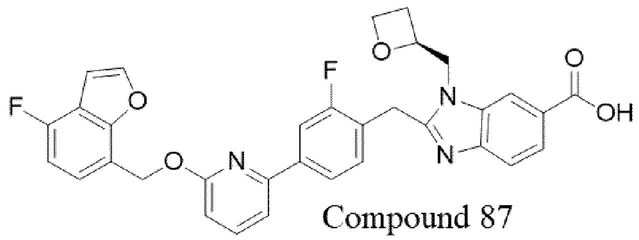

Compound 87

Synthesis route:

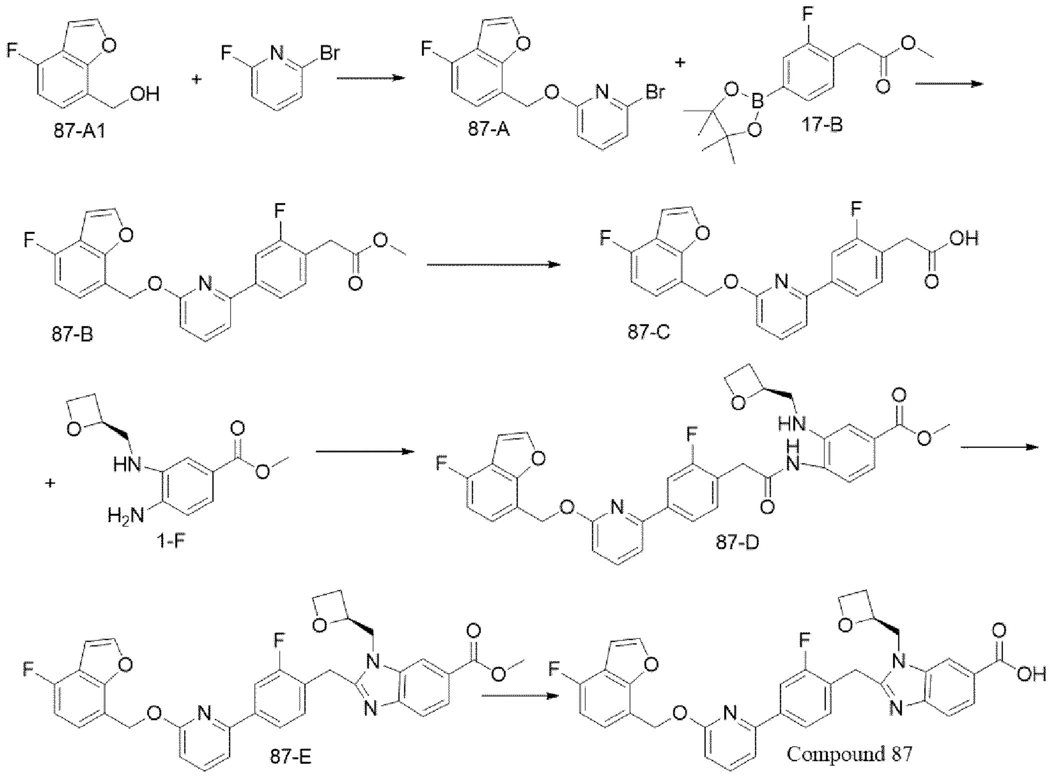


Referring to the synthesis method of compound 17, wherein compound 7-A was replaced with compound 87-A to finally obtain compound 87. MS m/z (ESI): 582.1837(M+H)$^+$, $^1$H NMR (400 MHz, DMSO) δ =8.23 (s, 1H), 8.12 (d, J = 2.0 Hz, 1H), 7.89-7.80 (m, 4H), 7.63 (d, J = 7.6 Hz, 1H), 7.55 (d, J = 8.4 Hz, 1H), 7.49 (dd, J = 8.4, 5.6 Hz, 1H), 7.42 (t, J = 8.0 Hz, 1H), 7.13-7.09 (m, 2H), 6.87 (d, J = 8.0 Hz, 1H), 5.75 (s, 2H), 5.08-5.03 (m, 1H), 4.70 (dd, J = 15.6, 6.8 Hz, 1H), 4.59-4.33 (m, 5H), 2.74-2.66 (m, 1H), 2.43-2.34 (m, 1H).

Example 36:

Synthesis of Compound 88:

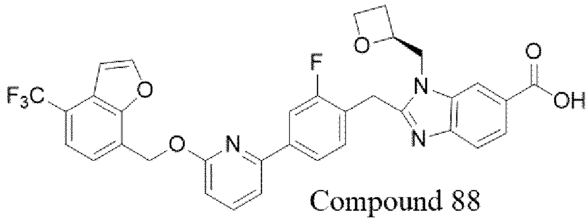

Compound 88

Synthesis route:

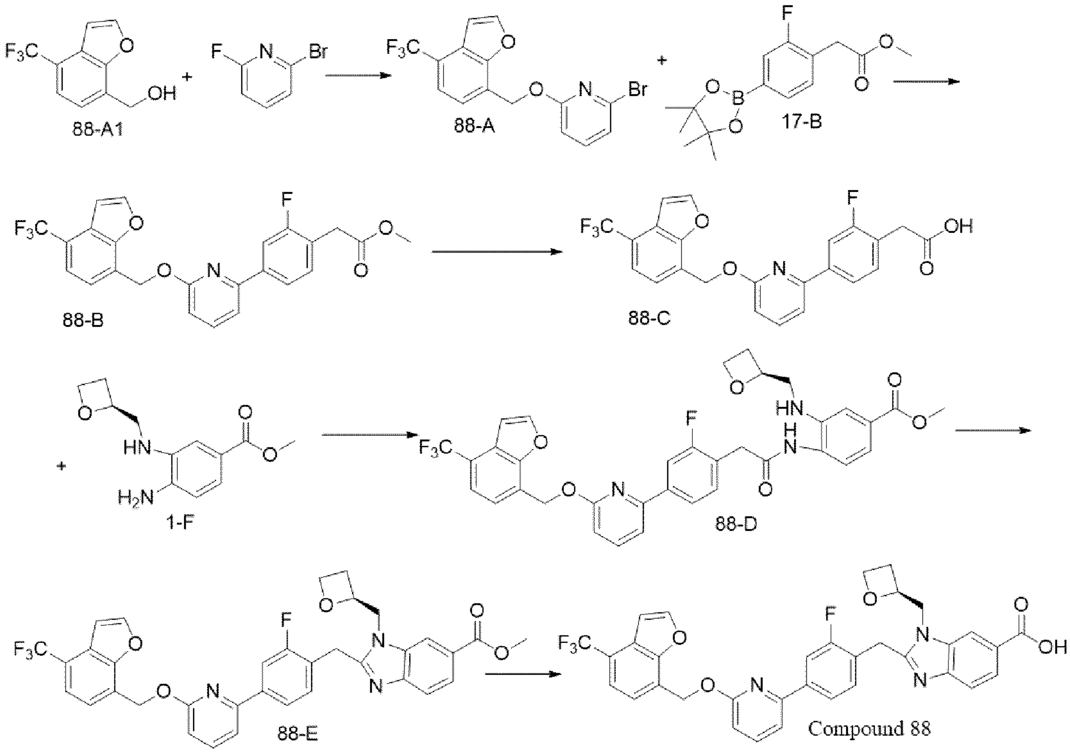


Referring to the synthesis method of compound 17, wherein compound 7-A was replaced with compound 88-A to finally obtain compound 88. MS m/z (ESI): 632.1802(M+H)$^+$.

Example 37:

Synthesis of Compound 89:

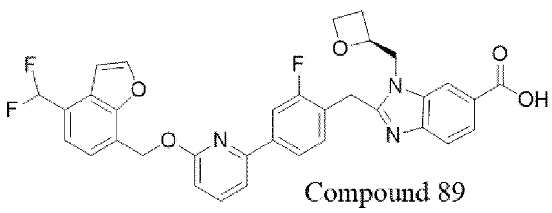

Compound 89

Synthesis route:

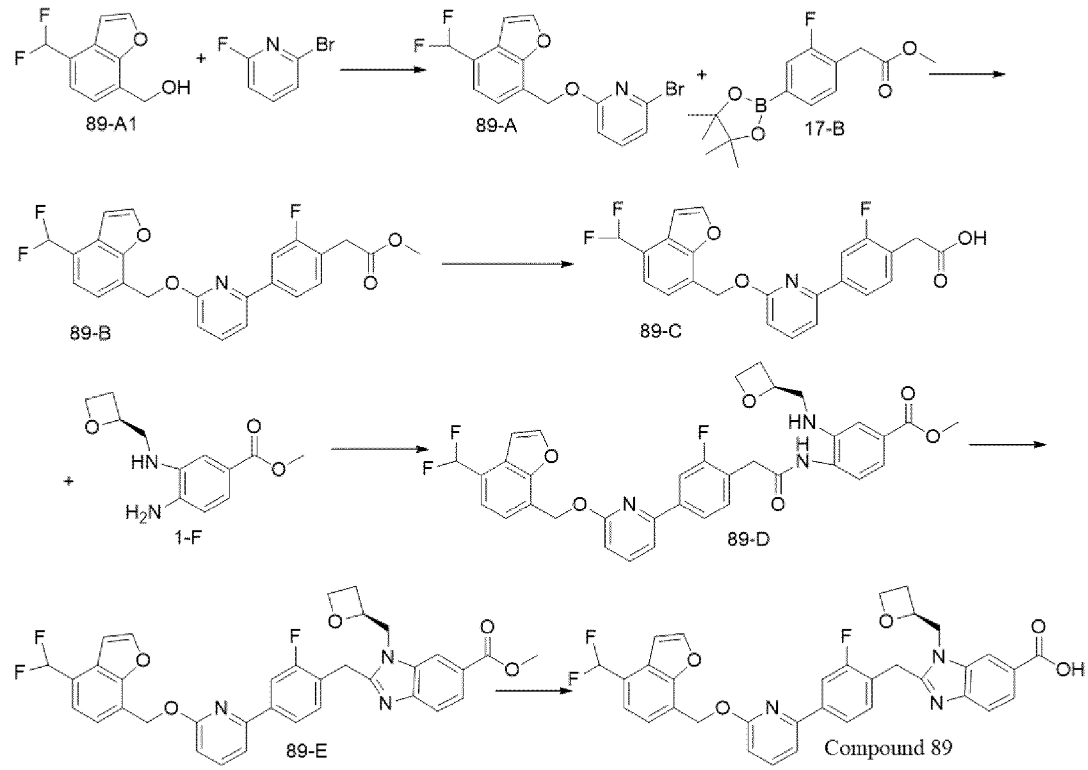

89-A1

89-A

17-B

89-B

89-C

1-F

89-D

89-E

Compound 89

Referring to the synthesis method of compound 17, wherein compound 7-A was replaced with compound 89-A to obtain compound 89. MS m/z (ESI):614.1898(M+H)$^+$.

Example 38:

Synthesis of Compound 91:

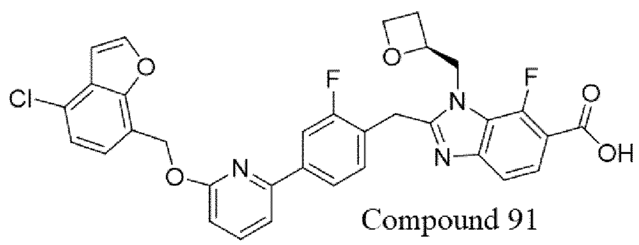

Compound 91

Synthesis route:

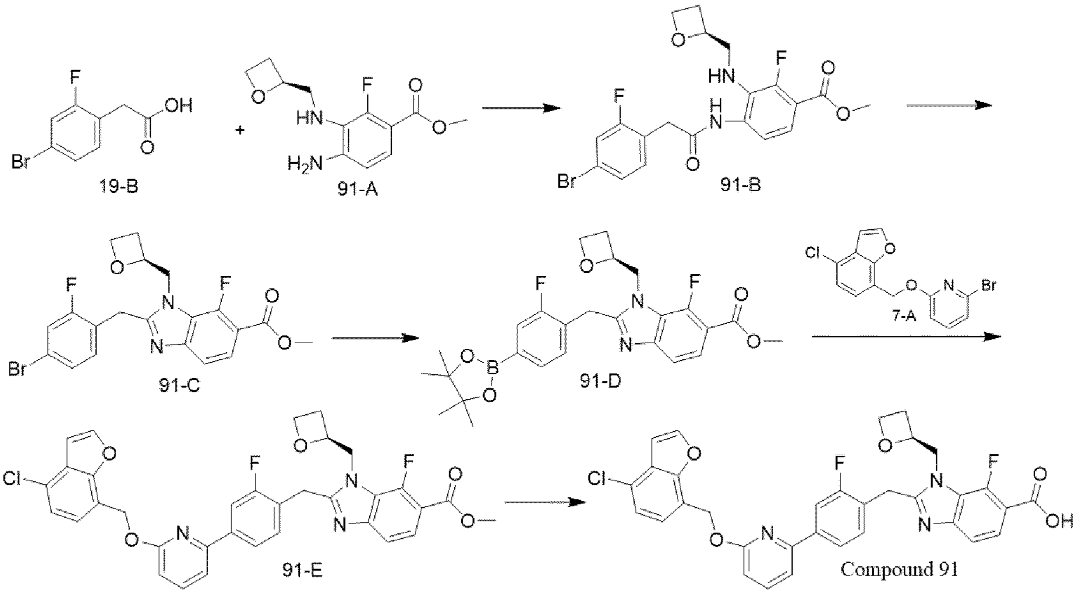


Compound 91-A:

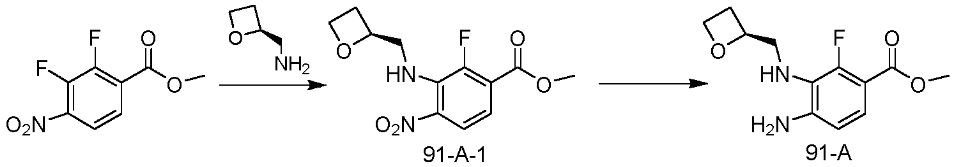


Compound 91-A-1:

1.02 g of methyl 2,3-difluoro-4-nitrobenzoate (1.0 eq.) was weighed and added to a 50mL round-bottom flask. 5 mL of DMF and 5 mL of THF were added, and then 0.95 g of TEA (2.0 eq.) was added. The reaction mixture was stirred evenly and then 0.5 g of (*S*)-2-(aminomethyl)oxetane (1.2 eq.) was added, and the thus obtained system was heated to 60°C for reaction. After reaction for 6 hours, the heating was stopped, the reaction solution was cooled to room temperature, then added with 20 mL of water and 20 mL of ethyl acetate, and subsequently left to stand. The organic phases were separated, the aqueous phase was extracted with 20 mL of ethyl acetate, and the organic phases were combined and washed with saturated salt solution, dried with anhydrous $Na_2SO_4$ for 5 minutes, and then filtered. The filtrate was concentrated to dryness to obtain compound 91-A-1 which was directly used in the next step.

Compound 91-A:

compound 91-A-1 (1.0 eq.) obtained in the previous step was weighed and added to a 50mL round-bottom flask. 20 mL of methanol was added. 0.14 g of Pd/C (10%) was then added. The reaction mixture was replaced with hydrogen thrice, reacted at room temperature for 15 hours under hydrogen atmosphere. Subsequently, the reaction solution was filtered by a funnel with diatomite, and the filter cake was washed with 20 mL×2 of ethyl acetate. The filtrate was concentrated to dryness to obtain 1.2 g of compound 91-A-1 which was directly used in the next step.

Compound 91-B:

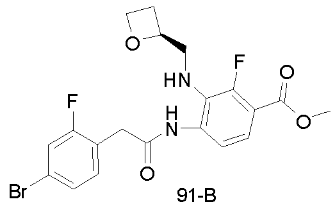
91-B

0.2 g of compound 19-B (1.0 eq.), 0.24 g of compound 91-A (1.1 eq.) and 0.148 g of N-methylimidazole (2.1 eq.)

were weighed and added to a 25mL round-bottom flask. 4 mL of acetonitrile was added. The reaction solution was stirred for 20 minutes in an ice bath, and then 0.265 g of N,N,N',N'-tetramethylchloroformamidinium hexafluorophosphate (TCFH) (1.1 eq.) was added. The reaction solution was continuously stirred for 0.5 hours in the ice bath, and then transferred to room temperature to be continuously reacted. After reaction for 3 hours, 12 mL of water was added to the reaction solution, the thus obtained system was stirred for 0.5 hours, and filtered. The filter cake was washed with 5mL×2 of water and purified by column chromatography to obtain 0.155 g of compound 91-B with a yield of 38.5%.

Compound 91-C:

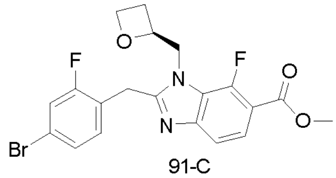

91-C

0.155 g of compound 91-B (1.0 eq.) was weighed and added to a 25mL round-bottom flask. 2.5 mL of 1,2-dichloroethane was added. 0.8 mL acetic acid (40.0eq.) was then added dropwise. After the dropwise addition, the reaction solution was heated for reaction at 80°C. After reaction for 3 hours, the reaction solution was cooled to room temperature and was concentrated. After the concentration, 3 mL of water was added to the residue, and 3% $K_2CO_3$ solution was added under stirring. The pH of the reaction system was adjusted to be 7-8, then the reaction solution was filtered, and the filter cake was washed with a small amount of water. The filter cake was dried to obtain 0.134 g of compound 91-C with a yield of 89.9%.

Compound 91-D:

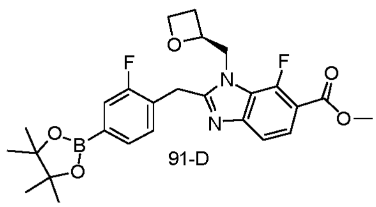

91-D

0.134 g of compound 91-C (1.0 eq.), 91 mg of bis(pinacolato)diboron (1.2 eq.), 7 mg of Pd(dppf)$Cl_2$ (0.03 eq.) and 74 mg of potassium acetate (2.5 eq.) were weighed and added to a 25mL round-bottom flask. 2 mL of 1,4-dioxane was added. Subsequently, the reaction solution was degassed with nitrogen twice, and heated to 100°C for reaction under nitrogen atmosphere. After reaction for 2 hours, the heating was stopped and the reaction solution was cooled to room temperature. Subsequently, the reaction solution was filtered by a funnel with diatomite and silica gel. The filter cake was washed with mixed solvent of petroleum ether and ethyl acetate (1: 1). Subsequently, the filtrate was concentrated to dryness to obtain compound 91-D which was directly used in the next step.

Compound 91-E:

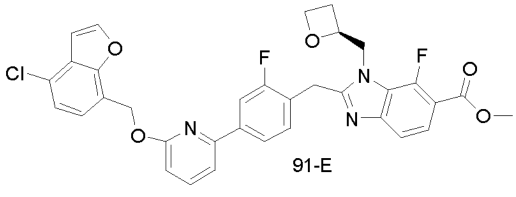

91-E

84 mg of compound 7-A (1.0 eq.), compound 91-D obtained in the previous step, 5.5 mg of Pd(dppf)$Cl_2$ (0.03 eq.) and 202 mg of $Cs_2CO_3$ (2.5 eq.) were weighed and added to a 50mL round-bottom flask. 2.5 mL of 1,4-dioxane and 0.4 mL of $H_2O$ were added. Subsequently, the reaction solution was degassed with nitrogen twice, and heated to 90°C for reaction under nitrogen atmosphere. After reaction for 3 hours, the heating was stopped and the reaction solution was cooled to room temperature. Subsequently, the reaction solution was filtered by a funnel with diatomite, and the filter cake was washed with 10 mL×2 of ethyl acetate. Subsequently, the filtrate was concentrated to dryness and purified by column chromatography to obtain 0.141 g of compound 91-E with a yield of 90.4%. MS m/z (ESI): 630.1602 $(M+H)^+$.

Compound 91

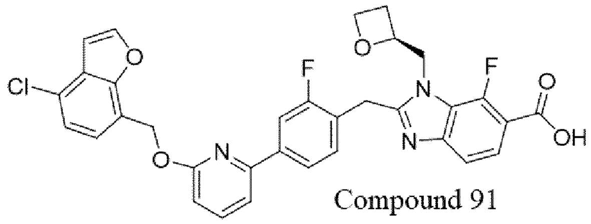

Compound 91

0.14 g of compound 91-E (1.0 eq.) was weighed and added to a 25mL round-bottom flask. 0.7 mL of methanol and 2.1 mL of tetrahydrofuran were added, and then 0.35 mL of 2 mol/L sodium hydroxide solution was added. Subsequently, the reaction solution was reacted at room temperature. After 20 hours, the reaction solution was concentrated. After the concentration, 4 mL of water and 0.5 mL of acetone were added to the residue. The pH of the reaction system was adjusted to be 4-5 with 1 mol/L HCl, the system was filtered and the filter cake was washed with a small amount of water. The filter cake was dried to obtain 104 mg of compound 91 with a yield of 76.7%. MS m/z (ESI): 616.1450 $(M+H)^+$, $^1H$ NMR (400 MHz, DMSO-d6) δ =8.18 (s, 1H), 7.89-7.81 (m, 3H), 7.66-7.63 (m, 2H), 7.48-7.35 (m, 4H), 7.06 (s, 1H), 6.89 (d, J = 8.0 Hz, 1H), 5.77 (s, 2H), 5.10 (d, J = 6.4 Hz, 1H), 4.78 (dd, J = 15.6, 7.2 Hz, 1H), 4.61 (d, J = 13.6 Hz, 1H), 4.54-4.50 (m, 2H), 4.45-4.38 (m, 2H), 2.80-2.72 (m, 1H), 2.47-2.39 (m, 1H).

Example 39:

Synthesis of Compound 93:

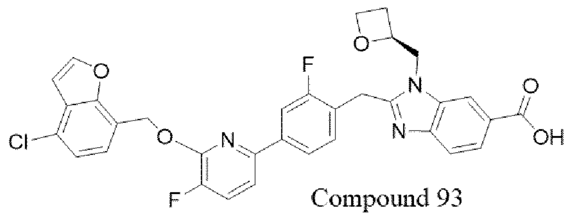

Compound 93

Synthesis route:

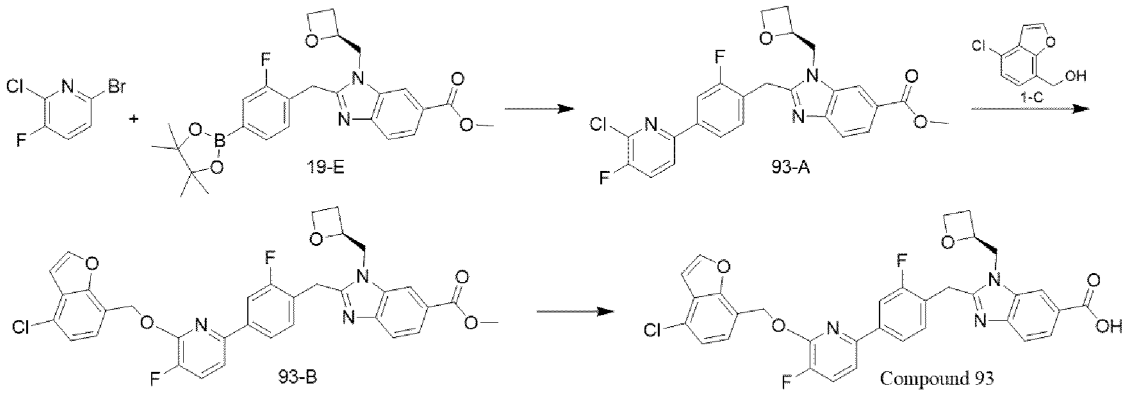


Compound 93-A:

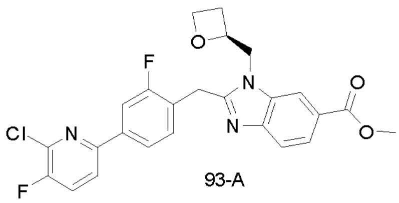

93-A

0.5 g of 6-bromo-2-chloro-3-fluoropyridine (1.0 eq.), 1.37 g of compound 19-E (1.2 eq.), 87 mg of $Pd(dppf)Cl_2$ (0.05 eq.) and 1.94 g of cesium carbonate (2.5 eq.) were weighed and added to a 50mL round-bottom flask. 15 mL of 1,4-dioxane and 2.5 mL of water were added. Subsequently, the reaction solution was degassed with nitrogen twice and heated to 80°C for reaction under nitrogen atmosphere. After reaction for 2 hours, the heating was stopped and the reaction solution was cooled to room temperature. Subsequently, the reaction solution was filtered by a funnel with diatomite, and the filter cake was washed with 15 mL×2 of ethyl acetate. Subsequently, the filtrate was concentrated to dryness and purified by column chromatography to obtain 1.07 g of compound 93-A with a yield of 93%. MS m/z (ESI): 484.1237 $(M+H)^+$.

Compound 93-B:

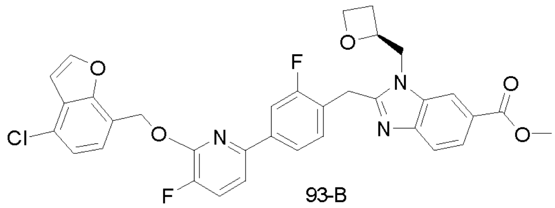

93-B

0.3 g of compound 93-A (1.0 eq.), 0.17 g of compound 1-C (1.5 eq.), 57 mg of $Pd_2(dba)_3$ (0.1 eq.), 59 mg of 2-dicyclohexylphosphino-2',6'-diisopropoxybiphenyl (0.2 eq.) and 0.404 g of cesium carbonate (2 eq.) were weighed and added to a 25mL round-bottom flask. 4.5 mL of toluene was added. Subsequently, the reaction solution was degassed with nitrogen twice and heated to 110°C for reaction under nitrogen atmosphere. After reaction for 12 hours, the heating was stopped and the reaction solution was cooled to room temperature. Subsequently, the reaction solution was filtered by a funnel with diatomite, and the filter cake was washed with 10 mL×2 of ethyl acetate. Subsequently, the filtrate was concentrated to dryness and purified by column chromatography to obtain 0.112 g of compound 93-B with a yield of 28.7%. MS m/z (ESI): 630.1609 $(M+H)^+$.

Compound 93

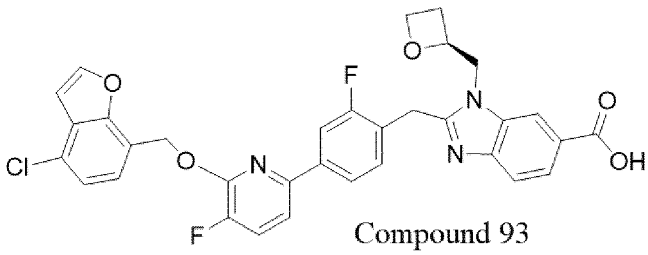

Compound 93

0.11 g of compound 93-B (1.0 eq.) was weighed and added to a 25mL round-bottom flask. 0.6 mL of methanol and 1.65 mL of tetrahydrofuran were added, and then 0.22 mL of 2 mol/L sodium hydroxide solution was added. Subsequently, the reaction solution was reacted at room temperature. After 20 hours, the reaction solution was concentrated. After the concentration, 4 mL of water and 0.5 mL of acetone were added to the residue. The pH of the reaction system was adjusted to be 4-5 with 1 mol/L HCl, the system was filtered and the filter cake was washed with a small amount of water. The filter cake was pulped with 1 mL of ethyl acetate and 0.2 mL of methanol, and then filtered. The filter cake was washed with 1 mL×2 of ethyl acetate, and dried to obtain 65 mg of compound 93 with a yield of 60.4%. MS m/z (ESI): 616.1442 $(M+H)^+$, $^1H$ NMR (400 MHz, DMSO-d6) δ= 8.22 (d, J = 24 Hz, 2H), 7.85-7.78 (m, 4H), 7.66 (d, J = 6.8 Hz, 1H), 7.59 (d, J = 8.4 Hz, 1H), 7.50 (d, J = 8 Hz, 1H), 7.42-7.37 (m, 2H), 7.04 (d, J = 20 Hz, 1H), 5.86 (s, 2H), 5.05 (d, J = 5.6 Hz, 1H), 4.72 (dd, J = 15.6, 7.2 Hz, 1H), 4.61-4.33 (m, 5H), 2.74-2.66 (m, 1H), 2.42-2.34 (m, 1H).

Example 40:

Synthesis of Compound 94:

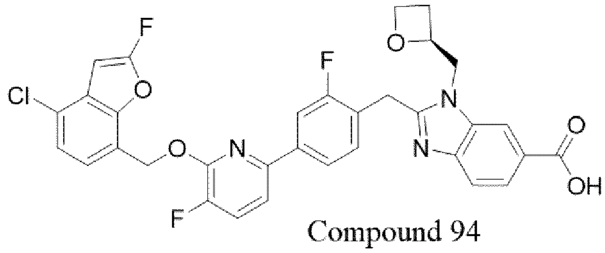

Compound 94

Synthesis route:

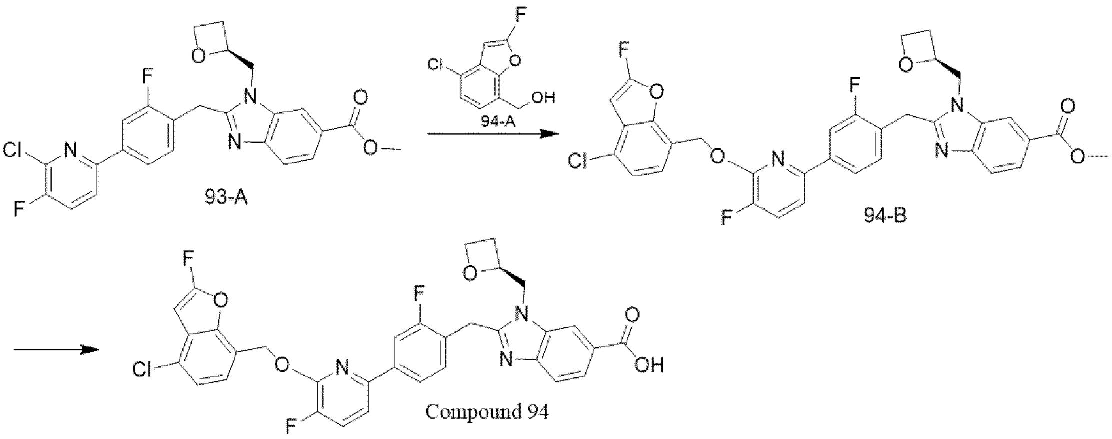


93-A

94-B

Compound 94

Referring to the synthesis method of compound 93, wherein compound 1-C was replaced with compound 94-A to finally obtain compound 94.

Compound 94-A:

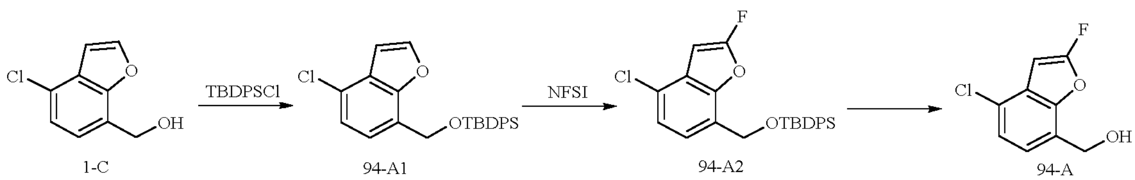


1-C

94-A1

94-A2

94-A

Compound 94-A1:

1.0 g of compound 1-C (1.0 eq.) and 0.565 g of imidazole (1.5 eq.) were weighed and added to a 50mL round-bottom flask. 22 mL of DMF was added. Then the reaction mixture was stirred evenly in an ice bath, cooled to 0°C, and TBDPSCl was added dropwise. After the dropwise addition, the reaction solution was kept at low temperature to react for 1 hour, then the ice bath was removed, and the reaction solution was recovered to room temperature, and stirred for reaction for 2 hours. TLC showed that the raw materials were completely reacted. Subsequently, the reaction solution was added with 44 mL of water and 30 mL of ethyl acetate for extraction. The organic phases were washed thrice with 15 mL of water each time, and washed thrice with 15 mL of saturated salt solution each time, then dried with anhydrous sodium sulfate and filtered. The filtrate was concentrated under reduced pressure to obtain an oily crude product, which was purified by column chromatography (PE as eluent) to obtain compound 94-A1 (2.30 g, yield 100%).

Compound 94-A2:

1.5 g of compound 94-A1 (1.0 eq.) was weighed and added to a 50mL three-necked flask. 20 mL of anhydrous THF was added under the protection of nitrogen. The reaction mixture was stirred to dissolve, and then cooled at -75°C. When the temperature of the reaction solution was cooled to -75°C, 5.16 mL of LDA (2 mol/L, 3.0 eq.) was added dropwise to the reaction solution. After the dropwise addition, the reaction solution was kept at low temperature to react for 1 hour. THF solution of NFSI (N-fluorobenzenesulfonimide) (1.63 g dissolved in 5 mL of THF, 1.5 eq.) was then added dropwise. After the dropwise addition, the reaction solution was kept at low temperature to react for 1 hour. The cooling was closed, and the reaction solution was slowly recovered to room temperature. saturated ammonium chloride solution was added to the reaction solution for quenching. The reaction solution was extracted twice with 25 mL of ethyl acetate each time, the organic phases were combined and washed with 20 mL of water twice, washed with 20 mL of saturated salt solution twice, dried with anhydrous sodium sulfate and filtered. The filtrate was concentrated under reduced pressure to obtain an oily crude product, which was purified by silica gel column chromatography (PE as eluent) to obtain compound 94-A2 (310 mg, yield 20.52%).

Compound 94-A:

310 mg of compound 94-A2 (1.0 eq.) was weighed and added to a 25mL round-bottom flask. 5.0 mL of tetrahydrofuran was added and was stirred to dissolve. 1.0 g of triethylamine trihydrofluoride (8.8 eq.) was added dropwise at room temperature. After the dropwise addition, the reaction solution was reacted overnight. TLC showed that the raw materials were completely reacted. Subsequently, the reaction solution was added with 15 mL of ethyl acetate for extraction. The organic phases were washed twice with 10 mL of water each time, washed twice with 10 mL of saturated salt solution each time, and then dried with anhydrous sodium sulfate and filtered. The filtrate was concentrated under reduced pressure to obtain a transparent oily substance, which was purified by column chromatography (PE/EA = 94/6) to obtain compound 94-A (125 mg, yield 88.03%). $^1$H NMR (400 MHz, DMSO-d6) $\delta$= 7.35 (q, *J* = 8.4 Hz, 2H), 6.46 (d, *J* = 9.6 Hz, 1H), 5.43 (t, *J* = 5.6 Hz, 1H), 4.71 (d, *J* = 5.6 Hz, 2H).

Compound 94-B:

281.60 mg of compound 93-A (1.0 eq.), 140.0 mg of compound 94-A (1.2 eq.), 64.0 mg of $Pd_2(dba)_3$ (0.12 eq.), 65.30 mg of RuPhos (2-dicyclohexylphosphino-2',6'-diisopropoxybiphenyl) (0.24 eq.) and 455.11 mg of cesium carbonate (2.4 eq.) were weighed and added to a 25mL round-bottom flask. 8 ml of toluene was added. Subsequently, the reaction solution was degassed with nitrogen twice and heated to 90°C for reaction under nitrogen atmosphere. After reaction for 3 hours, the heating was stopped, and the reaction solution was cooled to room temperature. Subsequently, the reaction solution was filtered by a funnel with diatomite. The filtrate was concentrated to dryness and purified by column chromatography (PE/EA = 60/40) to obtain compound 94-B (190 mg, yield 50.4%). MS m/z (ESI): 648.1514 $(M+H)^+$.

Compound 94:

145 mg of compound 94-B (1.0 eq.) was weighed and added to a 25mL round-bottom flask. 3 mL of tetrahydrofuran was added for dissolution, then 1.5 mL of water was added. The reaction solution was stirred evenly, and then 28.20 mg of $LiOH \cdot H_2O$ (3.0 eq.) was added. The reaction solution was stirred at room temperature for reaction overnight. TLC showed that the raw materials were completely reacted. The reaction solution was concentrated under reduced pressure to remove solvents and then 5 mL of water was added. The pH of the reaction solution was adjusted to be 4-5 with citric acid solution. Solids were precipitated and filtered. The filter cake was washed with a small amount of water. After being dried, the filter cake was pulped by a mixed solvent formed by 2 mL of acetone and 2 mL of ethyl acetate to obtain compound 94 (67 mg, yield 47.18%). MS m/z (ESI):634.1353 $(M+H)^+$, $^1$HNMR (400 MHz, DMSO-d6) $\delta$=12.61(bs, 1H), 8.25 (s, 1H), 7.92-7.75 (m, 4H), 7.67 (dd, J = 8.2, 2.8 Hz, 1H), 7.58 (d, J = 8.4 Hz, 1H), 7.49 (d, J = 8.2 Hz, 1H), 7.41 (dd, J = 11.3, 7.9 Hz, 2H), 6.53 (d, J = 6.3 Hz, 1H), 5.80 (s, 2H), 5.05 (m, 1H), 4.72 (dd, J = 15.6, 7.1 Hz, 1H), 4.64-4.30 (m, 5H), 2.70 (m, 1H), 2.38 (m, 1H).

Example 41:

Synthesis of Compound 95:

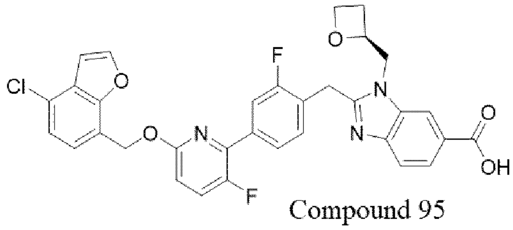

Compound 95

Synthesis route:

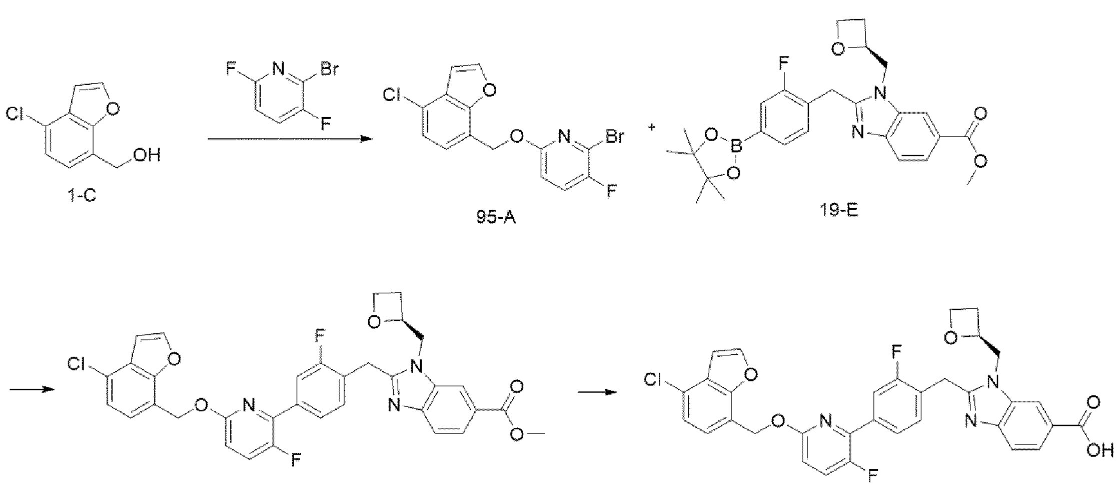

1-C

95-A

19-E

95-B

Compound 95

Compound 95-A:

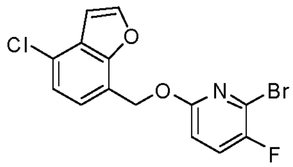

95-A

300 mg of compound 1-C (1.0 eq.), 319 mg of 2-bromo-3,6-difluoropyridine (1.0 eq.) and 1.07 g of cesium carbonate (2.0 eq.) were weighed and added to a 150mL round-bottom flask. 6 mL of DMF was added. The reaction mixture was heated to 90°C for reaction. After reaction for about 4 hours, until TLC showed that the reaction was completed, the reaction solution was cooled to room temperature, 18 mL of water was added, and extracted with 20 mL×3 of ethyl acetate. The organic phases were combined and washed with 20×2 of saturated sodium chloride solution, dried with anhydrous $Na_2SO_4$ and filtered. The filtrate was concentrated to obtain 547 mg of compound 95-A with a yield of 93.3%.

Compound 95-B:

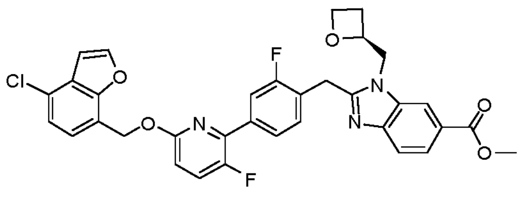

95-B

117 mg of compound 95-A (1.05 eq.), 150 mg of compound 19-E (1.0 eq.), 7 mg of Pd(dppf)$Cl_2$ (0.03 eq.) and 254

mg of $Cs_2CO_3$ (2.5 eq.) were weighed and added to a 50mL round-bottom flask. 5.0 mL of 1,4-dioxane and 1.0 mL of $H_2O$ were then added. Subsequently, the reaction solution was degassed with nitrogen twice and heated to 90°C for reaction under nitrogen atmosphere. After reaction for 4 hours, the heating was stopped, and the reaction solution was cooled to room temperature. Subsequently, the reaction solution was filtered by a funnel with diatomite and the filter cake was washed with about 20 mL of dichloromethane. Subsequently, the filtrate was concentrated to dryness and purified by column chromatography to obtain 138 mg of compound 95-B with a yield of 69.7%. MS m/z (ESI):630.1616 $(M+H)^+$.

Compound 95:

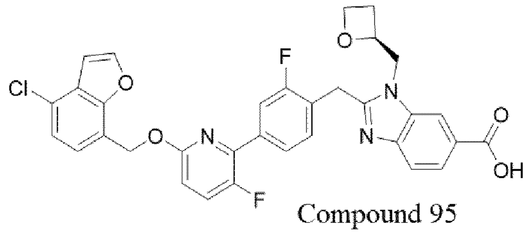

Compound 95

138 mg of compound 95-B (1.0 eq.) was weighed and added to a 25mL round-bottom flask. 3 mL of tetrahydrofuran and 1 mL of methanol were added. The thus obtained system was stirred to dissolve, and then 0.33 mL of 2 mol/L NaOH aqueous solution was added dropwise. After the addition, the reaction solution was reacted at room temperature for about 36 hours until TLC showed that the reaction was completed. The reaction solution was concentrated, 5 mL of water and 1 mL of acetone were added. The pH of the reaction solution was adjusted to be 4-5 with citric acid solution. A large amount of off-white solids were precipitated, and the reaction solution was continuously stirred for about 0.5 hours, subjected to suction filtration, and then washed with water. The filter cake was dried to obtain 135 mg of compound 95 with a yield of 100%. MS m/z (ESI):616.1457 $(M+H)^+$, $^1H$ NMR (400 MHz, DMSO) $\delta$ =8.25 (s, 1H), 8.17 (s, 1H), 7.90-7.67 (m, 4H), 7.59 (d, $J$ = 8.4 Hz, 1H), 7.52 - 7.43 (m, 2H), 7.36 (d, $J$ = 8.0 Hz, 1H), 7.06 (s, 1H), 7.02-6.93 (m, 1H), 5.72 (s, 2H), 5.06 (d, $J$ = 6.4 Hz, 1H), 4.76-4.70 (m, 1H), 4.62-4.41 (m, 4H), 4.39-4.34 (m, 1H), 2.73-2.66 (m, 1H), 2.45-2.29 (m, 1H).

Example 42:

Synthesis of Compound 97:

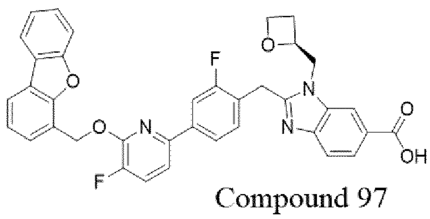

Compound 97

Synthesis route:

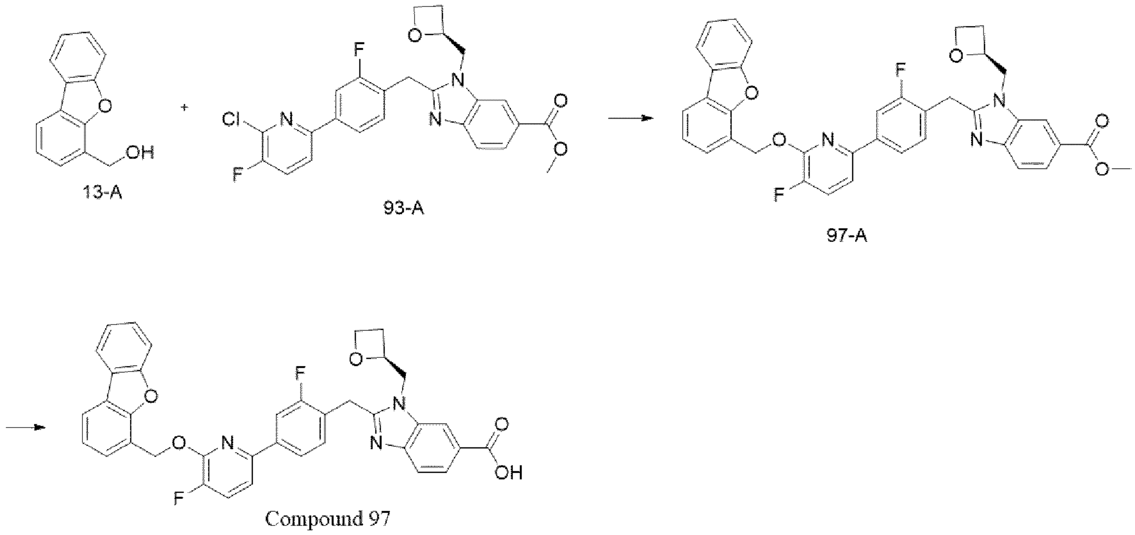

13-A

93-A

97-A

Compound 97

Compound 97-A:

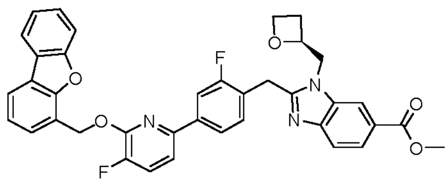

97-A

300 mg of compound 93-A (1.0 eq.), 184 mg of compound 13-A (1.5 eq.), 404 mg of cesium carbonate (2.0 eq.), 57 mg of $Pd_2(dba)_3$ (0.1 eq.) and 116 mg of 2-dicyclohexylphosphino-2',6'-diisopropoxybiphenyl (0.2 eq.) were weighed and added to a 25mL round-bottom flask. 5 mL of toluene was added. Subsequently, the reaction solution was degassed with nitrogen twice and heated to 110°C for reaction under nitrogen atmosphere for about 10 hours until TLC showed that the reaction was completed. The reaction solution was cooled to room temperature. Subsequently, the reaction solution was filtered by a funnel with diatomite, and the filter cake was washed with about 20 mL of dichloromethane. Subsequently, the filtrate was concentrated to dryness and purified by column chromatography to obtain 74 mg of compound 97-A with a yield of 18.5%. MS m/z (ESI):646.2149 $(M+H)^+$.

Compound 97:

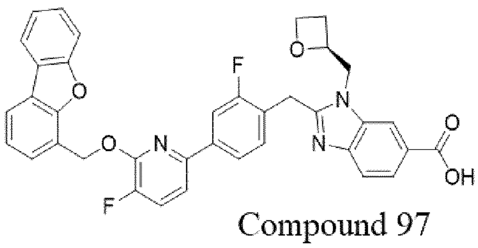

Compound 97

70 mg of compound 97-A (1.0 eq.) was weighed and added to a 25mL round-bottom flask. 3 mL of tetrahydrofuran and 1 mL of methanol were added. The thus obtained system was stirred to dissolve, and then 0.16 mL of 2 mol/L NaOH aqueous solution (3.0 eq.) was added dropwise. After the addition, the reaction solution was reacted at room temperature for about 36 hours until TLC showed that the reaction was completed. The reaction solution was concentrated, 4 mL of water and 0.5 mL of acetone were added. The pH of the reaction solution was adjusted to be 4-5 with citric acid solution. A large amount of off-white solids were precipitated, and the reaction solution was continuously stirred for about 0.5 hours, filtered, and then washed with water. The filter cake was dried to obtain 50 mg of compound 97 with a yield of 73.5%. MS m/z (ESI):632.1995 $(M+H)^+$, $^1H$ NMR (400 MHz, DMSO-d6) $\delta$=12.84 (s, 1H), 8.26 (s, 1H), 8.14 (t, $J$ = 6.6 Hz, 2H), 7.92-7.78 (m, 4H), 7.73 (d, $J$ = 8.2 Hz, 1H), 7.68-7.65 (m, 2H), 7.60 (d, $J$ = 8.4 Hz, 1H), 7.48-7.35(m, 3H), 7.29-7.25(m, 1H), 5.94 (s,

2H), 5.04 (d, *J* = 5.6 Hz, 1H), 4.72-4.67 (m, 1H), 4.58-4.31 (m, 5H), 2.76-2.60 (m, 1H), 2.42-2.26 (m, 1H).

Example 43:

Synthesis of Compound 98:

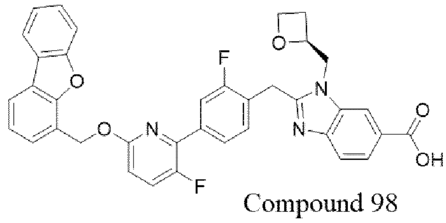

Compound 98

Synthesis route:

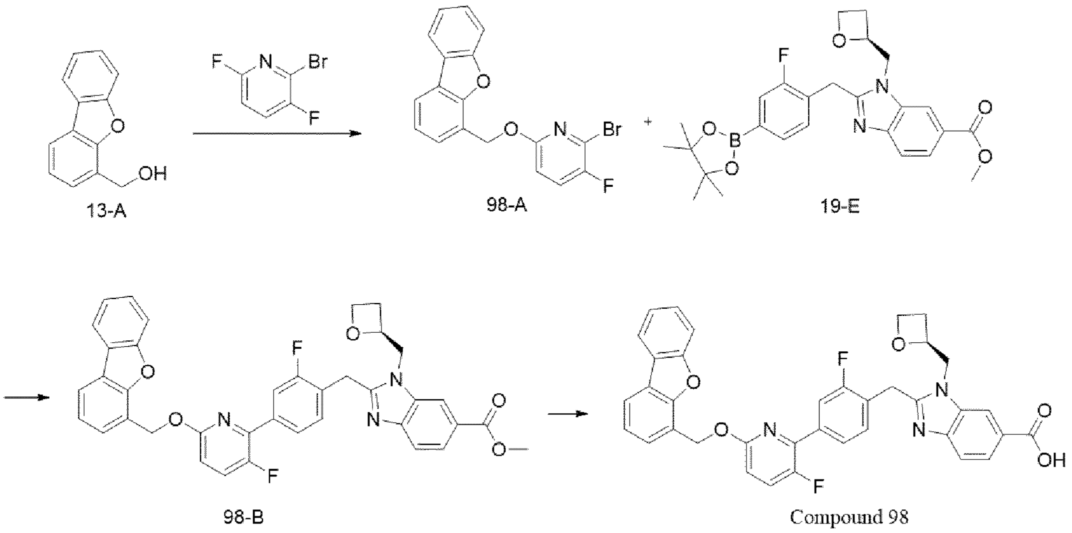

13-A

98-A

19-E

98-B

Compound 98

Referring to the preparation method of compound 95, wherein compound 1-C was replaced with compound 13-A to finally obtain compound 98. MS m/z (ESI):632.2004 $(M+H)^+$, $^1H$ NMR (400 MHz, DMSO-d6) δ=12.69 (s, 1H), 8.27 (s, 1H), 8.14 (t, *J* = 8.4 Hz, 2H), 7.88-7.69 (m, 5H), 7.63 (dd, *J* = 15.7, 7.9 Hz, 2H), 7.50-7.29 (m, 4H), 7.00 (dd, *J* = 8.9, 2.6 Hz, 1H), 5.80 (s, 2H), 5.07-5.03 (m, 1H), 4.74-4.68 (m, 1H), 4.61-4.32 (m, 5H), 2.74-2.65 (m, 1H), 2.41-2.33 (m, 1H).

Example 44:

Synthesis of Compound 99:

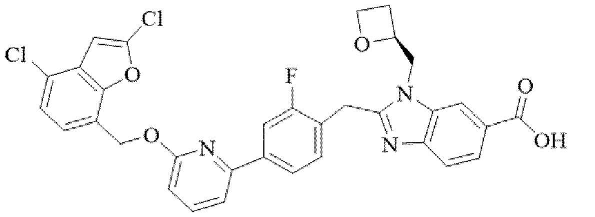

Compound 99

Synthesis route:

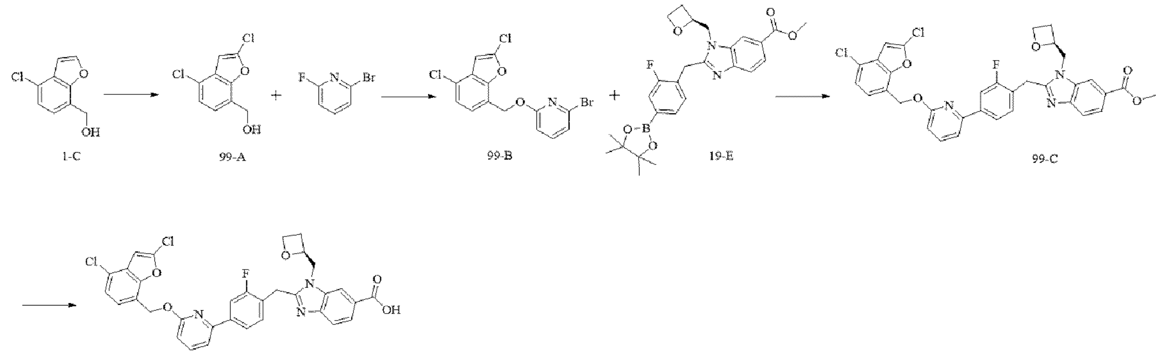


Compound 99

Compound 99-A:

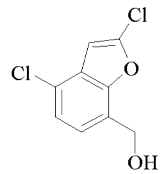

99- A

400 mg of compound 1-C (1.0 eq.) was weighed and added to a 50mL three-neck flask. 16 mL of anhydrous THF was added under the protection of nitrogen. The thus obtained system was stirred to dissolve, and then cooled at -75°C. When the temperature of the reaction solution was cooled to -75°C, 3.8 mL of LDA (2 mol/L, 3.5 eq.) was dropwise added to the reaction solution. After the dropwise addition, the reaction solution was kept at low temperature to react for 1 hour. THF solution of perchloroethane (780 mg dissolved in 8 mL of THF, 1.5 eq.) was then dropwise added to the reaction solution. After the dropwise addition, the reaction solution was kept at low temperature to react for 30 minutes. The cooling was closed, and the reaction solution was slowly recovered to room temperature. TLC showed that a small amount of raw materials left, the reaction was stopped and saturated ammonium chloride solution was added to the reaction solution for quenching. The reaction solution was extracted twice with 15 mL of ethyl acetate each time, the organic phases were combined and washed with 15 mL of water twice, washed with 15 mL of saturated salt solution twice, dried with anhydrous sodium sulfate and filtered. The filtrate was concentrated under reduced pressure to obtain an oily crude product, which was purified by silica gel column chromatography (PE/EA = 95/5) to obtain compound 99-A (299 mg, yield 62.73%).

Compound 99-B:

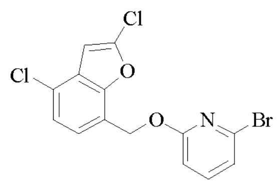

99-B

299 mg of compound 99-A (1.0 eq.), 900.57 mg of $Cs_2CO_3$ (2.0 eq.) and 267.50 mg of 2-bromo-6-fluoropyridine (1.1 eq.) were weighed and added to a 50mL round-bottom flask. 10 mL of DMF was added. The reaction mixture was stirred and dissolve, then was stirred evenly, and then placed at 90°C to react under heating for 4 hours. TLC showed that two raw materials were basically reacted completely. The heating was stopped and the reaction solution was cooled to room temperature. 30 mL of water and 20 mL of ethyl acetate were added to the reaction solution for extraction, and the aqueous phase was extracted with 10 mL of ethyl acetate. The organic phases were combined and washed with 15 mL×3 of water thrice, washed with 15 mL×3 of saturated salt solution thrice, dried with anhydrous sodium sulfate and filtered. The filtrate was concentrated under reduced pressure to obtain an oily crude product, which was dried to obtain compound

99-B (520 mg, yield 100%). The compound 99-B was directly used in the next feeding.

Compound 99-C:

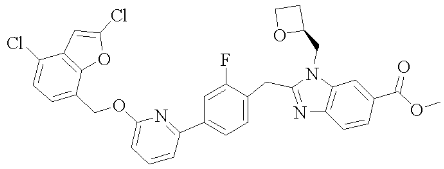

99-C

520 mg of compound 99-B (1.0 eq.), 803.13 mg of compound 19-E (1.2 eq.), 61.16 mg of Pd(dppf)$Cl_2$ (0.06 eq.) and 1.13 g of cesium carbonate (2.5 eq.) were weighed and added to a 50mL round-bottom flask. 10 mL of 1,4-dioxane was added. The reaction mixture was stirred evenly, replaced with nitrogen thrice, and heated to 90°C for reaction under nitrogen atmosphere for 2.5 hours. TLC showed that two raw materials were basically reacted completely. The heating was stopped and the reaction solution was cooled to room temperature. Subsequently, the reaction solution was filtered with diatomite and the filtrate was concentrated under reduced pressure to obtain a crude product which was purified by silica gel column chromatography (PE/EA = 60/40) to obtain compound 99-C (600 mg, yield 66.7%). MS m/z (ESI): 646.1310 $(M+H)^+$, $^1$HNMR (400 MHz, DMSO-d6) δ=8.28 (s, 1H), 7.91-7.77 (m, 4H), 7.63 (t, J = 7.2 Hz, 2H), 7.49 (d, J = 8.2 Hz, 1H), 7.41 (t, J = 8.0 Hz, 2H), 7.19 (s, 1H), 6.90 (d, J = 8.2 Hz, 1H), 5.73 (s, 2H), 5.12-5.00 (m, 1H), 4.74 (dd, J = 15.6, 7.2 Hz, 1H), 4.65-4.30 (m, 5H), 3.88 (s, 3H), 2.76-2.64 (m, 1H), 2.43-2.32 (m,1H).

Compound 99:

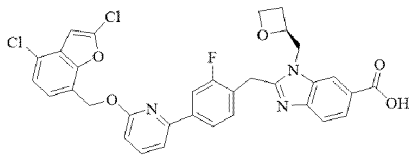

Compound 99

320 mg of compound 99-C (1.0 eq.) was weighed and added to a 25mL round-bottom flask. 6.5 mL of tetrahydrofuran and 1.0 mL of methanol were added. The thus obtained system was stirred to dissolve, and then 1.0 mL (4.0 eq.) of 2 mol/L NaOH solution was added. The reaction solution was stirred evenly, and then reacted overnight at room temperature. TLC showed that the raw materials were basically reacted completely. Subsequently, the reaction solution was cooled to room temperature and concentrated under reduced pressure. 10 mL of water was added to the concentrated solution, and the pH of the reaction solution was adjusted to be about 4 with citric acid solution. A large amount of white solids were precipitated, stirred for 30 minutes at room temperature and filtered. The filter cake was collected, and dried to obtain white solid compound 99 (250 mg, yield 79.85%). MS m/z (ESI): 632.1150 $(M+H)^+$, $^1$HNMR (400 MHz, DMSO-d6) δ=12.60 (brs, 1H), 8.25 (s, 1H), 7.89-7.76 (m, 4H), 7.62 (dd, J = 17.1, 7.9 Hz, 2H), 7.49 (d, J = 8.1 Hz, 1H), 7.40 (q, J = 6.8 Hz, 2H), 7.18 (s, 1H), 6.90 (d, J = 8.2 Hz, 1H), 5.73 (s, 2H), 5.05 (d,J = 6.5 Hz, 1H), 4.72 (dd, J =15.6, 7.2 Hz, 1H), 4.64-4.32 (m,5H), 2.75-2.65 (m, 1H), 2.42-2.32 (m, 1H).

Example 45:

Synthesis of Compound 100:

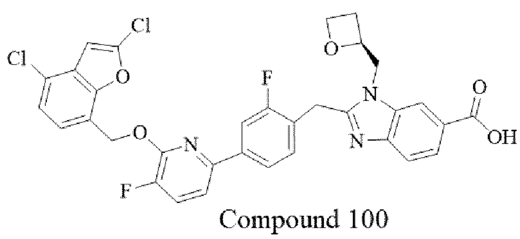

Compound 100

Synthesis route:

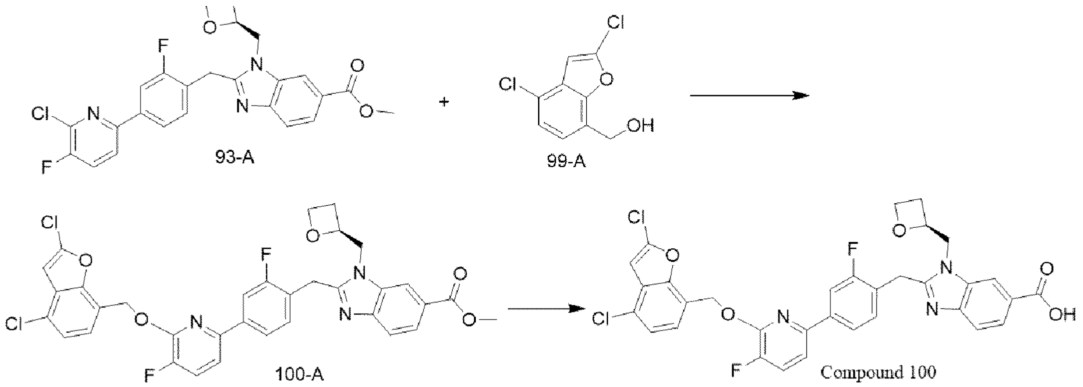

93-A

99-A

100-A

Compound 100

Compound 100-A:

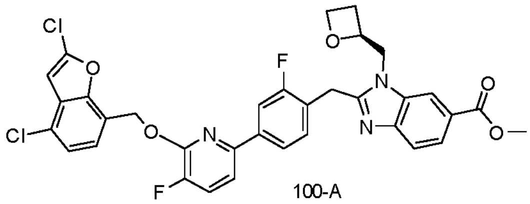

100-A

0.407 g of compound 93-A (1.0 eq.), 0.274 g of compound 94-A (1.5 eq.), 77 mg of $Pd_2(dba)_3$ (0.1 eq.), 79 mg of 2-dicyclohexylphosphino-2',6'-diisopropoxybiphenyl (0.2 eq.) and 0.548 g of cesium carbonate (2 eq.) were weighed and added to a 25mL round-bottom flask. 6 ml of toluene was added. Subsequently, the reaction solution was degassed with nitrogen twice and heated to 110°C for reaction under nitrogen atmosphere. After reaction for 5 hours, the heating was stopped, and the reaction solution was cooled to room temperature. Subsequently, the reaction solution was filtered by a funnel with diatomite and the filter cake was washed with about 10 mL×2 of ethyl acetate. Subsequently, the filtrate was concentrated to dryness and purified by column chromatography to obtain 47 mg of compound 100-A with a yield of 8.4%. MS m/z (ESI): 664.1220 $(M+H)^+$.

Compound 100:

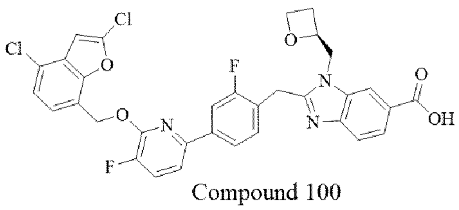

Compound 100

45 mg of compound 100-A (1.0 eq.) was weighed and added to a 25mL round-bottom flask. 0.25 mL of methanol and 0.75 mL of tetrahydrofuran were added, and then 0.15 mL of 2 mol/L sodium hydroxide solution was added. Subsequently, the reaction solution was reacted at room temperature. After 22 hours, the reaction solution was concentrated. After the concentration, 2 mL of water and 0.25 mL of acetone were added to the residue, and the pH of the reaction system was adjusted to be 4-5 with 1 mol/L HCl aqueous solution. The reaction solution was filtered, and the filter cake was washed with a small amount of water. The filter cake was dried to obtain 40 mg of compound 100 with a yield of 90.4%. MS m/z (ESI): 650.1068 $(M+H)^+$, $^1H$ NMR (400 MHz, DMSO-d6) $\delta$= 8.25 (s, 1H), 7.85-7.78 (m, 4H), 7.67 (dd, J = 8.4, 2.8 Hz, 1H), 7.58 (d, J = 8.4 Hz, 1H), 7.52-7.49 (m, 1H), 7.43-7.37 (m, 2H), 7.19 (s, 1H), 5.84 (s, 2H), 5.08-5.02 (m, 1H), 4.72 (dd, J = 15.6, 6.8 Hz, 1H), 4.59 (d, J = 15.6 Hz, 1H), 4.53-4.33 (m, 4H), 2.74-2.66 (m, 1H), 2.42-2.34 (m, 1H).

Example 46:

Synthesis of Compound 101:

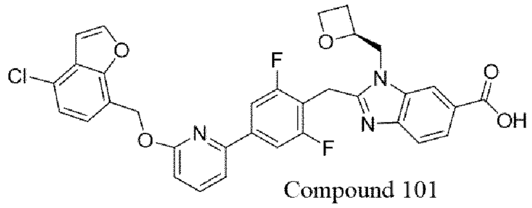

Compound 101

Synthesis route:

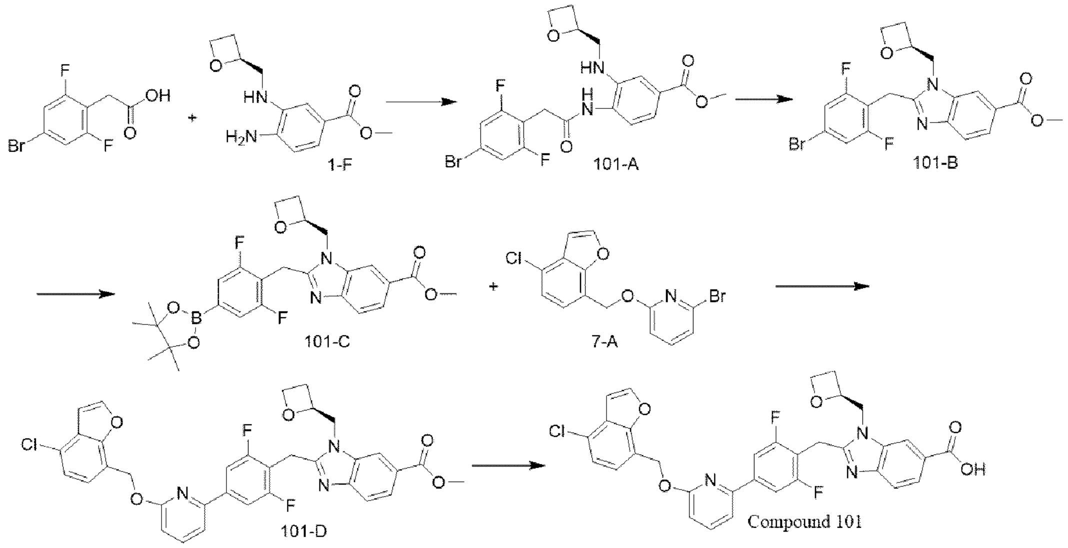

1-F, 101-A, 101-B, 101-C, 7-A, 101-D, Compound 101

Compound 101-A:

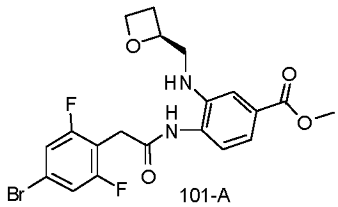

101-A

0.4 g of 4-bromo-2,6-difluorobenzeneacetic acid (1.0 eq.), 0.414 g of compound 1-F (1.1 eq.) and 0.275 g of N-methylimidazole (2.1 eq.) were weighed and added to a 25mL round-bottom flask. 10 mL of acetonitrile was added. The reaction solution was stirred for 20 minutes in an ice bath, and 0.492 g of N,N,N',N'-tetramethylchloroformamidinium hexafluorophosphate (TCFH) (1.1 eq.) was added. The reaction solution was continuously stirred for 0.5 hours in the ice bath, and then transferred to room temperature to be continuously reacted. After reaction for 3 hours, 30 mL of water was added to the reaction solution. The thus obtained system was stirred for 0.5 hours and then filtered. The filter cake was washed with 10 mL×2 of water and dried to obtain 0.703 g of compound 101-A with a yield of 94%.

Compound 101-B:

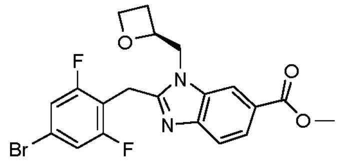

101-B

0.703 g of compound 101-A (1.0 eq.) was weighed and added to a 25mL round-bottom flask. 11 mL of 1,2-dichloroethane was added, and 3.5 mL of acetic acid (40.0 eq.) was then added dropwise. After the dropwise addition, the reaction solution was placed at 80°C for reaction under heating. After reaction for 3 hours, the reaction solution was cooled to room temperature and then the reaction solution was concentrated. After the concentration, 10 mL of water was added to the reaction solution, and 3% $K_2CO_3$ aqueous solution was added under stirring. The pH of the reaction system was adjusted to be 7-8. The system was then filtered and the filter cake was washed with a small amount of water, and then the filter cake was dried to obtain 0.642 g of compound 101-B with a yield of 94.9%.

Compound 101-C:

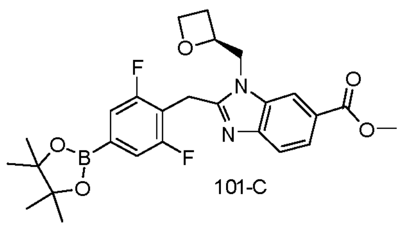

101-C

0.3 g of compound 101-B (1.0 eq.), 203 mg of bis(pinacolato)diboron (1.2 eq.), 15 mg of Pd(dppf)$Cl_2$ (0.03 eq.) and 163 mg of potassium acetate (2.5 eq.) were weighed and added to a 25mL round-bottom flask. 4.5 mL of 1,4-dioxane was added. Subsequently, the reaction solution was degassed with nitrogen twice and heated to 100°C for reaction under nitrogen atmosphere. After reaction for 2 hours, the heating was stopped, and the reaction solution was cooled to room temperature. Subsequently, the reaction solution was filtered by a funnel with diatomite and silica gel, and the filter cake was washed with a mixed solvent of petroleum ether and ethyl acetate (1: 1). The filtrate was collected and then the filtrate was concentrated to dryness to obtain compound 101-C, which was directly used in the next step.

Compound 101-D:

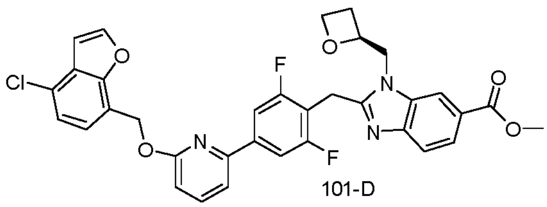

101-D

0.188 g of compound 7-A (1.0 eq.), compound 101-C obtained in the previous step, 12.2 mg of Pd(dppf)$Cl_2$ (0.03 eq.) and 0.451 g of $Cs_2CO_3$ (2.5 eq.) were weighed and added to a 50mL round-bottom flask. 5.6 mL of 1,4-dioxane and 1 mL of $H_2O$ were added. Subsequently, the reaction solution was degassed with nitrogen twice and heated to 90°C for reaction under nitrogen atmosphere. After reaction for 3 hours, the heating was stopped, and the reaction solution was cooled to room temperature. Subsequently, the reaction solution was filtered by a funnel with diatomite and the filter cake was washed with 10 mL×2 of ethyl acetate. Subsequently, the filtrate was concentrated to dryness and purified by column chromatography to obtain 0.248 g of compound 101-D with a yield of 71%. MS m/z (ESI): 630.1534 $(M+H)^+$.

Compound 101:

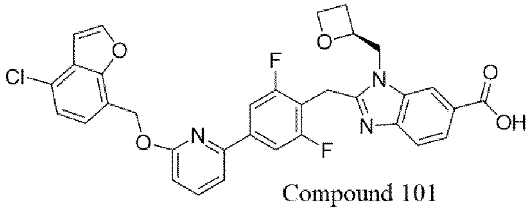

Compound 101

0.247 g of compound 101-D (1.0 eq.) was weighed and added to a 25mL round-bottom flask. 1.3 mL of methanol and 3.7 mL of tetrahydrofuran, and then 0.6 mL of 2 mol/L sodium hydroxide solution were added. Subsequently, the reaction solution was reacted at room temperature. After 20 hours, the reaction solution was concentrated. After the concentration, 6 mL of water and 0.5 mL of acetone were added to the reaction solution, and the pH of the reaction system was adjusted to be 4-5 with 1 mol/L HCl solution. The reaction solution was filtered and the filter cake was washed with a small amount of water then dried to obtain 0.228 g of compound 101 with a yield of 94.4%. MS m/z (ESI): 616.1446 $(M+H)^+$, $^1H$ NMR (400 MHz, DMSO-d6) $\delta$=8.25 (s, 1H), 8.17 (s, 1H), 7.88-7.77(m, 4H), 7.72 (d, J = 7.2 Hz, 1H), 7.55 (d, J = 8.4 Hz, 1H), 7.48 (d, J = 8.0 Hz, 1H), 7.36 (d, J = 8.0 Hz, 1H), 7.06 (s, 1H), 6.94 (d, J = 8.0 Hz, 1H), 5.79 (s, 2H), 5.14-5.10 (m, 1H), 4.80 (dd, J = 15.6, 6.8 Hz, 1H), 4.67 (d, J = 14.8 Hz, 1H), 4.58-4.42 (m, 3H), 4.36 (dd, J = 14.8, 6.0 Hz, 1H), 2.78-2.70 (m, 1H), 2.44-2.35 (m, 1H).

Example 47:

Synthesis of Compound 102:

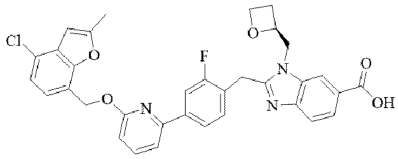

Compound 102

Synthesis route:

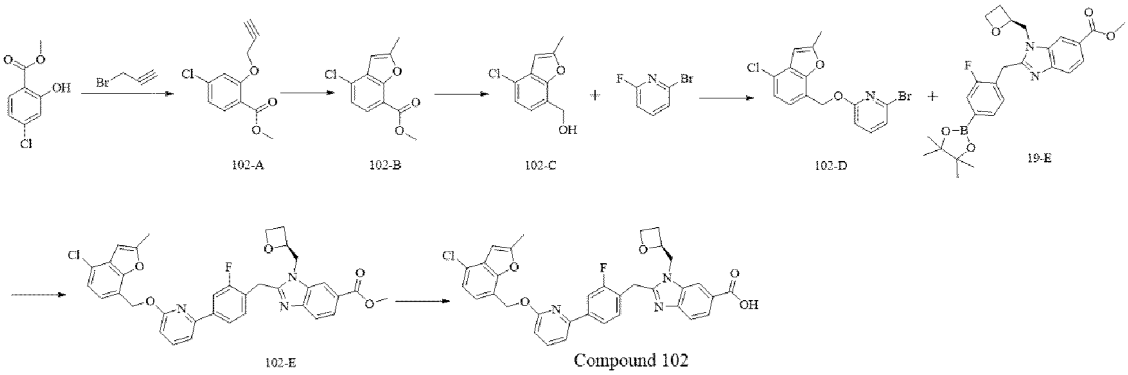

Compound 102-A:

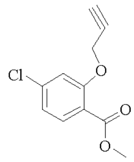

102-A

1.87 g of 4-chloro-2-hydroxy-benzoic acid methyl ester (1.0 eq.), 1.42 g of 3-bromopropyne (1.2 eq.) and 2.1 g of potassium carbonate (1.5 eq.) were weighed and added to a 50mL three-neck flask. 20 mL of N,N-dimethylformamide was added. The rection mixture was stirred evenly, and placed at 50°C for reaction under heating for 5 hours. TLC showed that the raw materials were basically reacted completely, the reaction was stopped and the reaction solution was cooled to room temperature. 40 mL of water and 20 mL of ethyl acetate were added to the reaction solution for extraction, the aqueous phase was extracted with 15 mL of ethyl acetate, and the organic phases were combined and then washed with 15 mL×2 of water twice, washed with 15 mL×3 of saturated salt solution thrice, dried with anhydrous sodium sulfate and filtered. The filtrate was concentrated under reduced pressure to obtain oily substance 102-A crude product. The product was directly used in the next feeding without purification.

Compound 102-B:

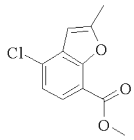

102-B

The 102-A crude product in the previous step was stirred to dissolve with 15 mL of N,N-diethylaniline, and 2.67 g of cesium fluoride (1.3 eq.) was weighed and added quickly. The reaction solution was stirred evenly and placed at 220°C for reaction under heating for 3 hours. TLC showed that the raw materials were basically reacted completely. The reaction was stopped and the reaction solution was cooled to room temperature. 30 mL of water was added to the reaction solution, and the pH of the reaction solution was adjusted to be 4 with 2 mol/L HCl solution. 20 mL of ethyl acetate was added for extraction, the aqueous phase was extracted with 20 mL of ethyl acetate once, and the organic phases were combined and then washed with 20 mL ×2 of saturated sodium chloride solution twice, dried with anhydrous sodium sulfate and filtered. The filtrate was concentrated under reduced pressure to obtain a black oily substance, which was purified by silica gel column chromatography (PE/EA = 95/5) to obtain compound 102-B (1.16 g, two-step yield 51.6%).

Compound 102-C:

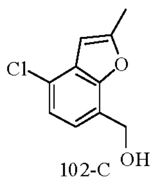

102-C

1.16 g of compound 102-B (1.0 eq.) was weighed and added to a 50mL round-bottom flask. 15 mL of methanol was added. The thus obtained system was stirred to dissolve at room temperature. 3.90 g of sodium borohydride (20.0 eq.) was weighed and added, and the solution was stirred for reaction overnight. TLC showed that the raw materials were basically reacted completely. The reaction was stopped. The reaction solution was slowly dropwise added with 30 mL of water, and then a large amount of solids were precipitated, which were stirred for about 30 minutes at room temperature and then filtered. The filter cake was collected and dried to obtain yellow solid compound 102-C (950 mg, yield 94.06%).

Compound 102-D:

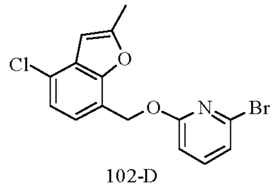

102-D

400 mg of compound 102-C (1.0 eq.), 1.32 g of $Cs_2CO_3$ (2.0 eq.) and 428.7 mg of 2-bromo-6-fluoropyridine (1.2 eq.) were weighed and added to a 25mL round-bottom flask. 12 mL of DMF was added. The reaction mixture was stirred evenly and then placed at 90°C for reaction under heating for 5 hours. TLC showed that two raw materials were basically reacted completely. The heating was stopped and cooled to room temperature. 24 mL of water was added to the reaction solution, and then a large amount of solids were precipitated, which were stirred for about 30 minutes at room temperature and then filtered. The filter cake was washed with water and dried to obtain solid compound 102-D (640 mg, yield 89.38%).

Compound 102-E:

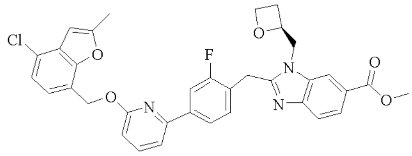

102-E

340 mg of compound 102-D (1.0 eq.), 509 mg of compound 19-E (1.0 eq.), 42.32 mg of Pd(dppf)$Cl_2$ (0.06 eq.) and 785.2 mg of cesium carbonate (2.5 eq.) were weighed and added to a 50mL round-bottom flask. 9 mL of 1,4-dioxane was added. Then the reaction mixture was stirred evenly, replaced with nitrogen thrice, and heated to 90°C for reaction under nitrogen atmosphere for 2.5 hours. TLC showed that two raw materials were basically reacted completely. The heating was stopped, and the reaction solution was cooled to room temperature. Subsequently, the reaction solution was filtered with diatomite, and the filtrate was concentrated under reduced pressure to obtain a crude product, which was purified by silica gel column chromatography (DCM/MeOH = 98/2) to obtain compound 102-E (530 mg, yield 87.8%).

Compound 102:

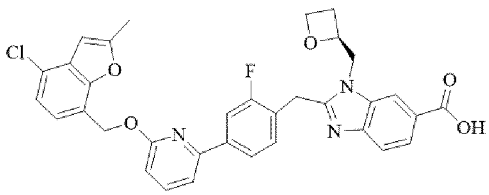

Compound 102

530 mg of compound 102-E (1.0 eq.) was weighed and added to a 25mL round-bottom flask. 9 mL of tetrahydrofuran and 1.5 mL of methanol were added. The thus obtained system was stirred to dissolve, and then 0.85 mL (2.0 eq.) of 2 mol/L NaOH solution was added. The reaction solution was stirred evenly, and reacted overnight at room temperature. TLC showed that the raw materials were basically reacted completely. Subsequently, the reaction solution was cooled to room temperature and concentrated under reduced pressure. 10 mL of water was added to the concentrated solution, and the pH of the reaction solution was adjusted to be about 4 with citric acid solution. A large amount of white solids were precipitated, stirred for 30 minutes at room temperature, and filtered. The filter cake was collected and pulped with a mixed solvent (DCM/MeOH = 1: 1), and purified to obtain white solid compound 102 (400 mg, yield 77.16%). MS m/z (ESI): 612.1696 $(M+H)^+$, $^1$HNMR (400 MHz, DMSO-d6) δ=12.71 (s, 1H), 8.26 (s, 1H), 7.95-7.74 (m, 4H), 7.61 (dd, J = 13.3, 7.9 Hz, 2H), 7.41(t,J=8.0Hz,1H),7.32 (dd, J = 35.5,8.0Hz, 2H), 6.89 (d, J = 8.2 Hz, 1H), 6.68 (s, 1H), 5.73 (s, 2H), 5.05 (dd, J = 7.3, 2.7 Hz, 1H), 4.72 (dd, J = 15.6, 7.1 Hz, 1H), 4.66-4.29 (m, 5H), 2.76-2.63 (m, 1H), 2.46 (d, J = 11.5 Hz, 3H), 2.38 (m, 1H).

Example 48:

Synthesis of Compound 103:

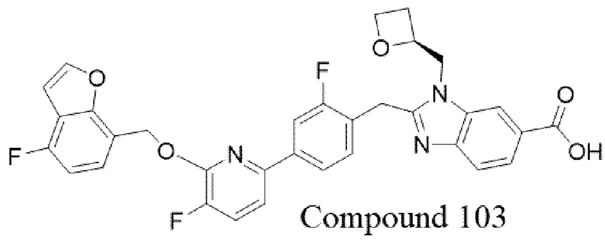

Compound 103

Synthesis route:

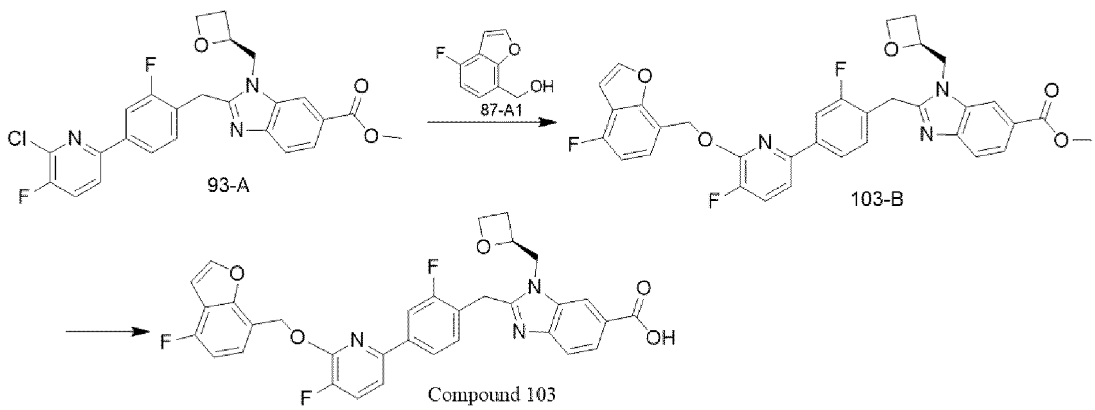


Referring to the synthesis method of compound 93, wherein compound 1-C was replaced with compound 87-A1 to finally obtain compound 103. MS m/z (ESI): 600.1743 $(M+H)^+$, $^1H$ NMR (400 MHz, DMSO-d6) δ= 8.25 (d, J = 24 Hz, 2H), 7.88-7.76 (m, 4H), 7.65 (d, J = 6.8 Hz, 1H), 7.57 (d, J = 8.4 Hz, 1H), 7.51 (d, J = 8.4 Hz, 1H), 7.43-7.38 (m, 2H), 7.03 (d, J = 20 Hz, 1H), 5.85 (s, 2H), 5.07 (d, J = 5.6 Hz, 1H), 4.75 (dd, J = 15.6, 7.2 Hz, 1H), 4.58-4.31 (m, 5H), 2.75-2.67 (m, 1H), 2.41-2.35 (m, 1H).

Example 49:

Synthesis of Compound 104:

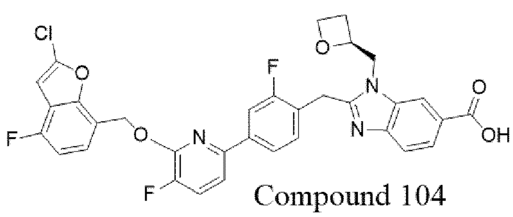

Compound 104

Snthesis route:

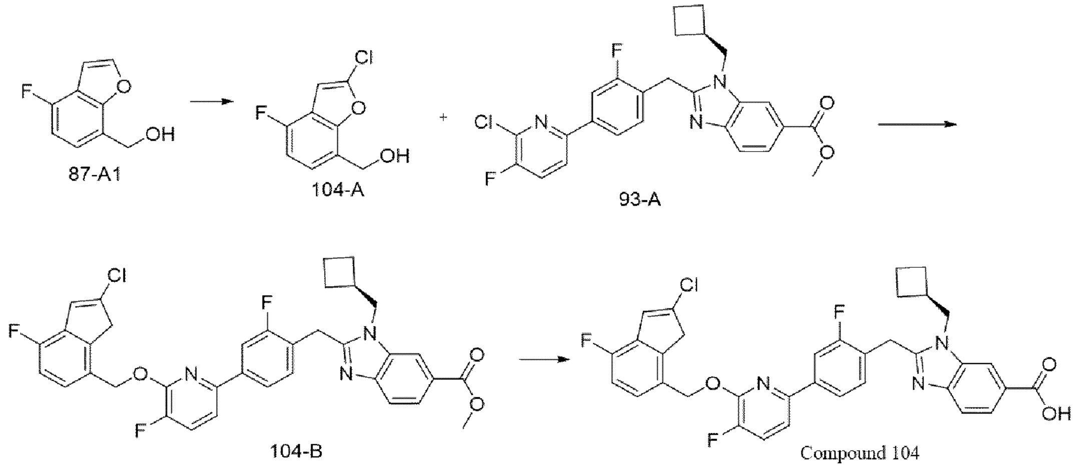

87-A1 104-A 93-A 104-B Compound 104

Referring to the synthesis method of compound 100, wherein compound 99-A was replaced with compound 104-A (wherein the preparation method of 104A referred to that of compound 99-A and wherein compound 1-C was replaced with compound 87-A1) to finally obtain compound 104. MS m/z (ESI): 634.1350$(M+H)^+$, $^1H$ NMR (400 MHz, DMSO-d6) δ= 8.28 (s, 1H), 7.87-7.77 (m, 4H), 7.68 (dd, J = 8.4, 2.6 Hz, 1H), 7.59 (d, J = 8.4 Hz, 1H), 7.50-7.46 (m, 1H), 7.45-7.36 (m, 2H), 7.20 (s, 1H), 5.85 (s, 2H), 5.10-5.03 (m, 1H), 4.75 (dd, J = 15.6, 6.8 Hz, 1H), 4.57 (d, J = 15.6 Hz, 1H), 4.55-4.31 (m, 4H), 2.75-2.68 (m, 1H), 2.45-2.36 (m, 1H).

Example 50:

Synthesis of Compound 105:

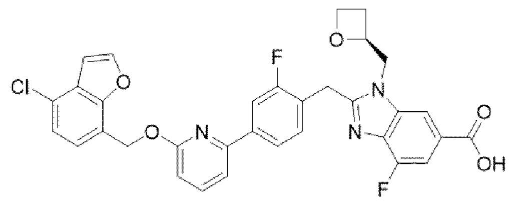

Compound 105

Synthesis route:

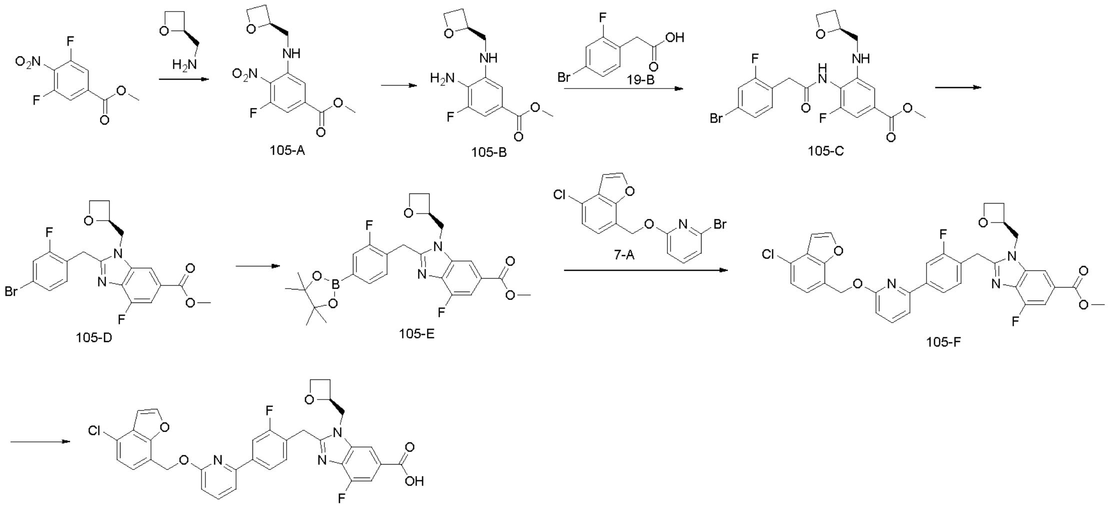

105-A 105-B 105-C 105-D 105-E 105-F 105

## Compound 105-A:

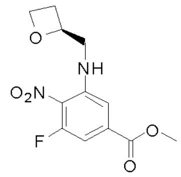
105-A

2 g of methyl 3,5-difluoro-4-nitrobenzoate (1.0 eq.), 802 mg of (*S*)-2-(aminomethyl)oxetane (1.0 eq.) and 1.87 g of triethylamine (2.0 eq.)were weighed and added to a 100mL round-bottom flask. 10 mL of N,N-dimethylformamide and 10 mL of tetrahydrofuran were added. The reaction mixture was stirred evenly, and then heated to 60°C for reaction for about 12 hours until TLC showed that the reaction was completed. The reaction solution was cooled to room temperature, 40 mL of water was added, and the thus obtained system was extracted with 3×30 mL of ethyl acetate. The organic phases were combined and washed with 2×30 mL of saturated sodium chloride solution, dried with anhydrous sodium sulfate, filtered, concentrated, separated and purified by silica gel column chromatography to obtain 1.91 g of compound 105-A with a yield of 72.9%.

## Compound 105-B:

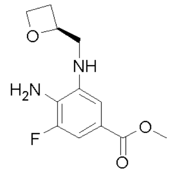
105-B

1.91 g of compound 105-A (1.0 eq.) was weighed and added to a 100mL round-bottom flask. 30 mL of methanol was added. The reaction mixture was then stirred to form a suspension. 287 mg of Pd/C (10%) was then added, and the system was replaced with hydrogen thrice. The reaction solution was reacted for 4 hours at room temperature under hydrogen atmosphere until TLC showed that the reaction was completed. Subsequently, the reaction solution was filtered with diatomite, and the filtrate was concentrated under reduced pressure to obtain 1.71 g of compound 105-B with a yield of 100%.

Compound 105-C:

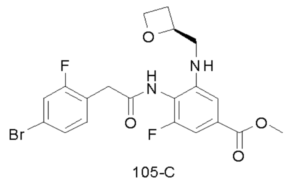

105-C

500 mg of compound 19-B (1.0 eq.), 600 mg of compound 105-B (1.1 eq.), 370 mg of NMI (2.1 eq.) and 662 mg of TCFH (1.1 eq.) were weighed and added to a 100mL round-bottom flask. 10 mL of acetonitrile was added. Then the reaction mixture was stirred for about 0.5 hours in an ice bath, and then reacted overnight at room temperature until TLC showed that the reaction was completed. 20 mL of water was added to the reaction solution to precipitate a large amount of off-white solids, the reaction system was continuously stirred for about 0.5 hours and then was subjected to suction filtration, washed with water. The filter cake was dried to obtain 507 mg of compound 105-C with a yield of 50.3%.

Compound 105-D:

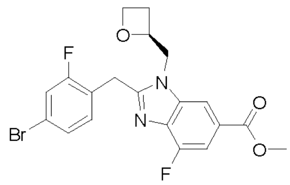

105-D

507 mg of compound 105-C (1.0 eq.) and 2.60 g of acetic acid (40.0 eq.) were weighed and added to a 100mL round-bottom flask. 10 mL of 1,2-dichloroethane was added. Then the rection mixture was stirred evenly, and then heated to 80°C for reaction for about 6 hours until TLC showed that the reaction was completed. The reaction solution was cooled to room temperature and then concentrated, and then 5 mL of water was added. The pH of the reaction solution was adjusted to be neutral with 5% sodium hydrogen carbonate solution. A large amount of solids were precipitated, the reaction system was stirred for a while and then was subjected to suction filtration, washed with a small amount of water to obtain the filter cake. The filter cake was purified by silica gel column chromatography to obtain 330 mg of compound 105-D with a yield of 67.6%.

Compound 105-E:

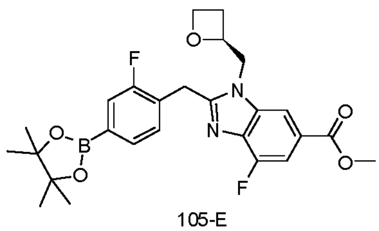

105-E

330 mg of compound 105-D (1.0 eq.), 223 mg of bis(pinacolato)diboron (1.2 eq.), 16.0 mg of $Pd(dppf)Cl_2$ (0.03 eq.) and 179 mg of potassium acetate (2.5 eq.) were weighed and added to a 50mL round-bottom flask. 5.0 mL of 1,4-dioxane was added. Subsequently, the reaction solution was degassed with nitrogen twice and heated to 100°C for reaction under nitrogen atmosphere. After reaction for 2 hours, the heating was stopped, and the reaction solution was cooled to room temperature. Subsequently, the reaction solution was filtered by a funnel with diatomite and the filter cake was washed with 20 mL×2 of ethyl acetate. Subsequently, the filtrate was concentrated to dryness and purified by column chromatography to obtain 261 mg of compound 105-E with a yield of 71.7%.

Compound 105-F:

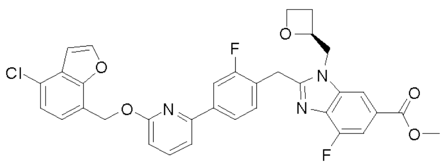

105-F

260 mg of compound 105-E (1.1 eq.), 161 mg of compound 7-A (1.0 eq.), 10.4 mg of Pd(dppf)$Cl_2$ (0.03 eq.) and 387 mg of $Cs_2CO_3$ (2.5 eq.) were weighed and added to a 50mL round-bottom flask. 5.0 mL of 1,4-dioxane and 1.0 mL of $H_2O$ were added. Subsequently, the reaction solution was degassed with nitrogen twice and heated to 90°C for reaction under nitrogen atmosphere. After reaction for 2 hours, the heating was stopped, and the reaction solution was cooled to room temperature. Subsequently, the reaction solution was filtered by a funnel with diatomite and the filter cake was washed with 10 mL×2 of ethyl acetate. Subsequently, the filtrate was concentrated to dryness and purified by column chromatography to obtain 185 mg of compound 105-F with a yield of 61.7%.

Compound 105

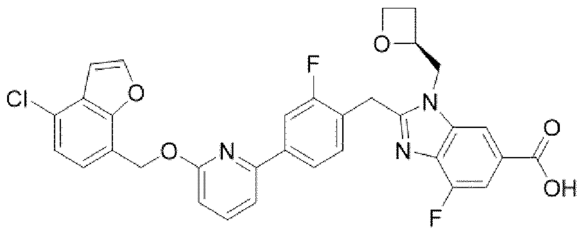

Compound 105

180 mg of compound 105-F (1.0 eq.) was weighed and added to a 25mL round-bottom flask. 3 mL of tetrahydrofuran and 1 mL of methanol were added. The thus obtained system was stirred to dissolve, and then 0.57 mL of 2 mol/L NaOH aqueous solution (4.0 eq.) was slowly dropwise added. After the addition, the reaction solution was reacted at room temperature for about 36 hours until TLC showed that the reaction was completed. The reaction solution was concentrated, and 5 mL of water and 1 mL of acetone were added. The pH of the reaction solution was adjusted to be 4-5 with citric acid solution. A large amount of off-white solids were precipitated, which were continuously stirred for about 0.5 hours and then filtered and washed with water. The filter cake was dried to obtain 157 mg of compound 105 with a yield of 89.2%. MS m/z (ESI): 616.1452 $(M+H)^+$, $^1H$ NMR (400 MHz, DMSO-d6) $\delta$= 8.18 (s, 1H), 8.13 (s, 1H), 7.96-7.77 (m, 3H), 7.64 (d, *J* = 7.4 Hz, 1H), 7.56-7.40 (m, 3H), 7.36 (d, *J* = 8.0 Hz, 1H), 7.06 (s, 1H), 6.89 (d, *J* = 8.2 Hz, 1H), 5.77 (s, 2H), 5.05 (d, *J* = 5.6 Hz, 1H), 4.78-4.72 (m, 1H), 4.67-4.31 (m, 5H), 2.77-2.62 (m, 1H), 2.45-2.30 (m, 1H).

Example 51:

Synthesis of Compound 106:

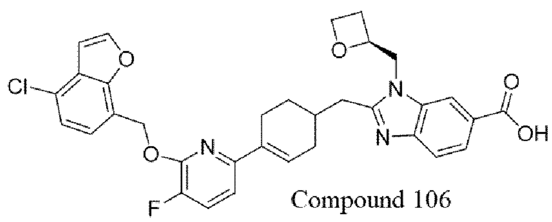

Compound 106

Synthesis route:

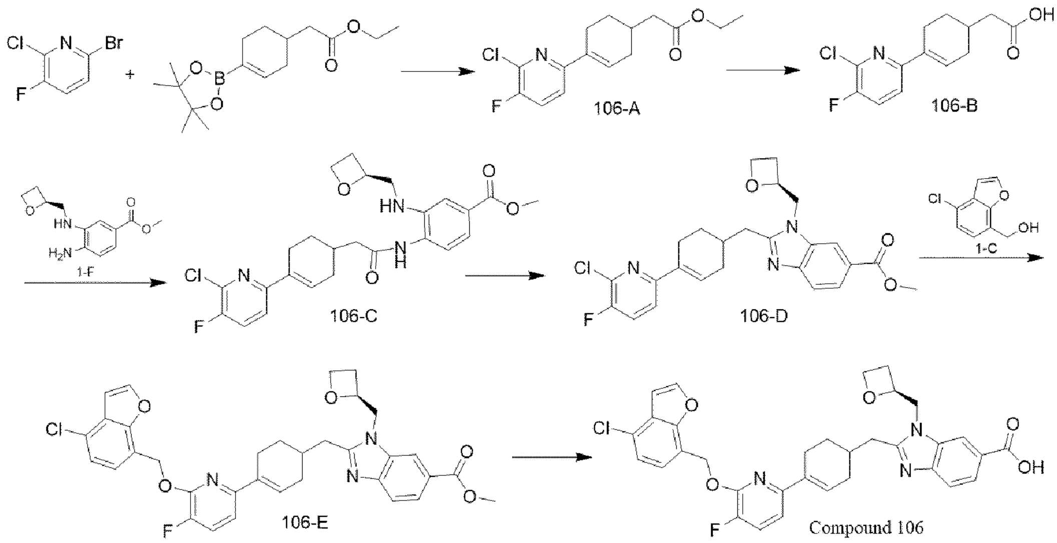


Compound 106-A:

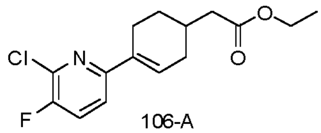
106-A

0.6 g of 2-chloro-3-fluoro-6-bromopyridine (1.0 eq.), 1.01 g of compound 1-pinacol borate-4-ethyl acetate-1-cyclohexene (1.2 eq.), 0.208 g of Pd(dppf)$Cl_2$ (0.1 eq.) and 2.32 g of cesium carbonate (2.5 eq.) were weighed and added to a 50mL round-bottom flask. 18 mL of 1,4-dioxane and 3 mL of water were added. Subsequently, the reaction solution was degassed with nitrogen twice and heated to 80°C for reaction under nitrogen atmosphere. After reaction for 2 hours, the heating was stopped, and the reaction solution was cooled to room temperature. Subsequently, the reaction solution was filtered by a funnel with diatomite and the filter cake was washed with 15 mL×2 of ethyl acetate. Subsequently, the filtrate was concentrated to dryness and purified by column chromatography to obtain 0.769 g of compound 106-A with a yield of 90.6%.

Compound 106-B:

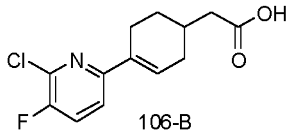
106-B

0.765 g of compound 106-A (1.0 eq.) was weighed and added to a 50mL round-bottom flask. 7.5 mL of methanol and 11.5 mL of tetrahydrofuran were added. Subsequently, 2.6 mL of 2 mol/L NaOH solution (2.0 eq.) was added dropwise to the reaction solution at room temperature. After the dropwise addition, the reaction solution was continuously reacted for 1 hour at room temperature. After the reaction, the reaction solution was concentrated. After the concentration, 20 mL of water was added, and the pH of the reaction system was adjusted to be 4-5 with 1 mol/L HCl solution. Then, the reaction solution was filtered, and the filter cake was washed with 10 mL×2 of water, and then dried to obtain 0.612 g of compound 106-B with a yield of 88.3%.

Compound 106-C:

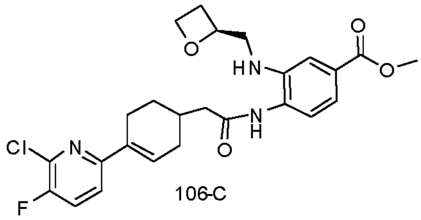

106-C

0.61 g of compound 106-B (1.0 eq.), 0.615 g of compound 1-F (1.1 eq.) and 0.390 g of N-methylimidazole (2.1 eq.) were weighed and added to a 50mL round-bottom flask. 13.0 mL of acetonitrile was added. The reaction solution was stirred for 20 minutes in an ice bath, and then 0.697 g of N,N,N',N'-tetramethylchloroformamidinium hexafluorophosphate (1.1 eq.) was weighed and added. The reaction solution was continuously stirred and reacted for 0.5 hours in the ice bath. Subsequently, the reaction solution was transferred to room temperature and continuously reacted for 3 hours. After the reaction, 36 mL of $H_2O$ was added to the reaction system, the system was stirred for 0.5 hours and filtered. The filter cake was washed with 20 mL×3 of $H_2O$, and dried at 50°C to obtain 1.0 g of compound 106-C with a yield of 90.6%. MS m/z (ESI): 488.1745 $(M+H)^+$.

Compound 106-D:

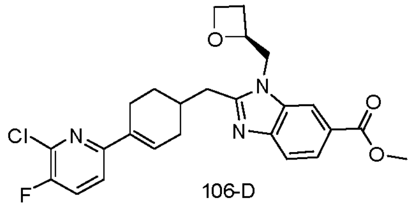

106-D

1.0 g of compound 106-C (1.0 eq.) was weighed and added to a 50mL round-bottom flask. 15.0 mL of 1,2-dichloroethane was added, and then 4.923 g of acetic acid (40.0 eq.) was added dropwise. After the dropwise addition, the reaction solution was placed at 90°C for reaction under heating. After 5 hours, the reaction solution was cooled to room temperature and concentrated to remove solvent, then 15 mL of water was added, and the pH of the reaction solution was adjusted to be 7-8 with 5% $NaHCO_3$ aqueous solution. The reaction solution was filtered. The filter cake was washed with a small amount of water and dried to obtain 0.81 g of compound 106-D with a yield of 84%. MS m/z (ESI): 470.1629 $(M+H)^+$.

Compound 106-E:

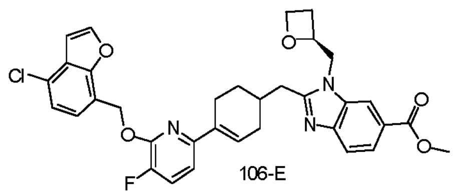

106-E

0.35 g of compound 106-D (1.0 eq.), 0.204 g of compound 1-C (1.5 eq.), 69 mg of $Pd_2(dba)_3$ (0.1 eq.), 69.5 mg of 2-dicyclohexylphosphino-2',6'-diisopropoxybiphenyl (0.2 eq.) and 0.485 g of cesium carbonate (2 eq.) were weighed and added to a 25mL round-bottom flask. 5.5 ml of toluene was added. Subsequently, the reaction solution was degassed with nitrogen twice and heated to 110°C for reaction under nitrogen atmosphere. After reaction for 12 hours, the heating was stopped, and the reaction solution was cooled to room temperature. Subsequently, the reaction solution was filtered by a funnel with diatomite and the filter cake was washed with 10 mL×2 of ethyl acetate. Subsequently, the filtrate was concentrated to dryness and purified by column chromatography to obtain 0.201 g of compound 106-E with a yield of 43.9%. MS m/z (ESI): 616.2022$(M+H)^+$.

Compound 106:

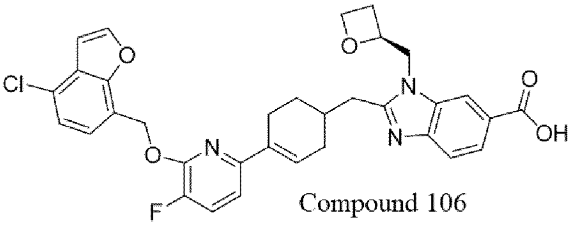

Compound 106

0.2 g of compound 106-E (1.0 eq.) was weighed and added to a 25mL round-bottom flask. 1 mL of methanol and 3 mL of tetrahydrofuran were added, and then 0.6 mL of 2 mol/L sodium hydroxide solution was added. Subsequently, the reaction solution was reacted at room temperature. After 20 hours, the reaction solution was concentrated. After the concentration, 5 mL of water and 0.5 mL of acetone was added to the reaction solution. The pH of the reaction system was adjusted to be 4-5 with 1 mol/L HCl solution. The reaction system was filtered and the filter cake was washed with a small amount of water, and then dried to obtain 0.182 g of compound 106 with a yield of 93.1%. MS m/z (ESI): 602.1858 $(M+H)^+$, $^1H$ NMR (400 MHz, DMSO-d6) δ= 8.21 (s, 1H), 8.17 (d, J = 2.4 Hz, 1H), 7.81 (dd, J = 8.4, 1.6 Hz, 1H), 7.61 (dd, J = 19.2, 10.4 Hz, 2H), 7.44 (d, J = 8.0 Hz, 1H), 7.36 (d, J = 8.0 Hz, 1H), 7.06 (dd, J = 8.4, 6.4 Hz, 2H), 6.66 (s, 1H), 5.74 (s, 2H), 5.05-5.00 (m, 1H), 4.64 (dd, J = 15.6, 7.2 Hz, 1H), 4.52-4.43 (m, 2H), 4.30 (m, 1H), 3.04-2.92 (m, 2H), 2.73-2.65 (m, 1H), 2.45- 2.33 (m, 4H), 2.06-1.96 (m, 2H), 1.50-1.17 (m, 2H).

Example 52:

Synthesis of Compound 107:

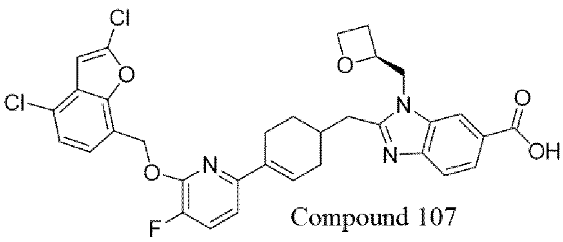

Compound 107

Synthesis route:

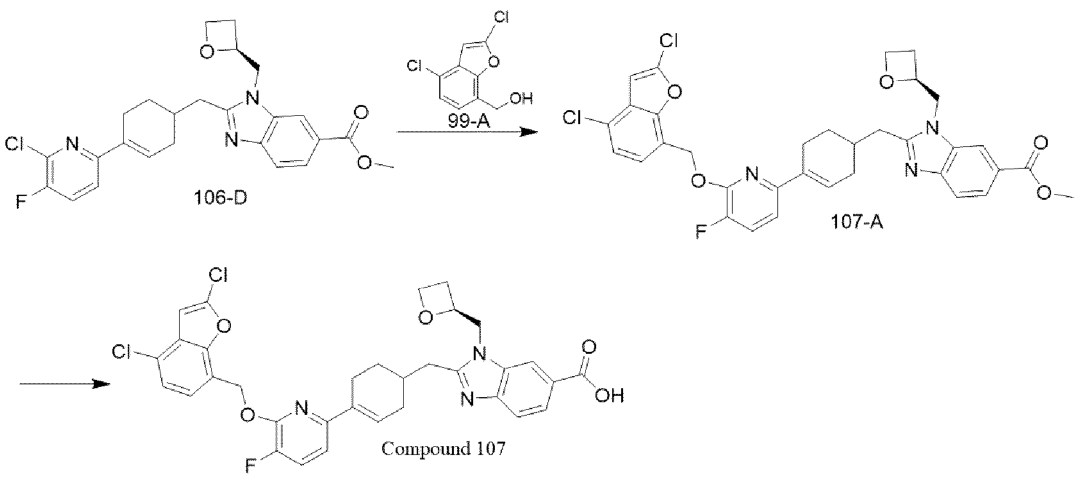


106-D

107-A

Compound 107

Compound 107-A:

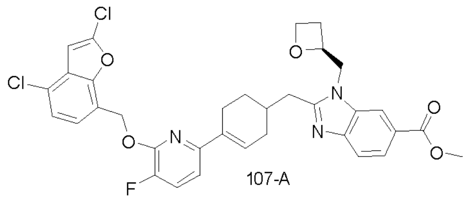

107-A

0.35 g of compound 106-D (1.0 eq.), 0.243 g of compound 99-A (1.5 eq.), 69 mg of $Pd_2(dba)_3$ (0.1 eq.), 70 mg of 2-dicyclohexylphosphino-2',6'-diisopropoxybiphenyl (0.2 eq.) and 0.485 g of cesium carbonate (2 eq.) were weighed and added to a 25mL round-bottom flask. 5.5 ml of toluene was added. Subsequently, the reaction solution was degassed with nitrogen twice and heated to 110°C for reaction under nitrogen atmosphere. After reaction for 12 hours, the heating was stopped, and the reaction solution was cooled to room temperature. Subsequently, the reaction solution was filtered by a funnel with diatomite and the filter cake was washed with 10 mL×2 of ethyl acetate. Subsequently, the filtrate was concentrated to dryness and purified by column chromatography to obtain 56 mg of compound 107-A with a yield of 11.5%. MS m/z (ESI): 650.1639 $(M+H)^+$.

Compound 107:

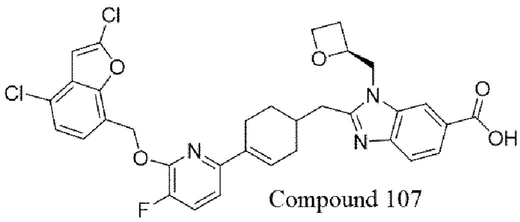

Compound 107

55 mg of compound 107-A (1.0 eq.) was weighed and added to a 25mL round-bottom flask. 0.3 mL of methanol and 0.9 mL of tetrahydrofuran were added, and then 0.15 mL of 2 mol/L sodium hydroxide solution was added. Subsequently, the reaction solution was reacted at room temperature. After 20 hours, the reaction solution was concentrated. After the concentration, 2 mL of water and 0.25 mL of acetone were added to the reaction solution. The pH of the reaction system was adjusted to be 4-5 with 1 mol/L HCl solution. The reaction system was filtered and the filter cake was washed with a small amount of water, and then dried to obtain 45 mg of compound 107 with a yield of 83.6%. MS m/z (ESI): 636.1473 $(M+H)^+$, $^1H$ NMR (400 MHz, DMSO-d6) $\delta$= 12.71 (s, 1H), 8.22 (s, 1H), 7.80 (dd, J = 8.4, 1.2 Hz, 1H), 7.64-7.58 (m, 2H), 7.46-7.35 (m, 2H), 7.17 (s, 1H), 7.06 (dd, J = 7.2, 2.4 Hz, 1H), 6.64 (d, J = 11.6 Hz, 1H), 5.70 (s, 2H), 5.03 (dd, J = 14, 7.2 Hz, 1H), 4.64 (dd, J = 15.6, 7.2 Hz, 1H), 4.53-4.43 (m, 2H), 4.30 (dd, J = 14.8, 6.4 Hz, 1H), 3.04-2.91 (m, 2H), 2.73-2.65 (m, 1H), 2.41-2.33 (m, 4H), 2.06-1.95 (m, 2H), 1.49-1.24 (m, 2H).

Example 53:

Synthesis of Compound 108:

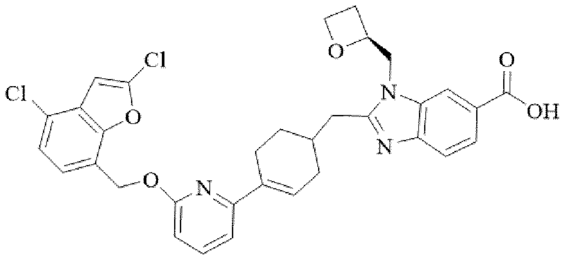

Compound 108

Synthesis route:

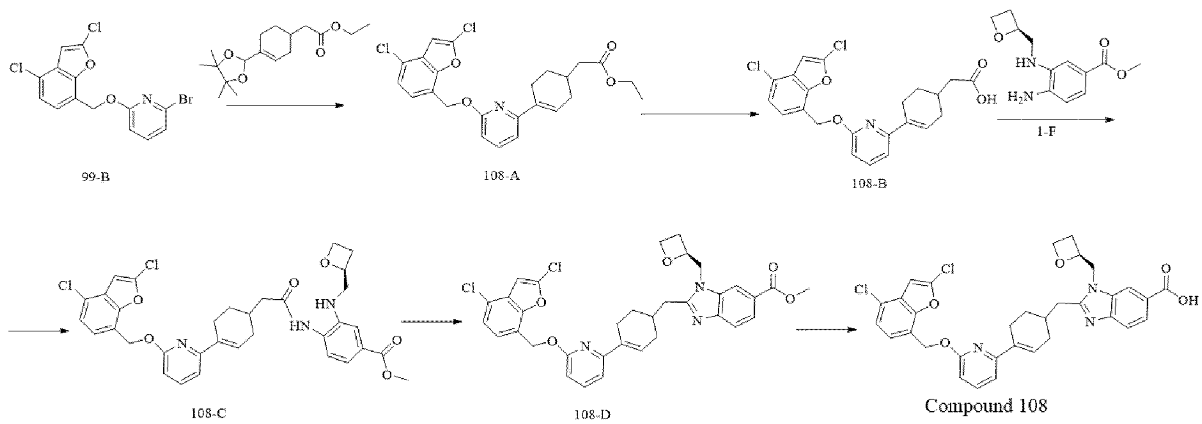


Compound 108-A:

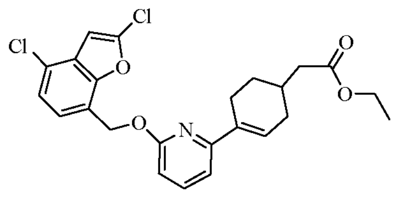

108-A

630 mg of compound 99-B (1.0 eq.), 497.2 mg of compound 1-pinacol borate-4-ethyl acetate-1-cyclohexene (1.0 eq.), 74.2 mg of Pd(dppf)$Cl_2$ (0.06 eq.) and 1.38 g of cesium carbonate (2.5 eq.) were weighed and added to a 50mL round-bottom flask. 15.0 mL of dioxane was added. Subsequently, the reaction solution was replaced with nitrogen thrice and heated to 90°C for reaction under nitrogen atmosphere. After reaction for 2.5 hours, the heating was stopped and the reaction solution was cooled to room temperature. Subsequently, the reaction solution was filtered by a funnel with diatomite. The filtrate was concentrated to dryness and purified by column chromatography to obtain compound 108-A 327 mg, yield 42.04%.

Compound 108-B:

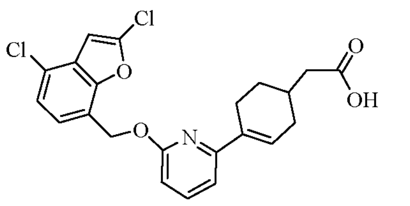

108-B

327 mg of compound 108-A (1.0 eq.) was weighed and added to a 25mL round-bottom flask. 2 mL of methanol and 2 mL of tetrahydrofuran were added. The thus obtained system was stirred to dissolve, and then 1.0 mL of 2 mol/L NaOH solution was added dropwise. After the dropwise addition, the reaction solution was continuously reacted for 3.0 hours at room temperature. After the reaction was completed, the reaction solution was concentrated. After the concentration, 10 mL of water and 30 mL of ethyl acetate were added to the reaction solution, and the pH of the reaction solution was adjusted to be 4-5 with 1 mol/L HCl solution. The reaction solution was left to stand for liquid separation, and the organic phases were collected. The organic phases were washed with 15 mL of saturated salt solution twice, dried with anhydrous $Na_2SO_4$, and filtered. The filtrate was concentrated to dryness to obtain white solid compound 108-B (320 mg).

Compound 108-C:

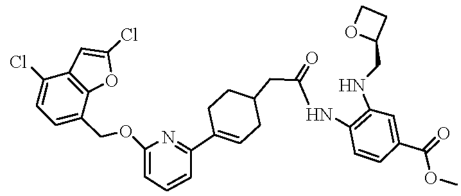

108-C

320 mg of compound 108-B (1.0 eq.) and 183.6 mg of compound 1-F (1.05 eq.) were weighed and added to a 50mL round-bottom flask. 10.0 mL of acetonitrile was added. The reaction solution was stirred for 15 minutes in an ice bath, and then 290.64 mg of N,N,N',N'-tetramethylchloroformamidinium hexafluorophosphate (1.4 eq.) and 243.0 mg of N-methylimidazole (4.0 eq.) were weighed and added. The reaction solution was continuously stirred and reacted for 1 hour in the ice bath. Subsequently, the reaction solution was transferred to room temperature and continuously reacted for 3 hours. After the reaction, 30 mL of $H_2O$ was added to the reaction system, the system was stirred for 0.5 hours, and filtered. The filter cake was washed with 20 mL of water, and then the filter cake was collected and dried to obtain compound 108-C (410 mg, yield 85.17%).

Compound 108-D:

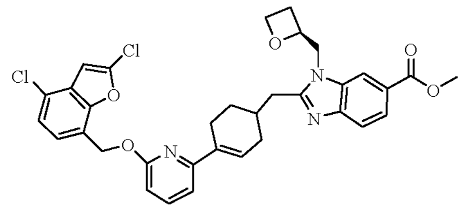

108-D

410 mg of compound 108-C (1.0 eq.) was weighed and added to a 25mL round-bottom flask. 4.0 mL of acetic acid was added. Then the reaction mixture was stirred evenly, and then heated at 90°C for reaction. After 1.5 hours, TLC showed that the raw materials were basically reacted completely. The reaction solution was cooled to room temperature and concentrated under reduced pressure. 10 mL of water and 25 mL of ethyl acetate were added to the residue, and the pH of the reaction solution was adjusted to be 7-8 with saturated $NaHCO_3$ aqueous solution. Subsequently, the reaction solution was left to stand for liquid separation, and the organic phases were separated. The organic phases were washed with 10 mL of saturated salt solution twice, dried with anhydrous $Na_2SO_4$, and filtered. The filtrate was concentrated to dryness and purified by column chromatography to obtain compound 108-D (220 mg, yield 55.21%).

Preparation of Compound 108:

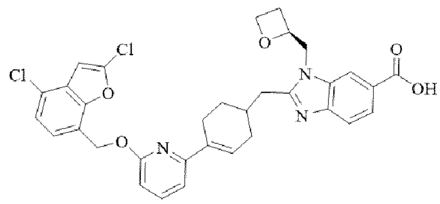

Compound 108

220 mg of compound 108-D (1.0 eq.) was weighed and added to a 25mL round-bottom flask. 1.5 mL of methanol and 2.5 mL of tetrahydrofuran were added. The thus obtained system was stirred to dissolve, and then 0.8 mL of 2 mol/L NaOH aqueous solution was added. Subsequently, the reaction solution was reacted at room temperature. TLC showed that the raw materials were basically reacted completely. After the reaction solution was concentrated, 5 mL of water and 3 mL of ethyl acetate were added to the residue, and the obtained system was left to stand for liquid separation. The aqueous phase was collected, and the pH of the aqueous phase was adjusted to be 4-5 with citric acid solution. 10 mL of ethyl

acetate was added for extraction, and the organic phases were washed with 5 mL of saturated salt solution twice, dried with anhydrous $Na_2SO_4$ and filtered. The filtrate was concentrated to dryness to obtain compound 108 (200 mg, yield 93%). MS m/z (ESI): 618.1560 (M+H)+, $^1$HNMR (400 MHz, CDCl3) δ=8.22 (s, 1H), 8.09 (d, J = 8.6 Hz, 1H), 7.88 (d, J = 8.5 Hz, 1H), 7.53 (t, J = 7.8 Hz, 1H), 7.35 (d, J = 8.0 Hz, 1H), 7.22 (d, J = 8.1 Hz, 1H), 6.94 (d, J = 7.5 Hz, 1H), 6.76 (s, 1H), 6.71 (s, 1H), 6.66 (d, J = 8.2 Hz, 1H), 5.65 (s, 2H), 5.21 (d, J = 7.2 Hz, 1H), 4.64 (d, J = 7.2 Hz, 1H), 4.58-4.33 (m, 3H), 3.15 (d, J = 6.5 Hz, 2H), 2.82-2.71 (m, 1H), 2.65 (d, J = 17.4 Hz, 1H), 2.48 (s, 4H), 2.16 (d, J = 13.7 Hz, 2H), 1.63 (m, 1H).

Example 54:

Synthesis of compound DB06:

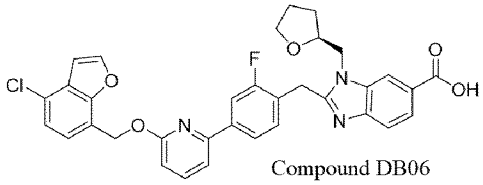

Compound DB06

Synthesis route:

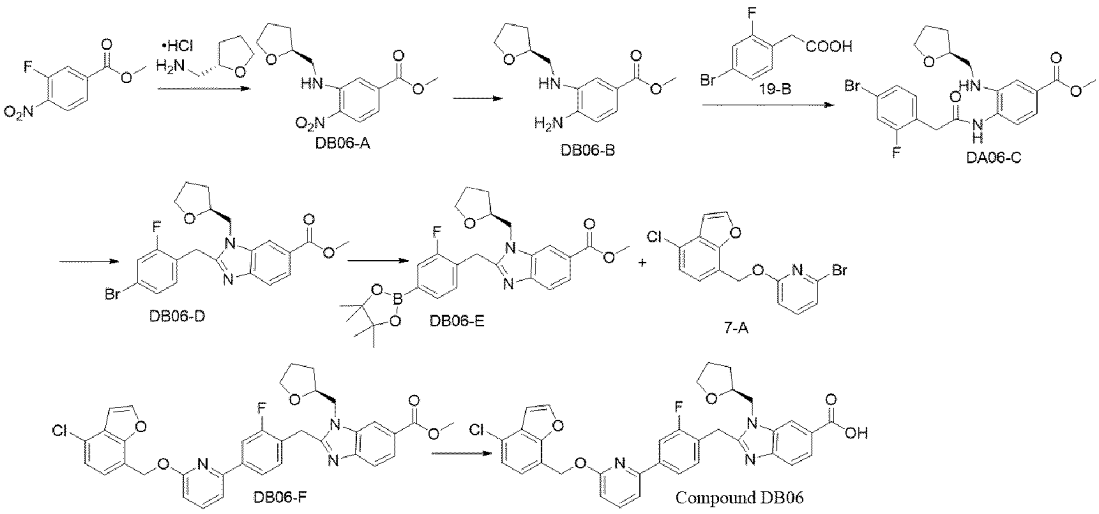


Compound DB06-A:

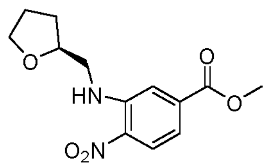

DB06-A

2.0 g of methyl 3-fluoro-4-nitrobenzoate (1.0 eq.) and 1.45 g of (S)-(tetrahydrofuran-2-yl) formamide hydrochloride (1.05 eq.) were weighed and added to a 100mL round-bottom flask. 10 mL of N,N-dimethylformamide, 10 mL of tetrahydrofuran and 4.07 g of triethylamine (4.0 eq.) were added. The rection mixture was stirred evenly, then heated to 50°C for reaction for about 6 hours. After the reaction was completed, the reaction solution was concentrated to remove tetrahydrofuran. The residue was added with 30 mL of water, and a large amount of yellow solids were precipitated. The reaction solution was continuously stirred for about 0.5 hours, and then was subjected to suction filtration, and washed with a small amount of water. The filter cake was dried to obtain 2.64 g of compound DB06-A with a yield of 94.0%, which was

directly used in the next reaction.

Compound DB06-B:

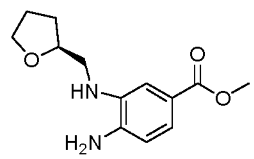

DB06-B

2.64 g of compound DB06-A (1.0 eq.) was weighed and added to a 100mL round-bottom flask. 26 mL of methanol was added. The reaction mixture was stirred evenly. 260 mg of Pd/C (10%) was then added, and the system was replaced with hydrogen thrice. The reaction solution was reacted for 3 hours at room temperature under hydrogen atmosphere. After the reaction, the reaction solution was filled with diatomite and was filtered. The filtrate was concentrated under reduced pressure to obtain 2.43 g of brown oily compound DB06-B with a yield greater than 100%, which was directly used in the next reaction.

Compound DB06-C:

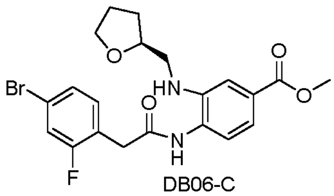

DB06-C

2.06 g of compound 19-B (1.0 eq.) and 2.43 g of compound DB06-B (1.1 eq.) were weighed and added to a 150mL round-bottom flask. 41 mL of acetonitrile was added. The reaction mixture was stirred and cooled in an ice bath, then 1.52 g of NMI (2.1 eq.) and 2.73 g of TCFH (1.1 eq.) were added. The reaction solution was continuously stirred for about 0.5 hours, then the ice bath was removed, and the reaction solution was reacted overnight at room temperature. After the reaction was completed, about 80 mL of water was added, and a large amount of off-white solids were precipitated. The reaction solution was continuously stirred for about 0.5 hours, and then was subjected to suction filtration, and washed with water. The filter cake was dried to obtain 2.71 g of compound DB06-C with a yield of 65.9%, which was directly used in the next reaction.

Compound DB06-D:

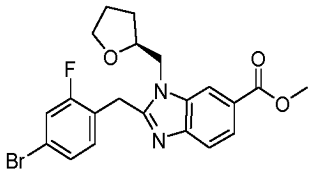

DB06-D

2.71 g of compound DB06-C was weighed and added to a 150mL round-bottom flask. 27.0 mL of 1,2-dichloroethane was added, and then 6.7 mL of acetic acid (20.0 eq.) was then added dropwise. After the dropwise addition, the reaction solution was placed at 60°C for reaction under heating. After reaction for 5 hours, the reaction solution was cooled to room temperature, and then the reaction solution was concentrated. After the concentration, 20.0 mL of acetonitrile and 20 mL of water were added, and the pH of the reaction solution was adjusted to be 7-8 with saturated sodium bicarbonate solution. A large amount of off-white solids were precipitated, and then the reaction solution was continuously stirred at room temperature for 0.5 hours, filtered and washed with water. The filter cake was dried to obtain 2.28 g of compound DB06-D with a yield of 87.5%, which was directly used in the next reaction.

Compound DB06-E:

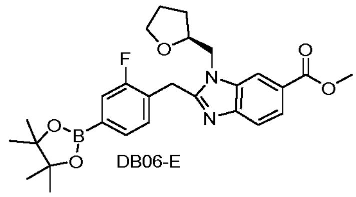

DB06-E

2.28 g of compound DB06-D (1.0 eq.), 1.56 g of bis(pinacolato)diboron (1.2 eq.), 112 mg of Pd(dppf)$Cl_2$ (0.03 eq.) and 1.25 g of potassium acetate (2.5 eq.) were weighed and added to a 150mL round-bottom flask. 34.0 mL of 1,4-dioxane was added. Subsequently, the reaction solution was degassed with nitrogen twice and heated to 100°C for reaction under nitrogen atmosphere. After reaction for 2 hours, the heating was stopped and the reaction solution was cooled to room temperature. Subsequently, the reaction solution was filtered by a funnel with diatomite and the filter cake was washed with 20 mL×3 of ethyl acetate. Subsequently, the filtrate was concentrated to dryness and purified by column chromatography to obtain 3.08 g of compound DB06-E with a yield greater than 100%, which was directly used in the next reaction.

Compound DB06-F:

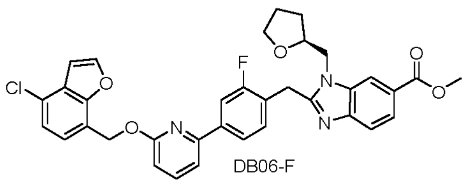

DB06-F

0.47 g of compound DB06-E (1.50 eq.), 0.20 g of compound 7-A (1.0 eq.), 14 mg of Pd(dppf)$Cl_2$ (0.03 eq.) and 515 mg of $Cs_2CO_3$ (2.5 eq.) were weighed and added to a 50mL round-bottom flask. 4.0 mL of 1,4-dioxane was added. Subsequently, the reaction solution was degassed with nitrogen twice and heated to 100°C for reaction under nitrogen atmosphere. After reaction for 2 hours, the heating was stopped and the reaction solution was cooled to room temperature. Subsequently, the reaction solution was filtered by a funnel with diatomite and the filter cake was washed with 10 mL×2 of ethyl acetate. Subsequently, the filtrate was concentrated to dryness and purified by column chromatography to obtain 0.29 g of compound DB06-F with a yield of 78.3%.

Compound DB06:

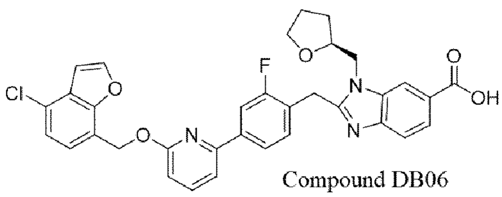

Compound DB06

0.15 g of compound DB06-F (1.0 eq.) was weighed and added to a 50mL round-bottom flask. 6.0 mL of tetrahydrofuran and 2.0 mL of methanol were added. The reaction mixture was stirred evenly, 0.51 mL of 2 mol/L sodium hydroxide solution (4.0 eq.) was slowly dropwise added, and the system was heated to 40°C for reaction for about 4 hours. After the reaction was completed, the reaction solution was concentrated, and 4 mL of water and 0.5 mL of acetone were added. The pH of the reaction solution was adjusted to be 4-5 with citric acid solution, and a large amount of off-white solids were precipitated. The reaction solution was continuously stirred for about 0.5 hours, then was subjected to suction filtration and washed with water. The filter cake was dried to obtain 127 mg of compound DB06 with a yield of 87.0%. MS m/z (ESI):612.1634 (M+H)+; $^1H$ NMR (400 MHz, DMSO) δ =12.87 (s, 1H), 8.22 (d, J = 0.9 Hz, 1H), 7.94 - 7.87 (m, 2H), 7.86 - 7.76 (m, 2H), 7.67 - 7.55 (m, 3H), 7.53 - 7.40 (m, 2H), 7.32 (dd, J = 8.2, 1.8 Hz, 1H), 6.88 (d, J = 8.2 Hz, 1H), 5.52 (s, 2H), 4.58 - 4.30 (m, 4H), 4.16 (dt, J = 12.2, 4.8 Hz, 1H), 3.79 (dd, J = 14.7, 6.9 Hz, 1H), 3.63 (dd, J = 14.1, 7.6 Hz, 1H), 2.06 (dt, J = 12.2, 5.9 Hz, 1H), 1.93 - 1.74 (m, 2H), 1.69 - 1.55 (m, 1H).

Comparative Example 1:

Compound PF-06882961:

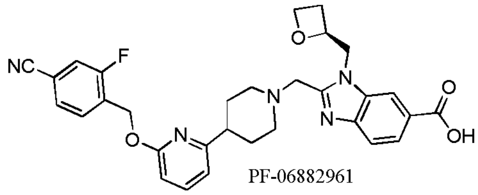

PF-06882961

Synthesis route:

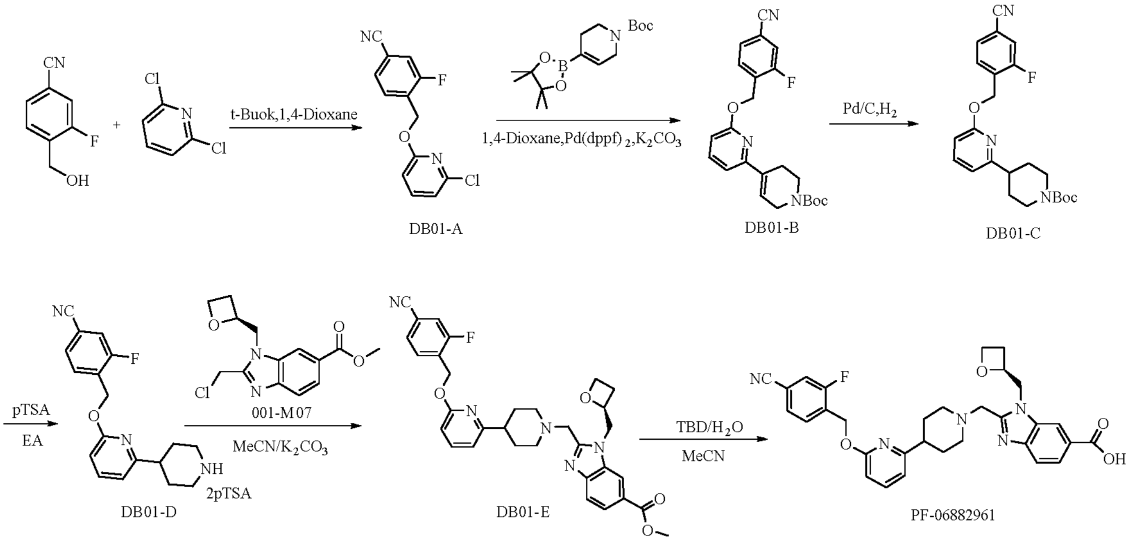


DB01-A DB01-B DB01-C DB01-D 2pTSA DB01-E PF-06882961

Compound DB01-A:

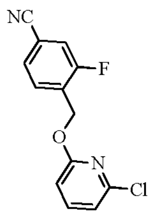

DB01-A

8.0 g of 2,6-dichloropyridine (1.0 eq.) and 8.6 g of 3-fluoro-4-(hydroxymethyl)benzonitrile (1.0 eq.) were weighed and added to a 250mL round-bottom flask. 80 mL of 1,4-dioxanewas added, the thus obtained system was stirred to dissolve. Mixed solution of THF (40 mL) containing 7.28 g of potassium tert-butoxide (1.2 eq.) was added dropwise to the above mixed solution. After the dropwise addition, the reaction solution was stirred at 40°C for 60 minutes. TLC showed that the raw materials disappeared. The heating of the reaction was stopped, and the reaction solution was cooled to room temperature. 240 mL of water was added to the reaction solution, then a large amount of solids were precipitated, which were filtered, then the filter cake was washed with water and dried to obtain light yellow solid DB01-A (12.42 g, yield 87.59%). $^1$HNMR (400 MHz, CDCl3) δ =7.68 (t, J = 7.5 Hz, 1H), 7.59 (dd, J = 8.1, 7.6 Hz, 1H), 7.50 (dd, J = 7.9, 1.5 Hz, 1H), 7.41 (dd, J = 9.2, 1.5 Hz, 1H), 6.98 (dd, J = 7.6, 0.6 Hz, 1H), 6.76 (dd, J = 8.2, 0.6 Hz, 1H), 5.50 (s, 2H).

Compound DB01-B:

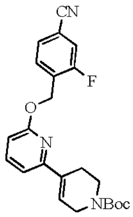

DB01-B

6.0 g of compound DB01-A (1.0 eq.), 7.73 g of 4-(4,4,5,5-tetramethyl-1,3,2-dioxaborolan-2-yl)-3,6-dihydro-2H-pyridine-1-carboxylic acid tert-butyl ester (1.1 eq.), 1.68 g of Pd(dppf)$Cl_2$ (0.1 eq.) and 2.9 g of $NaHCO_3$ (1.5 eq.) were weighed and added to a 250mL round-bottom flask. 90 mL of 1,4-dioxane and 18.0 mL of $H_2O$ were added. Then the reaction mixture was stirred evenly, replaced with nitrogen for four times, and placed at 90°C for reaction under heating for 3 hours under nitrogen atmosphere. TLC showed that the raw materials disappeared. The heating of the reaction was stopped and the reaction solution was cooled to room temperature. Subsequently, the reaction solution was concentrated and purified by column chromatography (PE/EtOAc = 95/5) 95/5) to obtain a solid product DB01-B (8.6 g, yield 92.0%).

Compound DB01-C:

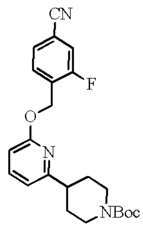

DB01-C

8.6 g of compound DB01-B (1.0 eq.) was weighed and added to a 500mL round-bottom flask. 2.58 g of Pd/C (10% content) and 215 mL of toluene were added. Then the reaction mixture was stirred evenly and replaced with hydrogen thrice, stirred for reaction under hydrogen atmosphere. HPLC showed that the raw materials were completely reacted. The reaction solution was filtered with diatomite, and the filter cake was washed with methanol. The filtrate was concentrated under reduced pressure to obtain oily compound DB01-C (8.17 g, yield 94.6%). $^1$HNMR (400 MHz, CDCl3) $\delta$ =7.64 (t, J = 7.5 Hz, 1H), 7.55 (t, J = 7.5 Hz, 1H), 7.46 (d, J = 7.9Hz, 1H), 7.40 (d, J = 9.3Hz, 1H), 6.77 (d, J = 7.3 Hz, 1H), 6.67 (d, J = 8.1 Hz, 1H), 5.51 (s, 2H), 4.23 (brs, 2H), 2.83 (t, J = 11.3 Hz, 2H), 2.72 (tt, J = 11.8, 3.7 Hz, 1H), 1.84 (d, J = 12.1 Hz, 2H), 1.69 (d, J = 15.9Hz, 2H), 1.51 (s, 9H).

Compound DB01-D:

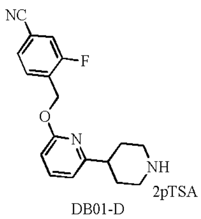

2pTSA

DB01-D

8.2 g of compound DB01-C (1.0 eq.) and 9.47 g (2.5 eq.) of pTSA monohydrate were weighed and added to a 250mL round-bottom flask. 190 mL of ethyl acetate was added. Then the reaction mixture was stirred evenly and reacted at 60°C for about 30 minutes. TLC showed that the raw materials disappeared. The heating of the reaction was stopped and the reaction solution was cooled to room temperature. A large amount of off-white solid compounds were precipitated, which were filtered and dried to obtain 2-molecule p-toluenesulfonate compound DB01-D (11.14 g, yield 85.69%).

Compound DB01-E:

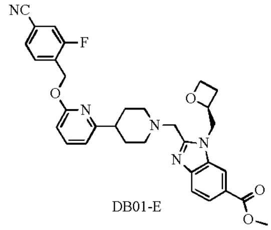

DB01-E

2.7 g of compound DB01-D (1.0 eq.), 1.21 g of compound 001-M07 (1.0 eq.) and 2.84 g of potassium carbonate (5.0 eq.) were weighed and added to a 150mL round-bottom flask. 54 mL of acetonitrile was added. The reaction solution was stirred evenly and heated at 50°C for reaction for 3 hours. TLC showed that two raw materials were completely reacted. The heating was stopped and then subsequently, the reaction solution was cooled to room temperature, 108 mL of water and 50 mL of ethyl acetate were added, and the thus obtained system was left to stand for liquid separation. The aqueous phase was extracted with 50 mL of ethyl acetate, the organic phases were combined and washed with 50 mL of saturated sodium chloride solution twice, dried with anhydrous sodium sulfate and filtered. The filtrate was concentrated under reduced pressure to obtain an oily substance. A mixed solvent (PE/EA= 3/1) was used for pulping and purifying to obtain a solid product DB01-E (2.07 g, yield 88.08%).

Compound PF-06882961

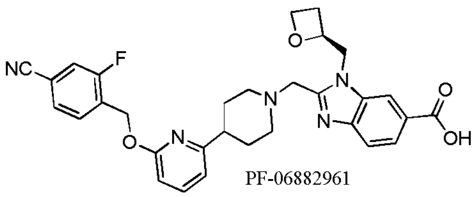

PF-06882961

500 mg of compound DB01-E (1.0 eq.) was weighed and added to a 50mL round-bottom flask. 9 mL of acetonitrile was added. Water solution (1.8 mL) containing 245 mg of TBD (2.0 eq.) was added dropwise under stirring, and the system was stirred for reaction for 24 hours at room temperature. TLC showed that the raw materials were completely reacted. The pH of the reaction solution was adjusted to be 5.0 with citric acid solution. 20 mL of ethyl acetate was added and the system was left to stand for liquid separation. The ethyl acetate organic phases were washed with 10 mL of saturated sodium chloride solution, dried with anhydrous sodium sulfate, filtered, and concentrated under reduced pressure to obtain an oily matter, which was subjected to column chromatography to obtain a white solid product PF-06882961 (0.44 g, 89.97%). MS m/z (ESI):556.2366 (M+H)+, $^1$HNMR (400 MHz, DMSO-d6) δ =12.84 (brs, 1H), 8.28 (s, 1H), 7.89 (d, J = 9.9 Hz, 1H), 7.81 (d, J = 8.9 Hz, 1H), 7.74-7.59 (m, 4H), 6.89 (d, J = 7.3 Hz, 1H), 6.72 (d, J = 8.2 Hz, 1H), 5.47 (s, 2H), 5.11 (d, J = 4.3 Hz, 1H), 4.84-4.79 (m, 1H), 4.69-4.66 (m, 1H), 4.51-4.46 (m, 1H), 4.41-4.36 (m, 1H), 3.95 (d, J = 13.5 Hz, 1H), 3.78 (d, J = 13.5 Hz, 1H), 2.98 (d, J = 10.9 Hz, 1H), 2.84 (d, J = 10.8 Hz, 1H), 2.73-2.67 (m, 1H), 2.61-2.56 (m, 1H), 2.51-2.41 (m, 1H), 2.25-2.13 (m, 2H), 1.79-1.60 (m, 4H).

Comparative Example 2:

Compound DB02:

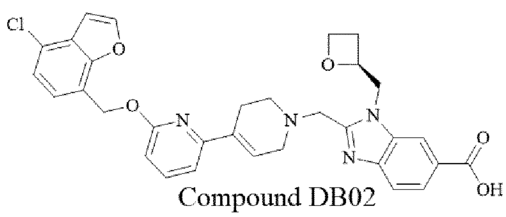

Compound DB02

Synthesis route:

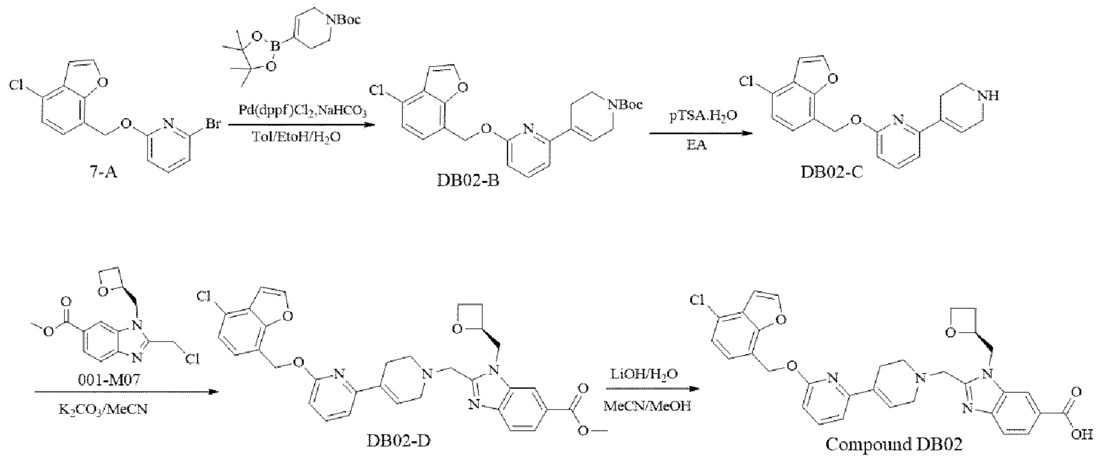


Compound DB02-B:

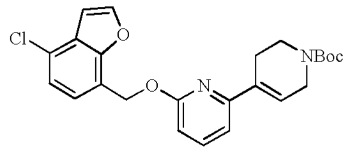

DB02-B

620 mg of compound 7-A (1.0 eq.) was weighed and added to a 100mL round-bottom flask. 622.78 mg of N-Boc-1,2,5,6-tetrahydropyridine-4-boronic acid pinacol ester (1.1 eq.), 307.61 mg of $NaHCO_3$ (2.0 eq.) and 134.0 mg of Pd(dppf)$Cl_2$ (0.1 eq.) were added. A mixed solvent (toluene/EtOH/water = 31 mL/15 mL/15 mL) was added. The reaction solution was stirred evenly and replaced with nitrogen thrice. Under the protection of nitrogen, the reaction solution was placed at 110°C for reaction under heating for 2 hours. TLC showed that the raw materials were completely reacted. Subsequently, the heating was stopped and the reaction solution was cooled to room temperature, and filtered with diatomite. 15 mL of water was added to the filtrate, the thus obtained system was stirred and left to stand for liquid separation, the organic phases were washed twice with 30 mL×2 of saturated salt solution, dried with anhydrous sodium sulfate and filtered. The filtrate was concentrated under reduced pressure to obtain oily compound DB02-B (902 mg, yield 1.12%), which was directly used in the next reaction without purification

Compound DB02-C:

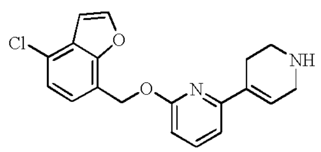

DB02-C

404 mg of compound DB02-B (1.0 eq.) and 522.72 mg of pTSA·$H_2O$ (3.0 eq.) were weighed and added to a 50mL round-bottom flask. 10 mL of ethyl acetate was added. The reaction solution was stirred evenly and placed at 65°C for reaction under heating for 2 hours. TLC showed that the raw materials were completely reacted. The heating was stopped and the reaction solution was cooled to room temperature. 5 mL of water was added to the reaction solution, and the pH of the reaction solution was adjusted to be about 8 with saturated sodium bicarbonate. The reaction solution was stirred for 10 minutes, and left to stand for liquid separation. The organic phases were collected, washed with 5 mL×2 of water twice and washed with 5 mL×2 of saturated sodium chloride solution twice, dried with anhydrous sodium sulfate and filtered. The filtrate was concentrated under reduced pressure to obtain oily compound DB02-C. The compound was directly used in the next feeding without calculating the yield and purification.

Compound DB02-D:

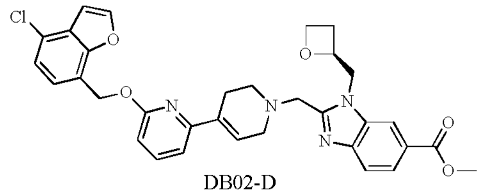

DB02-D

The compound DB02-C (1.0 eq.) obtained in the previous step was weighed and added to a 50mL round-bottom flask. 9 mL of acetonitrile, 270 mg of compound 001-M07 (1.0 eq.) and 253.20 mg of potassium carbonate (2.0 eq.) were added. The reaction solution was stirred evenly and heated at 50°C for reaction for 2 hours. TLC showed that two raw materials were completely reacted. Subsequently, the heating was stopped and the reaction solution was cooled to room temperature. 18 mL of water and 10 mL of ethyl acetate were added to the reaction solution, and the thus obtained system was left to stand for liquid separation. The aqueous phase was extracted with 10 mL of ethyl acetate. The organic phases were combined, washed with 10 mL×2 of saturated sodium chloride solution twice, dried with anhydrous sodium sulfate and filtered. The filtrate was concentrated under reduced pressure to obtain an oily crude product, which was purified by column chromatography (PE/EA = 10/90-50/50 as developing agent) to obtain compound DB02-D (460 mg, yield 83.94%).

Compound DB02:

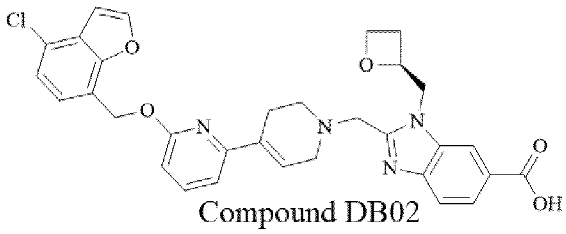

Compound DB02

460 mg of compound DB02-D (1.0 eq.) was weighed and added to a 50mL round-bottom flask. A mixed solvent (acetonitrile/methanol/water = 9 mL/3 mL/3 mL) was added, and then 48.40 mg of LiOH·$H_2O$ (1.5 eq.) was added. The reaction solution was stirred evenly and placed at 45°C for reaction under heating for 4-5 hours. TLC showed that the raw materials were completely reacted. After the reaction solution was cooled to room temperature, the solvent was evaporated under reduced pressure, and then 10 mL of water was added to the residue. The impurities were extracted with 15 mL of ethyl acetate and the aqueous phase was adjusted with citric acid till a pH of the reaction solution was 4-5. 10 mL of ethyl acetate was added to the aqueous phase and the thus obtained system was left to stand for liquid separation. The aqueous phase was continuously extracted with 10 mL×2 of ethyl acetate twice, and the ethyl acetate organic phases were combined. The organic phases were washed with saturated salt solution, dried with anhydrous sodium sulfate and filtered. The filtrate was concentrated under reduced pressure and purified by column chromatography to obtain off-white solid comparative compound DB02 (400 mg, yield 89.02%).MS m/z (ESI):585.1904 (M+H)+; $^1$HNMR (400 MHz, MeOD) δ =8.35 (d, J = 0.9 Hz, 1H), 8.05-7.99 (m, 1H), 7.87 (d, J = 2.2 Hz, 1H), 7.76 (d, J = 8.5 Hz, 1H), 7.64 (t, J = 7.9 Hz, 1H), 7.36 (d, J = 8.0 Hz, 1H), 7.23 (d, J = 8.0 Hz, 1H), 7.08 (d, J = 7.4 Hz, 1H), 6.93 (d, J = 2.2 Hz, 1H), 6.73 (d, J = 8.2 Hz, 1H), 6.67-6.69 (brs, 1H), 5.68 (s, 2H), 5.27-5.18 (m, 1H), 4.87-4.81 (dd, J = 15.5, 7.1 Hz, 1H), 4.69 (dd, J = 15.5,2.5 Hz, 1H), 4.62(m, 1H),4.49-4.38 (m, 3H), 3.65 (s, 2H), 3.21 (m, 2H), 2.79-2.76 (m,1H), 2.74-2.72 (m, 2H), 2.54-2.45 (m, 1H).

Comparative Example 3

Compound DB03:

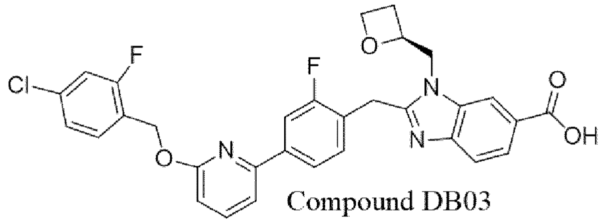

Compound DB03

Synthesis route:

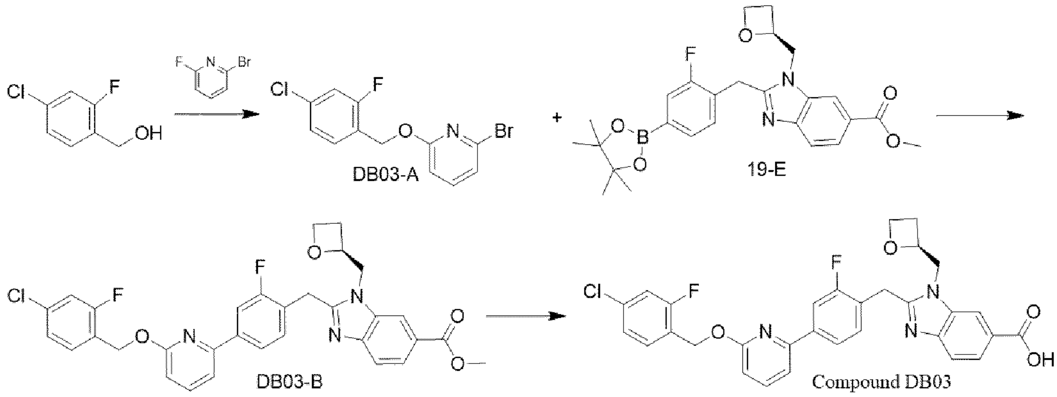

DB03-A 19-E DB03-B Compound DB03

Compound DB03-A:

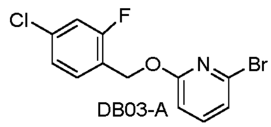

DB03-A

0.5 g of compound 2-chloro-4-fluorobenzyl alcohol (1.0 eq.), 0.822 g of 2-bromo-6-fluoropyridine (1.5 eq.) and 2.04 g of cesium carbonate (2.0 eq.) were weighed and added to a 50mL round-bottom flask. 15 mL of DMF was added. Subsequently, the reaction solution was heated to 90°C for reaction. After reaction for 4 hours, the heating was stopped, and the reaction solution was cooled to room temperature. 60 mL of water and 30 mL of ethyl acetate were added, the thus obtained system was stirred for 5 minutes, and then left to stand. The organic phases were separated, the aqueous phase was continuously washed with 30 mL×2 of ethyl acetate twice, and the organic phases were combined. Subsequently, the organic phases were washed with saturated salt solution, dried with anhydrous $Na_2SO_4$ for 5 minutes, and then filtered. The filtrate was concentrated to dryness and purified by column chromatography to obtain 0.84 g of compound DB03-A with a yield of 85%.

Compound DB03-B:

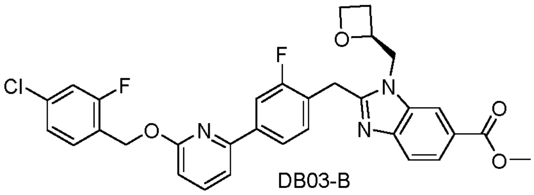

DB03-B

0.2 g of compound DB03-A (1.0 eq.), 0.364 g of compound 19-E (1.2 eq.), 14 mg of $Pd(dppf)Cl_2$ (0.03 eq.) and 0.515 g of $Cs_2CO_3$ (2.5 eq.) were weighed and added to a 25mL round-bottom flask. 6.0 mL of 1,4-dioxane and 1.0 mL of $H_2O$ were added. Subsequently, the reaction solution was degassed with nitrogen twice and heated to 90°C for reaction under nitrogen atmosphere. After reaction for 3 hours, the heating was stopped and the reaction solution was cooled to

room temperature. Subsequently, the reaction solution was filtered by a funnel with diatomite and the filter cake was washed with 10 mL×2 of ethyl acetate. Subsequently, the filtrate was concentrated to dryness and purified by column chromatography to obtain 0.275 g of compound DB03-B with a yield of 74%. MS m/z (ESI): 590.1583 (M+H)+.

Compound DB03:

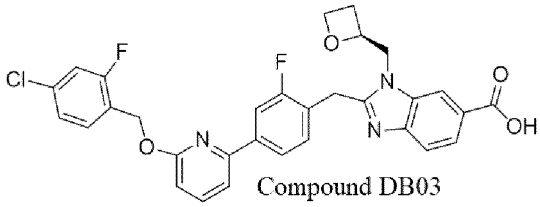

Compound DB03

0.272 g of compound DB03-B (1.0 eq.) was weighed and added to a 25mL round-bottom flask. 1.5 mL of methanol and 4.5 mL of tetrahydrofuran were added, and then 0.47 mL of 2N sodium hydroxide solution was added. Subsequently, the reaction solution was reacted at room temperature. After 15 hours, the reaction solution was concentrated. After the concentration, 5 mL of water was added to the residue, and the pH of the reaction system was adjusted to be 4-5 with 1 mol/L HCl solution. Subsequently, the reaction solution was filtered. The filter cake was pulped with 5 mL of ethyl acetate and 1 mL of methanol, and then filtered. The filter cake was washed with 3 mL×3 of ethyl acetate and dried to obtain 0.173 g of compound DB03 with a yield of 65%. MS m/z (ESI): 576.1499 (M+H)+, $^1$H NMR (400 MHz, DMSO-d6) δ = 8.26 (s, 1H), 7.93-7.79 (m, 4H), 7.65-7.58 (m, 3H), 7.48-7.42 (m, 2H), 7.32 (d, J = 8.0 Hz, 1H), 6.88 (d, J = 8.0 Hz, 1H), 5.52 (s, 2H), 5.07-5.02 (m, 1H), 4.73 (dd, J = 15.6, 7.2 Hz, 1H), 4.52-4.33 (m, 5H), 2.74-2.66 (m, 1H), 2.42-2.34 (m, 1H).

Comparative Example 4:

Compound DB04:

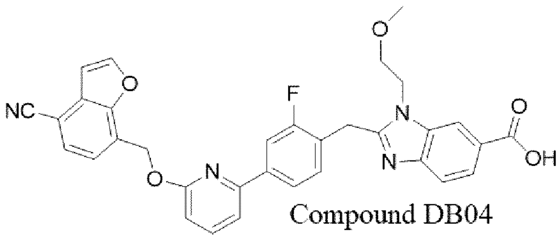

Compound DB04

Synthesis route:

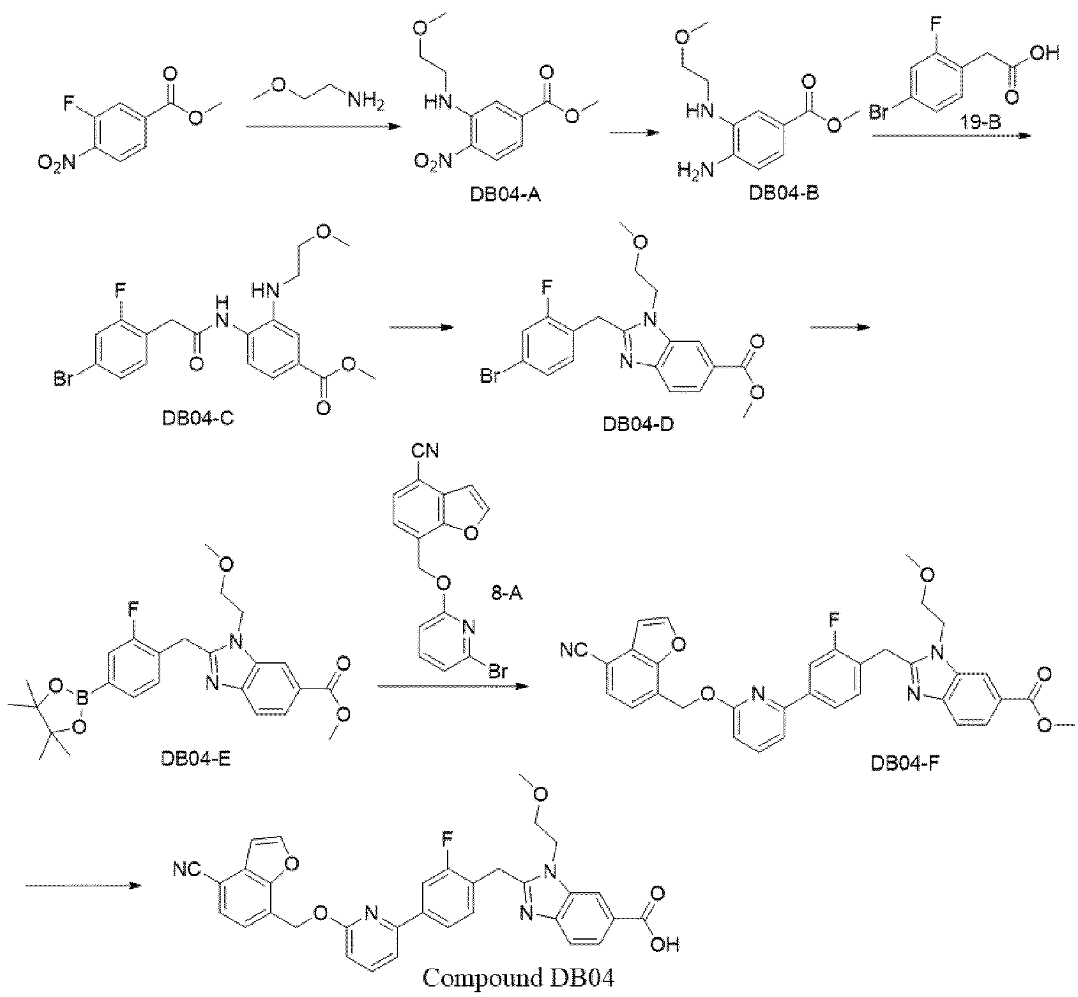

Compound DB04

Compound DB04-A:

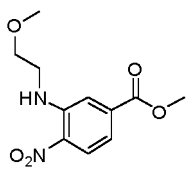
DB04-A

3 g of methyl 3-fluoro-4-nitrobenzoate (1.0 eq.), 1.36 g of 2-methoxyethanamine (1.2 eq.) and 3.05 g of triethylamine (2.0 eq.) were weighed and added to a 250mL round-bottom flask. 15 mL of N,N-dimethylformamide and 15 mL of tetrahydrofuran were added. The reaction mixture was stirred evenly and then reacted at 60°C for about 8 hours until TLC showed that the reaction was completed. The reaction solution was cooled to room temperature and concentrated to remove most tetrahydrofuran, then 50 mL of water was added to precipitate a large amount of yellow solids, the system was continuously stirred for about 0.5 hours and then subjected to suction filtration, then washed. The filter cake was dried to obtain 3.39 g of compound DB04-A with a yield of 88.5%.

Compound DB04-B:

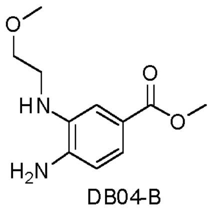

DB04-B

3.39 g of compound DB04-A (1.0 eq.) was weighed and added to a 250mL round-bottom flask. 50 mL of methanol was added, the thus obtained system was stirred evenly, and then 0.51 g of Pd/C (10%) was added. The reaction mixture was replaced with hydrogen thrice, reacted for about 2 hours at room temperature under hydrogen atmosphere until TLC showed that the reaction was completed. The reaction solution was filtered with diatomite, and the filtrate was concentrated to dryness to obtain 2.99 g of compound DB04-B with a yield of 100%.

Compound DB04-C:

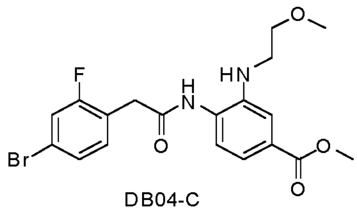

DB04-C

2.0 g of compound 19-B (1.0 eq.) and 2.12 g of compound DB04-B (1.1 eq.) were weighed and added to a 250mL round-bottom flask. 40 mL of acetonitrile was added, stirred and cooled in an ice bath, then 1.48 g of NMI (2.1 eq.) and 2.65 g of TCFH (1.1 eq.) were added. The reaction mixture was continuously stirred for about 0.5 hours. The ice bath was then removed, and the reaction solution was reacted overnight at room temperature until TLC showed that the reaction was completed. About 100 mL of water was added into the reaction solution and a large amount of off-white solids were precipitated. The reaction solution was continuously stirred for about 0.5 hours, and then filtered, and washed with water. The filter cake was dried to obtain 2.98 g of compound DB04-C with a yield of 79.0%.

Compound DB04-D:

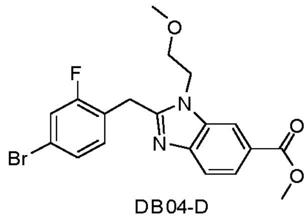

DB04-D

2.98 g of compound DB04-C (1.0 eq.) was weighed and added to a 250mL round-bottom flask. 45 mL of 1,2-dichloroethane was added. The thus obtained system was stirred evenly, and 8.15 g of glacial acetic acid (20.0 eq.) was slowly dropwise added. After the addition, the reaction solution was heated to 80°C and reacted for about 3 hours until TLC showed that the reaction was completed. After the reaction solution was cooled to room temperature, 100 mL of ethyl acetate and 50 mL of 3% potassium carbonate solution were added, the thus obtained system was stirred for a while, and then subjected to liquid separation. The aqueous phase was extracted with 50 mL of ethyl acetate. The ethyl acetate phases were combined, washed with 50 mL×2 of saturated sodium chloride solution twice, dried with anhydrous sodium sulfate, filtered and concentrated to obtain 2.86 g of compound DB04-D with a yield of 100%.

Compound DB04-E:

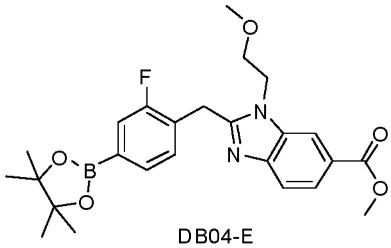

DB04-E

2.86 g of compound DB04-D (1.0 eq.), 2.07 g of bis(pinacolato)diboron (1.2 eq.), 149 mg of Pd(dppf)$Cl_2$ (0.03 eq.) and 1.67 g of potassium acetate (2.5 eq.) were weighed and added to a 250mL round-bottom flask. 45 mL of 1,4-dioxane was added. Then the reaction mixture was stirred evenly, degassed with nitrogen twice, and heated to 100°C for reaction for about 2 hours under nitrogen atmosphere until TLC showed that the reaction was completed. Subsequently, the reaction solution was cooled to room temperature, filtered with diatomite, and washed with dichloromethane. After concentration, the filtrate was purified by column chromatography to obtain 3.12 g of compound DB04-E with a yield of 98.1%.

Compound DB04-F:

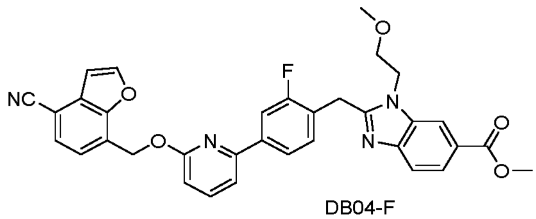

DB04-F

200 mg of compound 8-A (1.0 eq.), 299 mg of compound DB04-E (1.05 eq.), 13.3 mg of Pd(dppf)$Cl_2$ (0.03 eq.) and 495 mg of cesium carbonate (2.5 eq.) were weighed and added to a 25mL round-bottom flask. 5 mL of 1,4-dioxane and 1 mL of water were added. The reaction solution was stirred evenly, degassed with nitrogen twice, and heated to 90°C for reaction under nitrogen atmosphere for about 2 hours until TLC showed that the reaction was completed. The reaction solution was cooled to room temperature, filtered with diatomite, and washed with dichloromethane. After concentration, the filtrate was purified by column chromatography to obtain 173 mg of compound DB04-F with a yield of 48.2%. MS m/z (ESI): 591.2046 $(M+H)^+$.

Compound DB04:

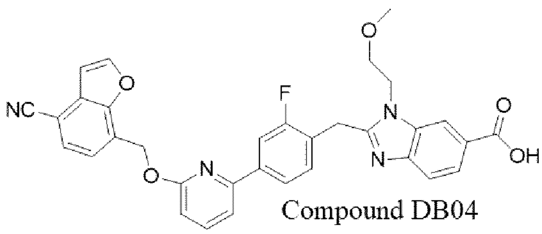

Compound DB04

170 mg of compound DB04-F (1.0 eq.) was weighed and added to a 25mL round-bottom flask. 5 mL of acetonitrile and 1 mL of water were added. The thus obtained system was stirred evenly, 100 mg of TBD (2.5 eq.) was then added. Then the reaction mixture was stirred for reaction at room temperature for about 48 hours until TLC showed that the reaction was completed. The reaction solution was concentrated, 8 mL of water was added to the residue, and the pH of the reaction solution was adjusted to be about 5.0 with citric acid. A number of off-white solids were precipitated. The reaction solution was continuously stirred for about 0.5 hours and was then subjected to suction filtration, and washed with a small amount of water. The obtained filter cake was recrystallized with dichloromethane/methanol to obtain 76 mg of compound DB04 with a yield of 45.8%. $^1H$ NMR (400 MHz, DMSO) $\delta$ =12.56 (s, 1H), 8.36 (d, $J$ = 1.7 Hz, 1H), 8.19 (s, 1H), 7.90 - 7.74 (m, 5H), 7.69 - 7.53 (m, 3H), 7.39 (t, $J$ = 8.0 Hz, 1H), 7.23 (d, $J$ = 1.8 Hz, 1H), 6.93 (d, J = 8.2 Hz, 1H), 5.86 (s, 2H), 4.55 (s, 2H), 4.41 (s, 2H), 3.65 (t, $J$ = 4.6 Hz, 2H), 3.20 (s, 3H).

Comparative Example 5:

Compound DB05:

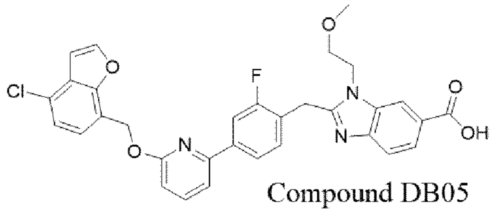

Compound DB05

Synthesis route:

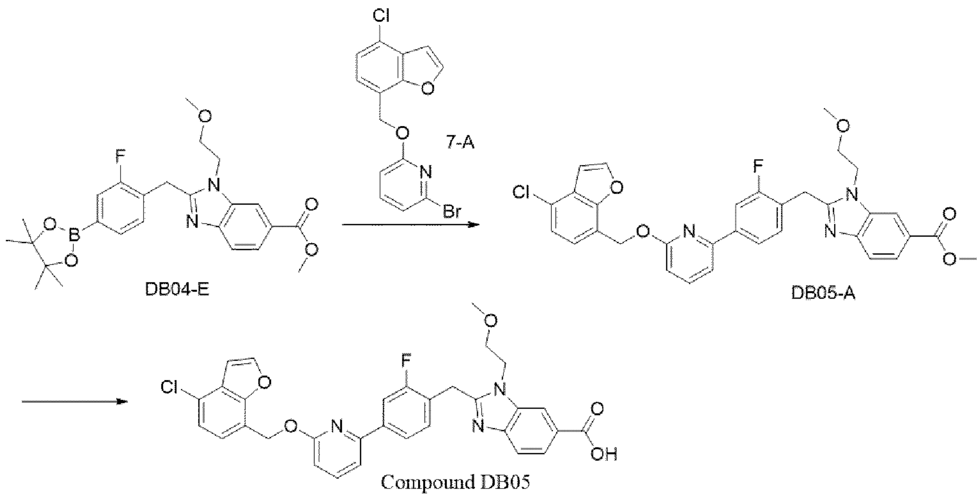

Compound DB05-A:

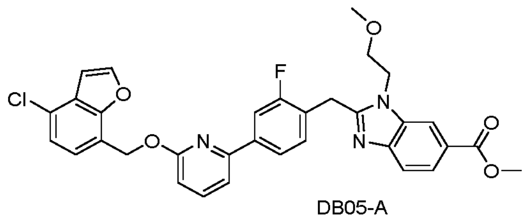

DB05-A

200 mg of compound 7-A (1.0 eq.), 290 mg of compound DB04-E (1.05 eq.), 13 mg of Pd(dppf)$Cl_2$ (0.03 eq.) and 481 mg of cesium carbonate (2.5 eq.) were weighed and added to a 25mL round-bottom flask. 5 mL of 1,4-dioxane and 1 mL of water were added. The reaction solution was stirred evenly, degassed with nitrogen twice, and heated to 90°C for reaction under nitrogen atmosphere for about 2 hours until TLC showed that the reaction was completed. The reaction solution was cooled to room temperature, filtered with diatomite, and washed with dichloromethane. After concentration, the filtrate was purified by column chromatography to obtain 270 mg of compound DB05-A with a yield of 76.3%. MS m/z (ESI): 600.1701 $(M+H)^+$.

Compound DB05:

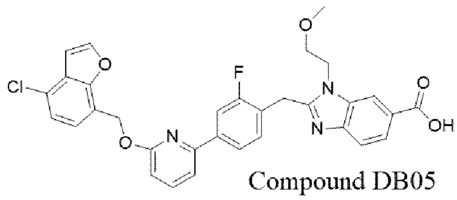

Compound DB05

263 mg of compound DB05-A (1.0 eq.) was weighed and added to a 25mL round-bottom flask. 6 mL of tetrahydrofuran and 2 mL of methanol were added. The thus obtained system was stirred to dissolve at room temperature, 0.55 mL of 2 mol/L NaOH solution (2.5 eq.) was then slowly dropwise added. Then the reaction mixture was stirred overnight at room temperature until TLC showed that the reaction was completed. The reaction solution was concentrated. After the concentration, 5 mL of water was added to the residue, and the pH of the reaction solution was adjusted to be 4-5 with 0.5 mol/L HCl solution. Subsequently, the reaction solution was filtered. The filter cake was pulped with 5 mL of ethyl acetate and 1 mL of methanol, and then filtered. The filter cake was washed with a small amount of ethyl acetate and dried to obtain 196 mg of compound DB05 with a yield of 76.3%. MS m/z (ESI): 586.1545 $(M+H)^+$; $^1H$ NMR (400 MHz, DMSO) $\delta$ =12.50 (s, 1H), 8.18 (d, *J* = 2.4 Hz, 2H), 7.94 - 7.76 (m, 4H), 7.63 (d, *J* = 7.4 Hz, 1H), 7.57 (d, *J* = 8.4 Hz, 1H), 7.47 (d, *J* = 8.0 Hz, 1H), 7.44 - 7.33 (m, 2H), 7.06 (d, *J* = 2.2 Hz, 1H), 6.89 (d, *J* = 8.2 Hz, 1H), 5.77 (s, 2H), 4.55 (t, *J* = 4.8 Hz, 2H), 4.42 (s, 2H), 3.66 (t, *J* = 5.0 Hz, 2H), 3.21 (s, 3H).

**Experimental Example 1: in-vitro activity evaluation**

**Experimental method:**

Logarithmically growing GLP1R/CRE-LUC HEK293 cells (purchased from Nanjing Cobioer) were digested, the digested cells were re-suspended in a DMEM+10%FBS culture medium, and a cell density was adjusted to be $3.5*10^5$/mL. The cell solution was added into a 96-well cell culture plate by 100 $\mu$L/well, and the plate was cultured overnight in an incubator (37°C, 5% $CO_2$).

A compound was dissolved with DMSO to prepare a storage solution at an initial concentration of 10 mM, an initial concentration of the small molecular compound was 0.2 mM, and the compound was diluted by 3 times and 10 points were diluted, while the $11^{th}$ point was DMSO. Another 96-well plate was taken, added with 95 $\mu$L of cell culture solution (DMEM+10% FBS) in each well, then added with 5 $\mu$L of to-be-tested samples at different concentrations in each well, mixed evenly, and then added with 11 $\mu$L of to-be-tested samples at different concentrations in each well of the cell culture plate, and each sample had two wells. The culture plate was incubated in an incubator for 6 hours (37°C, 5% $CO_2$). The 96-well cell culture plate was taken out, added with 100 $\mu$L of ONE-Glo™ reagent in each well, and incubated at room temperature for 10 minutes, and a chemiluminescence signal value was measured with an enzyme-labeled instrument. Data were processed and analyzed by Microsoft Excel and Graphpad Prism6 to obtain an $EC_{50}$ value of the compound.

**Experimental results:**

Table 1. Determination of agonist activities of compounds on GLP1R/CRE-LUC HEK293 cells

| Compound | $EC_{50}$ (nM) | Compound | $EC_{50}$ (nM) |
|---|---|---|---|
| Compound 1 | 114.20 nM | Compound 2 | 101.40 nM |
| Compound 3 | 65.10 nM | Compound 7 | 20.87 nM |
| Compound 13 | 13.57 nM | Compound 14 | 6.76 nM |
| Compound 15 | 25.81nM | Compound 17 | 4.13 nM |
| Compound 18 | 7.11nM | Compound 19 | 3.20 nM |
| Compound 21 | 0.96 nM | Compound 25 | 11.47 nM |
| Compound 38 | 7.53 nM | Compound 67 | 1.97nM |
| Compound 87 | 1.56nM | Compound 91 | 0.76nM |

(continued)

| Compound | $EC_{50}$ (nM) | Compound | $EC_{50}$ (nM) |
|---|---|---|---|
| Compound 92 | 1.76 nM | Compound 93 | 2.89nM |
| Compound 94 | 0.89nM | Compound 95 | 2.33nM |
| Compound 96 | 3.02 nM | Compound 97 | 7.57nM |
| Compound 99 | 0.82nM | Compound 100 | 2.02nM |
| Compound 101 | 0.69nM | Compound 102 | 9.10nM |
| Compound 103 | 1.17nM | Compound 104 | 1.76nM |
| Compound 105 | 1.09nM | Compound 106 | 5.87nM |
| Compound 107 | 1.90nM | Compound 108 | 3.11nM |
| Comparative Example 1-Compound PF-06882961 | 39.23 nM | Comparative Example 2-Compound DB02 | 13.64 nM |
| Comparative Example 3-Compound DB03 | 18.43 nM | Comparative Example 4-Compound DB04 | 275.10 nM |
| Comparative Example 5-Compound DB05 | 311.80 nM | Compound DB06 | 69.07 nM |

Conclusion: as shown in Table 1, the compounds of the present invention could activate the GLP1R of hGLP-1R HEK293 cells, wherein,
the activities of the compound 13, the compound 14, the compound 17, the compound 18, the compound 19, the compound 21, the compound 25, the compound 38, the compound 67, the compound 87, the compound 91, the compound 92, the compound 93, the compound 94, the compound 95, the compound 96, the compound 97, the compound 99, the compound 100, the compound 101, the compound 102, the compound 103, the compound 104, the compound 105, the compound 106, the compound 107 and the compound 108 were higher than those of the comparative example compound PF-06882961, the comparative example compound DB02, the comparative example compound DB03 and the comparative example compound DB06, and were much higher than those of the comparative example compound DB04 and the comparative example compound DB05, especially the activities of the compound 21, the compound 91, the compound 94, the compound 99, the compound 101, the compound 103 and the compound 105 were all more than 10 times higher than that of the comparative compound DB02 with better activity.

**Experimental Example 2: in-vivo pharmacological experiment**

**Experimental method:**

Genetically engineered hGLP1R mice, male, purchased from Biocytogen Jiangsu Gene Biotechnology Co., Ltd., were fasted overnight before the glucose tolerance test (IPTGG), without water deprivation. A solvent or corresponding compound was orally administered once, a glucose solution (2 g/kg) was intraperitoneally injected 60 minutes after administration, and a blood glucose value before administration of glucose (equivalent to a blood glucose value of 0 minute) and blood glucose values 15 minutes, 30 minutes, 60 minutes and 120 minutes after administration of glucose were measured. The solvent was 5%DMSO+40%PEG400+55% physiological saline, and a dosage of the compound was 1 mg/kg (n=3).

**Experimental results:**

As shown in FIG. 1 and FIG. 2, when the compound 17, the compound 19, the compound 38 and the compound 61, and the PF-06882961 and the comparative example compound DB05 were orally administered at the same dosage (1 mg/kg) once, it could be seen from the blood glucose curves (FIG. 1) and the blood glucose AUC (0 to 120 minutes) (FIG. 2) that blood glucose reduction effects of the compounds of the present invention were significantly better than that of the PF-06882961, and were also significantly better than that of the comparative example compound DB05 at the same time, and the compounds of the present invention took effect more quickly in blood glucose reduction compared with the comparative example compound DB05.

As shown in FIG. 3 and FIG. 4, when the compound 17, the compound 93 and the compound 100, and the PF-06882961 and the comparative example compound DB03 were orally administered at the same dosage (1 mg/kg)

once, it could be seen from the blood glucose curves (FIG. 3) and the blood glucose AUC (0 to 120 minutes) (FIG. 4) that blood glucose reduction effects of the compounds of the present invention were significantly better than that of the PF-06882961, and were also absolutely better than that of the comparative example compound DB03 at the same time, and the compounds of the present invention took effect more quickly in blood glucose reduction compared with the comparative example compound DB03.

As shown in FIG. 5 and FIG. 6, when the compound 21, the compound 94, the compound 99, the compound 106 and the compound 107, and the PF-06882961 were orally administered at the same dosage (1 mg/kg) once, it could be seen from the blood glucose curves (FIG. 5) and the blood glucose AUC (0 to 120 minutes) (FIG. 6) that blood glucose reduction effects of the compounds of the present invention were significantly better than that of the PF-06882961.

**Experimental example 3: hERG test**

Test object: influences of compounds on an hERG potassium channel current were tested by a full-automatic patch clamp method.

Experimental method:

(1) An HEK-293 cell line stably expressing an hERG potassium channel was adopted, and hERG potassium channel cells were purchased from Creacell Company.
(2) The HEK-293 cell line stably expressing the hERG potassium channel was cultured in a DMEM culture medium containing 10% fetal bovine serum and 0.8 mg/mL G418 at 37°C under a carbon dioxide concentration of 5%.
(3) Cell passage: the old culture medium was removed, and the cells were washed once with PBS, then added with 1 mL of TrypLE™ Express solution, and incubated at 37°C for about 0.5 minutes. When the cells were separated from a dish bottom, about 5 mL of complete culture medium preheated at 37°C was added. A cell suspension was gently blown with a straw to separate the aggregated cells. The cell suspension was transferred to a sterile centrifuge tube and centrifuged at 1000 rpm for 5 minutes to collect the cells. In amplification or maintenance of culture, the cells were inoculated into a 6 cm cell culture dish, and the number of cells inoculated into each cell culture dish was $2.5\times10^5$ cells (final volume: 5 mL). In order to maintain an electrophysiological activity of cells, a cell density should not exceed 80%.
(4) In patch clamp detection, before the test, the cells were separated by TrypLE™ Express, and $4\times10^3$ cells were spread on a cover slip and cultured in a 24-well plate (final volume: 500 μL). After 18 hours, the test was carried out for detection.

**Data analysis**:

A current after an effect of each drug concentration and a blank control current ( $\frac{Peak\ tail\ current\ compound}{Peak\ tail\ current\ vehicle}$ ) were standardized first, and then an inhibition rate ( $1-\frac{Peak\ tail\ current\ compound}{Peak\ tail\ current\ vehicle}$ ) corresponding to each drug concentration was calculated. A mean number and a standard error were calculated for each concentration, and a semi-inhibitory concentration of each compound was calculated by the following equation:

$$\text{inhibition} = \frac{1}{1+\left(\frac{IC_{50}}{C}\right)^h}$$

A dosage-dependent effect was nonlinearly fitted by the above equation, wherein C represented a concentration of a test sample, $IC_{50}$ represented a semi-inhibitory concentration, and h represented a Hill coefficient. Curve fitting and $IC_{50}$ calculation were completed by IGOR software. Determination results were shown in Table 2.

Table 2. Testing of $IC_{50}$ values of inhibitory effects of compounds on hERG potassium channel current

| Compound | $IC_{50}$ (μM) |
|---|---|
| Comparative Example 1-Compound PF-06882961 | 9.35 |
| Comparative Example 3-Compound DB03 | >10 |
| Compound 7 | >30 |

(continued)

| Compound | $IC_{50}$ (μM) |
|---|---|
| Compound 17 | >30 |
| Compound 21 | >30 |
| Compound 87 | >30 |
| Compound 93 | >30 |
| Compound 94 | >30 |
| Compound 99 | >30 |
| Compound 100 | >30 |
| Compound 104 | >30 |

Conclusion: compared with the comparative example compounds, the compounds of the present invention had weaker inhibitory effects on the hERG potassium channel current, lower risks of cardiotoxicity and higher safeties.

Pharmacokinetic studies showed that: half-lives of the compound 21, the compound 93, the compound 94, the compound 99 and the compound 100 of the present invention in SD rats were 6 hours to 10 hours, and compared with the comparative example PF-06882961 and the comparative example 3-compound DB03, the half-lives could be significantly and effectively prolonged (half-lives of the comparative example PF-06882961 and the comparative example 3-compound DB03 in SD rats were 3.13 hours and 2.94 hours respectively). Meanwhile, a plasma exposure quantity was high, and an administration frequency and a single administration dosage were reduced, thus achieving the purpose of reducing the dosage and the number of times of administration, so as to provide therapeutic advantages.

## Claims

1. A compound of general formula (II) or a pharmaceutically acceptable salt, a stereoisomer, a solvate or a hydrate thereof:

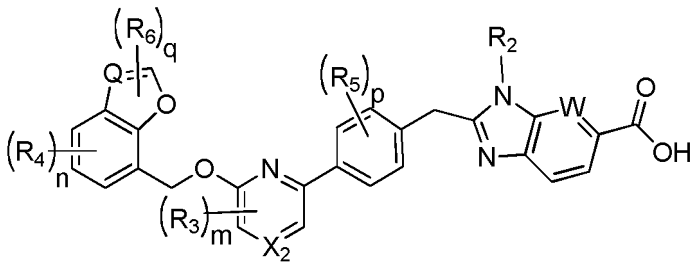

formula (II)

wherein:

$R_2$ is selected from

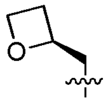

or

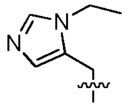

;

$R_3$ is independently selected from hydrogen atom and halogen;
$R_4$ is independently selected from hydrogen atom, halogen, cyano and C1-C6 alkyl, wherein the C1-C6 alkyl is further substituted by one or more halogens;
$R_5$ is independently selected from halogen;
$R_6$ is independently selected from hydrogen atom, halogen, C1-C6 alkyl, C3-C6 cycloalkyl, phenyl, furan and C2-C4 alkenyl; or two adjacent $R_6$ are connected to form a ring;
W is selected from N and $CR_{10}$;
$X_2$ is selected from N and $CR_{10}$;

Q is selected from N and CH;
p is selected from 0, 1, 2 or 3;
m is selected from 0, 1 or 2;
n is selected from 0, 1, 2 or 3;
q is selected from 0, 1 or 2;
$R_{10}$ is selected from hydrogen atom and halogen;
preferably, the compound or the pharmaceutically acceptable salt, the stereoisomer, the solvate or the hydrate thereof has a structure shown in general formula (II-1):

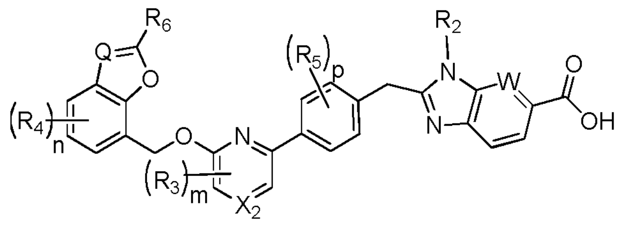

formula (II-1);

further preferably, $R_3$ is selected from hydrogen atom and F; $R_4$ is selected from hydrogen atom, F, Cl, cyano, $CF_3$ and $CHF_2$; $X_2$ and Q are selected from CH; $R_{10}$ is selected from hydrogen atom and F; further preferably, the compound or the pharmaceutically acceptable salt, the stereoisomer, the solvate or the hydrate thereof has a structure shown in general formula (II-1-1):

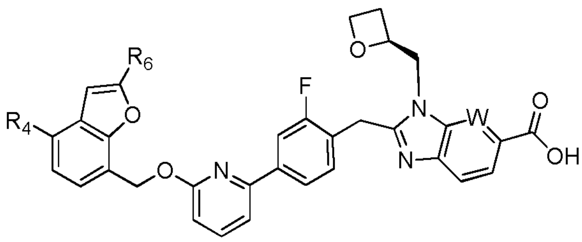

formula (II-1-1),

wherein $R_4$ is selected from Cl and cyano; $R_6$ is selected from hydrogen atom and F; and $R_{10}$ is selected from hydrogen atom;
or, preferably, the compound or the pharmaceutically acceptable salt, the stereoisomer, the solvate or the hydrate thereof has a structure shown in general formula (II-2):

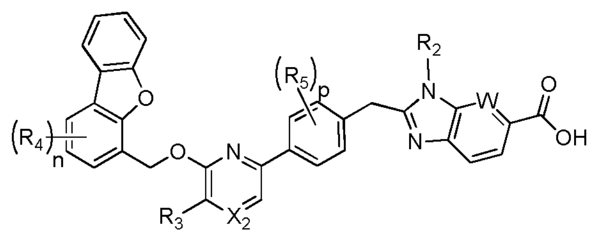

formula (II-2);

further preferably, $R_5$ is selected from F; $R_3$ is selected from hydrogen atom and F; $X_2$ is selected from CH; $R_{10}$ is selected from hydrogen atom and F; and further preferably, the compound or the pharmaceutically acceptable salt, the stereoisomer, the solvate or the hydrate thereof has a structure shown in general formula (II-2-1):

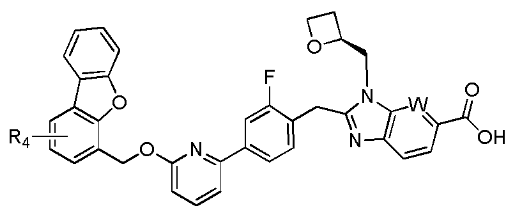

formula (II-2-1),

wherein $R_4$ is from hydrogen atom, Cl and cyano; and $R_{10}$ is selected from hydrogen atom.

2. A compound or a pharmaceutically acceptable salt, a stereoisomer, a solvate or a hydrate thereof, selected from any of the following compounds:

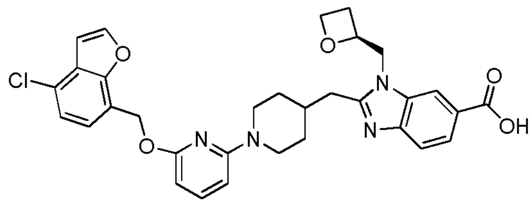
,

compound 1

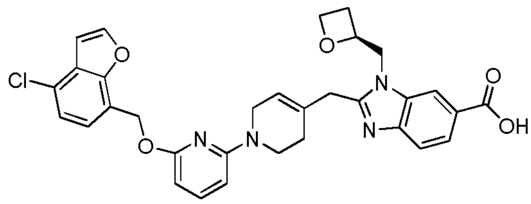
,

compound 2

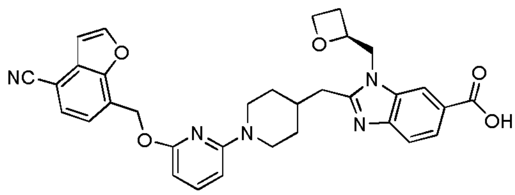
,

compound 3

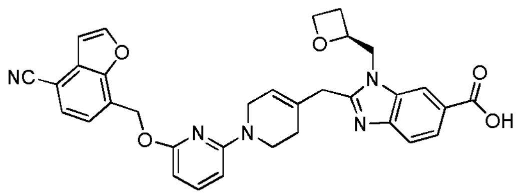
,

compound 4

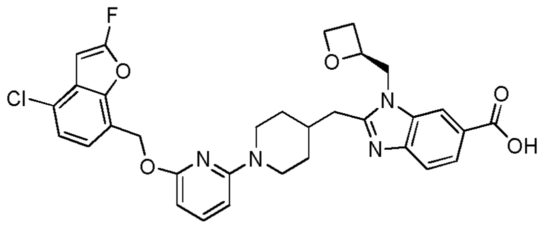
,

compound 5

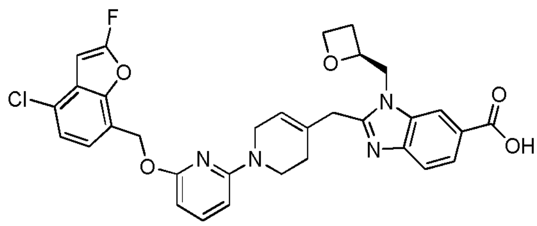
,

compound 6

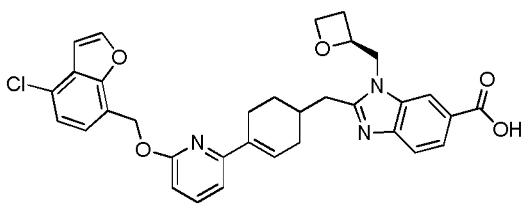
,

compound 7

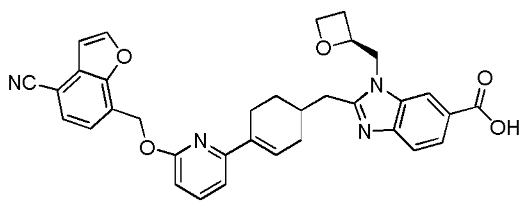
,

compound 8

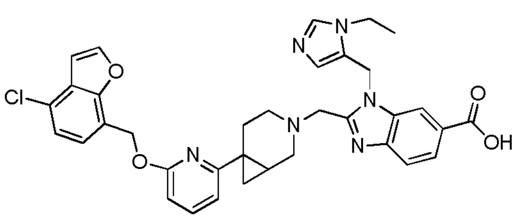
,

compound 9

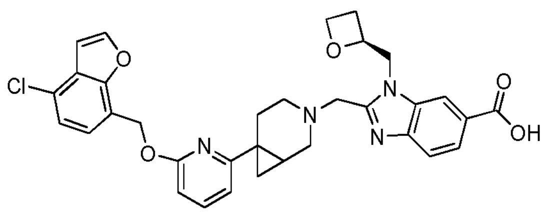
,

compound 10

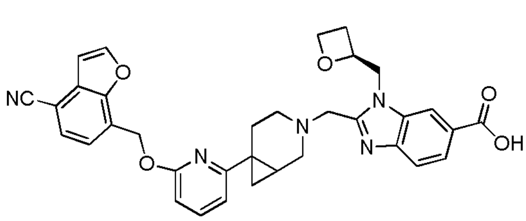
,

compound 11

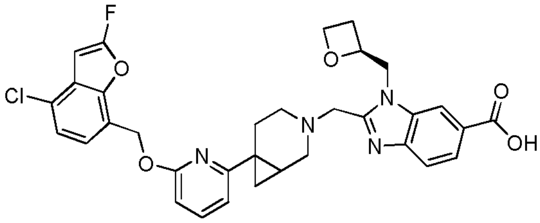
,

compound 12

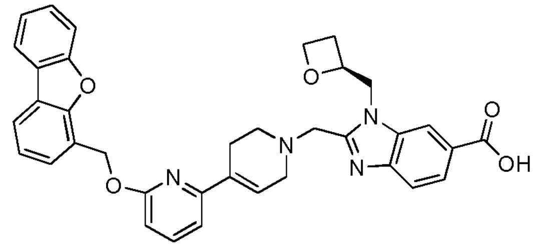
,

compound 13

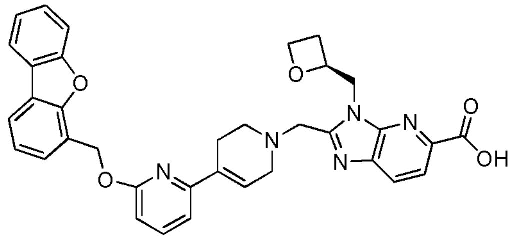
,

compound 14

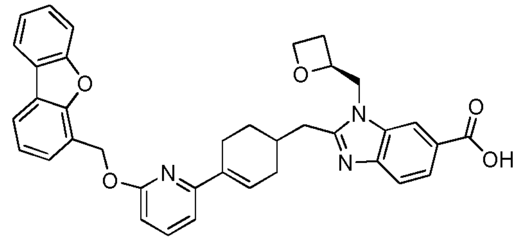
,

compound 15

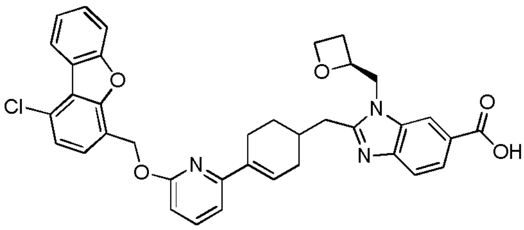
,

compound 16

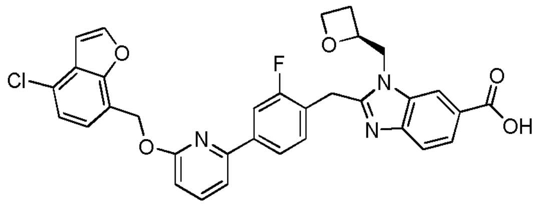
,

compound 17

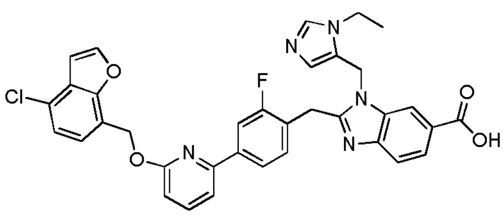
,

compound 18

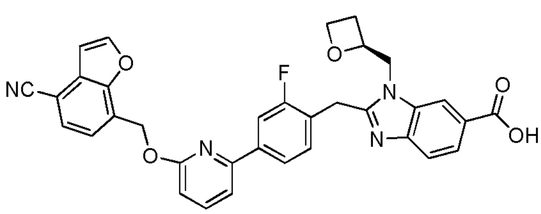
,

compound 19

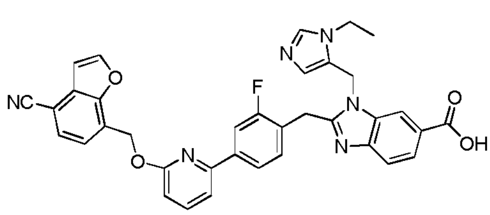
,

compound 20

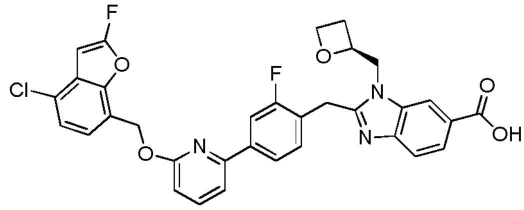,

compound 21

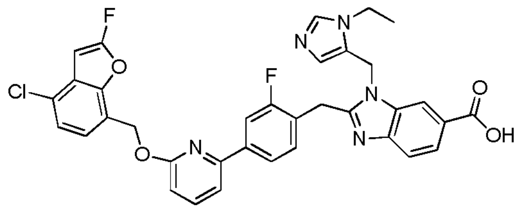,

compound 22

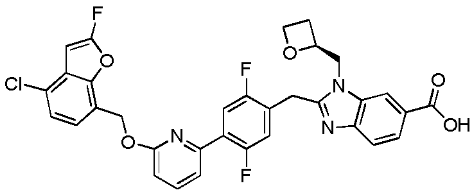,

compound 23

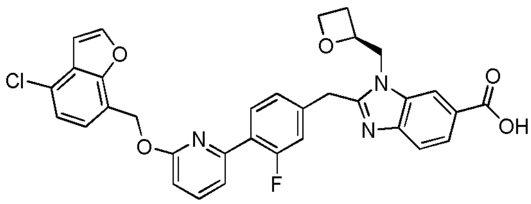,

compound 24

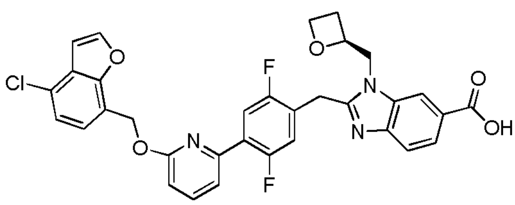,

compound 25

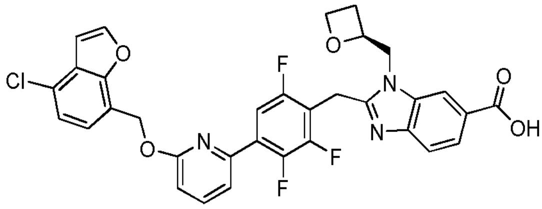,

compound 26

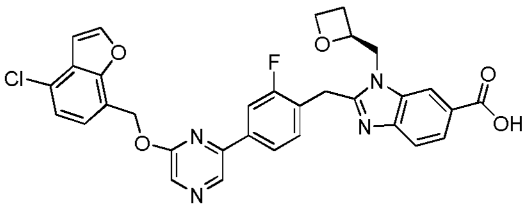,

compound 27

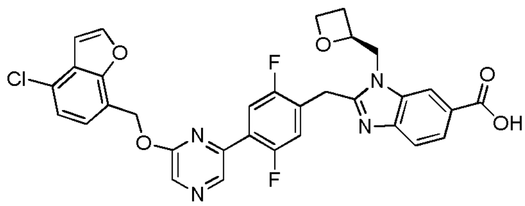,

compound 28

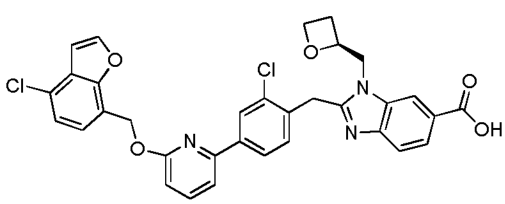,

compound 29

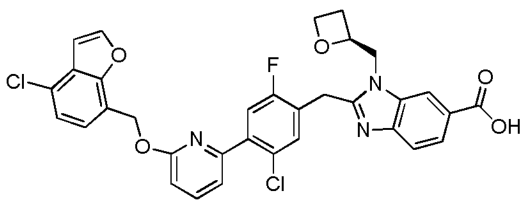,

compound 30

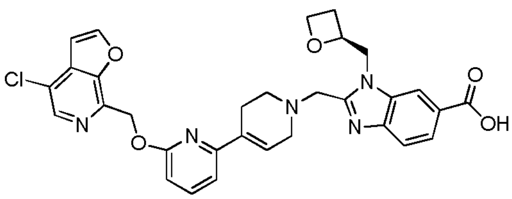

,

compound 31

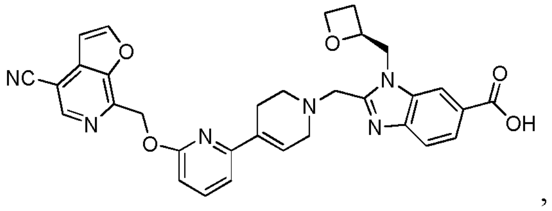

,

compound 32

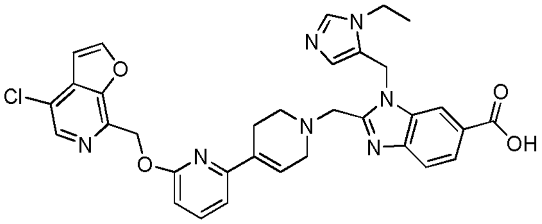

,

compound 33

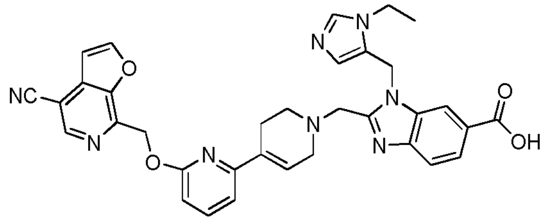

,

compound 34

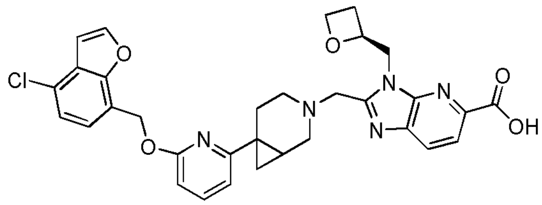

,

compound 35

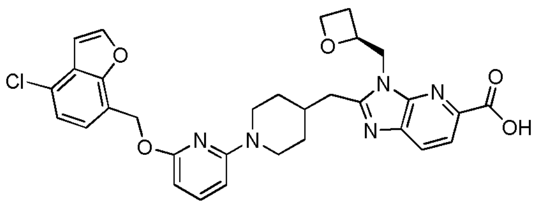

,

compound 36

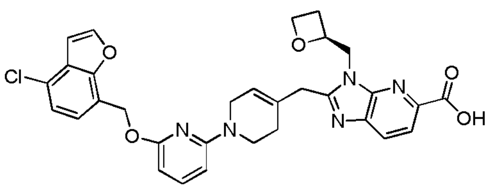

,

compound 37

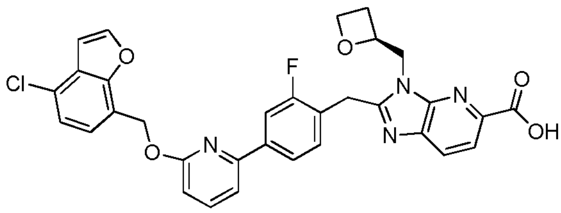

,

compound 38

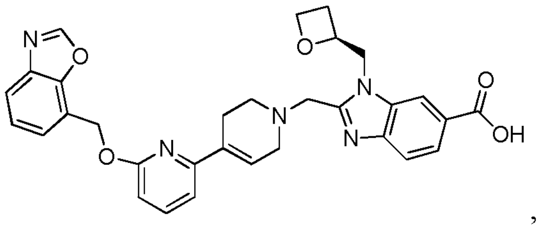

,

compound 39

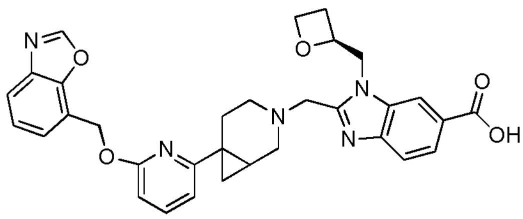

,

compound 40

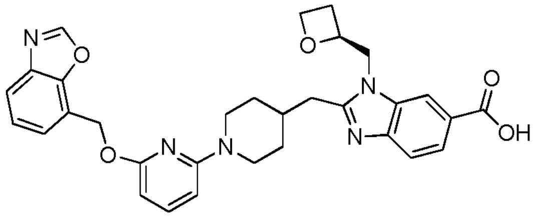

,

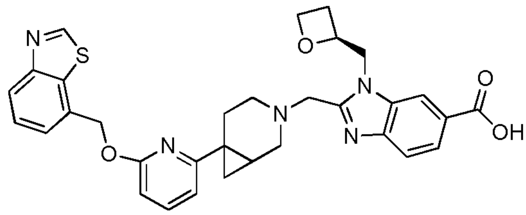

,

compound 41

compound 42

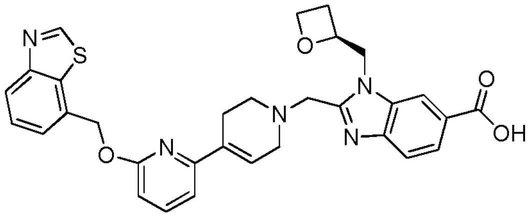

,

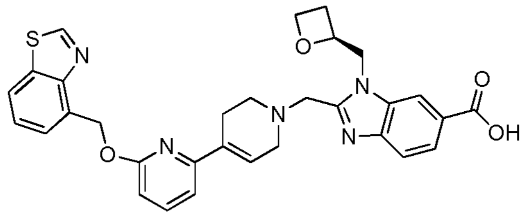

,

compound 43

compound 44

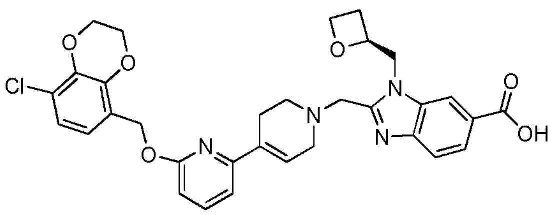

,

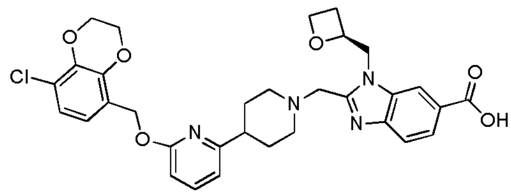

,

compound 45

compound 46

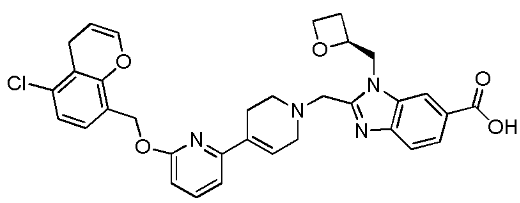

,

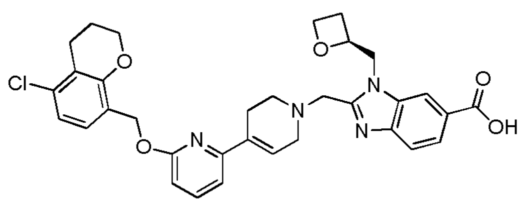

,

compound 47

compound 48

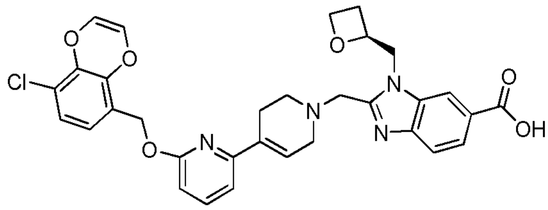

,

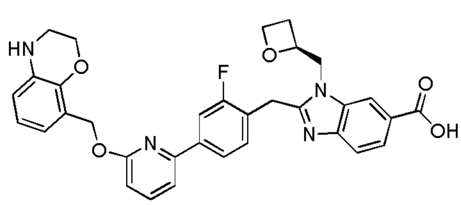

,

compound 49

compound 50

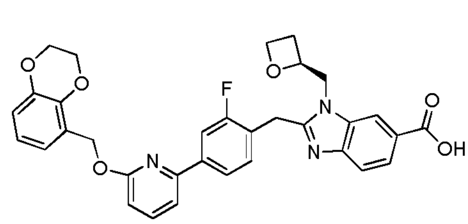,

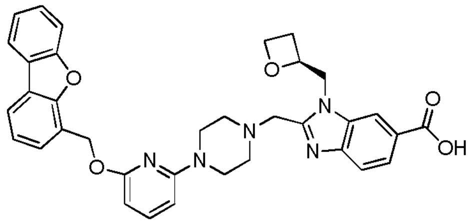,

compound 51 compound 52

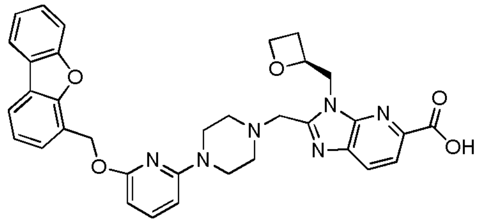,

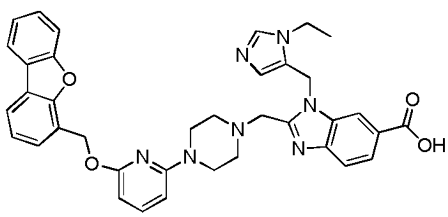,

compound 53 compound 54

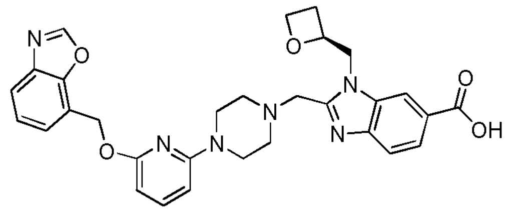,

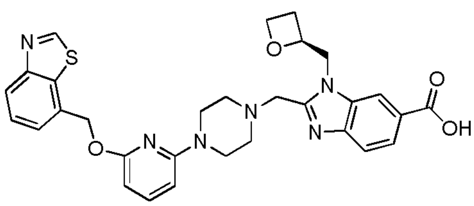,

compound 55 compound 56

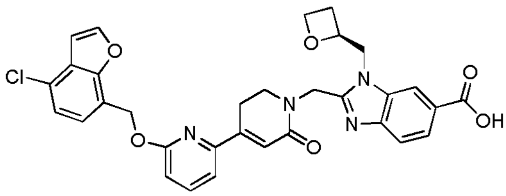,

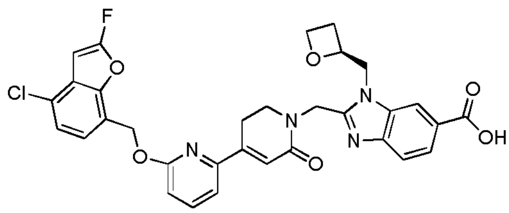,

compound 57 compound 58

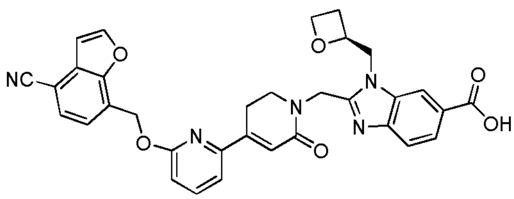,

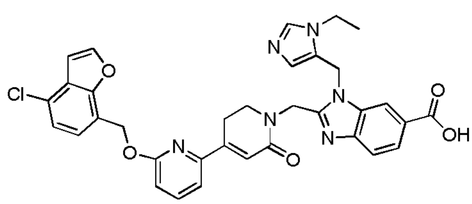,

compound 59 compound 60

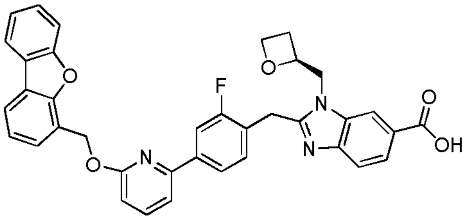
,

compound 61

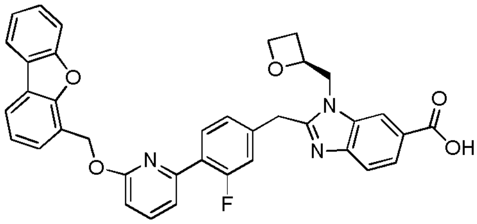
,

compound 62

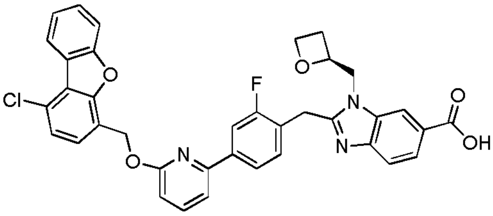
,

compound 63

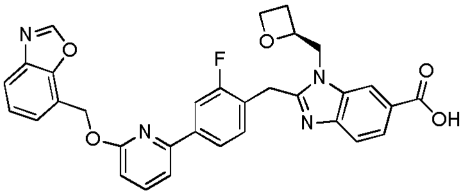
,

compound 64

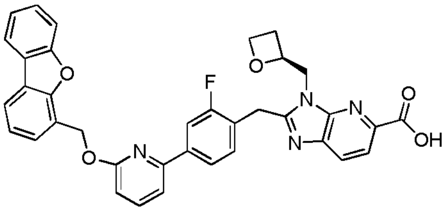
,

compound 65

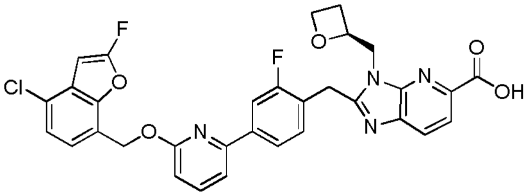
,

compound 66

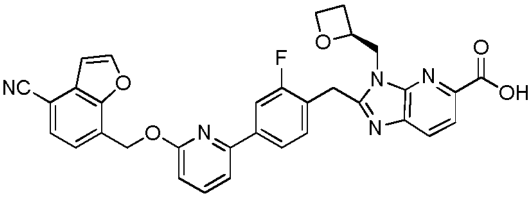
,

compound 67

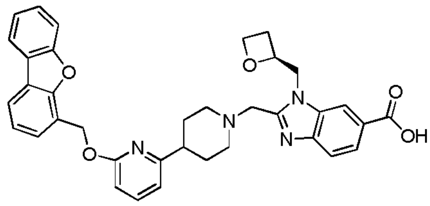
,

compound 68

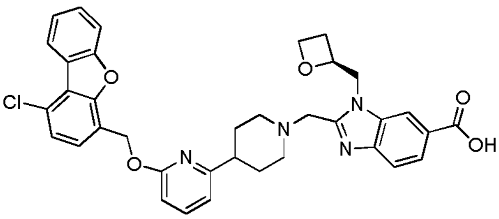
,

compound 69

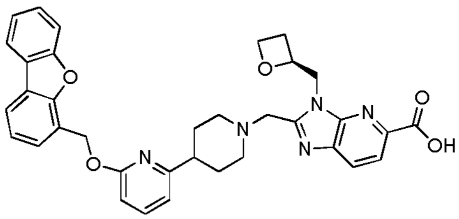
,

compound 70

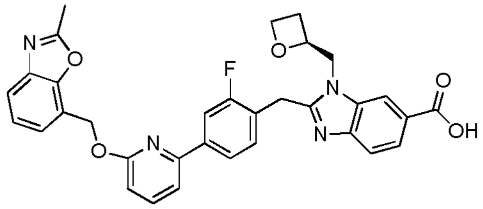

,

compound 71

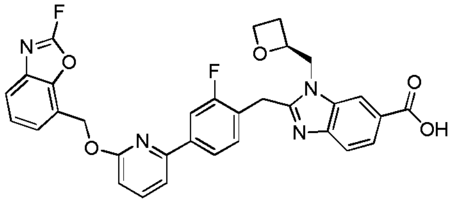

,

compound 72

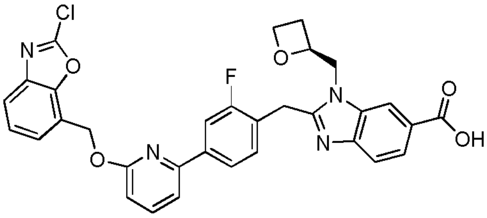

,

compound 73

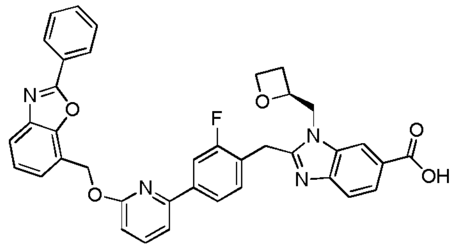

,

compound 74

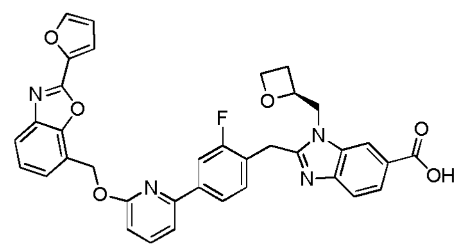

,

compound 75

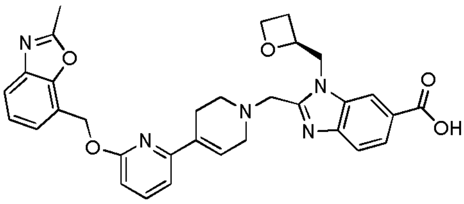

,

compound 76

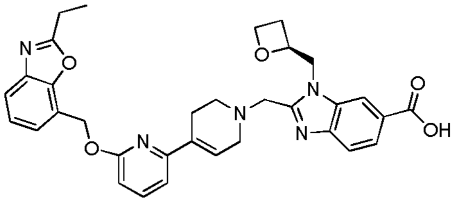

,

compound 77

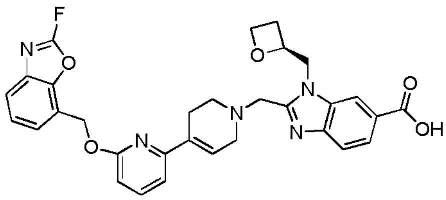

,

compound 78

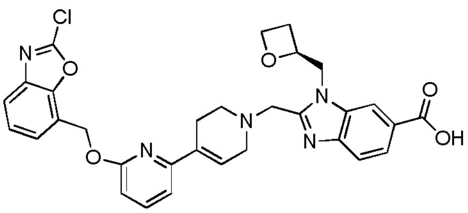

,

compound 79

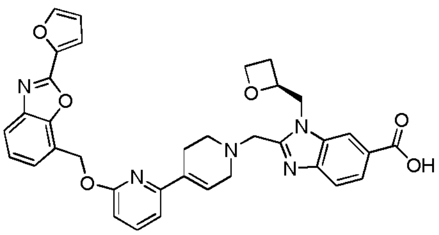

,

compound 80

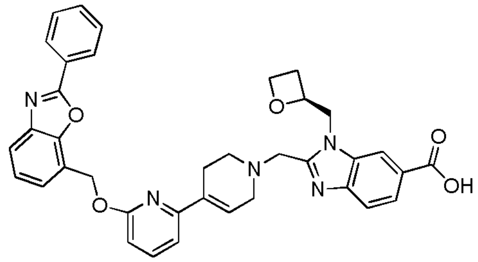
,

compound 81

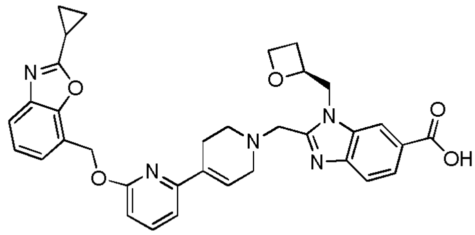
,

compound 82

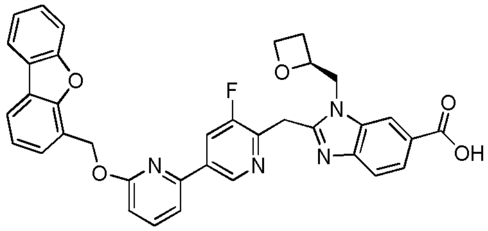
,

compound 83

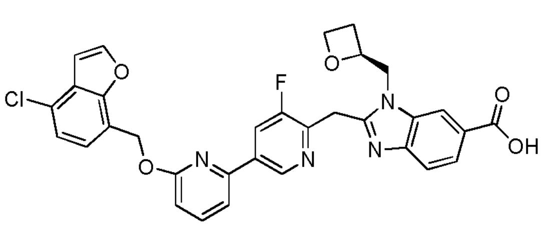
,

compound 84

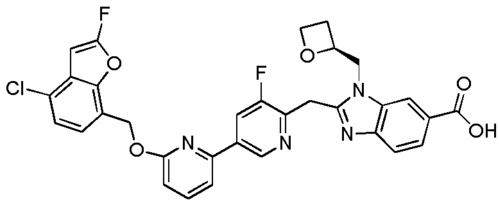
,

compound 85

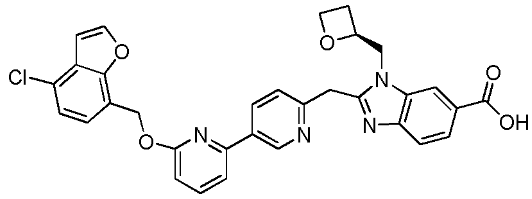
,

compound 86

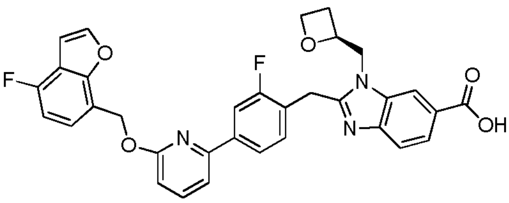
,

compound 87

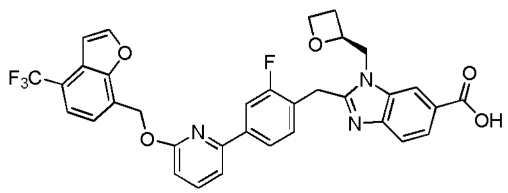
,

compound 88

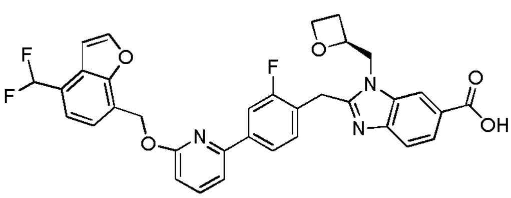
,

compound 89

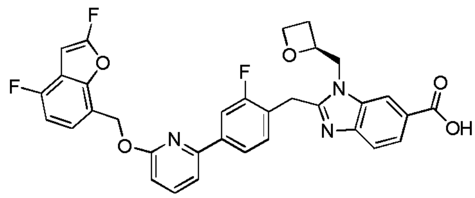
,

compound 90

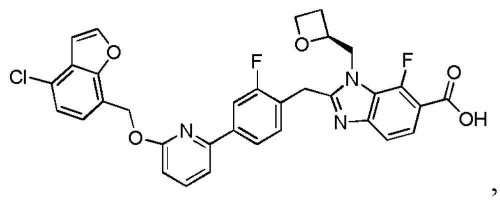

,

compound 91

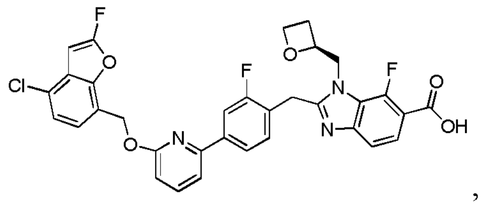

,

compound 92

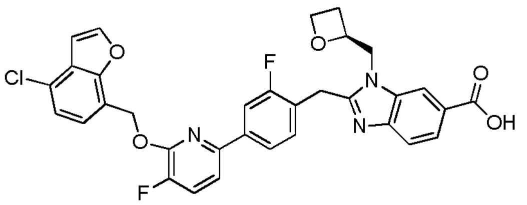

,

compound 93

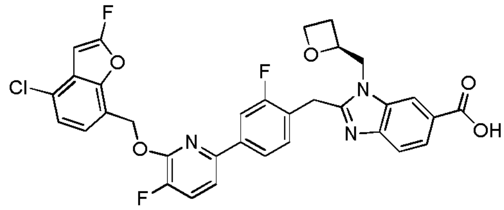

,

compound 94

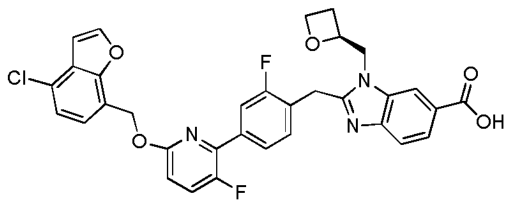

,

compound 95

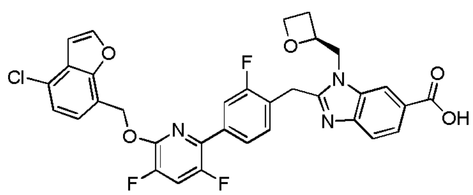

,

compound 96

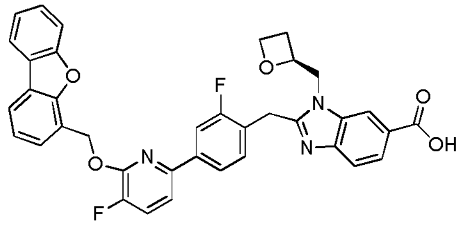

,

compound 97

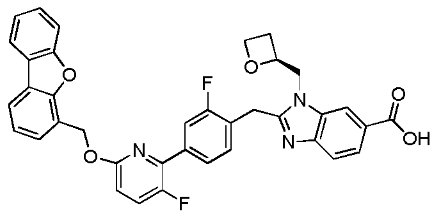

compound 98

**3.** A compound of general formula (II-3) or a pharmaceutically acceptable salt, a stereoisomer, a solvate or a hydrate thereof:

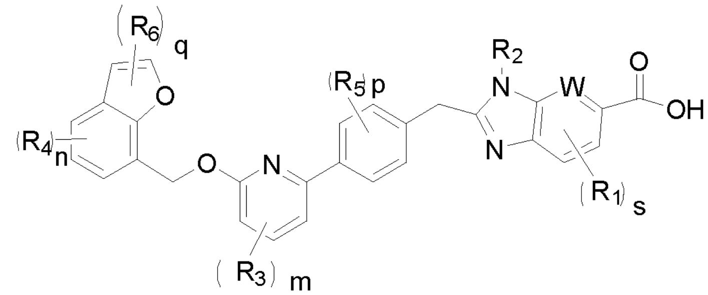

formula (II-3)

wherein:

$R_1$ is selected from hydrogen atom and halogen;

$R_2$ is independently selected from

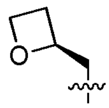 or 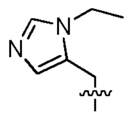 ;

$R_3$ is independently selected from hydrogen atom and halogen;
$R_4$ is independently selected from hydrogen atom, halogen and cyano;
$R_5$ is independently selected from halogen;
$R_6$ is independently selected from hydrogen atom, halogen and C1-C6 alkyl;
W is selected from N and CH;
p is selected from 0, 1, 2 or 3;
m is selected from 0, 1, 2 or 3;
n is selected from 0, 1, 2 or 3;
q is selected from 0, 1 or 2;
s is selected from 0, 1 or 2;
preferably, the compound or the pharmaceutically acceptable salt, the stereoisomer, the solvate or the hydrate thereof has a structure shown in general formula (II-3-1):

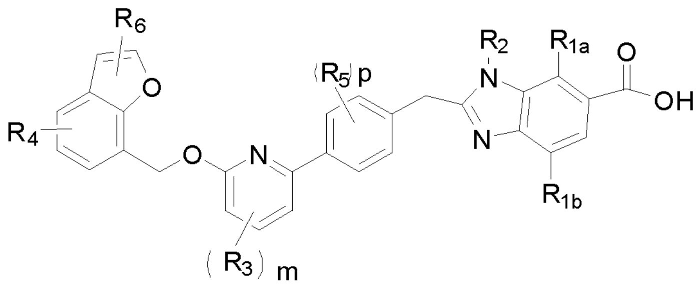

formula (II-3-1)

wherein, $R_{1a}$ and $R_{1b}$ are independently selected from hydrogen atom and F;
$R_2$ is

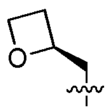 ;

$R_6$ is independently selected from hydrogen atom and halogen;
m is selected from 1 or 2;
p is selected from 1, 2 or 3;
further preferably, the compound or the pharmaceutically acceptable salt, the stereoisomer, the solvate or the hydrate thereof has a structure shown in general formula (II-3-2):

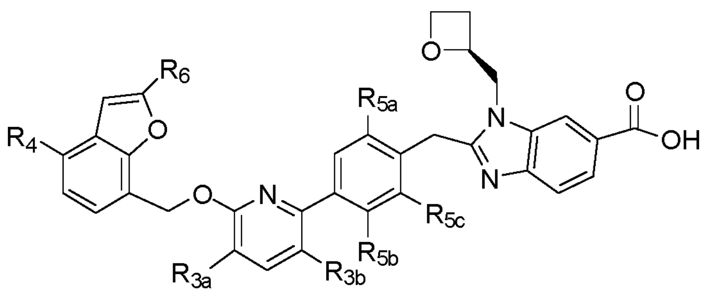

formula (II-3-2)

wherein, $R_{3a}$ and $R_{3b}$ are independently selected from F and hydrogen atom;
$R_4$ is independently selected from F, Cl and cyano;

$R_{5a}$, $R_{5b}$ and $R_{5c}$ are independently selected from hydrogen atom and F;
$R_6$ is independently selected from hydrogen atom, F and Cl; and
further preferably, $R_{3a}$ and $R_{3b}$ are not F at the same time, $R_{5a}$ and $R_{5c}$ are independently selected from hydrogen atom and F, and $R_{5b}$ is hydrogen atom.

**4.** A compound or a pharmaceutically acceptable salt, a stereoisomer, a solvate or a hydrate thereof, selected from any of the following compounds:

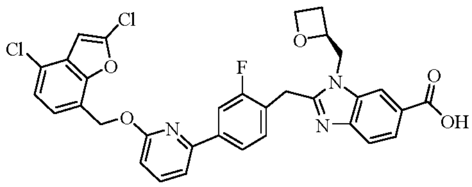
,
compound 99

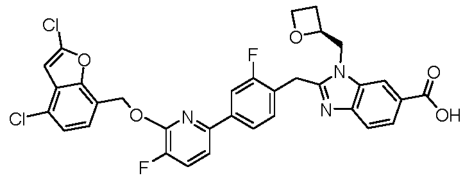
,
compound 100

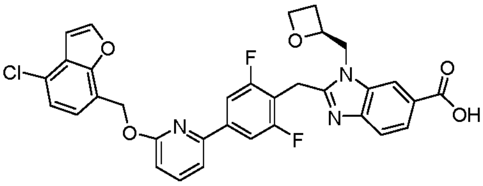
,
compound 101

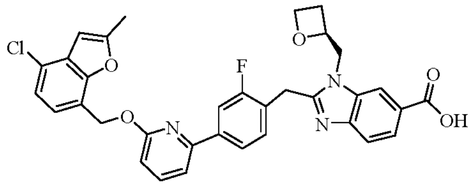
,
compound 102

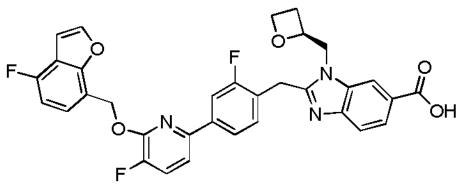
,
compound 103

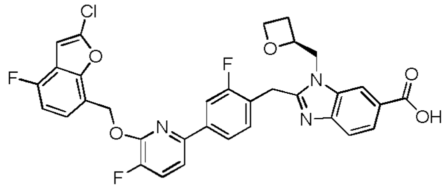
,
compound 104

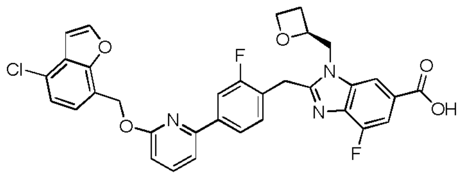
,
compound 105

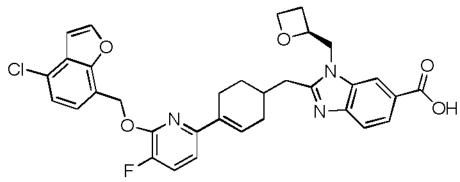
,
compound 106

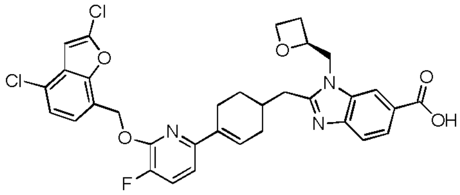
,
compound 107

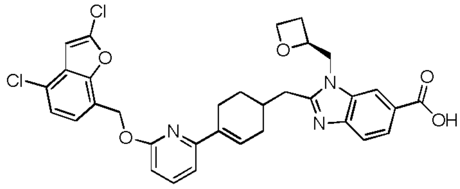
compound 108

**5.** A compound of general formula (II') or a pharmaceutically acceptable salt, a stereoisomer, a solvate or a hydrate thereof:

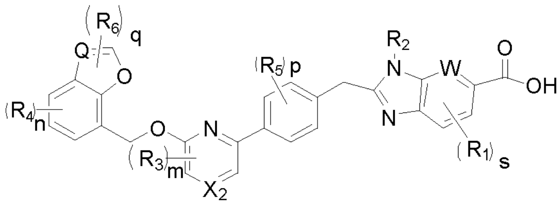

formula (II')

wherein:

$R_1$ is selected from hydrogen atom and halogen;
$R_2$ is selected from

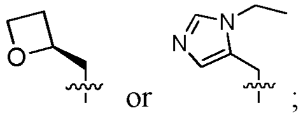

$R_3$ is independently selected from hydrogen atom and halogen;
$R_4$ is independently selected from hydrogen atom, halogen, cyano and C1-C6 alkyl, wherein the C1-C6 alkyl is further substituted by one or more halogens;
$R_5$ is independently selected from hydrogen atom and halogen;
$R_6$ is independently selected from hydrogen atom, halogen, C1-C6 alkyl, C3-C6 cycloalkyl, phenyl, furan and C2-C4 alkenyl;
W is selected from N and $CR_{10}$;
$X_2$ is selected from N and $CR_{10}$;
Q is selected from N and CH;
p is selected from 0, 1, 2 or 3;
m is selected from 0, 1, 2 or 3;
n is selected from 0, 1, 2 or 3;
q is selected from 0, 1 or 2;
s is selected from 0, 1 or 2;
$R_{10}$ is selected from hydrogen atom and halogen;
preferably, the compound or the pharmaceutically acceptable salt, the stereoisomer, the solvate or the hydrate thereof has a structure shown in general formula (II'-1):

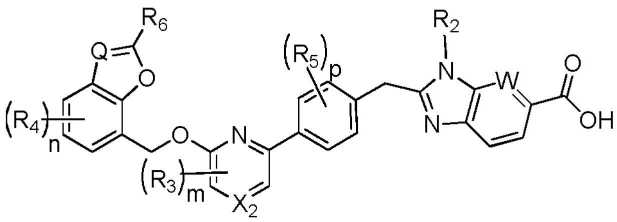

formula (II'-1)

wherein:

$R_3$ is selected from hydrogen atom and F;
$R_4$ is selected from hydrogen atom, F, Cl, cyano, $CF_3$ and $CHF_2$;
$X_2$ and Q are selected from CH; and $R_{10}$ is selected from hydrogen atom and F;
or,
preferably, the compound or the pharmaceutically acceptable salt, the stereoisomer, the solvate or the hydrate thereof has a structure shown in general formula (II'-3):

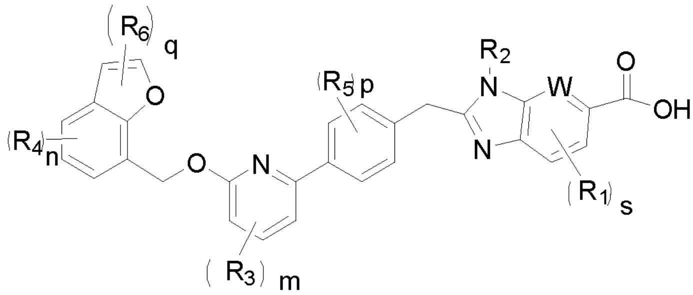

formula (II'-3)

wherein:

$R_4$ is independently selected from hydrogen atom, halogen and cyano;
$R_6$ is independently selected from hydrogen atom, halogen and C1-C6 alkyl; and
W is selected from N and CH.

**6.** The compound of general formula (II') or the pharmaceutically acceptable salt, the stereoisomer, the solvate or the hydrate thereof according to claim 5, wherein:
the structure shown in general formula (II'-1) is further the structure shown in general formula (II'-1-1):

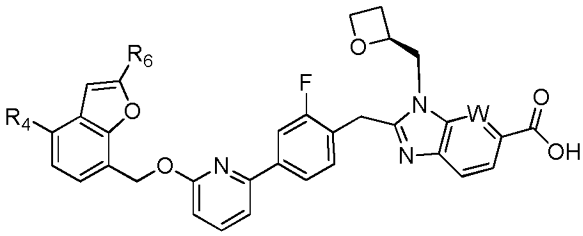

formula (II'-1-1)

wherein:

$R_4$ is selected from Cl and cyano;
$R_6$ is selected from hydrogen atom and F;
$R_{10}$ is selected from hydrogen atom;
the structure shown in general formula (II'-3) is further the structure shown in general formula (II'-3-1):

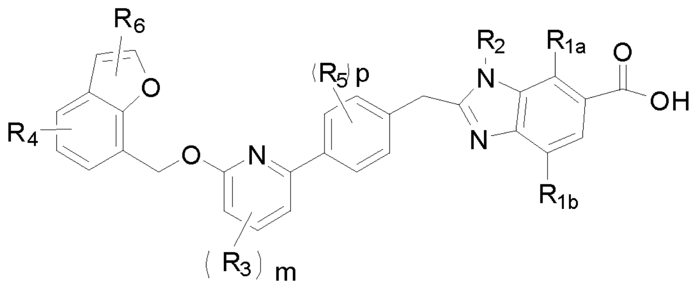

formula (II'-3-1)

wherein:

$R_{1a}$ and $R_{1b}$ are independently selected from hydrogen atom and F;
$R_2$ is:

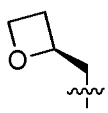

;

$R_6$ is independently selected from hydrogen atom and halogen;
m is selected from 1 or 2;
p is selected from 1, 2 or 3;
preferably, the structure shown in general formula (II’-3) is further the structure shown in general formula (II’-3-2):

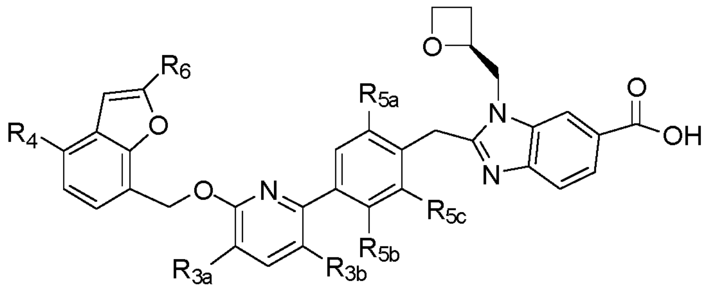

formula (II'-3-2)

wherein:

$R_{3a}$ and $R_{3b}$ are independently selected from F and hydrogen atom;
$R_4$ is independently selected from F, Cl and cyano;
$R_{5a}$, $R_{5b}$ and $R_{5c}$ are independently selected from hydrogen atom and F;
$R_6$ is independently selected from hydrogen atom, F and Cl; and
further preferably, $R_{3a}$ and $R_{3b}$ are not F at the same time, $R_{5a}$ and $R_{5c}$ are independently selected from hydrogen atom and F, and $R_{5b}$ is hydrogen atom.

**7.** A pharmaceutical composition, comprising the compound or the pharmaceutically acceptable salt, the stereoisomer, the solvate or the hydrate thereof according to claim 1 or 2, or claim 3 or 4, or claim 5 or 6, and a pharmaceutically acceptable carrier.

**8.** Use of the compound or the pharmaceutically acceptable salt, the stereoisomer, the solvate or the hydrate thereof according to claim 1 or 2, or claim 3 or 4, or claim 5 or 6, or the pharmaceutical composition according to claim 7 in preparing a medicament for a GLP-1 receptor agonist.

**9.** Use of the compound or the pharmaceutically acceptable salt, the stereoisomer, the solvate or the hydrate thereof according to claim 1 or 2, or claim 3 or 4, or claim 5 or 6, or the pharmaceutical composition according to claim 7 in preparing a medicament for treating and/or preventing type I diabetes, type II diabetes, malnutrition-related diabetes, diabetic complications, obesity, metabolic syndrome, hyperglycemia, glucose intolerance, nonalcoholic fatty liver disease (NAFLD), nonalcoholic steatohepatitis (NASH), cardiovascular disease, dyslipidemia, cerebral infarction, stroke, Parkinson’s disease, dementia, insulin resistance and hepatic insulin resistance; and preferably use in preparing the medicament for treating and/or preventing type I diabetes, type II diabetes, malnutrition-related diabetes, diabetic complications, obesity, metabolic syndrome, hyperglycemia, glucose intolerance, nonalcoholic fatty liver disease (NAFLD), nonalcoholic steatohepatitis (NASH), cardiovascular disease and dyslipidemia.

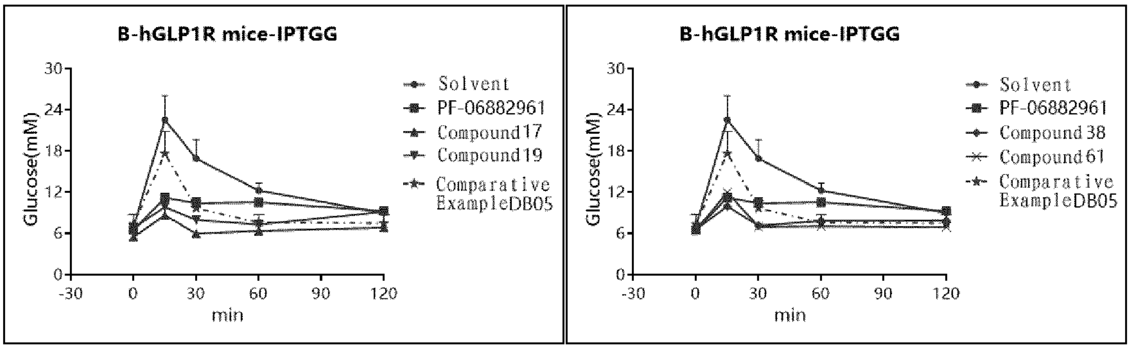


Figure 1

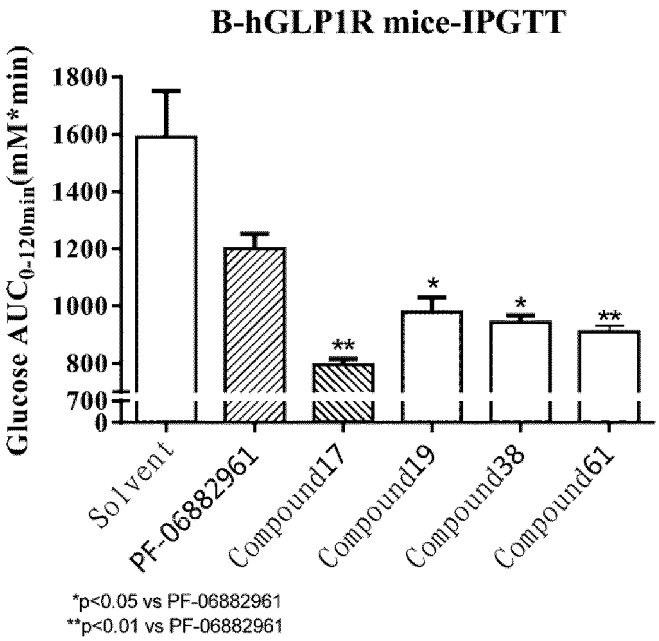


Figure 2

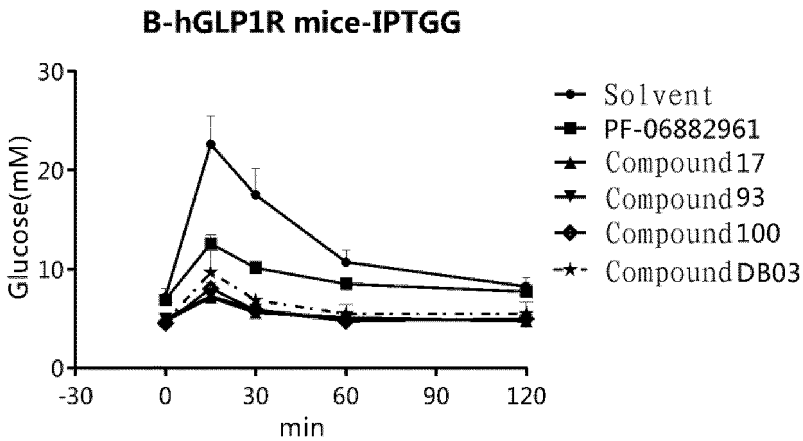


Figure 3

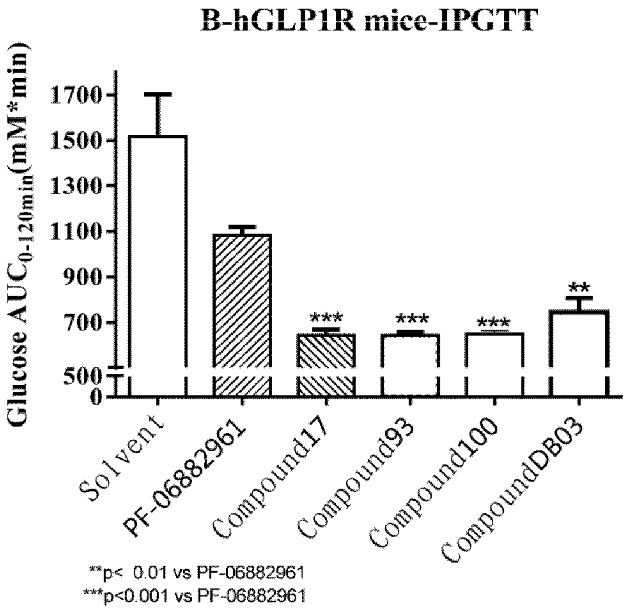


Figure 4

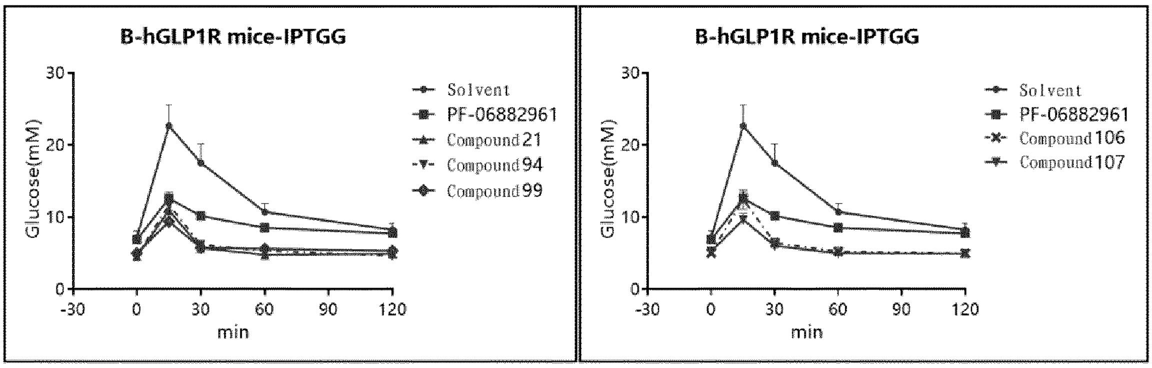


Figure 5

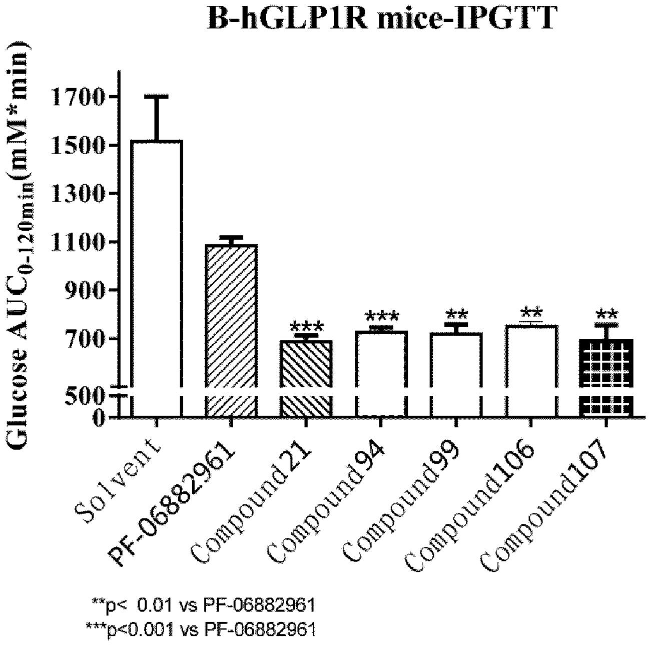
B-hGLP1R mice-IPGTT
Glucose $AUC_{0\text{-}120min}$(mM*min)
1700
1500
1300
1100
900
700
500
0
***
***
**
**
**
Solvent
PF-06882961
Compound21
Compound94
Compound99
Compound106
Compound107
**p< 0.01 vs PF-06882961
***p<0.001 vs PF-06882961

Figure 6

**INTERNATIONAL SEARCH REPORT**

International application No. **PCT/CN2023/095059**

**A. CLASSIFICATION OF SUBJECT MATTER**

C07D405/14(2006.01)i; C07D413/14(2006.01)i; C07D417/14(2006.01)i; C07D491/048(2006.01)i; C07D471/04(2006.01)i; A61K31/4545(2006.01)i; A61K31/444(2006.01)i; A61K31/4439(2006.01)i; A61K31/497(2006.01)i; A61K31/496(2006.01)i; A61P3/10(2006.01)i

According to International Patent Classification (IPC) or to both national classification and IPC

**B. FIELDS SEARCHED**

Minimum documentation searched (classification system followed by classification symbols)

IPC: C07D405/-,C07D413/-,C07D417/-,C07D471/-,C07D491/-,A61K31/-

Documentation searched other than minimum documentation to the extent that such documents are included in the fields searched

Electronic data base consulted during the international search (name of data base and, where practicable, search terms used)

CNTXT, CNKI, VEN, STN: 苯并呋喃, 苯并咪唑, 苯基, 羧酸, 代谢, benzofuran, benzoimidazol, benzyl, GLP, 根据权利要求1中的通式进行了子结构检索, Substructure search is performed according to the general formula in claim 1

**C. DOCUMENTS CONSIDERED TO BE RELEVANT**

| Category* | Citation of document, with indication, where appropriate, of the relevant passages | Relevant to claim No. |
|---|---|---|
| X | WO 2021081207 A1 (GILEAD SCIENCES, INC.) 29 April 2021 (2021-04-29) description, page 477, embodiment 152, and claims 1, 34, 53, and 66 | 1-9 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 September 2023** | **08 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**
**Information on patent family members**

International application No.
**PCT/CN2023/095059**

| Patent document cited in search report | Publication date (day/month/year) | Patent family member(s) | Publication date (day/month/year) |
|---|---|---|---|
| WO 2021081207 A1 | 29 April 2021 | AU 2020369568 A1 | 05 May 2022 |
| | | US 2021171499 A1 | 10 June 2021 |
| | | US 11702404 B2 | 18 July 2023 |
| | | JP 2022552735 A | 19 December 2022 |
| | | CO 2022005077 A2 | 10 May 2022 |
| | | IL 292399 A | 01 June 2022 |
| | | CA 3157525 A1 | 29 April 2021 |
| | | KR 20220092909 A | 04 July 2022 |
| | | TW 202317535 A | 01 May 2023 |
| | | PE 20221040 A1 | 17 June 2022 |
| | | TW 202130630 A | 16 August 2021 |
| | | TWI 790492 B | 21 January 2023 |
| | | CL 2022001030 A1 | 03 February 2023 |
| | | CR 20220178 A | 15 June 2022 |
| | | BR 112022007627 A2 | 12 July 2022 |
| | | EP 4048664 A1 | 31 August 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Sichuan Medical Journal*, 2020, vol. 41 (10), 1089-1093